# EUROPEAN PATENT APPLICATION

(11) **EP 4 603 475 A1**
(43) Date of publication of application: **20.08.2025**
(21) Application number: 23876813.9
(22) Date of filing: 13.10.2023
(51) Int. Cl.: C07C 219/16, C07D 207/06, C07D 211/10, A61K 9/127, A61K 31/40, C12N 15/12

(54) **AMINO LIPID COMPOUND AND LIPID NANOPARTICLE FOR DELIVERING BIOACTIVE INGREDIENT**

(30) Priority: 13.10.2022 WO PCT/CN2022/125103; 06.12.2022 WO PCT/CN2022/136902; 17.03.2023 WO PCT/CN2023/082176; 01.09.2023 WO PCT/CN2023/116507
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); CHEN, Yonghao, Shenzhen, Guangdong 518000 (CN); HUANG, Xing, Shenzhen, Guangdong 518000 (CN)
(74) Representative: Nederlandsch Octrooibureau
(86) International application number: PCT/CN2023/124497
(87) International publication number: WO 2024/078614

(57) **Abstract**

Provided are an amino lipid compound for preparing a lipid nanoparticle for delivering an active ingredient and a preparation method therefor, a lipid nanoparticle and a pharmaceutical composition containing the amino lipid compound, and the use thereof.

## Description

### Technical Field

The present disclosure relates to an amino lipid compound that can be used to prepare a lipid nanoparticle for delivering an active ingredient and a preparation method therefor. The present disclosure also relates to a lipid nanoparticle and a pharmaceutical composition, containing the amino lipid compound, and use thereof.

### Background Art

Safe and effective targeted delivery of biologically active ingredients (e.g., nucleic acids, proteins, and small molecule drugs) into cells, tissues, and/or organs has been a technical challenge in the field of drug delivery, especially for nucleic acid drugs which are susceptible to degradation by nucleases in blood plasma. Nucleic acid drugs, including mRNA, antisense oligonucleotides (ASOs), microRNA (miRNA), small interfering RNA (siRNA) and aptamers, are gradually being developed as a new type of therapeutic approach, and have great therapeutic potential in the treatment of cancers, the prevention of infectious diseases, and the treatment of genetic diseases.

The delivery of nucleic acid drugs by lipid nanoparticles has been widely used. However, the targeting, safety, and delivery efficiency of nucleic acid drug delivery remain to be improved. Different nucleic acid drugs, cells (or tissues, organs), and application scenarios need to be matched with lipid nanoparticles of different properties. Thus, there is a great demand for the development of different lipid nanoparticles, especially amino lipid compounds for preparing lipid nanoparticles, to accommodate the need of delivering nucleic acid drugs in different application scenarios.

### Summary of the Invention

One aspect of the present disclosure provides an amino lipid compound represented by formula (I): 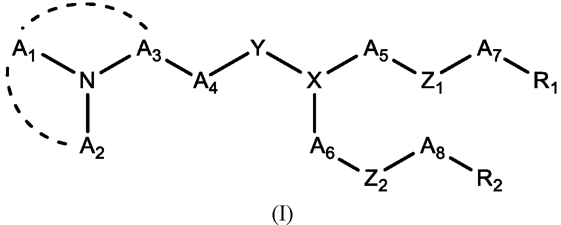 wherein A₁, A₂, A₃, A₄, A₅, A₆, A₇, A₈, Z₁, Z₂, Y, X, R₁, and R₂ are each as defined below.

Another aspect of the present disclosure provides a method for preparing the amino lipid compound.

Another aspect of the present disclosure provides a use of the amino lipid compound in the manufacture of a vehicle for an active ingredient.

Another aspect of the present disclosure provides a lipid nanoparticle comprising the amino lipid compound.

Another aspect of the present disclosure provides a pharmaceutical composition comprising the lipid nanoparticle.

Another aspect of the present disclosure provides a method for delivering a biologically active ingredient into cells, tissues, or organs by the lipid nanoparticle or pharmaceutical composition.

Another aspect of the present disclosure provides a method for producing a polypeptide and/or protein of interest in mammalian cells by the lipid nanoparticle or pharmaceutical composition.

Another aspect of the present disclosure provides a use of the amino lipid compound, lipid nanoparticle, or pharmaceutical composition in the manufacture of a medicament.

Another aspect of the present disclosure provides a method for preventing and/or treating a disease or disorder in a mammal in need thereof by the lipid nanoparticle or pharmaceutical composition.

Another aspect of the present disclosure provides a use of the amino lipid compound, lipid nanoparticle, or pharmaceutical composition in the manufacture of a medicament for nucleic acid transfer.

### Detailed Description of the Invention

### Definitions

Unless otherwise defined below, all technical and scientific terms used herein are intended to have the same meaning as commonly understood by one of ordinary skill in the art. Reference to a technique as used herein is intended to mean a technique as commonly understood in the art, including those variations that are apparent to those skilled in the art, or substitutions of equivalence technique. While it is believed that the following terms are well understood by those skilled in the art, the following definitions are set forth to better explain the present invention.

As used herein, the terms "comprising", "including", "having", "containing", or "involving" as well as other variations thereof, are inclusive or open-ended and do not exclude other non-recited elements or method steps.

As used herein, the term "hydrocarbyl" refers to the group remaining after the loss of one hydrogen atom from an aliphatic hydrocarbon, including straight or branched, saturated or unsaturated hydrocarbyl groups. A hydrocarbyl group includes, but is not limited to, alkyl, alkenyl, and alkynyl groups. Preferably, the hydrocarbyl group has from 1 to 24 carbon atoms (C₁-C₂₄ hydrocarbyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ... C₂₁, C₂₂, C₂₃, or C₂₄ hydrocarbyl). Examples of the hydrocarbyl group include, but are not limited to, C₁-C₂₄ hydrocarbyl, C₁-C₂₂ hydrocarbyl, C₁-C₂₀ hydrocarbyl, C₁-C₁₈ hydrocarbyl, C₁-C₁₆ hydrocarbyl, C₁-C₁₂ hydrocarbyl, C₁-C₁₀ hydrocarbyl, C₁-C₈ hydrocarbyl, C₁-C₇ hydrocarbyl, C₁-C₆ hydrocarbyl, C₁-C₄ hydrocarbyl, C₁-C₃ hydrocarbyl, C₁-C₂ hydrocarbyl, C₂-C₈ hydrocarbyl, C₂-C₄ hydrocarbyl, C₄-C₈ hydrocarbyl, C₄-C₉ hydrocarbyl, C₅-C₈ hydrocarbyl, C₁-C₄ hydrocarbyl, C₂-C₈ hydrocarbyl, C₃ hydrocarbyl, C₄ hydrocarbyl, C₅ hydrocarbyl, C₆ hydrocarbyl, C₇ hydrocarbyl, and C₈ hydrocarbyl. Unless explicitly stated otherwise in this specification, the hydrocarbyl group is optionally substituted, and for the substituents, reference is made to the definition of "optionally substituted" below. In certain embodiments, the hydrocarbyl group has no branches (i.e., is a straight chain), one branch, two branches, or multiple branches.

As used herein, the term "hydrocarbylene" refers to a divalent group remaining after further loss of one hydrogen atom from the hydrocarbyl as defined above. Unless expressly stated otherwise in this specification, the hydrocarbylene group is also optionally substituted.

As used herein, the term "alkyl" is a straight or branched saturated monovalent hydrocarbyl. Preferably, an alkyl group has from 1 to 24 carbon atoms (C₁-C₂₄ alkyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ... C₂₁, C₂₂, C₂₃, or C₂₄ alkyl). Examples of the alkyl group include, but are not limited to, C₁-C₂₄ alkyl, C₁-C₂₂ alkyl, C₁-C₂₀ alkyl, C₁-C₁₈ alkyl, C₁-C₁₆ alkyl, C₁-C₁₂ alkyl, C₁-C₁₀ alkyl, C₁-C₈ alkyl, C₁-C₇ alkyl, C₁-C₆ alkyl, C₁-C₄ alkyl, C₁-C₃ alkyl, C₁-C₂ alkyl, C₂-C₈ alkyl, C₂-C₄ alkyl, C₄-C₈ alkyl, C₄-C₉ alkyl, C₅-C₈ alkyl, C₁-C₄ alkyl, C₂-C₈ alkyl, methyl, ethyl, n-propyl, isopropyl, n-butyl, sec-butyl, isobutyl, tert-butyl, n-pentyl, n-hexyl, n-heptyl, n-octyl, n-nonyl, n-decyl, n-undecyl, n-dodecyl, n-tridecyl, and tridecan-7-yl. Unless explicitly stated otherwise in this specification, the alkyl group is optionally substituted.

As used herein, the term "alkylene" refers to a divalent group remaining after further loss of one hydrogen atom from the alkyl as defined above. Unless expressly stated otherwise in this specification, the alkylene is also optionally substituted.

As used herein, the term "alkenyl" is a straight or branched monovalent hydrocarbyl containing one or more double bonds (C=C). Preferably, an alkenyl group has from 2 to 24 carbon atoms (C₂-C₂₄ alkenyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ... C₂₁, C₂₂, C₂₃, or C₂₄ alkenyl), and has 1, 2, 3, 4, or more double bonds. The alkenyl group includes, but is not limited to, C₂-C₂₄ alkenyl, C₂-C₂₂ alkenyl, C₂-C₂₀ alkenyl, C₂-C₁₈ alkenyl, C₂-C₁₆ alkenyl, C₂-C₁₂ alkenyl, C₂-C₁₀ alkenyl, C₂-C₈ alkenyl, C₂-C₇ alkenyl, C₂-C₆ alkenyl, C₂-C₄ alkenyl, C₂-C₃ alkenyl, C₄-C₈ alkenyl, C₄-C₉ alkenyl, C₅-C₈ alkenyl having 1, 2, 3, 4 or more double bonds. Some more specific examples include, but are not limited to, ethenyl, propenyl, but-1-enyl, but-2-enyl, pent-1-enyl, pent-2-enyl, hex-1-enyl, hex-2-enyl, hex-3-enyl, hept-1-enyl, hept-2-enyl, hept-3-enyl, oct-1-enyl, oct-2-enyl, oct-3-enyl, non-1-enyl, non-2-enyl, and non-3-enyl. In some embodiments, the alkenyl group has one double bond. Unless expressly stated otherwise in this specification, the alkenyl group is optionally substituted.

As used herein, the term "alkenylene" refers to a divalent group remaining after further loss of one hydrogen atom from the alkenyl as defined above. Unless expressly stated otherwise in the specification, the alkenylene group is also optionally substituted.

As used herein, the term "alkynyl" is a straight or branched monovalent hydrocarbyl group containing one or more triple bonds (C≡C). Preferably, an alkynyl group has from 2 to 24 carbon atoms (C₂-C₂₄ alkynyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ... C₂₁, C₂₂, C₂₃, or C₂₄ alkynyl), and having 1, 2, 3, 4, or more triple bonds. The alkynyl group includes, but is not limited to, C₂-C₂₄ alkynyl, C₂-C₂₂ alkynyl, C₂-C₂₀ alkynyl, C₂-C₁₈ alkynyl, C₂-C₁₆ alkynyl, C₂-C₁₂ alkynyl, C₂-C₁₀ alkynyl, C₂-C₈ alkynyl, C₂-C₇ alkynyl, C₂-C₆ alkynyl, C₂-C₄ alkynyl, C₂-C₃ alkynyl, C₄-C₈ alkynyl, C₄-C₉ alkynyl, C₅-C₈ alkynyl having 1, 2, 3, 4 or more triple bonds. Some more specific examples include, but are not limited to, ethynyl, propynyl, but-1-ynyl, but-2-ynyl, pent-1-ynyl, pent-2-ynyl, hex-1-ynyl, hex-2-ynyl, hex-3-ynyl, hept-1-ynyl, hept-2-ynyl, hept-3-ynyl, oct-1-ynyl, oct-2-ynyl, oct-3-ynyl, non-1-ynyl, non-2-ynyl, and non-3-ynyl. In some embodiments, the alkynyl group has one triple bond. Unless explicitly stated otherwise in this specification, the alkynyl group is optionally substituted.

As used herein, the term "alkynylene" refers to a divalent group remaining after further loss of one hydrogen atom from the alkynyl as defined above. Unless explicitly stated otherwise in the specification, the alkynylene group is also optionally substituted.

As used herein, the terms "cyclohydrocarbyl", "cyclohydrocarbylene", and "hydrocarbon ring" refer to saturated (i.e., "cycloalkyl" and "cycloalkylene") or unsaturated (i.e., having one or more double bonds (cycloalkenyl) and/or triple bonds (cycloalkynyl) in the ring) monocyclic or polycyclic hydrocarbon rings having ring carbon atoms. In certain embodiments, "cyclohydrocarbyl", "cyclohydrocarbylene", and "hydrocarbon ring" have, for example, from 3 to 10, suitably from 3 to 8, more suitably from 3 to 6, such as from 5 to 6 or from 5 to 7, ring carbon atoms. "Cyclohydrocarbyl", "cyclohydrocarbylene", and "hydrocarbon ring" include, but are not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), etc. Unless expressly stated otherwise in this specification, the cyclohydrocarbyl, cyclohydrocarbylene, and hydrocarbon rings are optionally substituted.

As used herein, the term "cycloalkyl" refers to a saturated monocyclic or polycyclic (such as bicyclic) hydrocarbon ring (e.g., monocyclic, such as cyclopropyl, cyclobutyl, cyclopentyl, cyclohexyl, cycloheptyl, cyclooctyl, cyclononyl, or bicyclic, including spirocyclic, fused, or bridged systems, such as bicyclo[1.1.1] pentyl, bicyclo[2.2.1]heptyl, bicyclo[3.2.1]octyl or bicyclo[5.2.0]nonyl, decalin, etc.). In certain embodiments, the cycloalkyl group has, for example, 3 to 10, such as 3-7, 5-6, or 5-7 carbon atoms. Unless expressly stated otherwise in this specification, the cycloalkyl group is optionally substituted.

As used herein, the term "heterohydrocarbyl" or its subordinate concepts (e.g., heteroalkyl, heteroalkenyl, heteroalkynyl, heteroaryl, etc.) refer to a stable straight, branched, or cyclic hydrocarbon radical or combination thereof, consisting of a specified number of carbon atoms and at least one heteroatom. The heteroatom refer to an atom other than carbon and hydrogen. In certain embodiments, the heterohydrocarbyl group contains one, two, or more heteroatoms. In certain embodiments, the heterohydrocarbyl group contains one or more identical or different heteroatoms. Preferably, the heteroatom is selected from O, N and S. Examples of the heterohydrocarbyl group include, but are not limited to, -CH₂-CH₂-O-CH₃, -CH₂-CH₂-CH₂-O-CH₂-CH₃, -CH₂-(CH₂)₃-O-(CH₂)₅-CH₃, -CH₂-CH₂-NH-CH₃, -CH₂-CH₂-N(CH₃)-CH₃, -CH₂-S-CH₂-CH₃, -CH₂-CH₂, -CH=CH-O-CH₃, -CH₂-CH=N-OCH₃, -CH=CH-N(CH₃)-CH₃, and -CH₂-NH-OCH₃. Unless explicitly stated otherwise in this specification, the heterohydrocarbyl group is optionally substituted.

As used herein, the term "heterohydrocarbylene" or its subordinate concepts (such as heteroalkylene, heteroalkenylene, heteroalkynylene, heteroarylene, etc.) refer to a divalent group remaining after further loss of one hydrogen atom from the heterohydrocarbyl as defined above. Unless explicitly stated otherwise in this specification, the heterohydrocarbylene group or its subordinate concepts (such as heteroalkylene, heteroalkenylene, heteroalkynylene, heteroarylene, etc.) are also optionally substituted.

As used herein, the term "heterocycle," "heterocyclyl," or "heterocyclylene" means a cyclic group having a cyclic structure and containing one or more heteroatoms in the ring-forming atoms. In certain embodiments, the ring-forming atoms include one or more heteroatoms which are the same or different. In certain embodiments, the one or more heteroatoms included in the ring-forming atoms are selected from N, O, and S. The "heterocycle", "heterocyclyl" or "heterocyclylene" as disclosed herein is saturated or unsaturated. In certain embodiments, the "heterocycle", "heterocyclyl", or "heterocyclylene" comprises a monocyclic ring, a bicyclic ring, or a polycyclic ring. In certain embodiments, the "heterocycle", "heterocyclyl", or "heterocyclylene" is a 4- to 10-membered heterocycle, e.g., 4- to 7-membered heterocycle, 5- to 7-membered heterocycle. Preferably, in certain embodiments, the heterocycle group is a 4- to 10-membered heterocycle which may be optionally substituted, wherein the ring-forming atoms contain 1, 2, 3, 4, 5, or 6 heteroatoms selected from N, O, and S. More preferably, the heterocycle group is a 4- to 7-membered saturated heterocycle which may be optionally substituted, wherein the ring-forming atoms contain 1, 2, 3 or 4 heteroatoms selected from N, O and S; more preferably, the heterocycle group is a 5- to 7-membered (e.g., 5- to 6-membered) saturated heterocycle which may be optionally substituted, wherein the ring-forming atoms contain 1, 2 or 3 heteroatoms selected from N, O and S. Examples of heterocycle include, but are not limited to, azetidine, oxetanyl, tetrahydrofuran, pyrrolidine, imidazolidine, pyrazolidine, tetrahydropyran, piperidine, morpholine, thiomorpholine, piperazine, and preferably pyrrolidine, piperidine, piperazine, and morpholine. The heterocycle may be optionally substituted with one or more substituents, and for the substituents, reference is made to the definition for "optionally substituted" below. Unless expressly stated otherwise in this specification, the heterocycle, heterocyclyl, or heterocyclylene is optionally substituted.

As used herein, the term "aryl" refers to an all-carbon monocyclic or fused polycyclic aromatic group having a conjugated π-electron system. For example, as used herein, the term "C₆₋₁₄ aryl" means an aromatic group containing 6 to 14 (e.g., 6 to 12) carbon atoms, such as phenyl or naphthyl. Unless explicitly stated otherwise in this specification, the aryl group is optionally substituted.

As used herein, the term "heteroaryl" refers to a monocyclic or polycyclic (e.g., bicyclic or tricyclic) aromatic group having a conjugated π-electron system, of which ring atoms consist of carbon atoms and at least one heteroatom, for example, it has from 5 to 14 (e.g., 5, 6, 7, 8, 9, 10, 11, 12, 13, or 14) ring atoms, including 1, 2, 3, 4, 5, 6, 7, 8, 9, 10, 11, 12, or 13 carbon atoms and 1, 2, 3, 4, or 5 identical or different heteroatoms independently selected from N, O, S, and S(O)₂. One or more ring carbon atoms in the heteroaryl group may be substituted with C(O). The heteroaryl group may be benzo-fused. Unless explicitly stated otherwise in this specification, the heteroaryl group is optionally substituted.

As used herein, the term "optionally substituted" means that one or more hydrogen atoms attached to an atom or group are independently unsubstituted, or independently substituted with one or more (e.g., 1, 2, 3, or 4) substituents. The substituents are independently selected from, but are not limit to, deuterium (D), tritium (T), halogen, -OH, mercapto, cyano, -CD₃ , C₁-C₆ alkyl (preferably C₁-C₃ alkyl), C₂-C₆ alkenyl, C₂-C₆ alkynyl, cycloalkyl (preferably C₃-C₈ cycloalkyl), aryl, heterocyclyl (preferably 3- to 8-membered heterocyclyl), heteroaryl, arylC₁-C₆ alkyl, heteroarylC₁-C₆ alkyl, C₁-C₆ haloalkyl, -OC₁-C₆ alkyl (preferably -OC₁-C₃ alkyl), -OC₂-C₆ alkenyl, OC₁-C₆ alkylphenyl, C₁-C₆ alkyl-OH (preferably C₁-C₄ alkyl-OH), C₁-C₆ alkyl-SH, C₁-C₆ alkyl-O-C₁-C₆ alkyl, OC₁-C₆ haloalkyl, -NH₂, C₁-C₆ alkyl-NH₂ (preferably C₁-C₃ alkyl-NH₂), -N(C₁-C₆ alkyl)₂ (preferably -N(C₁-C₃ alkyl)₂), -NH(C₁-C₆ alkyl) (preferably -NH(C₁-C₃ alkyl)), -N(C₁-C₆ alkyl)(C₁-C₆ alkylphenyl), -NH(C₁-C₆ alkylphenyl), nitro, -C(O)-OH, -C(O)OC₁-C₆ alkyl (preferably -C(O)OC₁-C₃ alkyl), -CONRiRii (wherein Ri and/or Rii is H, D and C₁-C₆ alkyl, preferably C₁-C₃ alkyl), -NHC(O)(C₁-C₆ alkyl), -NHC(O)(phenyl), -N(C₁-C₆ alkyl)C(O)(C₁-C₆ alkyl), -N(C₁-C₆ alkyl)C(O)(phenyl), -C(O)C₁-C₆ alkyl, -C(O)heteroaryl (preferably -C(O)-5- to 7-membered heteroaryl), -C(O)C₁-C₆ alkylphenyl, -C(O)C₁-C₆ haloalkyl, -OC(O)C₁-C₆ alkyl (preferably -OC(O)C₁-C₃ alkyl), -S(O)₂-C₁-C₆ alkyl, -S(O)-C₁-C₆ alkyl, -S(O)₂-phenyl, -S(O)₂-C₁-C₆haloalkyl, -S(O)₂NH₂, -S(O)₂NH (C₁-C₆ alkyl), -S(O)₂NH(phenyl), -NHS(O)₂(C₁-C₆ alkyl), -NHS(O)₂(phenyl) and -NHS(O)₂(C₁-C₆ haloalkyl), wherein each of the alkyl, cycloalkyl, phenyl, aryl, heterocyclyl, and heteroaryl is optionally further substituted with one or more substituents selected from, but not limitated to, halogen, -OH, -NH₂, cycloalkyl, 3- to 8-membered heterocyclyl, C₁-C₄ alkyl, C₁-C₄ haloalkyl-, -OC₁-C₄ alkyl, -C₁-C₄ alkyl-OH, -C₁-C₄ alkyl-O-C₁-C₄ alkyl, -OC₁-C₄ haloalkyl, cyano, nitro, -C(O)-OH, -C(O)OC₁-C₆ alkyl, -CON(C₁-C₆ alkyl)₂, -CONH(C₁ -C₆ alkyl), -CONH₂ , -NHC(O)(C₁-C₆ alkyl), -NH(C₁-C₆ alkyl)C(O)(C₁-C₆ alkyl), -SO₂(C₁-C₆ alkyl), -SO₂(phenyl), -SO₂(C₁-C₆ haloalkyl), -SO₂NH₂, -SO₂NH(C₁-C₆ alkyl), -SO₂NH(phenyl), -NHSO₂(C₁-C₆ alkyl), -NHSO₂(phenyl), and -NHSO₂(C₁-C₆ haloalkyl). When an atom or group is substituted with a plurality of substituents, the plurality of substituents may be the same or different.

In certain embodiments, the substituents may be independently selected from, but are not limit to, a halogen (such as a chlorine, bromine, fluorine, or iodine), a carboxylic acid (such as -C(=O)-OH), an oxygen (such as =O), a sulfur (such as =S), a hydroxyl (such as -OH), an ester group (such as -C(=O)ORiii or -OC(=O)Riii), an aldehyde group (such as -C(=O)H), a carbonyl (such as -C(=O)Riii, or represented by C=O), an acyl halide (such as -C(=O)X, wherein X is selected from bromine, fluorine, chlorine, or iodine), a carbonic ester group (such as -OC(=O)ORiii), an alkoxy (such as -ORiii), an acetal (such as -C(ORiii)₂Riii, wherein each ORiii is the same or different and is an alkoxy group), a phosphate (such as P(=O)₄³⁻), a thiol (such as -SH), a sulfoxide (such as -S(=O)Riii), a sulfinic acid (such as -S(=O)OH), a sulfonic acid (such as -S(=O)₂OH), a thioaldehyde (such as -C(=S)H), a sulfate (such as S(=O)₄²⁻), a sulfonyl (such as -S(=O)₂Riii), a sulfinyl (such as -S(=O)Riii), an amide group (such as -C(=O)N(Riii)₂ or -N(Riii)C(=O)Riii), an azido (such as -N₃), a nitro (such as -NO₂), a cyano (such as -CN), an isocyano (such as -NC), an acyloxy (such as -OC(=O)Riii), an amino (such as -N(Riii)₂, -N(Riii)H or -NH₂), a carbamoyl (such as -OC(=O)N(Riii)₂, -OC(=O)N(Riii)H or -OC(=O)NH₂), a sulfonamide group (such as -S(=O)₂N(Riii)₂, -S(=O)₂N(Riii)H, -S(=O)₂NH₂, -N(Riii)S(=O)₂Riii, -N(H)S(=O)₂Riii, -N(Riii)S(=O)₂H or -N(H)S(=O)₂H), an alkyl, an alkenyl, an alkynyl, a cyclohydrocarbyl (such as cycloalkyl, cycloalkenyl or cycloalkynyl), a heterocyclohydrocarbyl (such as heterocycloalkyl containing one or more heteroatoms selected from S, N, and O, or heterocycloalkenyl containing one or more heteroatoms selected from S, N, and O), an aryl (such as phenyl, or a fused ring group), a heteroaryl (such as an 8- to 10-membered bicyclic heteroaryl containing 1 to 4 heteroatoms independently selected from nitrogen, oxygen, or sulfur), -C(=O)SRiii, -C(=N-CN)N(Riii)₂, -C(=N-O-CH₃)N(Riii)₂, -C(=N-SO₂-NH₂)N(Riii)₂, -C(=CH-NO₂)N(Riii)₂, -OC(=O)N(Riii)₂, -CHN(Riii)N(Riii)₂, -C(=O)N(Riii)ORiii, -N(Riii)₂C(=O)ORiii, -OP(=O)(ORiii)₂, -P(=O)(ORiii)₂, -N(ORiii)C(=O)Riii, -N(ORiii)S(=O)₂Riii, -N(ORiii)C(=O)ORiii, -N(ORiii)C(=O)N(Riii)₂, -N(ORiii)C(=S)N(Riii)₂, -N(ORiii)C(NRiii)N(Riii)₂, -N(ORiii)C(CHRiii)N(Riii)₂. In any of the foregoing, Riii is a hydrogen, or alkyl, or alkenyl, or alkynyl, or heteroalkyl, or heteroalkenyl, or heteroalkynyl, as defined herein. In some embodiments, Riii is a hydrogen, or C₁-C₁₂ alkyl, or C₂-C₁₂ alkenyl, or C₂-C₁₂ alkynyl, or C₂-C₁₂ heteroalkyl, or C₃-C₁₂ heteroalkenyl, or C₃-C₁₂ heteroalkynyl, as defined herein.

In certain embodiments, the substituents itself may be further substituted with, for example, one or more substituents as defined herein. For example, the C₁-C₆ alkyl as a substituent may be further substituted with one or more substituents as define herein.

As use herein, that term "halo" or "halogen" group is defined to include F, Cl, Br, or I.

As used herein, "pharmaceutically acceptable salt" refers to an acid-addition salt or a base-addition salt of a compound of the present disclosure which retains the biological effectiveness and properties of the compound of the present disclosure and is not typically biologically or otherwise undesirable. In many cases, the compound of the present invention can form an acid and/or base salt due to the presence of an amino and/or carboxyl group or a similar group.

Pharmaceutically acceptable acid addition salts can be formed from the compound of the present disclosure and inorganic and/or organic acids, the inorganic acids being such as, but not limited to, hydrochloric acid, hydrobromic acid, sulfuric acid, nitric acid, and phosphoric acid; and the organic acids being such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, hexanoic acid, octanoic acid, carbonic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, and undecylenic acid.

Pharmaceutically acceptable base addition salts can be formed from the compound of the present disclosure and inorganic and/or organic bases. Salts derived from inorganic bases include, but are not limited to, sodium, potassium, lithium, ammonium, calcium, magnesium, iron, zinc, copper, manganese, aluminum salts, etc. Preferred inorganic salts are ammonium, sodium, potassium, calcium, and magnesium salts. Salts derived from organic bases include, but are not limited to, salts of primary amines, secondary amines, tertiary amines, substituted amines (including naturally occurring substituted amines), cyclic amines, and basic ion exchange resins as following: such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, tromethamine, purine, piperazine, piperidine, N-ethylpiperidine, and polyamine resins. Particularly preferred organic bases are isopropylamine, diethylamine, ethanolamine, trimethylamine, dicyclohexylamine, choline, and caffeine.

A numerical range stated herein should be understood to encompass the boundary values and any and all subranges contained therein. For example, a range of "1 to 10" should be understood to include not only the explicitly recited values of 1 and 10, but also any individual values in the range of 1 to 10 (e.g., 2, 3, 4, 5, 6, 7, 8, and 9) and subranges (e.g., 1 to 2, 1.5 to 2.5, 1 to 3, 1.5 to 3.5, 2.5 to 4, 3 to 4.5, etc.). This principle also applies to ranges that use only one value as a minimum or maximum.

As use herein, that term "isomer" means different compounds having the same molecular formula. "Stereoisomers" are isomers that differ only in the way the atoms are arranged in space. "Atropisomers" are stereoisomers resulting from hindered rotation about a single bond. "Enantiomers" are a pair of stereoisomers that are non-overlapping mirror images of each other. A mixture of any ratio of a pair of enantiomers may be referred to as a "racemic" mixture. "Diastereoisomers" are stereoisomers that have at least two asymmetric atoms and are not mirror-images of one another. "Tautomers" refer to isomeric forms of a compound that are in equilibrium with each other. The concentration of the isomeric form will depend on the environment in which the compound is found and may vary, for example, depending on whether the compound is a solid or in an organic or aqueous solution.

In certain embodiments, "stereoisomers" may also include the E and Z isomers, or mixtures thereof, as well as the *cis* and *trans* isomers, or mixtures thereof.

Nucleic acids and/or polynucleotides useful in the present disclosure include a coding region encoding a polypeptide of interest, a 5'-UTR at the 5'-end of the coding region, a 3'-UTR at the 3'-end of the coding region. In some embodiments, the nucleic acid or polynucleotide further comprises at least one of a polyadenylation region and a Kozak sequence. In some embodiments, the nucleic acid or polynucleotide (e.g., mRNA) may also include a 5' cap structure. Any region of a nucleic acid may include one or more alternative nucleosides, such as 5-substituted uridine (e.g., 5-methoxyuridine), 1-substituted pseudouridine (e.g., 1-methyl-pseudouridine or 1-ethyl-pseudouridine), and/or 5-substituted cytidine (e.g., 5-methyl-cytidine).

The term "5'-UTR" or "5'-untranslated region" may be a RNA sequence in an mRNA that is located upstream of the coding sequence and is not translated into protein. The 5'-UTR in a gene typically begins at the transcription start site and ends at a nucleotide upstream of the translation start codon of the coding sequence. The 5'-UTR may contain an element that controls gene expression, such as a ribosome binding site, a 5'-terminal oligopyrimidine tract, and a translation initiation signal such as a Kozak sequence. The mRNA can be post-transcriptionally modified by the addition of a 5' cap. Thus, the 5'-UTR in mature mRNA can also refer to the RNA sequence between the 5' cap and the start codon. As used herein, that term "3' untranslated region" or "3'-UTR" can be a RNA sequence in an mRNA that is located downstream of the code sequence and is not translated into protein. The 3'-UTR in the mRNA is located between the stop codon of the coding sequence and the poly(A) sequence, for example, beginning at a nucleotide downstream of the stop codon and ending at a nucleotide upstream of the poly(A) sequence. The sequence of the 5'-UTR and/or 3'-UTR may be homologous or heterologous to the sequence of the coding region. The 3'-UTR may comprise a 3'-UTR derived from at least one gene of albumin gene, alpha-globin gene, beta-globin gene, tyrosine hydroxylase gene, lipoxygenase gene, and collagen alpha gene.

As used herein, the term "polyadenylation region", "poly(A) sequence" and "poly(A)tail" are used interchangeably. A naturally occurring poly(A) sequence typically consists of adenine ribonucleotides. Preferably, a "polyadenylation region" refers to a poly(A) sequence comprising nucleotides or nucleotide segments other than adenine ribonucleotides. The poly(A) sequence is usually located at the 3' end of the mRNA, such as at the 3' end (downstream) of the 3'-UTR. Poly-A region may have different lengths. In particular, in some embodiments, the poly-A region of the nucleic acid molecules of the present disclosure is at least 30 nucleotides in length; in some embodiments, the poly-A region of the nucleic acid molecules of the present disclosure is at least 80 nucleotides in length; and in some embodiments, the poly-A region of the nucleic acid molecules of the present disclosure is at least 100 nucleotides in length.

As used herein, the term "5' cap structure" is the 5' cap structure which is typically located at the 5' end of the mature mRNA. In some embodiments, the 5' cap structure is linked to the 5'-end of the mRNA by a 5'-5'-triphosphate bond. The 5' cap structure is typically formed from modified (e.g., methylated) ribonucleotides (especially from guanine nucleotide derivatives). For example, m7GpppN (cap 0, or "cap0", is a cap structure formed by the 5'-phosphate group of hnRNA interacting with the 5'-phosphate group of m7GTP under the action of guanylate transferase to form a 5',5'-phosphodiester bond), where N is the terminal 5' nucleotide of the nucleic acid carrying the 5'-cap structure. In some embodiments, the 5' cap structure includes, but is not limited to, cap 0, cap 1 (a cap structure formed by further methylation of the 2'-OH of the ribose on the first nucleotide of hnRNA on the basis of cap 0, or "cap1"), cap 2 (a cap structure formed by further methylation of the 2'-OH of the ribose on the second nucleotide of hnRNA on the basis of cap 1, or "cap 2"), cap 4, cap 0 analog, cap 1 analog, cap 2 analog, or cap 4 analog.

### Amino lipid compound

In one aspect, the present disclosure provides an amino lipid compound represented by the following formula (I): 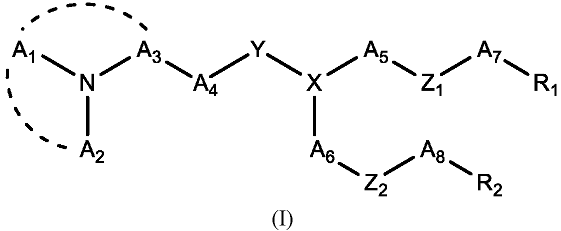
or a pharmaceutically acceptable salt thereof, or a stereoisomer thereof,
wherein,
A₁, A₂, and A₃ are one of the following:
   (1) when the dashed line connecting A₁ and A₂ and the dashed line connecting A₁ and A₃ are absent, A₁ is C₁-C₅ hydrocarbyl or C₁-C₅ heterohydrocarbyl, A₂ is C₁-C₅ hydrocarbyl or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₃ hydrocarbylene or C₁-C₃ heterohydrocarbylene, A₂ is C₁-C₃ hydrocarbylene or C₁-C₃ heterohydrocarbylene, A₃ is C₁-C₅ hydrocarbylene or a bond, and A₁ and A₂, together with the nitrogen atom to which they are both attached, form a four-, five-, or six-membered ring; or
   (3) when the dashed line connecting A₁ and A₃ is a bond and the dashed line connecting A₁ and A₂ is absent, A₁ is C₁-C₃ hydrocarbylene or C₁-C₃ heterohydrocarbylene, A₂ is C₁-C₅ hydrocarbyl or C₁-C₅ heterohydrocarbyl, A₃ is C₁-C₅ hydrocarbylene, and A₁ and A₃, together with the nitrogen atom to which they are both attached, form a four-, five-, or six-membered ring;
A₄ is C₁-C₅ hydrocarbylene or a bond;
A₅ is C₁-C₁₆ hydrocarbylene or a bond;
A₆ is C₁-C₁₆ hydrocarbylene or a bond;
A₇ is C₁-C₁₈ hydrocarbylene or a bond;
A₈ is C₁-C₁₈ hydrocarbylene or a bond;
Z₁ is -C(=O)O-, -OC(=O)-, -O-, -C(=O)-, -S-, -C(=O)S-, -SC(=O)-, -C(=O)NR₃-, or -NR₃C(=O)-;
R₃ is H or C₁-C₈ hydrocarbylene;
Z₂ is -C(=O)O-, -OC(=O)-, -O-, -C(=O)-, -S-, -C(=O)S-, -SC(=O)-, -C(=O)NR₄-, or -NR₄C(=O)-;
R₄ is H or C₁-C₃ hydrocarbylene;
Y is -Z₃C(=O)Z₄-, or -N(R₅)-;
Z₃ is -O-, -N(R₆)-, -S-, or a bond;
Z₄ is -O-, -N(R₇)-, -S-, or a bond;
R₅ is -C(=O)A₉Z₅A₁₀ or -C(=O)A₁₁;
Z₅ is -O- or -S-;
A₉ is C₁-C₃ hydrocarbylene or a bond;
A₁₀ is C₁-C₈ hydrocarbyl;
A₁₁ is C₁-C₃ hydrocarbyl;
R₆ is H, or C₁-C₈ hydrocarbyl;
R₇ is H, or C₁-C₈ hydrocarbyl;
X is C or N;
R₁ is H or C₁-C₁₈ hydrocarbyl, Z₆R₈, or -CH(OR₉)₂;
Z₆ is -C(=O)O-, -OC(=O)-, or -O-;
R₈ is H, C₁-C₁₈ hydrocarbyl, or C₁-C₁₈ heterohydrocarbyl containing O or S;
R₉ is C₁-C₁₈ hydrocarbyl, or C₁-C₁₈ heterohydrocarbyl containing O or S;
R₂ is H or C₁-C₁₈ hydrocarbyl, Z₇R₁₀, or -CH(OR₁₁)₂;
Z₇ is -C(=O)O-, -OC(=O)-, or -O-;
R₁₀ is H, C₁-C₁₈ hydrocarbyl, or C₁-C₁₈ heterohydrocarbyl containing O or S;
R₁₁ is C₁-C₁₈ hydrocarbyl, or C₁-C₁₈ heterohydrocarbyl containing O or S;
preferably, the hydrocarbyl is an alkyl, alkenyl, or alkynyl;
preferably, the hydrocarbylene is an alkylene, alkenylene, or alkynylene;
preferably, the heterohydrocarbyl is a heteroalkyl, heteroalkenyl, or heteroalkynyl.

In some embodiments, the present disclosure provides the amino lipid compound of formula (I), or a pharmaceutically acceptable salt thereof, or a stereoisomer thereof, as described above, wherein R₁ is Z₆R₈ or -CH(OR₉)₂, and R₂ is Z₇R₁₀ or -CH(OR₁₁)₂.

In some embodiments, in the amino lipid compound represented by formula (I), when the dashed line connecting A₁ and A₂ and the dashed line connecting A₁ and A₃ are absent, A₁ is C₁-C₅ hydrocarbyl or C₁-C₅ heterohydrocarbyl, A₂ is C₁-C₅ hydrocarbyl or C₁-C₅ heterohydrocarbyl, and A₃ is C₁-C₅ hydrocarbylene or a bond, i.e., having a structure represented by formula (Ia): 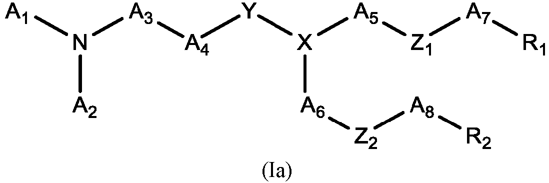 wherein A₄, A₅, A₆, A₇, A₈, Z₁, Z₂, Y, X, R₁, and R₂ are as defined for formula (I).

In some embodiments, in the amino lipid compound represented by formula (I), when the dashed line connecting A₁ and A₂ is a bond and the dashed line connecting A₁ and A₃ is absent, A₁ is C₁-C₃ hydrocarbylene or C₁-C₃ heterohydrocarbylene, A₂ is C₁-C₃ hydrocarbylene or C₁-C₃ heterohydrocarbylene, A₃ is C₁-C₅ hydrocarbylene or a bond, and A₁ and A₂, together with the nitrogen atom to which they are both attached, form a four-, five-, or six-membered ring, i.e., having a structure represented by formula (Ib): 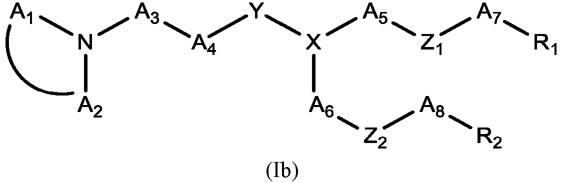 wherein A₄, A₅, A₆, A₇, A₈, Z₁, Z₂, Y, X, R₁, and R₂ are as defined for formula (I).

In some embodiments, in the amino lipid compound represented by formula (I), when the dashed line connecting A₁ and A₃ is a bond and the dashed line connecting A₁ and A₂ is absent, A₁ is C₁-C₃ hydrocarbylene or C₁-C₃ heterohydrocarbylene, A₂ is C₁-C₅ hydrocarbyl or C₁-C₅ heterohydrocarbyl, A₃ is C₁-C₅ hydrocarbylene, and A₁ and A₃, together with the nitrogen atom to which they are both attached, form a four-, five-, or six-membered ring, i.e., having a structure represented by formula (Ic): 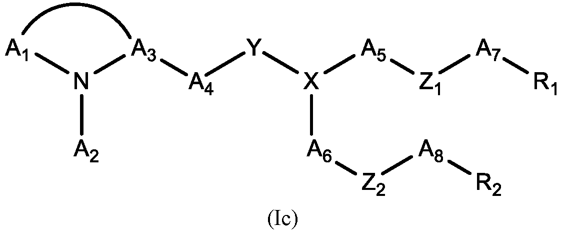 wherein A₄, A₅, A₆, A₇, A₈, Z₁, Z₂, Y, X, R₁, and R₂ are as defined for formula (I).

In some embodiments, the amino lipid compound represented by formula (I), when X is C, has a structure represented by formula (Id): 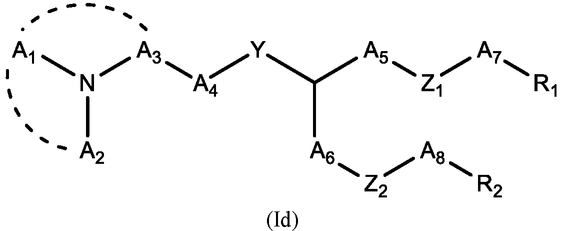 wherein A₁, A₂, A₃, A₄, A₅, A₆, A₇, A₈, Z₁, Z₂, Y, R₁, and R₂ are as defined for formula (I).

In some embodiments, the amino lipid compound represented by formula (I), when X is N, has a structure represented by formula (Ie): 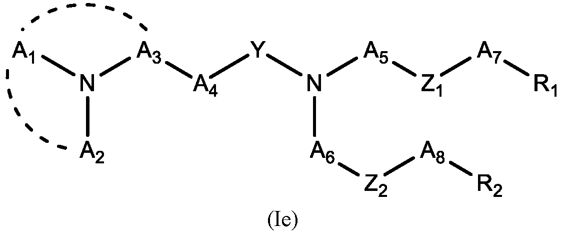 wherein A₁, A₂, A₃, A₄, A₅, A₆, A₇, A₈, Z₁, Z₂, Y, R₁, and R₂ are as defined for formula (I).

In some embodiments, the amino lipid compound represented by formula (I), when R₁ is -CH(OR₉)₂, has a structure represented by formula (II): 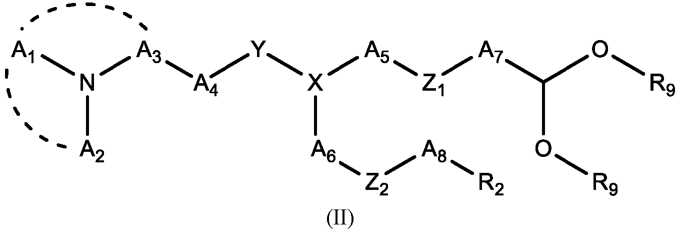 wherein A₁, A₂, A₃, A₄, A₅, A₆, A₇, A₈, Z₁, Z₂, Y, X, R₂, and R₉ are as defined for formula (I).

In some embodiments, the amino lipid compound represented by formula (II), when X is C, has a structure represented by formula (IIa): 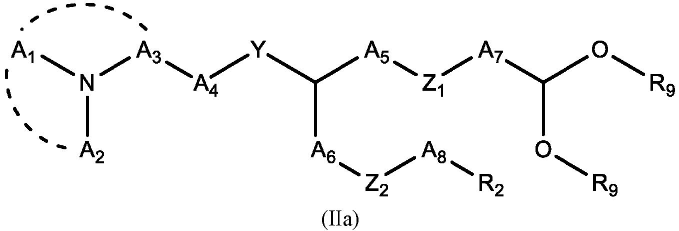 wherein A₁, A₂, A₃, A₄, A₅, A₆, A₇, A₈, Z₁, Z₂, Y, R₂, and R₉ are as defined for formula (I).

In some embodiments, the amino lipid compound represented by formula (II), when X is N, has a structure represented by formula (IIb): 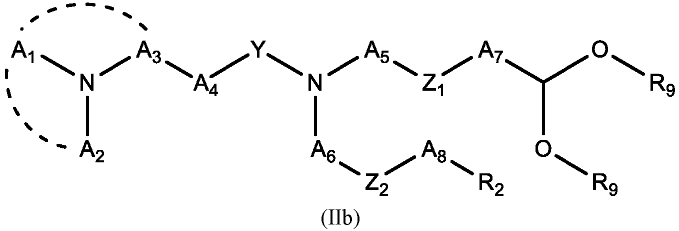 wherein A₁, A₂, A₃, A₄, A₅, A₆, A₇, A₈, Z₁, Z₂, Y, R₂, and R₉ are as defined for formula (I).

In some embodiments, the amino lipid compound represented by formula (I), when
R₁ is -CH(OR₉)₂; and
R₂ is -CH(OR₁₁)₂,
has a structure represented by formula (III): 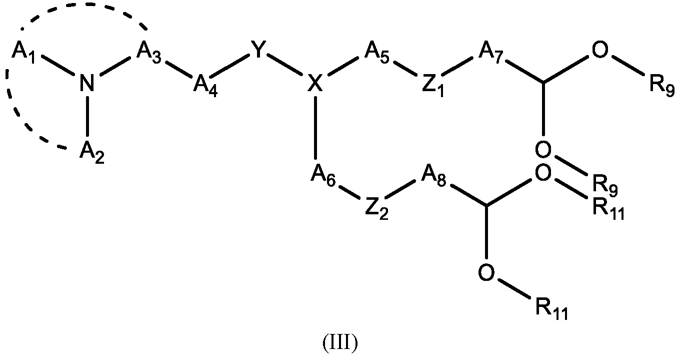
wherein A₁, A₂, A₃, A₄, A₅, A₆, A₇, A₈, Z₁, Z₂, Y, X, R₉, and R₁₁ are as defined for formula (I).

In some embodiments, the amino lipid compound represented by formula (III), when X is C, has a structure represented by formula (IIIa): 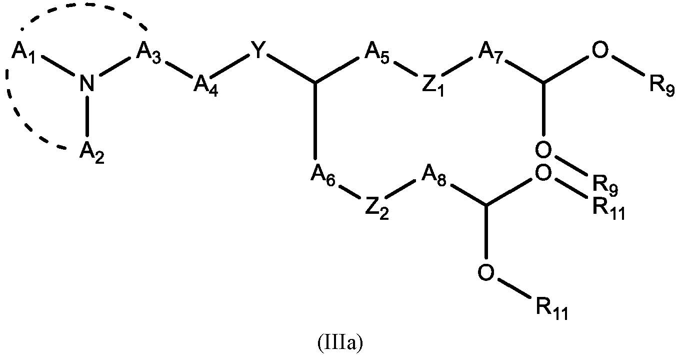 wherein A₁, A₂, A₃, A₄, A₅, A₆, A₇, A₈, Z₁, Z₂, Y, R₉, and R₁₁ are as defined for formula (I).

In some embodiments, the amino lipid compound represented by formula (III), when X is N, has a structure represented by formula (IIIb): 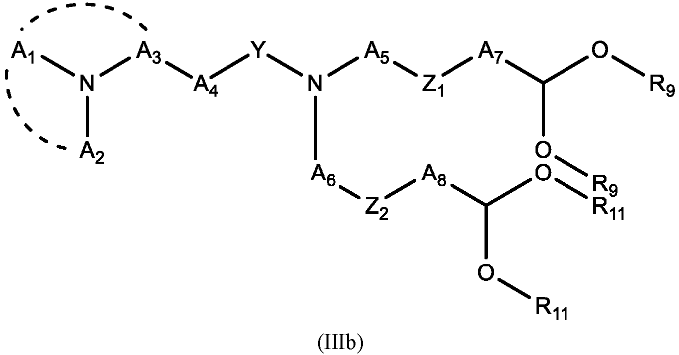 wherein A₁, A₂, A₃, A₄, A₅, A₆, A₇, A₈, Z₁, Z₂, Y, R₉, and R₁₁ are as defined for formula (I).

In some embodiments, the amino lipid compound represented by formula (I), when
Y is -Z₃C(=O)Z₄-;
Z₃ is -O-;
Z₄ is -O-;
X is C;
R₁ is -CH(OR₉)₂, and R₂ is -CH(OR₁₁)₂,
has a structure represented by formula (IV): 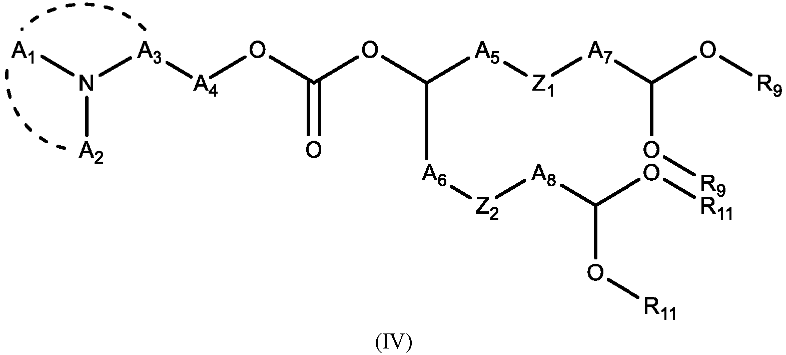 wherein A₁, A₂, A₃, A₄, A₅, A₆, A₇, A₈, Z₁, Z₂, R₉, and R₁₁ are as defined for formula (I).

In some embodiments, the amino lipid compound represented by formula (I), when
Y is -Z₃C(=O)Z₄-;
Z₃ is -O-;
Z₄ is -O-;
X is C;
Z₁ is -C(=O)O-, or -OC(=O)-;
Z₂ is -C(=O)O-, or -OC(=O)-;
R₁ is -CH(OR₉)₂, and R₂ is -CH(OR₁₁)₂,
has a structure represented by formula (Va), (Vb), (Vc), or (Vd): 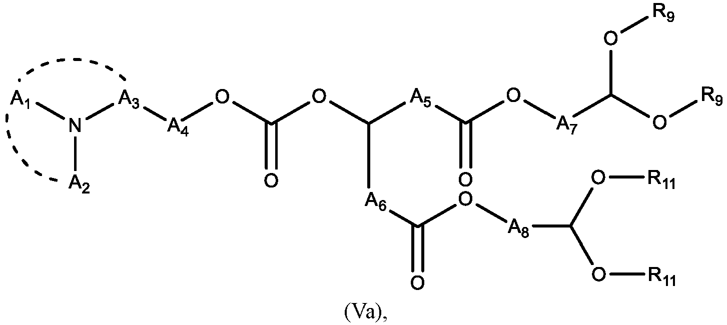 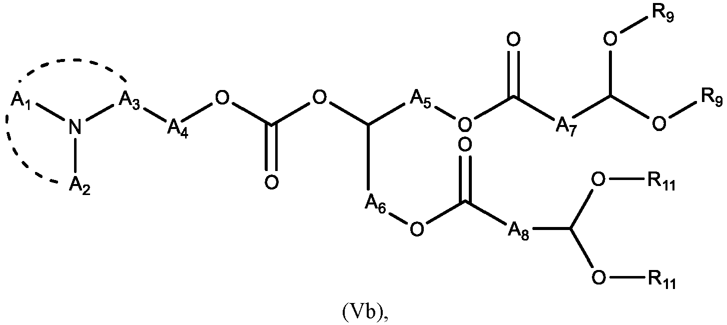 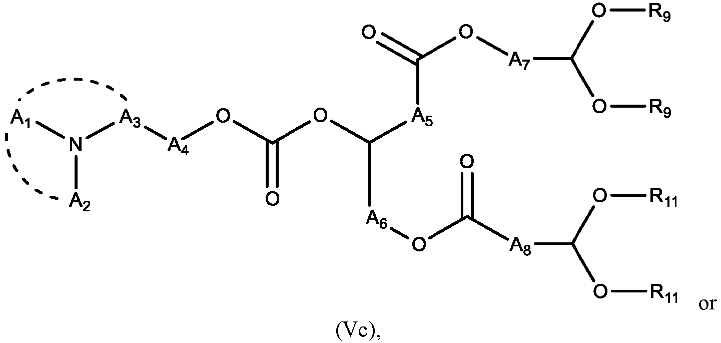 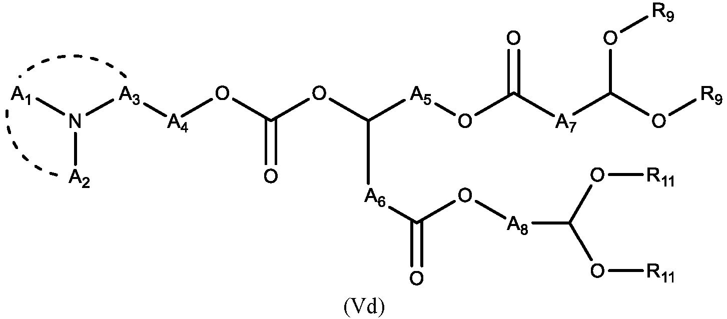 wherein A₁, A₂, A₃, A₄, A₅, A₆, A₇, A₈, R₉, and R₁₁ are as defined for formula (I).

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₁ is C₁, C₂, C₃, C₄, or C₅ hydrocarbyl, C₁, C₂, C₃, C₄, or C₅ heterohydrocarbyl, C₁, C₂, or C₃ hydrocarbylene, or C₁, C₂, or C₃ heterohydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₂ is C₁, C₂, C₃, C₄, or C₅ hydrocarbyl, C₁, C₂, C₃, C₄, or C₅ heterohydrocarbyl, C₁, C₂, or C₃ hydrocarbylene, or C₁, C₂, or C₃ heterohydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₃ is C₁, C₂, C₃, C₄, or C₅ hydrocarbylene or a bond.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₄ is C₁, C₂, or C₃ hydrocarbylene or a bond.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
As is C₁, C₂, C₃, C₄, C₅, C₆, C₇, Cs, C₉, C₁₀, C₁₁, C₁₂, C₁₃, C₁₄, C₁₅, or C₁₆ hydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₆ is C₁, C₂, C₃, C₄, C₅, C₆, C₇, C₈, C₉, C₁₀, C₁₁, C₁₂, C₁₃, C₁₄, C₁₅, or C₁₆ hydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₇ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
A₈ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, the present disclosure provides the amino lipid compound of formula (I), (Ia), (Ib), (Ic), (Id), (Ie), (II), (IIa), (IIb), (III), (IIIa), or (IIIb), or a pharmaceutically acceptable salt thereof, or a stereoisomer thereof, as described above, wherein:
A₉ is C₁, C₂, or C₃ hydrocarbylene.

In some embodiments, the present disclosure provides the amino lipid compound of formula (I), (Ia), (Ib), (Ic), (Id), (Ie), (II), (IIa), (IIb), (III), (IIIa), or (IIIb), or a pharmaceutically acceptable salt thereof, or a stereoisomer thereof, as described above, wherein:
A₁₀ is C₁, C₂, C₃, C₄, C₅, C₆, C₇, or C₈ hydrocarbylene.

In some embodiments, the present disclosure provides the amino lipid compound of formula (I), (Ia), (Ib), (Ic), (Id), (Ie), (II), (IIa), (IIb), (III), (IIIa), or (IIIb), or a pharmaceutically acceptable salt thereof, or a stereoisomer thereof, as described above, wherein:
A₁₁ is C₁, C₂, or C₃ hydrocarbylene.

In some embodiments, the present disclosure provides the amino lipid compound of formula (I), (Ia), (Ib), (Ic), (Id), (Ie), (II), (IIa), (IIb), (III), (IIIa), or (IIIb), or a pharmaceutically acceptable salt thereof, or a stereoisomer thereof, as described above, wherein:
R₆ is H, or C₁, C₂, C₃, C₄, C₅, C₆, C₇, or C₈ hydrocarbyl.

In some embodiments, the present disclosure provides the amino lipid compound of formula (I), (Ia), (Ib), (Ic), (Id), (Ie), (II), (IIa), (IIb), (III), (IIIa), or (IIIb), or a pharmaceutically acceptable salt thereof, or a stereoisomer thereof, as described above, wherein:
R₇ is H, or C₁, C₂, C₃, C₄, C₅, C₆, C₇, or C₈ hydrocarbyl.

In some embodiments, the present disclosure provides the amino lipid compound of formula (I), (Ia), (Ib), (Ic), (Id), (Ie), (II), (IIa), (IIb), (III), (IIIa), (IIIb), or (IV), or a pharmaceutically acceptable salt thereof, or a stereoisomer thereof, as described above, wherein:
R₃ is H, or C₁, C₂, C₃, C₄, C₅, C₆, C₇, or C₈ hydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), or (IV), or a pharmaceutically acceptable salt thereof, or a stereoisomer thereof, as described above, wherein:
R₄ is H, or C₁, C₂, or C₃ hydrocarbylene.

In some embodiments, the present disclosure provides the amino lipid compound of formula (I), (Ia), (Ib), (Ic), (Id), (Ie), (II), (IIa), (IIb), (III), (IIIa), (IIIb), or (IV), or a pharmaceutically acceptable salt thereof, or a stereoisomer thereof, as described above, wherein:
R₄ is optionally substituted C₁-C₃ hydrocarbylene;
preferably, R₄ is C₁-C₃ hydrocarbylene optionally substituted with hydroxyl, an ester group, hydrocarbyloxy, hydrocarbyl, halogen, oxygen, sulfur, amino, or an amide group.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₁ is H, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₁ is H, a straight C₁-C₁₈ alkyl, a straight C₂-C₁₈ alkenyl, or a straight C₂-C₁₈ alkynyl.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₁ is a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₁ is H, a branched C₃-C₁₈ alkyl, a branched C₃-C₁₈ alkenyl, or a branched C₄-C₁₈ alkynyl.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₁ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbyl containing O or S.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is a straight C₁-C₁₈ alkyl, or a straight C₂-C₁₈ alkenyl, or a straight C₂-C₁₈ alkynyl, or a straight C₁-C₁₈ heteroalkyl containing O or S, or a straight C₂-C₁₈ heteroalkenyl containing O or S, or a straight heteroalkynyl containing O or S.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl, or a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is a branched C₃-C₁₈ alkyl, or a branched C₃-C₁₈ alkenyl, or a branched C₄-C₁₈ alkynyl, or a branched C₃-C₁₈ heteroalkyl containing O or S, or a branched C₃-C₁₈ heteroalkenyl containing O or S, or a branched C₄-C₁₈ heteroalkynyl containing O or S.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), or (Ie), or a pharmaceutically acceptable salt thereof, or a stereoisomer thereof, as described above, wherein:
R₈ is

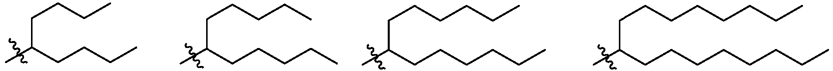

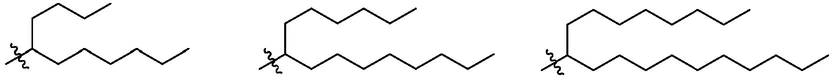

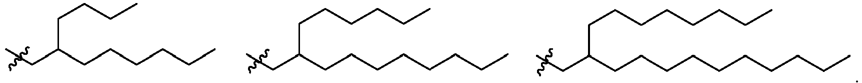

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is a straight C₁-C₁₈ alkyl, or a straight C₂-C₁₈ alkenyl, or a straight C₂-C₁₈ alkynyl, or a straight C₁-C₁₈ heteroalkyl containing O or S, or a straight C₂-C₁₈ heteroalkenyl containing O or S, or a straight C₂-C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl, or a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is a branched C₃-C₁₈ alkyl, or a branched C₃-C₁₈ alkenyl, or a branched C₄-C₁₈ alkynyl, or a branched C₃-C₁₈ heteroalkyl containing O or S, or a branched C₃-C₁₈ heteroalkenyl containing O or S, or a branched C₄-C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₉ is

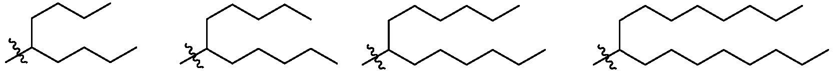

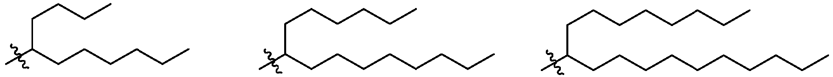

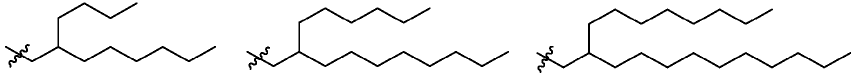

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₂ is H, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₂ is H, a straight C₁-C₁₈ alkyl, a straight C₂-C₁₈ alkenyl, or a straight C₂-C₁₈ alkynyl.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₂ is a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₂ is H, a branched C₃-C₁₈ alkyl, a branched C₃-C₁₈ alkenyl, or a branched C₄-C₁₈ alkynyl.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₂ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbyl containing O or S.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is a straight C₁-C₁₈ alkyl, or a straight C₂-C₁₈ alkenyl, or a straight C₂-C₁₈ alkynyl, or a straight C₁-C₁₈ heteroalkyl containing O or S, or a straight C₂-C₁₈ heteroalkenyl containing O or S, or a straight C₂-C₁₈ heteroalkynyl containing O or S.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl, or a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is a branched C₃-C₁₈ alkyl, or a branched C₃-C₁₈ alkenyl, or a branched C₄-C₁₈ alkynyl, or a branched C₃-C₁₈ heteroalkyl containing O or S, or a branched C₃-C₁₈ heteroalkenyl containing O or S, or a branched C₄-C₁₈ heteroalkynyl containing O or S.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), or (IIb), or a pharmaceutically acceptable salt thereof, or a stereoisomer thereof, as described above, wherein:
R₁₀ is

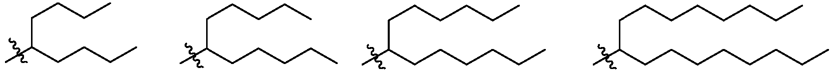

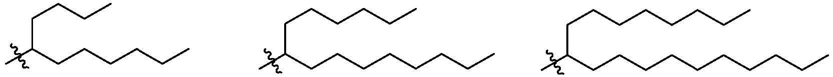

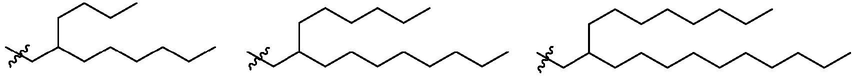

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is a straight C₁-C₁₈ alkyl, or a straight C₂-C₁₈ alkenyl, or a straight C₂-C₁₈ alkynyl, or a straight C₁-C₁₈ heteroalkyl containing O or S, or a straight C₂-C₁₈ heteroalkenyl containing O or S, or a straight C₂-C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is a straight C₁, C₂, C₃, C₄, C₅, C₆, C₇, Cs, C₉, C₁₀, C₁₁, C₁₂, C₁₃, C₁₄, C₁₅, C₁₆, C₁₇, or C₁₈ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynyl, or a straight C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a straight C₂, C₃, C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is a branched C₃-C₁₈ alkyl, or a branched C₃-C₁₈ alkenyl, or a branched C₄-C₁₈ alkynyl, or a branched C₃-C₁₈ heteroalkyl containing O or S, or a branched C₃-C₁₈ heteroalkenyl containing O or S, or a branched C₄-C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is a branched C₃, C₄, C₅, C₆, C₇, C₈, C₉, C₁₀, C₁₁, C₁₂, C₁₃, C₁₄, C₁₅, C₁₆, C₁₇, or C₁₈ alkyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ alkenyl, or a branched C₄, C₅, C₆, C₇, C₈, C₉, C₁₀, C₁₁, C₁₂, C₁₃, C₁₄, C₁₅, C₁₆, C₁₇, or C₁₈ alkynyl, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, or C₁₈ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, or C₁₈ heteroalkynyl containing O or S.

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
R₁₁ is

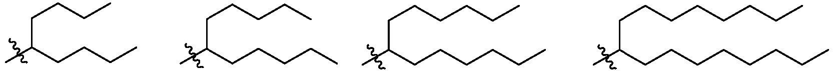

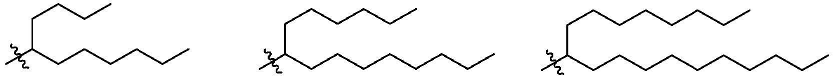

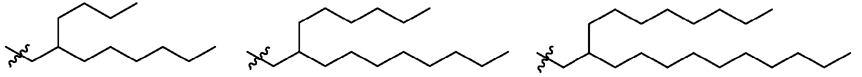

In some embodiments, the present disclosure provides the amino lipid compound of formula (I), (Ia), (Ib), (Ic), (Id), (Ie), (II), (IIa), (IIb), (III), (IIIa), (IIIb), (IV), (Va), (Vb), (Vc), or (Vd), or a pharmaceutically acceptable salt thereof, or a stereoisomer thereof, as described above, wherein:
the hydrocarbyl, hydrocarbylene, alkyl, alkylene, alkenyl, alkenylene, alkynyl, alkynylene, and heterohydrocarbyl are optionally substituted;
optionally, the hydrocarbyl, hydrocarbylene, alkyl, alkylene, alkenyl, alkenylene, alkynyl, alkynylene, and heterohydrocarbyl are optionally substituted with hydroxyl, an ester group, hydrocarbyloxy, hydrocarbyl, halogen, oxygen, sulfur, amino, or an amide group.

In yet another aspect, the present disclosure provides an amino lipid compound represented by the following formula (II-I): 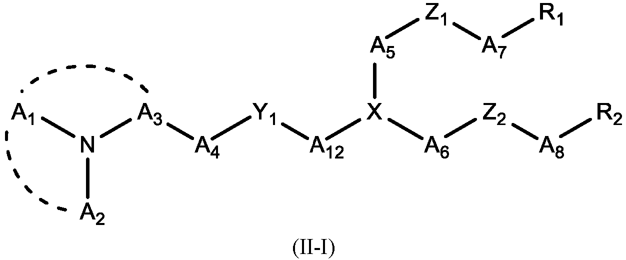
or a pharmaceutically acceptable salt thereof, or a stereoisomer thereof,
wherein,
A₁, A₂, and A₃ are one of the following:
   (1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring; or
   (3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
   (4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene or a bond;
A₅, A₆, A₇, A₈, and A₁₂ are each independently C₁-C₁₈ hydrocarbylene or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
Y₁ is -Z₃C(=O)Z₄-, -N(R₅)-, -Z₃C(=S)Z₄-, -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-, -OP(=O)O-, -OP(=O)(OH)O-, -OP(=O)(H)O-, -O-, -S-, -NHS(=O)₂O-, -NHP(=O)O-, -OP(=O)NH-, -NHP(=O)(OH)O-, -OP(=O)(OH)NH-, -OC(=O)S-, or -SC(=O)O-;
X is C or N;
R₁ is H, C₁-C₂₄ hydrocarbyl, C₁-C₂₄ heterohydrocarbyl, Z₆R₈, or -CH(OR₉)₂;
R₂ is H, C₁-C₂₄ hydrocarbyl, C₁-C₂₄ heterohydrocarbyl, Z₇R₁₀, or -CH(OR₁₁)₂;
Z₃ and Z₄ are each independently -O-, -N(R₆)-, -S-, or a bond;
Z₆ and Z₇ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
R₅ is -C(=O)A₉Z₅A₁₀, -C(=O)A₁₁, H, or C₁-C₈ hydrocarbyl;
Z₅ is -O- or -S-;
each R₆ is independently H or C₁-C₈ hydrocarbyl;
R₈ and R₁₀ are each independently H, C₁-C₂₄ hydrocarbyl, or C₁-C₂₄ heterohydrocarbyl containing O or S;
R₉ and R₁₁ are each independently C₁-C₂₄ hydrocarbyl, or C₁-C₂₄ heterohydrocarbyl containing O or S;
A₉ is C₁-C₈ hydrocarbylene or a bond;
A₁₀ and A₁₁ are each independently C₁-C₈ hydrocarbyl;
preferably, the hydrocarbyl is alkyl, alkenyl, or alkynyl;
preferably, the hydrocarbylene is alkylene, alkenylene, or alkynylene;
preferably, the heterohydrocarbyl is heteroalkyl, heteroalkenyl, or heteroalkynyl;
preferably, the heterohydrocarbylene is heteroalkylene, heteroalkenylene, or heteroalkynylene.

In yet another aspect, the present disclosure provides an amino lipid compound represented by the following formula (II-I): 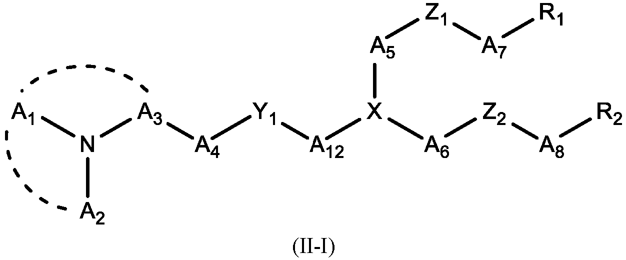
or a pharmaceutically acceptable salt thereof, or a stereoisomer thereof,
wherein,
A₁, A₂, and A₃ are one of the following:
   (1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring;
   (3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
   (4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene or a bond;
A₅, A₆, A₇, A₈, and A₁₂ are each independently C₁-C₁₈ hydrocarbylene, C₁-C₁₈ heterohydrocarbylene, or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
Y₁ is -Z₃C(=O)Z₄-, -N(R₅)-, -Z₃C(=S)Z₄-, -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-, -OP(=O)O-, -OP(=O)(OH)O-, -OP(=O)(H)O-, -O-, -S-, -NHS(=O)₂O-, -NHP(=O)O-, -OP(=O)NH-, -NHP(=O)(OH)O-, -OP(=O)(OH)NH-, -OC(=O)S-, or -SC(=O)O-;
X is C or N;
R₁ is H, C₁-C₂₄ hydrocarbyl, C₁-C₂₄ heterohydrocarbyl, Z₆R₈, -C(R₆)(OA₁₃Z₈R₉)₂, -C(R₆)(SA₁₃Z₈R₉)₂, or -C(R₆)(SA₁₃Z₈R₉)(OA₁₃Z₈R₉); R₂ is H, C₁-C₂₄ hydrocarbyl, C₁-C₂₄ heterohydrocarbyl, Z₇R₁₀, -C(R₆)(OA₁₄Z₉R₁₁)₂, -C(R₆)(SA₁₄Z₉R₁₁)₂, or -C(R₆)(SA₁₄Z₉R₁₁)(OA₁₄Z₉R₁₁);
Z₃ and Z₄ are each independently -O-, -N(R₆)-, -S-, or a bond;
Z₆ and Z₇ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
R₅ is -C(=O)A₉Z₅A₁₀, -C(=O)A₁₁, H, or C₁-C₈ hydrocarbyl;
Z₅ is -O- or -S-;
Z₈ and Z₉ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, -NHS(=O)₂O-, or a bond;
each R₆ is independently H or C₁-C₈ hydrocarbyl;
R₈ and R₁₀ are each independently H, C₁-C₂₄ hydrocarbyl, or C₁-C₂₄ heterohydrocarbyl containing O or S;
R₉ and R₁₁ are each independently C₁-C₂₄ hydrocarbyl, or C₁-C₂₄ heterohydrocarbyl containing O or S;
A₉ is C₁-C₈ hydrocarbylene or a bond;
A₁₀ and A₁₁ are each independently C₁-C₈ hydrocarbyl;
A₁₃ and A₁₄ are each independently C₁-C₈ hydrocarbylene, C₁-C₈ heterohydrocarbylene, or a bond;
preferably, the hydrocarbyl is alkyl, alkenyl, or alkynyl;
preferably, the hydrocarbylene is alkylene, alkenylene, or alkynylene;
preferably, the heterohydrocarbyl is heteroalkyl, heteroalkenyl, or heteroalkynyl;
preferably, the heterohydrocarbylene is heteroalkylene, heteroalkenylene, or heteroalkynylene.

The present disclosure provides the amino lipid compound of formula (II-I), or a pharmaceutically acceptable salt thereof, or a stereoisomer thereof, as described above, including one or more of the following features where applicable.

In some embodiments, the present disclosure provides the amino lipid compound of formula (II-I), or a pharmaceutically acceptable salt thereof, or a stereoisomer thereof, as described above, wherein:
A₁ is H, and A₂ is H; or A₁ is H, and A₂ is not H; or A₁ is not H, and A₂ is H.

In some embodiments, the present disclosure provides the amino lipid compound of formula (II-I), or a pharmaceutically acceptable salt thereof, or a stereoisomer thereof, as described above, wherein:
A₁ and A₂ are each independently C₁, C₂, C₃, C₄, or C₅ hydrocarbyl, or C₁, C₂, C₃, C₄, or C₅ heterohydrocarbyl, or C₁, C₂, C₃, C₄, or C₅ hydrocarbylene, or C₁, C₂, C₃, C₄, or C₅ heterohydrocarbylene.

In some embodiments, the present disclosure provides the amino lipid compound of formula (II-I), or a pharmaceutically acceptable salt thereof, or a stereoisomer thereof, as described above, wherein:
A₁ and A₂ are each independently C₁-C₅ alkyl, or C₂-C₅ alkenyl with 1 or 2 double bonds, or C₂-C₅ alkynyl with 1 or 2 triple bonds, or C₁-C₅ heteroalkyl containing S or O, or C₂-C₅ heteroalkenyl containing S or O with 1 or 2 double bonds, or C₂-C₅ heteroalkynyl containing S or O with 1 or 2 triple bonds, or C₁-C₅ alkylene, or C₂-C₅ alkenylene with 1 or 2 double bonds, or C₂-C₅ alkynylene with 1 or 2 triple bonds, or C₁-C₅ heteroalkylene containing S or O, or C₂-C₅ heteroalkenylene containing S or O with 1 or 2 double bonds, or C₂-C₅ heteroalkynylene containing S or O with 1 or 2 triple bonds.

In some embodiments, the present disclosure provides the amino lipid compound of formula (II-I), or a pharmaceutically acceptable salt thereof, or a stereoisomer thereof, as described above, wherein:
A₁ and/or A₂ is unsubstituted C₁-C₅ hydrocarbyl, or unsubstituted C₁-C₅ heterohydrocarbyl, or unsubstituted C₁-C₅ hydrocarbylene, or unsubstituted C₁-C₅ heterohydrocarbylene.

In some embodiments, the present disclosure provides the amino lipid compound of formula (II-I), or a pharmaceutically acceptable salt thereof, or a stereoisomer thereof, as described above, wherein:
A₁ and/or A₂ is substituted C₁-C₅ hydrocarbyl, or substituted C₁-C₅ heterohydrocarbyl, or substituted C₁-C₅ hydrocarbylene, or substituted C₁-C₅ heterohydrocarbylene.

In some embodiments, the present disclosure provides the amino lipid compound of formula (II-I), or a pharmaceutically acceptable salt thereof, or a stereoisomer thereof, as described above, wherein:
A₁ and/or A₂ is substituted C₁-C₅ alkyl, or substituted C₂-C₅ alkenyl, or substituted C₃-C₅ alkynyl, or substituted C₁-C₅ alkylene, or substituted C₂-C₅ alkenylene, or substituted C₃-C₅ alkynylene.

In some embodiments, the present disclosure provides the amino lipid compound of formula (II-I), or a pharmaceutically acceptable salt thereof, or a stereoisomer thereof, as described above, wherein:
A₁ and/or A₂ is C₁-C₅ alkyl, C₂-C₅ alkenyl, C₃-C₅ alkynyl, C₁-C₅ alkylene, C₂-C₅ alkenylene, or C₃-C₅ alkynylene, which is substituted with hydroxyl, cyano, phenyl, C₃-C₇ cyclohydrocarbyl, C₃-C₇ cyclohydrocarbyl containing 1-3 heteroatoms independently selected from nitrogen, oxygen, or sulfur, 5- to 6-membered monocyclic heteroaryl containing 1-4 heteroatoms independently selected from nitrogen, oxygen, and sulfur, 8- to 10-membered bicyclic heteroaryl containing 1-4 heteroatoms independently selected from nitrogen, oxygen, or sulfur, -OR₁₂, -C(=O)R₁₂, -C(=O)SR₁₂, -OC(=O)R₁₂, -OC(=O)OR₁₂, -N(R₁₂)₂, -C(=O)N(R₁₂)₂, -C(=S)N(R₁₂)₂, -S(=O)₂R₁₂, -S(=O)₂N(R₁₂)₂, -OC(=O)N(R₁₂)₂, -CH(NR₁₂)N(R₁₂)₂, -C(NR₁₂)₂R₁₂, -C(=O)N(R₁₂)OR₁₂, -CH(R₁₂)N(R₁₂)₂C(=O)OR₁₂, -C(R₁₂)₃, -N(R₁₂)C(=O)R₁₂, -N(R₁₂)C(=O)OR₁₂, -N(R₁₂)S(=O)₂R₁₂, -N(R₁₂)C(=O)N(R₁₂)₂, -N(R₁₂)C(=S)N(R₁₂)₂, -N(R₁₂)C(NR₁₂)N(R₁₂)₂, -N(R₁₂)C(CHR₁₂)N(R₁₂)₂, -N(OR₁₂)C(=O)R₁₂, -N(OR₁₂)S(=O)₂R₁₂, -N(OR₁₂)C(=O)OR₁₂, -N(OR₁₂)C(=O)N(R₁₂)₂, -N(OR₁₂)C(=S)N(R₁₂)₂, -N(OR₁₂)C(NR₁₂)N(R₁₂)₂, -N(OR₁₂)C(CHR₁₂)N(R₁₂)₂, -OP(=O)(OR₁₂)₂, -P(=O)(OR₁₂)₂, or a 3- to 7-membered cycloaliphatic ring and a 3- to 7-membered heterocyclyl ring each containing 1-3 heteroatoms independently selected from nitrogen, oxygen and sulfur, -C(=N-CN)N(R₁₂)₂, -C(=N-O-CH₃)N(R₁₂)₂, -C(=N-SO₂-NH₂)N(R₁₂)₂, -C(=CH-NO₂)N(R₁₂)₂, or -C(=O)OR₁₂,
wherein each R₁₂ is independently hydrogen, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
A₁ and/or A₂ is C₃-C₇ cyclohydrocarbyl containing 1-3 heteroatoms independently selected from nitrogen, oxygen, or sulfur, substituted with C₁-C₅ alkyl, =O, -C₀-C₅ alkylene-hydroxyl, -C(=O)N(R₁₅)₂; 8- to 10-membered bicyclic heteroaryl containing 1-4 heteroatoms independently selected from nitrogen, oxygen, or sulfur, optionally substituted with C₁-C₅ alkyl, hydroxyl; C₁-C₅ alkyl, C₂-C₅ alkenyl, C₃-C₅ alkynyl, C₁-C₅ alkylene, C₂-C₅ alkenylene, or C₃-C₅ alkynylene, which is substituted with 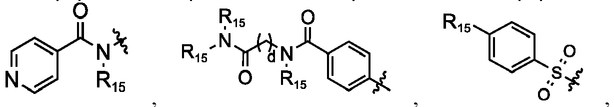 or 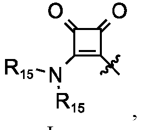 wherein each R₁₅ is independently H or C₁-C₃ alkyl, and d is 0, 1, 2, 3, 4, or 5.

In some embodiments, the present disclosure provides the amino lipid compound of formula (II-I), or a pharmaceutically acceptable salt thereof, or a stereoisomer thereof, as described above, wherein:
A₁ and/or A₂ is

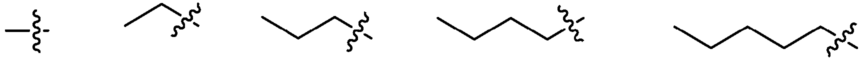

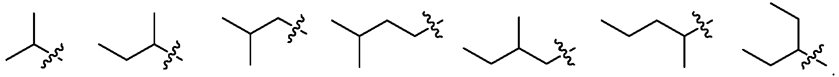

In some embodiments, the present disclosure provides the amino lipid compound of formula (II-I), or a pharmaceutically acceptable salt thereof, or a stereoisomer thereof, as described above, wherein:
A₁ and/or A₂ may also be 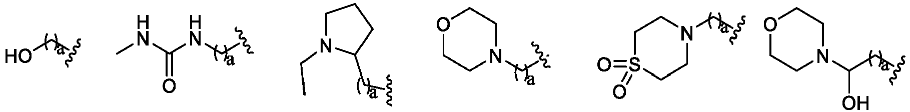 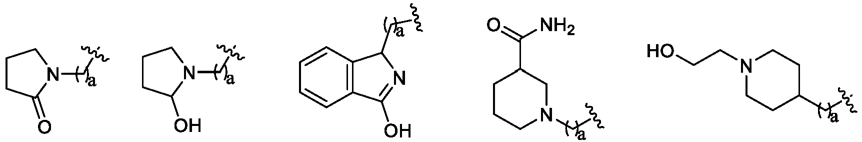 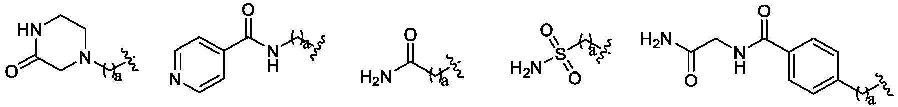 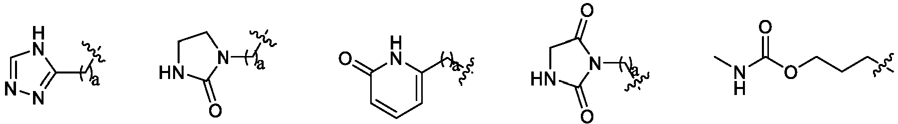 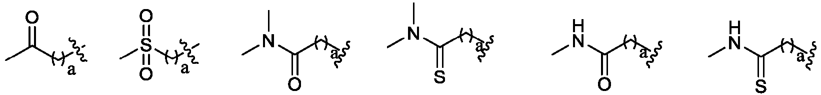 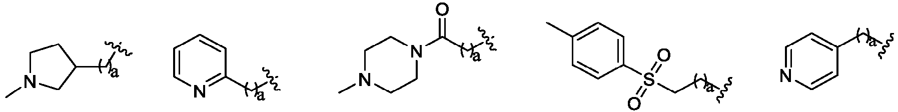 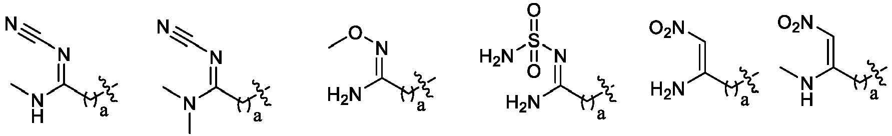 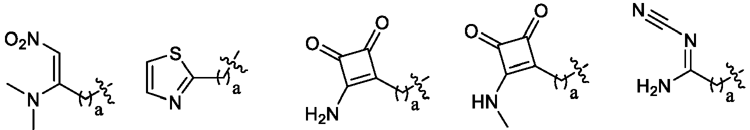
wherein a is 1, 2, 3, 4, or 5;
preferably, the a is 1, 2, or 3.

In some embodiments, the present disclosure provides the amino lipid compound of formula (II-I), or a pharmaceutically acceptable salt thereof, or a stereoisomer thereof, as described above, wherein:
A₃ is a bond.

In some embodiments, the present disclosure provides the amino lipid compound of formula (II-I), or a pharmaceutically acceptable salt thereof, or a stereoisomer thereof, as described above, wherein:
A₃ is C₁, C₂, C₃, C₄, or C₅ hydrocarbylene.

In some embodiments, the present disclosure provides the amino lipid compound of formula (II-I), or a pharmaceutically acceptable salt thereof, or a stereoisomer thereof, as described above, wherein:
A₃ is C₁-C₅ alkylene, or C₂-C₅ alkenylene with 1 or 2 double bonds, or C₂-C₅ alkynylene with 1 or 2 triple bonds.

In some embodiments, the present disclosure provides the amino lipid compound of formula (II-I), or a pharmaceutically acceptable salt thereof, or a stereoisomer thereof, as described above, wherein:
A₃ is unsubstituted C₁-C₅ alkylene, or unsubstituted C₂-C₅ alkenylene, or unsubstituted C₃-C₅ alkynylene.

In some embodiments, the present disclosure provides the amino lipid compound of formula (II-I), or a pharmaceutically acceptable salt thereof, or a stereoisomer thereof, as described above, wherein:
A₃ is substituted C₁-C₅ alkylene, or substituted C₂-C₅ alkenylene, or substituted C₃-C₅ alkynylene.

In some embodiments, the present disclosure provides the amino lipid compound of formula (II-I), or a pharmaceutically acceptable salt thereof, or a stereoisomer thereof, as described above, wherein:
A₃ is C₁-C₅ alkylene, C₂-C₅ alkenylene, or C₃-C₅ alkynylene, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when A₃ is C₁-C₅ alkylene, C₂-C₅ alkenylene, or C₃-C₅ alkynylene, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on A₃ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
when the dashed line connecting A₁ and A₂ is a bond and the dashed line connecting A₁ and A₃ is absent, A₁ and A₂, together with the nitrogen atom to which they are both attached, form an N-containing three-, four-, five-, six-, seven-, or eight-membered heterocyclic ring.

In some embodiments, A₁ and A₂, together with the nitrogen atom to which they are both attached, form an N-containing three-, four-, five-, or six-membered heterocyclic ring.

In some embodiments, A₁ and A₂, together with the nitrogen atom to which they are both attached, form an N-containing four-, five-, or six-membered heterocyclic ring.

In some embodiments, the present disclosure provides the amino lipid compound of formula (II-I), or a pharmaceutically acceptable salt thereof, or a stereoisomer thereof, as described above, wherein:
when the dashed line connecting A₁ and A₃ is a bond and the dashed line connecting A₁ and A₂ is absent, A₁ and A₃, together with the nitrogen atom to which they are both attached, form an N-containing three-, four-, five-, six-, seven-, or eight-membered heterocyclic ring.

In some embodiments, A₁ and A₃, together with the nitrogen atom to which they are both attached, form an N-containing three-, four-, five-, or six-membered heterocyclic ring.

In some embodiments, A₁ and A₃, together with the nitrogen atom to which they are both attached, form an N-containing four-, five-, or six-membered heterocyclic ring.

In some embodiments, the present disclosure provides the amino lipid compound of formula (II-I), or a pharmaceutically acceptable salt thereof, or a stereoisomer thereof, as described above, wherein: 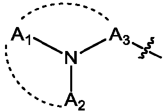 is 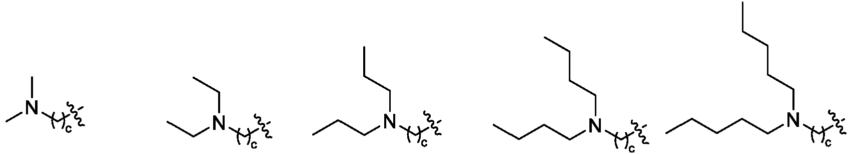 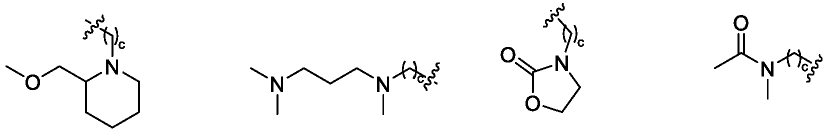 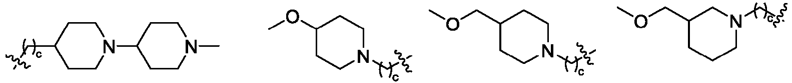 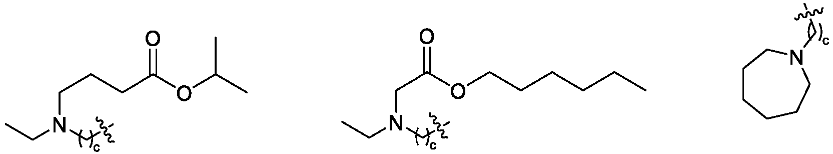 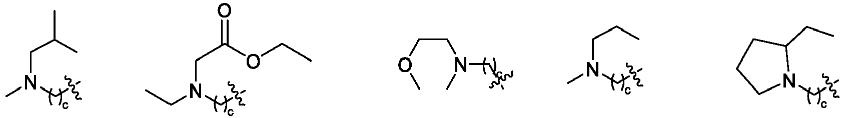 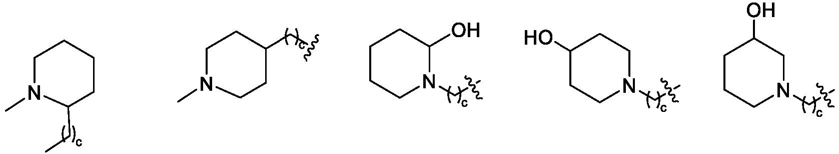 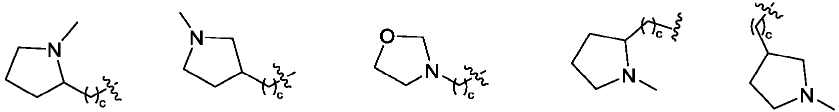 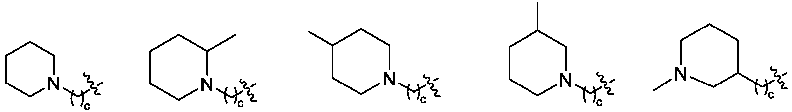 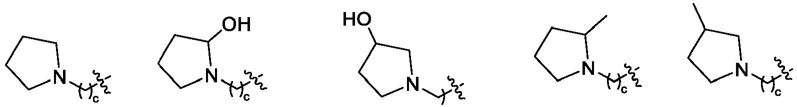 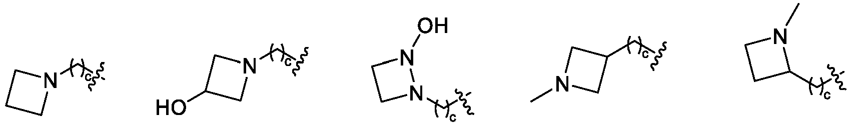 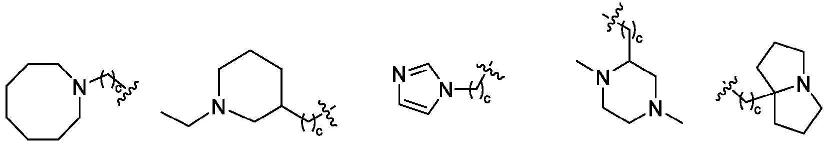 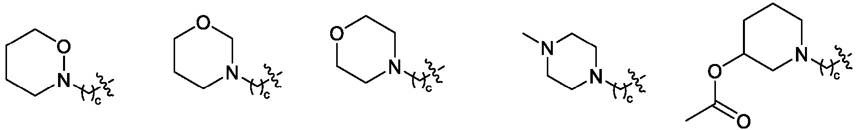 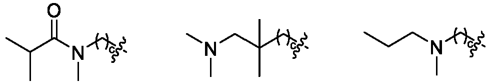
wherein c is 1, 2, 3, 4, or 5;
preferably, the c is 2, 3, or 4;
preferably, the c is 2 or 3.

In some embodiments, the present disclosure provides the amino lipid compound of formula (II-I), or a pharmaceutically acceptable salt thereof, or a stereoisomer thereof, as described above, wherein:
A₄ is a bond.

In some embodiments, the present disclosure provides the amino lipid compound of formula (II-I), or a pharmaceutically acceptable salt thereof, or a stereoisomer thereof, as described above, wherein:
A₄ is C₁, C₂, C₃, C₄, or C₅ hydrocarbylene.

In some embodiments, the present disclosure provides the amino lipid compound of formula (II-I), or a pharmaceutically acceptable salt thereof, or a stereoisomer thereof, as described above, wherein:
A₄ is C₁-C₅ alkylene, or C₂-C₅ alkenylene with 1 or 2 double bonds, or C₂-C₅ alkynylene with 1 or 2 triple bonds.

In some embodiments, the present disclosure provides the amino lipid compound of formula (II-I), or a pharmaceutically acceptable salt thereof, or a stereoisomer thereof, as described above, wherein:
A₄ is unsubstituted C₁-C₅ alkylene, or unsubstituted C₂-C₅ alkenylene, or unsubstituted C₃-C₅ alkynylene.

In some embodiments, the present disclosure provides the amino lipid compound of formula (II-I), or a pharmaceutically acceptable salt thereof, or a stereoisomer thereof, as described above, wherein:
A₄ is substituted C₁-C₅ alkylene, or substituted C₂-C₅ alkenylene, or substituted C₃-C₅ alkynylene.

In some embodiments, the present disclosure provides the amino lipid compound of formula (II-I), or a pharmaceutically acceptable salt thereof, or a stereoisomer thereof, as described above, wherein:
A₄ is C₁-C₅ alkylene, C₂-C₅ alkenylene, or C₃-C₅ alkynylene, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when A₄ is C₁-C₅ alkylene, C₂-C₅ alkenylene, or C₃-C₅ alkynylene, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on A₄ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
A₅, A₆, A₇, A₈, and A₁₂ are each independently C₁-C₁₈ hydrocarbylene or a bond.

In some embodiments, the present disclosure provides the amino lipid compound of formula (II-I), or a pharmaceutically acceptable salt thereof, or a stereoisomer thereof, as described above, wherein:
A₅, A₆, A₇, A₈, and A₁₂ are each independently C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene, or a bond.

In some embodiments, A₅, A₆, A₇, A₈, and A₁₂ are each independently a bond.

In some other embodiments, A₅, A₆, A₇, A₈, and A₁₂ are each independently C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, the present disclosure provides the amino lipid compound of formula (II-I), or a pharmaceutically acceptable salt thereof, or a stereoisomer thereof, as described above, wherein:
A₅, A₆, A₇, A₈, and A₁₂ are each independently C₁-C₁₈ alkylene, or C₂-C₁₈ alkenylene with 1, 2, 3, or more double bonds, or C₂-C_{l 8} alkynylene with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, the present disclosure provides the amino lipid compound of formula (II-I), or a pharmaceutically acceptable salt thereof, or a stereoisomer thereof, as described above, wherein:
A₁₂ is a bond, and A₅ and A₆ are not both C₁ hydrocarbylene.

In some embodiments, the present disclosure provides the amino lipid compound of formula (II-I), or a pharmaceutically acceptable salt thereof, or a stereoisomer thereof, as described above, wherein:
A₇ and A₈ are each independently C₁, C₂, C₃, C₄, C₅, C₆, or C₇ hydrocarbylene, or a bond.

In some embodiments, the present disclosure provides the amino lipid compound of formula (II-I), or a pharmaceutically acceptable salt thereof, or a stereoisomer thereof, as described above, wherein:
A₁₂ is C₁ hydrocarbylene, and A₅ is C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene; or A₁₂ is C₁ hydrocarbylene, and A₆ is C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, the present disclosure provides the amino lipid compound of formula (II-I), or a pharmaceutically acceptable salt thereof, or a stereoisomer thereof, as described above, wherein:
A₅ and/or A₆ is unsubstituted C₁-C₁₂ alkylene, or unsubstituted C₂-C₁₂ alkenylene, or unsubstituted C₃-C₁₂ alkynylene. For example, A₅ and/or A₆ is unsubstituted C₁-C₁₂ alkylene, or unsubstituted C₃-C₉ alkylene, or unsubstituted C₄-C₉ alkylene, or unsubstituted C₅-C₈ alkylene, or unsubstituted C₈ alkylene.

In some embodiments, the present disclosure provides the amino lipid compound of formula (II-I), or a pharmaceutically acceptable salt thereof, or a stereoisomer thereof, as described above, wherein:
A₅ and/or A₆ is substituted C₁-C₁₂ alkylene, or substituted C₂-C₁₂ alkenylene, or substituted C₃-C₁₂ alkynylene. For example, A₅ and/or A₆ is substituted C₁-C₁₂ alkylene, or substituted C₃-C₉ alkylene, or substituted C₄-C₉ alkylene, or substituted C₅-C₈ alkylene, or substituted C₈ alkylene.

In some embodiments, the present disclosure provides the amino lipid compound of formula (II-I), or a pharmaceutically acceptable salt thereof, or a stereoisomer thereof, as described above, wherein:
A₇ and/or A₈ is unsubstituted C₁-C₁₂ alkylene, or unsubstituted C₂-C₁₂ alkenylene, or unsubstituted C₃-C₁₂ alkynylene. For example, A₇ and/or A₈ is unsubstituted C₁-C₅ alkylene, or unsubstituted C₂-C₄ alkylene, or unsubstituted C₃ alkylene.

In some embodiments, the present disclosure provides the amino lipid compound of formula (II-I), or a pharmaceutically acceptable salt thereof, or a stereoisomer thereof, as described above, wherein:
A₇ and/or A₈ is substituted C₁-C₁₂ alkylene, or substituted C₂-C₁₂ alkenylene, or substituted C₃-C₁₂ alkynylene. For example, A₇ and/or A₈ is substituted C₁-C₅ alkylene, or substituted C₂-C₄ alkylene, or substituted C₃ alkylene.

In some embodiments, the present disclosure provides the amino lipid compound of formula (II-I), or a pharmaceutically acceptable salt thereof, or a stereoisomer thereof, as described above, wherein:
A₁₂ is unsubstituted C₁-C₁₂ alkylene, or unsubstituted C₂-C₁₂ alkenylene, or unsubstituted C₃-C₁₂ alkynylene. For example, A₁₂ is unsubstituted C₁-C₅ alkylene or unsubstituted C₁-C₃ alkylene.

In some embodiments, the present disclosure provides the amino lipid compound of formula (II-I), or a pharmaceutically acceptable salt thereof, or a stereoisomer thereof, as described above, wherein:
A₁₂ is substituted C₁-C₁₂ alkylene, or substituted C₂-C₁₂ alkenylene, or substituted C₃-C₁₂ alkynylene. For example, A₁₂ is substituted C₁-C₅ alkylene or substituted C₁-C₃ alkylene.

In some embodiments, the present disclosure provides the amino lipid compound of formula (II-I), or a pharmaceutically acceptable salt thereof, or a stereoisomer thereof, as described above, wherein:
at least one of A₅, A₆, A₇, A₈ and A₁₂ is C₁-C₁₂ alkylene, C₂-C₁₂ alkenylene, or C₃-C₁₂ alkynylene, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when at least one of A₅, A₆, A₇, A₈ and A₁₂ is C₁-C₁₂ alkylene, C₂-C₁₂ alkenylene, or C₃-C₁₂ alkynylene, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted A₅, A₆, A₇, A₈, or A₁₂ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
A₉ is a bond.

In some embodiments, the present disclosure provides the amino lipid compound of formula (II-I), or a pharmaceutically acceptable salt thereof, or a stereoisomer thereof, as described above, wherein:
A₉ is C₁, C₂, C₃, C₄, C₅, C₆, C₇, or C₈ hydrocarbylene, or C₁, C₂, C₃, C₄, C₅, C₆, C₇, or C₈ heterohydrocarbylene.

In some embodiments, the present disclosure provides the amino lipid compound of formula (II-I), or a pharmaceutically acceptable salt thereof, or a stereoisomer thereof, as described above, wherein:
A₉ is C₁-C₈ alkylene, or C₂-C₈ alkenylene with 1, 2, or more double bonds, or C₂-C₈ alkynylene with 1, 2, or more triple bonds.

In some embodiments, the present disclosure provides the amino lipid compound of formula (II-I), or a pharmaceutically acceptable salt thereof, or a stereoisomer thereof, as described above, wherein:
each A₉ is independently unsubstituted C₁-C₈ alkylene, or unsubstituted C₂-C₈ alkenylene, or unsubstituted C₃-C₈ alkynylene.

In some embodiments, the present disclosure provides the amino lipid compound of formula (II-I), or a pharmaceutically acceptable salt thereof, or a stereoisomer thereof, as described above, wherein:
at least one of the A₉ groups is substituted C₁-C₈ alkylene, or substituted C₂-C₈ alkenylene, or substituted C₃-C₈ alkynylene.

In some embodiments, the present disclosure provides the amino lipid compound of formula (II-I), or a pharmaceutically acceptable salt thereof, or a stereoisomer thereof, as described above, wherein:
at least one of the A₉ groups is C₁-C₈ alkylene, C₂-C₈ alkenylene, or C₃-C₈ alkynylene, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when A₉ is C₁-C₈ alkylene, C₂-C₈ alkenylene, or C₃-C₈ alkynylene, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted A₉ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
A₁₀ and A₁₁ are each independently is C₁, C₂, C₃, C₄, C₅, C₆, C₇, or C₈ hydrocarbyl, or C₁, C₂, C₃, C₄, C₅, C₆, C₇, or C₈ heterohydrocarbyl.

In some embodiments, the present disclosure provides the amino lipid compound of formula (II-I), or a pharmaceutically acceptable salt thereof, or a stereoisomer thereof, as described above, wherein:
A₁₀ and A₁₁ are each independently C₁-C₈ alkyl, or C₂-C₈ alkenyl with 1, 2, or more double bonds, or C₂-C₈ alkynyl with 1, 2, or more triple bonds.

In some embodiments, the present disclosure provides the amino lipid compound of formula (II-I), or a pharmaceutically acceptable salt thereof, or a stereoisomer thereof, as described above, wherein:
each A₁₀ and/or each A₁₁ is unsubstituted C₁-C₈ alkyl, or unsubstituted C₂-C₈ alkenyl, or unsubstituted C₃-C₈ alkynyl.

In some embodiments, the present disclosure provides the amino lipid compound of formula (II-I), or a pharmaceutically acceptable salt thereof, or a stereoisomer thereof, as described above, wherein:
at least one of the A₁₀ groups and/or the A₁₁ groups is substituted C₁-C₈ alkyl, or substituted C₂-C₈ alkenyl, or substituted C₃-C₈ alkynyl.

In some embodiments, the present disclosure provides the amino lipid compound of formula (II-I), or a pharmaceutically acceptable salt thereof, or a stereoisomer thereof, as described above, wherein:
at least one of the A₁₀ groups and/or the A₁₁ groups is C₁-C₈ alkyl, C₂-C₈ alkenyl, or C₃-C₈ alkynyl, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when A₁₀ and/or A₁₁ is C₁-C₈ alkyl, C₂-C₈ alkenyl, or C₃-C₈ alkynyl, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted A₁₀ or A₁₁ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein R₅ is unsubstituted C₁-C₈ alkyl, or unsubstituted C₂-C₈ alkenyl, or unsubstituted C₂-C₈ alkynyl.

In some embodiments, the present disclosure provides the amino lipid compound of formula (II-I), or a pharmaceutically acceptable salt thereof, or a stereoisomer thereof, as described above, wherein R₅ is substituted C₁-C₈ alkyl, or substituted C₂-C₈ alkenyl, or substituted C₂-C₈ alkynyl.

In some embodiments, the present disclosure provides the amino lipid compound of formula (II-I), or a pharmaceutically acceptable salt thereof, or a stereoisomer thereof, as described above, wherein:
R₅ is C₁-C₈ alkyl, C₂-C₈ alkenyl, or C₂-C₈ alkynyl, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when R₅ is C₁-C₈ alkyl, C₂-C₈ alkenyl, or C₂-C₈ alkynyl, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on R₅ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
Y₁ is -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -C(=S)O-, -OC(=S)-, -C(=S)-, -C(=S)S-, -SC(=S)-, -C(=S)N(R₆)-, -N(R₆)C(=S)-, -OC(=S)O-, -OC(=S)S-, -SC(=S)O-, -SC(=S)S-, -N(R₆)C(=S)O-, -OC(=S)N(R₆)-, -N(R₆)C(=S)N(R₆)-, -SC(=S)N(R₆)-, -N(R₆)C(=S)S-, -N(C(=S)A₉OA₁₀)-, -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-OP(=O)O-, -OP(=O)(OH)O-, -OP(=O)(H)O-, -NHS(=O)₂O-, -NHP(=O)O-, -OP(=O)NH-, -NHP(=O)(OH)O-, -OP(=O)(OH)NH-, -OC(=O)S-, or -SC(=O)O-.

In some embodiments, Y₁ is -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₁₁), -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-, -OS(=O)₂O-, -OS(=O)O-, -OP(=O)O-, -OP(=O)(OH)O-, -OP(=O)(H)O-, -NHS(=O)₂O-, -NHP(=O)O-, -OP(=O)NH-, -NHP(=O)(OH)O-, -OP(=O)(OH)NH-, -OC(=O)S-, or -SC(=O)O-.

In some embodiments, Y₁ is -N(H)C(=O)N(H)-, -S-, -OC(=O)O-, -N(H)C(=O)O-, -OC(=O)N(H)-, -C(=O)O-, -OC(=O)-, -N((CH₂)₃C(=O)OC₅H₁₁)C(=O)O-, -OC(=O)N((CH₂)₃C(=O)OC₅H₁₁)-, -N(H)C(=O)-, -C(=O)N(H)-, -N(C(=O)(CH₂)₃OC₈H₁₇), -N(C(=O)CH₂OCH₃), -N(C(=O)CH₃), -N(C(=O)C₃H₇), -N(C₃H₇)C(=O)O-, -OC(=O)N(C₆H₁₃)-, -C(=O)N(C₄H₉)-, -N(CH(CH₃)₂)C(=O)-, -N((CH₂)₃C(=O)OC₂H₅)C(=O)O-, -N((CH₂)₃C(=O)OCH₂CH=CHC₃H₇)C(=O)O-, -N(C₄H₉)C(=O)-, -O-, -S(=O)₂O-, -OS(=O)₂-, -OS(=O)₂O-, -OS(=O)O-, -S(=O)₂NH-, -NHS(=O)₂-, -OS(=O)₂NH-, -NHS(=O)₂NH-, -OP(=O)(H)O-, -OP(=O)(OH)O-, -OC(=O)S-, -SC(=O)O-, -SC(=O)N(H)-, -N(H)C(=O)S-, -C(=O)S-, or -SC(=O)-.

In some embodiments, the present disclosure provides the amino lipid compound of formula (II-I), or a pharmaceutically acceptable salt thereof, or a stereoisomer thereof, as described above, wherein: Y₁ is -C(=O)O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -C(=S)O-, -OC(=S)-, -C(=S)-, -C(=S)S-, -SC(=S)-, -C(=S)N(R₆)-, -N(R₆)C(=S)-, -OC(=S)O-, -OC(=S)S-, -SC(=S)O-, -SC(=S)S-, -N(R₆)C(=S)O-, -OC(=S)N(R₆)-, -N(R₆)C(=S)N(R₆)-, -SC(=S)N(R₆)-, -N(R₆)C(=S)S-, -N(C(=S)A₉OA₁₀)-, -OS(=O)₂O-, -OS(=O)O-, -OS(=O)NH-, -NHS(=O)₂NH-, -NHS(=O)₂-, -S(=O)₂NH-, -S(=O)₂O-, -OS(=O)₂-, -OP(=O)O-, -OP(=O)(OH)O-, -OP(=O)(H)O-, -NHS(=O)₂O-, -NHP(=O)O-, -OP(=O)NH-, -NHP(=O)(OH)O-, -OP(=O)(OH)NH-, -OC(=O)S-, or -SC(=O)O-.

In some embodiments, the present disclosure provides the amino lipid compound of formula (II-I), or a pharmaceutically acceptable salt thereof, or a stereoisomer thereof, as described above, wherein:
Z₁ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, the present disclosure provides the amino lipid compound of formula (II-I), or a pharmaceutically acceptable salt thereof, or a stereoisomer thereof, as described above, wherein:
Z₂ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, the present disclosure provides the amino lipid compound of formula (II-I), or a pharmaceutically acceptable salt thereof, or a stereoisomer thereof, as described above, wherein:
R₁ and R₂ are each independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heterohydrocarbyl.

In some embodiments, the present disclosure provides the amino lipid compound of formula (II-I), or a pharmaceutically acceptable salt thereof, or a stereoisomer thereof, as described above, wherein:
R₁ and/or R₂ is C₁-C₂₄ alkyl (e.g., C₅-C₁₆ alkyl, C₆-C₁₄ alkyl, C₆-C₁₂ alkyl), C₂-C₂₄ alkenyl (e.g., C₅-C₁₆ alkenyl, C₆-C₁₄ alkenyl, C₆-C₁₂ alkenyl), C₂-C₂₄ alkynyl (e.g., C₅-C₁₆ alkynyl, C₆-C₁₄ alkynyl, C₆-C₁₂ alkynyl).

In some embodiments, the present disclosure provides the amino lipid compound of formula (II-I), or a pharmaceutically acceptable salt thereof, or a stereoisomer thereof, as described above, wherein:
R₁ and/or R₂ is unsubstituted C₁-C₂₄ alkyl (e.g., unsubstituted C₅-C₁₆ alkyl, unsubstituted C₆-C₁₄ alkyl, unsubstituted C₆-C₁₂ alkyl), unsubstituted C₂-C₂₄ alkenyl (e.g., unsubstituted C₅-C₁₆ alkenyl, unsubstituted C₆-C₁₄ alkenyl, unsubstituted C₆-C₁₂ alkenyl), unsubstituted C₂-C₂₄ alkynyl (e.g., unsubstituted C₅-C₁₆ alkynyl, unsubstituted C₆-C₁₄ alkynyl, unsubstituted C₆-C₁₂ alkynyl).

In some embodiments, the present disclosure provides the amino lipid compound of formula (II-I), or a pharmaceutically acceptable salt thereof, or a stereoisomer thereof, as described above, wherein:
R₁ and/or R₂ is substituted C₁-C₂₄ alkyl (e.g., substituted C₅-C₁₆ alkyl, substituted C₆-C₁₄ alkyl, substituted C₆-C₁₂ alkyl), substituted C₂-C₂₄ alkenyl (e.g., substituted C₅-C₁₆ alkenyl, substituted C₆-C₁₄ alkenyl, substituted C₆-C₁₂ alkenyl), substituted C₂-C₂₄ alkynyl (e.g., substituted C₅-C₁₆ alkynyl, substituted C₆-C₁₄ alkynyl, substituted C₆-C₁₂ alkynyl, substituted C₁₀ alkynyl).

In some embodiments, the present disclosure provides the amino lipid compound of formula (II-I), or a pharmaceutically acceptable salt thereof, or a stereoisomer thereof, as described above, wherein:
R₁ and R₂ are each independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₂₄ heteroalkyl containing O or S, or a straight C₂-C₂₄ heteroalkenyl containing O or S with 1, 2, 3, 4, or more double bonds, or a straight C₂-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₁ and R₂ are each independently a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl, or a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl.

In some embodiments, the present disclosure provides the amino lipid compound of formula (II-I), or a pharmaceutically acceptable salt thereof, or a stereoisomer thereof, as described above, wherein:
R₁ and R₂ are each independently a branched C₃-C₂₄ alkyl, or a branched C₃-C₂₄ alkenyl with 1, 2, 3, 4, or more double bonds, or a branched C₄-C₂₄ alkynyl with 1, 2, 3, 4, or more triple bonds, or a branched C₃-C₂₄ heteroalkyl containing O or S, or a branched C₃-C₂₄ heteroalkenyl containing O or S with 1, 2, 3, 4, or more double bonds, or a branched C₄-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₁ and R₂ are each independently a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl containing O or S.

In some embodiments, the present disclosure provides the amino lipid compound of formula (II-I), or a pharmaceutically acceptable salt thereof, or a stereoisomer thereof, as described above, wherein:
R₅ and R₁₀ are each independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heterohydrocarbyl.

In some embodiments, the present disclosure provides the amino lipid compound of formula (II-I), or a pharmaceutically acceptable salt thereof, or a stereoisomer thereof, as described above, wherein:
R₅ and/or R₁₀ is C₁-C₂₀ alkyl (e.g., C₂-C₁₀ alkyl, C₂-C₈ alkyl, C₇-C₁₈ alkyl), or C₁-C₁₆ alkenyl (e.g., C₅-C₁₂ alkenyl, C₅-C₈ alkenyl, C₅ alkenyl, C₆ alkenyl), or C₅-C₁₆ alkynyl (e.g., C₅-C₁₂ alkynyl, C₁₀ alkynyl).

In some embodiments, the present disclosure provides the amino lipid compound of formula (II-I), or a pharmaceutically acceptable salt thereof, or a stereoisomer thereof, as described above, wherein:
R₈ and/or R₁₀ is unsubstituted C₁-C₂₀ alkyl (e.g., unsubstituted C₂-C₁₀ alkyl, unsubstituted C₂-C₈ alkyl, unsubstituted C₇-C₁₈ alkyl), or unsubstituted C₁-C₁₆ alkenyl (e.g., unsubstituted C₅-C₁₂ alkenyl, unsubstituted C₅-C₈ alkenyl, unsubstituted C₅ alkenyl, unsubstituted C₆ alkenyl), or unsubstituted C₅-C₁₆ alkynyl (e.g., unsubstituted C₅-C₁₂ alkynyl, unsubstituted C₁₀ alkynyl).

In some embodiments, the present disclosure provides the amino lipid compound of formula (II-I), or a pharmaceutically acceptable salt thereof, or a stereoisomer thereof, as described above, wherein:
R₈ and/or R₁₀ is substituted C₁-C₂₀ alkyl (e.g., substituted C₂-C₁₀ alkyl, substituted C₂-C₈ alkyl, substituted C₇-C₁₈ alkyl), or substituted C₁-C₁₆ alkenyl (e.g., substituted C₅-C₁₂ alkenyl, substituted C₅-C₈ alkenyl, substituted C₅ alkenyl, substituted C₆ alkenyl), or substituted C₅-C₁₆ alkynyl (e.g., substituted C₅-C₁₂ alkynyl, substituted C₁₀ alkynyl).

In some embodiments, the present disclosure provides the amino lipid compound of formula (II-I), or a pharmaceutically acceptable salt thereof, or a stereoisomer thereof, as described above, wherein:
R₈ and R₁₀ are each independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₂₄ heteroalkyl containing O or S, or a straight C₂-C₂₄ heteroalkenyl containing O or S with 1, 2, 3, 4, or more double bonds, or a straight C₂-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₈ and R₁₀ are each independently a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl; or a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl.

In some embodiments, the present disclosure provides the amino lipid compound of formula (II-I), or a pharmaceutically acceptable salt thereof, or a stereoisomer thereof, as described above, wherein:
R₈ and R₁₀ are each independently a branched C₃-C₂₄ alkyl, or a branched C₃-C₂₄ alkenyl with 1, 2, 3, 4, or more double bonds, or a branched C₄-C₂₄ alkynyl with 1, 2, 3, 4, or more triple bonds, or a branched C₃-C₂₄ heteroalkyl containing O or S, or a branched C₃-C₂₄ heteroalkenyl containing O or S with 1, 2, 3, 4, or more double bonds, or a branched C₄-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₈ and R₁₀ are each independently a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl containing O or S.

In some embodiments, the present disclosure provides the amino lipid compound of formula (II-I), or a pharmaceutically acceptable salt thereof, or a stereoisomer thereof, as described above, wherein:
R₈ and/or R₁₀ is C₁-C₂₀ alkyl (e.g., substituted C₂-C₁₀ alkyl, substituted C₂-C₈ alkyl, substituted C₇-C₁₈ alkyl), C₁-C₁₆ alkenyl (e.g., substituted C₅-C₁₂ alkenyl, substituted C₅-C₈ alkenyl, substituted C₅ alkenyl, substituted C₆ alkenyl), or C₅-C₁₆ alkynyl (e.g., substituted C₅-C₁₂ alkynyl, substituted C₁₀ alkynyl), which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or 0 with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when R₈ and/or R₁₀ is each independently substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted R₈ or R₁₀ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein R₈ and/or R₁₀ is each independently substituted aryl. In some embodiments, R₈ or R₁₀ is phenyl substituted with alkyl, such as

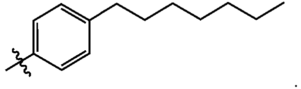

In some embodiments, the present disclosure provides the amino lipid compound of formula (II-I), or a pharmaceutically acceptable salt thereof, or a stereoisomer thereof, as described above, wherein:
R₈ and R₁₀ are each independently

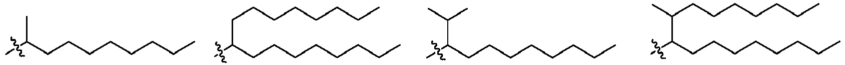

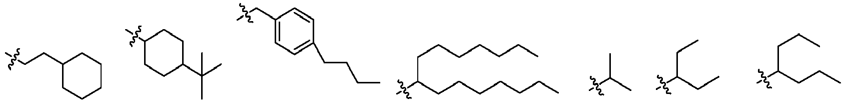

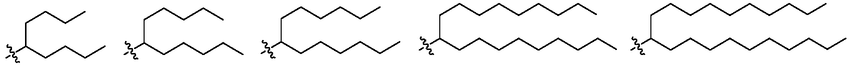

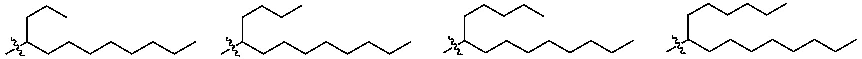

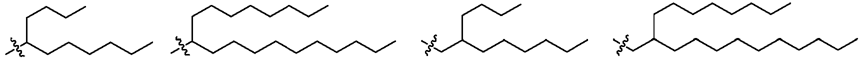

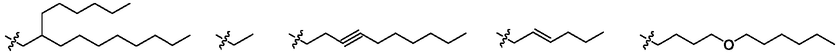

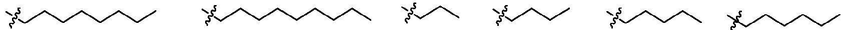

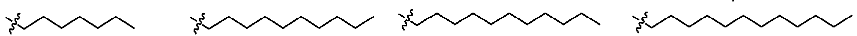

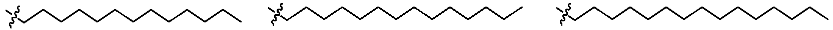

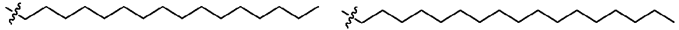

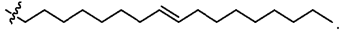

In some embodiments, the present disclosure provides the amino lipid compound of formula (II-I), or a pharmaceutically acceptable salt thereof, or a stereoisomer thereof, as described above, wherein:
each R₆ is independently C₁, C₂, C₃, C₄, C₅, C₆, C₇, or C₈ hydrocarbyl, or C₁, C₂, C₃, C₄, C₅, C₆, C₇, or C₈ heterohydrocarbyl.

In some embodiments, the present disclosure provides the amino lipid compound of formula (II-I), or a pharmaceutically acceptable salt thereof, or a stereoisomer thereof, as described above, wherein:
each R₆ is independently C₁-C₈ alkyl, or C₂-C₈ alkenyl with 1, 2, or more double bonds, or C₂-C₈ alkynyl with 1, 2, or more triple bonds.

In some embodiments, the present disclosure provides the amino lipid compound of formula (II-I), or a pharmaceutically acceptable salt thereof, or a stereoisomer thereof, as described above, wherein:
each R₆ is independently C₁, C₂, C₃, C₄, C₅, C₆, C₇, or C₈ alkyl.

In some embodiments, the present disclosure provides the amino lipid compound of formula (II-I), or a pharmaceutically acceptable salt thereof, or a stereoisomer thereof, as described above, wherein:
each R₆ is independently unsubstituted C₁-C₈ alkyl, or unsubstituted C₂-C₈ alkenyl, or unsubstituted C₂-C₈ alkynyl, or unsubstituted C₁-C₈ heteroalkyl, or unsubstituted C₂-C₈ heteroalkenyl, or unsubstituted C₂-C₈ heteroalkynyl. For example, each R₆ is independently unsubstituted C₁-C₅ alkyl, unsubstituted C₁-C₃ alkyl, or unsubstituted C₁ alkyl.

In some embodiments, the present disclosure provides the amino lipid compound of formula (II-I), or a pharmaceutically acceptable salt thereof, or a stereoisomer thereof, as described above, wherein:
at least one of the R₆ groups is substituted C₁-C₈ alkyl, or substituted C₂-C₈ alkenyl, or substituted C₂-C₈ alkynyl, for example, one of the R₆ groups is substituted C₁-C₅ alkyl, substituted C₁-C₃ alkyl, or substituted C₃ alkyl.

In some embodiments, the present disclosure provides the amino lipid compound of formula (II-I), or a pharmaceutically acceptable salt thereof, or a stereoisomer thereof, as described above, wherein:
at least one of the R₆ groups is C₁-C₈ alkyl, C₂-C₈ alkenyl, or C₂-C₈ alkynyl, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when R₆ is C₁-C₈ alkyl, C₂-C₈ alkenyl, or C₂-C₈ alkynyl, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted R₆ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
A₁₃ and A₁₄ are each independently C₁, C₂, C₃, C₄, C₅, C₆, C₇, or C₈ hydrocarbylene, or C₁, C₂, C₃, C₄, C₅, C₆, C₇, or C₈ heterohydrocarbylene, or a bond.

In some embodiments, A₁₃ and A₁₄ are each independently a bond.

In some embodiments, the present disclosure provides the amino lipid compound of formula (II-I), or a pharmaceutically acceptable salt thereof, or a stereoisomer thereof, as described above, wherein:
A₁₃ and A₁₄ are each independently C₁-C₈ alkylene, or C₂-C₈ alkenylene with 1, 2, or more double bonds, or C₂-C₈ alkynylene with 1, 2, or more triple bonds.

In some embodiments, the present disclosure provides the amino lipid compound of formula (II-I), or a pharmaceutically acceptable salt thereof, or a stereoisomer thereof, as described above, wherein:
each A₁₃ and each A₁₄ are independently C₁-C₈ alkylene, C₂-C₈ alkenylene, or C₂-C₈ alkynylene.

In some embodiments, the present disclosure provides the amino lipid compound of formula (II-I), or a pharmaceutically acceptable salt thereof, or a stereoisomer thereof, as described above, wherein:
each A₁₃ and/or each A₁₄ is unsubstituted C₁-C₅ alkylene, or unsubstituted C₂-C₈ alkenylene, or unsubstituted C₂-C₈ alkynylene. For example, A₁₃ and A₁₄ are each independently unsubstituted C₂-C₅ alkylene, or unsubstituted C₃-C₄ alkylene.

In some embodiments, the present disclosure provides the amino lipid compound of formula (II-I), or a pharmaceutically acceptable salt thereof, or a stereoisomer thereof, as described above, wherein:
at least one of the A₁₃ groups and/or the A₁₄ groups is substituted C₁-C₅ alkylene, or substituted C₂-C₈ alkenylene, or substituted C₂-C₈ alkynylene.

In some embodiments, the present disclosure provides the amino lipid compound of formula (II-I), or a pharmaceutically acceptable salt thereof, or a stereoisomer thereof, as described above, wherein:
at least one of the A₁₃ groups and/or the A₁₄ groups is C₁-C₅ alkylene, C₂-C₈ alkenylene, or C₂-C₈ alkynylene, which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein: when A₁₃ and/or A₁₄ is C₁-C₅ alkylene, C₂-C₈ alkenylene, or C₂-C₈ alkynylene, which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted A₁₃ or A₁₄ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein:
R₉ and R₁₁ are each independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heterohydrocarbyl.

In some embodiments, the present disclosure provides the amino lipid compound of formula (II-I), or a pharmaceutically acceptable salt thereof, or a stereoisomer thereof, as described above, wherein:
each R₉ is independently C₁-C₁₂ alkyl (e.g., C₃-C₉ alkyl, C₅-C₈ alkyl, C₆ alkyl, C₇ alkyl), C₅-C₁₆ alkenyl (e.g., C₅-C₁₂ alkenyl, C₅-C₈ alkenyl, C₆ alkenyl), or C₅-C₁₆ alkynyl (e.g., C₉-C₁₁ alkynyl, C₁₀ alkynyl).

In some embodiments, the present disclosure provides the amino lipid compound of formula (II-I), or a pharmaceutically acceptable salt thereof, or a stereoisomer thereof, as described above, wherein:
each R₉ is unsubstituted C₁-C₁₂ alkyl (e.g., unsubstituted C₃-C₉ alkyl, unsubstituted C₅-C₈ alkyl, unsubstituted C₆ alkyl, unsubstituted C₇ alkyl), unsubstituted C₅-C₁₆ alkenyl (e.g., unsubstituted C₅-C₁₂ alkenyl, unsubstituted C₅-C₈ alkenyl, unsubstituted C₆ alkenyl), or unsubstituted C₅-C₁₆ alkynyl (e.g., unsubstituted C₉-C₁₁ alkynyl, unsubstituted C₁₀ alkynyl).

In some embodiments, the present disclosure provides the amino lipid compound of formula (II-I), or a pharmaceutically acceptable salt thereof, or a stereoisomer thereof, as described above, wherein:
at least one of the R₉ groups is substituted C₁-C₁₂ alkyl (e.g., substituted C₃-C₉ alkyl, substituted C₅-C₈ alkyl, substituted C₆ alkyl, substituted C₇ alkyl), substituted C₅-C₁₆ alkenyl (e.g., substituted C₅-C₁₂ alkenyl, substituted C₅-C₈ alkenyl, substituted C₆ alkenyl), or substituted C₅-C₁₆ alkynyl (e.g., substituted C₉-C₁₁ alkynyl, substituted C₁₀ alkynyl).

In some embodiments, the present disclosure provides the amino lipid compound of formula (II-I), or a pharmaceutically acceptable salt thereof, or a stereoisomer thereof, as described above, wherein:
each R₁₁ is independently C₁-C₁₂ alkyl (e.g., C₃-C₉ alkyl, C₅-C₈ alkyl, C₆ alkyl, C₇ alkyl), C₅-C₁₆ alkenyl (e.g., C₅-C₁₂ alkenyl, C₅-C₈ alkenyl, C₆ alkenyl), or C₅-C₁₆ alkynyl (e.g., C₉-C₁₁ alkynyl, C₁₀ alkynyl).

In some embodiments, the present disclosure provides the amino lipid compound of formula (II-I), or a pharmaceutically acceptable salt thereof, or a stereoisomer thereof, as described above, wherein:
each R₁₁ is unsubstituted C₁-C₁₂ alkyl (e.g., unsubstituted C₃-C₉ alkyl, unsubstituted C₅-C₈ alkyl, unsubstituted C₆ alkyl, unsubstituted C₇ alkyl), unsubstituted C₅-C₁₆ alkenyl (e.g., unsubstituted C₅-C₁₂ alkenyl, unsubstituted C₅-C₈ alkenyl, unsubstituted C₆ alkenyl), or unsubstituted C₅-C₁₆ alkynyl (e.g., unsubstituted C₉-C₁₁ alkynyl, unsubstituted C₁₀ alkynyl).

In some embodiments, the present disclosure provides the amino lipid compound of formula (II-I), or a pharmaceutically acceptable salt thereof, or a stereoisomer thereof, as described above, wherein:
at least one of the R₁₁ groups is substituted C₁-C₁₂ alkyl (e.g., substituted C₃-C₉ alkyl, substituted C₅-C₈ alkyl, substituted C₆ alkyl, substituted C₇ alkyl), substituted C₅-C₁₆ alkenyl (e.g., substituted C₅-C₁₂ alkenyl, substituted C₅-C₈ alkenyl, substituted C₆ alkenyl), substituted C₅-C₁₆ alkynyl (e.g., substituted C₉-C₁₁ alkynyl, substituted C₁₀ alkynyl).

In some embodiments, the present disclosure provides the amino lipid compound of formula (II-I), or a pharmaceutically acceptable salt thereof, or a stereoisomer thereof, as described above, wherein:
R₉ and R₁₁ are each independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₂₄ heteroalkyl containing O or S, or a straight C₂-C₂₄ heteroalkenyl containing O or S with 1, 2, 3, 4, or more double bonds, or a straight C₂-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₉ and R₁₁ are each independently a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl; or a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl.

In some embodiments, the present disclosure provides the amino lipid compound of formula (II-I), or a pharmaceutically acceptable salt thereof, or a stereoisomer thereof, as described above, wherein:
R₉ and R₁₁ are each independently a branched C₃-C₂₄ alkyl, or a branched C₃-C₂₄ alkenyl with 1, 2, 3, 4, or more double bonds, or a branched C₄-C₂₄ alkynyl with 1, 2, 3, 4, or more triple bonds, or a branched C₃-C₂₄ heteroalkyl containing O or S, or a branched C₃-C₂₄ heteroalkenyl containing O or S with 1, 2, 3, 4, or more double bonds, or a branched C₄-C₂₄ heteroalkynyl containing O or S with 1, 2, 3, 4, or more triple bonds.

In some embodiments, the present disclosure provides the amino lipid compound of formula (II-I), or a pharmaceutically acceptable salt thereof, or a stereoisomer thereof, as described above, wherein:
R₉ and R₁₁ are each independently a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl containing O or S, or a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl containing O or S, or a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl containing O or S.

In some embodiments, the present disclosure provides the amino lipid compound of formula (II-I), or a pharmaceutically acceptable salt thereof, or a stereoisomer thereof, as described above, wherein:
at least one of the R₉ groups and/or the R₁₁ is groups C₁-C₁₂ alkyl (C₃-C₉ alkyl, C₅-C₈ alkyl, C₆ alkyl, C₇ alkyl), C₅-C₁₆ alkenyl (e.g., C₅-C₁₂ alkenyl, C₅-C₈ alkenyl, C₆ alkenyl), substituted C₅-C₁₆ alkynyl (e.g., C₉-C₁₁ alkynyl, C₁₀ alkynyl), which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when R₉ and/or R₁₁ is C₁-C₁₂ alkyl (C₃-C₉ alkyl, C₅-C₈ alkyl, C₆ alkyl, C₇ alkyl), C₅-C₁₆ alkenyl (e.g., C₅-C₁₂ alkenyl, C₅-C₈ alkenyl, C₆ alkenyl), or substituted C₅-C₁₆ alkynyl (e.g., C₉-C₁₁ alkynyl, C₁₀ alkynyl), each of which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted R₉ or R₁₁ to form a three-, four-, five-, or six-membered ring.

In some embodiments, the present disclosure provides the amino lipid compound of formula (II-I), or a pharmaceutically acceptable salt thereof, or a stereoisomer thereof, as described above, wherein at least one of the R₉ groups and/or the R₁₁ groups is independently substituted aryl. In some embodiments, R₉ and R₁₁ are phenyl substituted with alkyl, such as

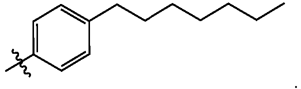

In some embodiments, the present disclosure provides the amino lipid compound of formula (II-I), or a pharmaceutically acceptable salt thereof, or a stereoisomer thereof, as described above, wherein:
R₉ and R₁₁ are each independently 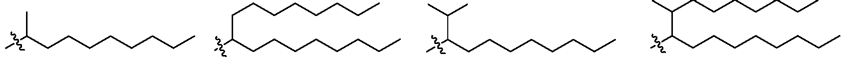 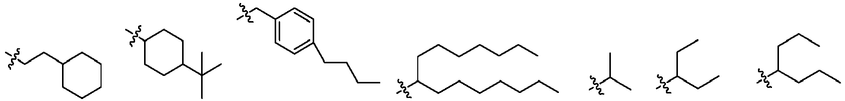 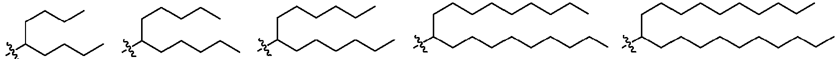 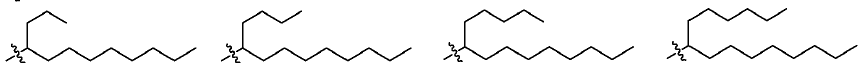 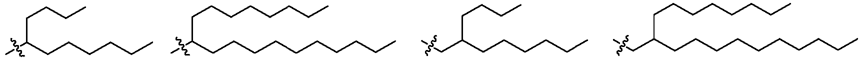 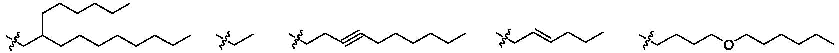 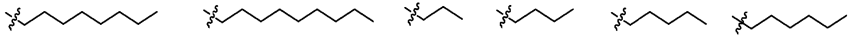 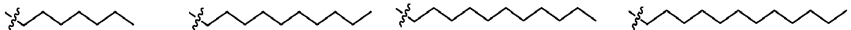 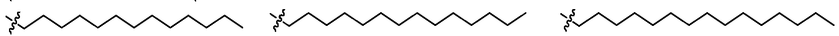 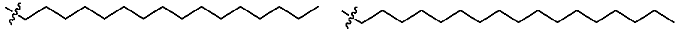 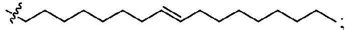
preferably

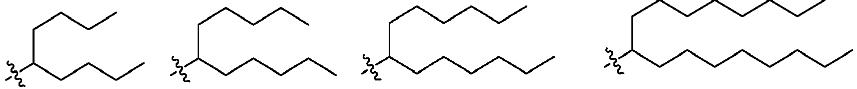

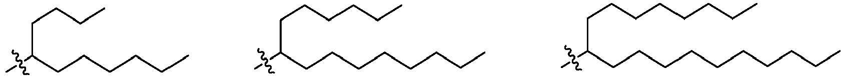

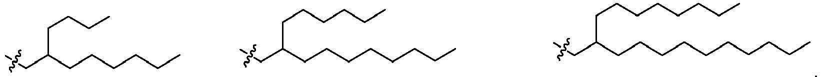

In some embodiments, the present disclosure provides the amino lipid compound of formula (II-I), or a pharmaceutically acceptable salt thereof, or a stereoisomer thereof, as described above, wherein:
each Z₈ is independently a bond.

In some embodiments, the present disclosure provides the amino lipid compound of formula (II-I), or a pharmaceutically acceptable salt thereof, or a stereoisomer thereof, as described above, wherein:
each Z₉ is independently a bond.

In some embodiments, the present disclosure provides the amino lipid compound of formula (II-I), or a pharmaceutically acceptable salt thereof, or a stereoisomer thereof, as described above, wherein:
R₁ is H, C₁-C₂₄ hydrocarbyl, C₁-C₂₄ heterohydrocarbyl, Z₆R₈, or -CH(OR₉)₂.

In some embodiments, R₁ is -CH(OR₉)₂.

In some embodiments, the present disclosure provides the amino lipid compound of formula (II-I), or a pharmaceutically acceptable salt thereof, or a stereoisomer thereof, as described above, wherein:
R₂ is H, C₁-C₂₄ hydrocarbyl, C₁-C₂₄ heterohydrocarbyl, Z₇R₁₀, or -CH(OR₁₁)₂.

In some embodiments, R₂ is -CH(OR₁₁)₂.

In some embodiments, the present disclosure provides the amino lipid compound of formula (II-I), or a pharmaceutically acceptable salt thereof, or a stereoisomer thereof, as described above, wherein:
R₁ and/or R₂ is C₁-C₂₄ alkyl (e.g., C₅-C₁₆ alkyl, C₆-C₁₄ alkyl, C₆-C₁₂ alkyl), C₂-C₂₄ alkenyl (e.g., C₅-C₁₆ alkenyl, C₆-C₁₄ alkenyl, C₆-C₁₂ alkenyl), or C₂-C₂₄ alkynyl (e.g., C₅-C₁₆ alkynyl, C₆-C₁₄ alkynyl, C₆-C₁₂ alkynyl, C₁₀ alkynyl), which is substituted with hydroxyl, halogen, =O, =S, cyano, cyclohydrocarbyl, aryl, heterocycle, -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄,
wherein,
each R₁₃ is independently C₁-C₁₂ alkylene, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds;
each R₁₄ is independently H, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-I), or a pharmaceutically acceptable salt thereof, or a stereoisomer thereof, as described above, wherein:
when R₁ and/or R₂ is each independently C₁-C₂₄ alkyl (e.g., C₅-C₁₆ alkyl, C₆-C₁₄ alkyl, C₆-C₁₂ alkyl), C₂-C₂₄ alkenyl (e.g., C₅-C₁₆ alkenyl, C₆-C₁₄ alkenyl, C₆-C₁₂ alkenyl), or C₂-C₂₄ alkynyl (e.g., C₅-C₁₆ alkynyl, C₆-C₁₄ alkynyl, C₆-C₁₂ alkynyl, C₁₀ alkynyl), which is substituted with -R₁₄, -R₁₃OR₁₄, -R₁₃SR₁₄, -R₁₃OC(=O)OR₁₄, -R₁₃OC(=O)SR₁₄, -R₁₃C(=O)N(R₁₄)₂, -R₁₃OC(=O)R₁₄, -R₁₃SC(=O)OR₁₄, -R₁₃N(R₁₄)C(=O)R₁₄, or -R₁₃C(=O)OR₁₄, R₁₃ and/or R₁₄ may be attached to the C atom on the substituted R₁ or R₂ to form a three-, four-, five-, or six-membered ring.

In some embodiments, each R₁₄ may also be independently substituted aryl, substituted cyclohydrocarbyl, or substituted heterocyclyl. In some embodiments, R₁₄ is aryl substituted with alkyl. In some embodiments, R₁₄ is phenyl substituted with alkyl, such as

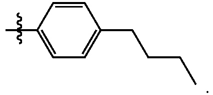

In some embodiments, R₁₄ is unsubstituted cycloalkyl, such as cyclobutyl, cyclopentyl, or cyclohexyl.

In some embodiments, the present disclosure provides the amino lipid compound of formula (II-I), or a pharmaceutically acceptable salt thereof, or a stereoisomer thereof, as described above, wherein:
R₁ and R₂ are each independently C₅-C₁₆ alkyl substituted with hydroxyl, such as C₆-C₁₄ alkyl substituted with hydroxyl, C₆-C₁₂ alkyl substituted with hydroxyl, C₁₀ alkyl substituted with hydroxy. In some embodiments, R₁ and R₂ are each independently C₁₀ alkyl, such as

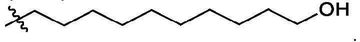

In some embodiments, the present disclosure provides the amino lipid compound of formula (II-I), or a pharmaceutically acceptable salt thereof, or a stereoisomer thereof, as described above, wherein:
R₁ and R₂ are each independently 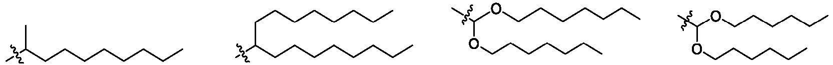 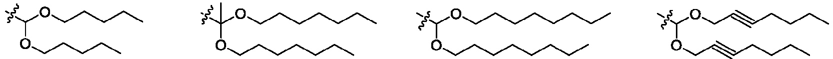 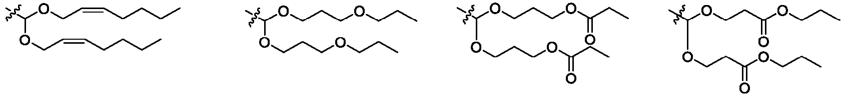 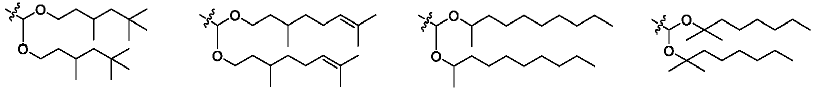 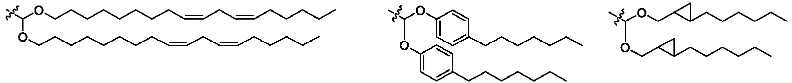 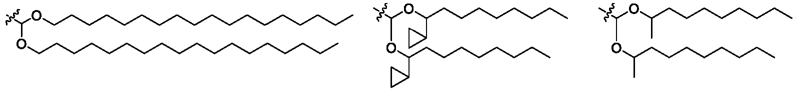 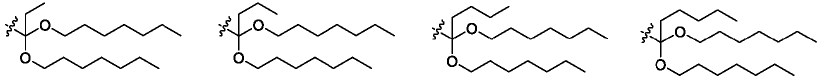 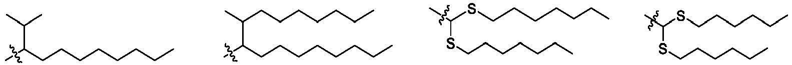 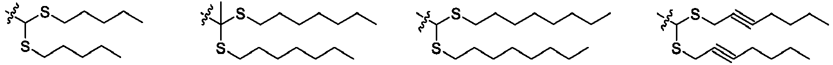 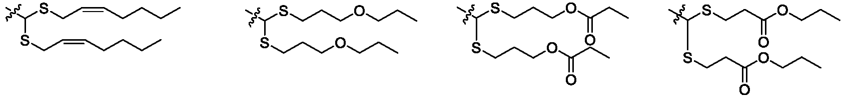 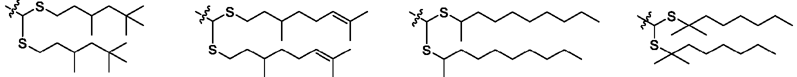 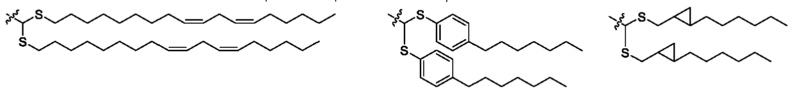 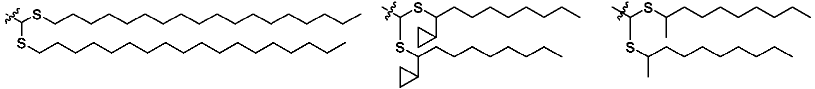 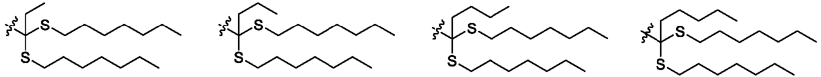 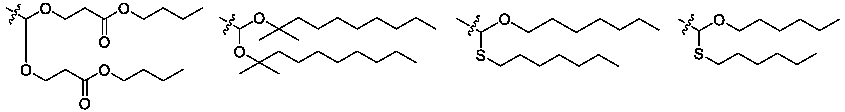 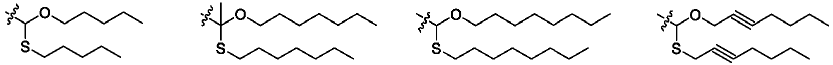 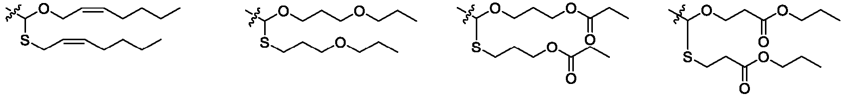 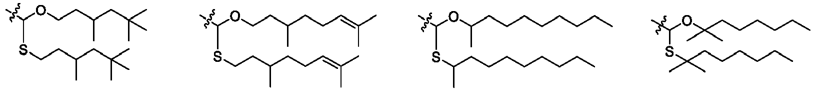 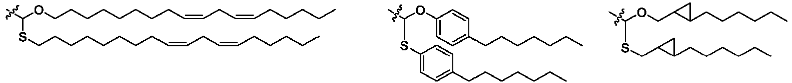 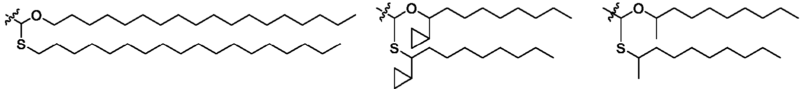 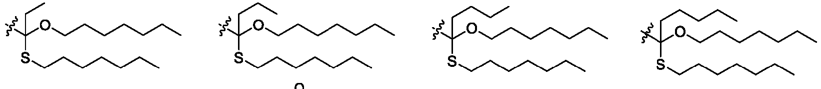 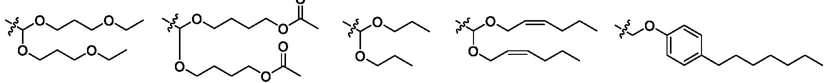 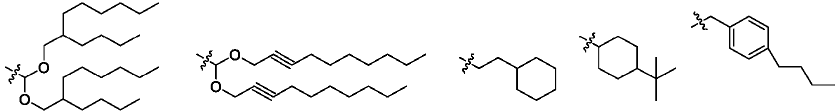 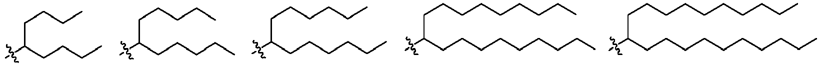 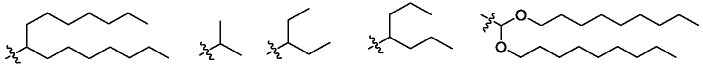 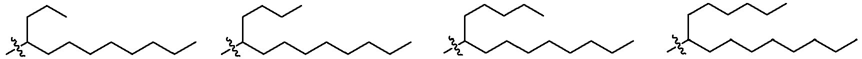 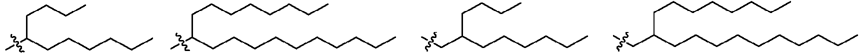 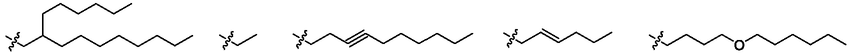 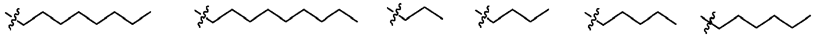 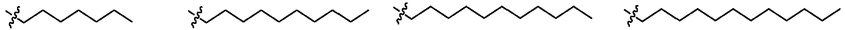 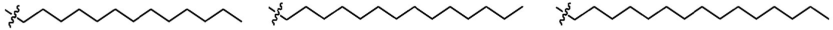 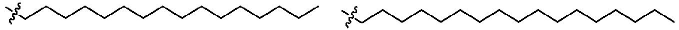 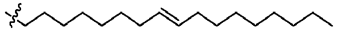 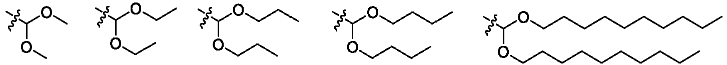 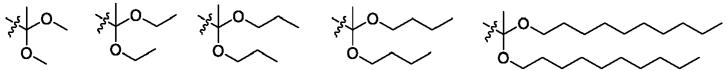 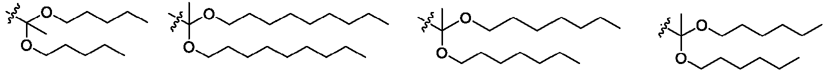 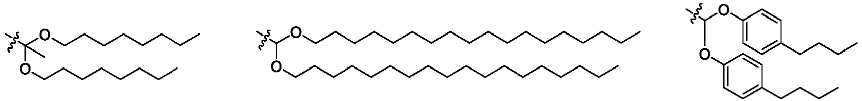 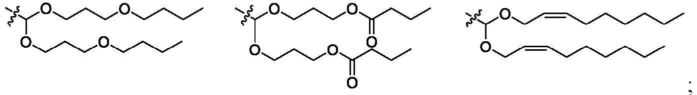
preferably

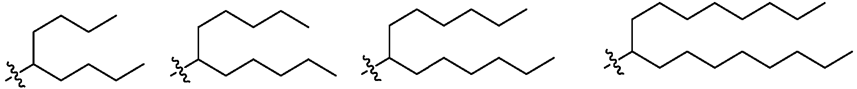

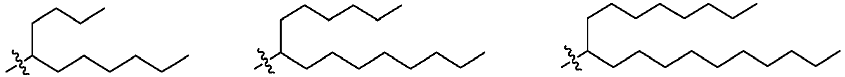

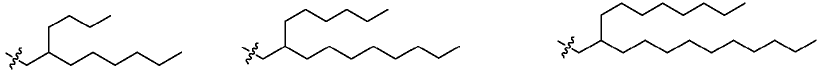

In some embodiments, the present disclosure provides the amino lipid compound of formula (II-I), or a pharmaceutically acceptable salt thereof, or a stereoisomer thereof, as described above, wherein R₁ is Z₆R₈ or -CH(OR₉)₂, and R₂ is Z₇R₁₀ or -CH(OR₁₁)₂.

In some embodiments, the amino lipid compound represented by formula (II-I), or a pharmaceutically acceptable salt thereof, or a stereoisomer thereof, has a structure represented by formula (II-Ia): 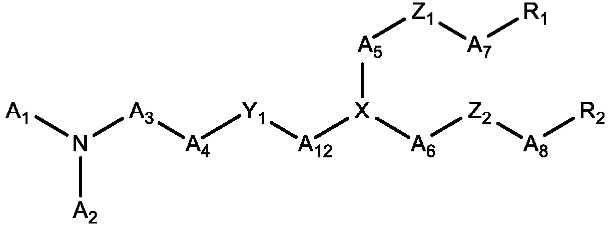 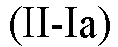 wherein,
A₁ is H, C₁-C₅ hydrocarbyl, or C₁-C₅ heterohydrocarbyl;
A₂ is H, C₁-C₅ hydrocarbyl, or C₁-C₅ heterohydrocarbyl;
A₃ is C₁-C₅ hydrocarbylene or a bond;
A₄, A₅, A₆, A₇, A₈, Z₁, Z₂, Y₁, X, R₁, R₂, and A₁₂ are as defined for formula (II-I).

The present disclosure provides the amino lipid compound of formula (II-Ia), or a pharmaceutically acceptable salt thereof, or a stereoisomer thereof, as described above, including one or more of the following features where applicable.

In some embodiments, the present disclosure provides the amino lipid compound of formula (II-Ia), or a pharmaceutically acceptable salt thereof, or a stereoisomer thereof, as described above, wherein:
A₁ and A₂ are each independently H.

In some embodiments, the present disclosure provides the amino lipid compound of formula (II-Ia), or a pharmaceutically acceptable salt thereof, or a stereoisomer thereof, as described above, wherein:
A₁ and A₂ are each independently C₁, C₂, C₃, C₄, or C₅ hydrocarbyl, or C₁, C₂, C₃, C₄, or C₅ heterohydrocarbyl.

In some embodiments, the present disclosure provides the amino lipid compound of formula (II-Ia), or a pharmaceutically acceptable salt thereof, or a stereoisomer thereof, as described above, wherein:
A₁ and A₂ are each independently C₁-C₅ alkyl, or C₂-C₅ alkenyl with 1 or 2 double bonds, or C₂-C₅ alkynyl with 1 or 2 triple bonds, or C₁-C₅ heteroalkyl containing S or O, or C₂-C₅ heteroalkenyl containing S or O with 1 or 2 double bonds, or C₂-C₅ heteroalkynyl containing S or O with 1 or 2 triple bonds.

In some embodiments, the present disclosure provides the amino lipid compound of formula (II-Ia), or a pharmaceutically acceptable salt thereof, or a stereoisomer thereof, as described above, wherein:
A₁ and A₂ are each independently optionally substituted with hydroxyl, cyano, phenyl, C₃-C₇ cyclohydrocarbyl, C₃-C₇ cyclohydrocarbyl containing 1-3 heteroatoms independently selected from nitrogen, oxygen, or sulfur, 5- to 6-membered monocyclic heteroaryl containing 1-4 heteroatoms independently selected from nitrogen, oxygen, and sulfur, 8- to 10-membered bicyclic heteroaryl containing 1-4 heteroatoms independently selected from nitrogen, oxygen, or sulfur, -OR₁₂, -C(=O)R₁₂, -C(=O)SR₁₂, -OC(=O)R₁₂, -OC(=O)OR₁₂, -N(R₁₂)₂, -C(=O)N(R₁₂)₂, C(=S)N(R₁₂)₂, -S(=O)₂R₁₂, -S(=O)₂N(R₁₂)₂, -OC(=O)N(R₁₂)₂, -CH(NR₁₂)N(R₁₂)₂, -C(NR₁₂)₂R₁₂, -C(=O)N(R₁₂)OR₁₂, -CH(R₁₂)N(R₁₂)₂C(=O)OR₁₂, -C(R₁₂)₃, -N(R₁₂)C(=O)R₁₂, -N(R₁₂)C(=O)OR₁₂, -N(R₁₂)S(=O)₂R₁₂, -N(R₁₂)C(=O)N(R₁₂)₂, -N(R₁₂)C(=S)N(R₁₂)₂, -N(R₁₂)C(NR₁₂)N(R₁₂)₂, -N(R₁₂)C(CHR₁₂)N(R₁₂)₂, -N(OR₁₂)C(=O)R₁₂, -N(OR₁₂)S(=O)₂R₁₂, -N(OR₁₂)C(=O)OR₁₂, -N(OR₁₂)C(=O)N(R₁₂)₂, -N(OR₁₂)C(=S)N(R₁₂)₂, -N(OR₁₂)C(NR₁₂)N(R₁₂)₂, -N(OR₁₂)C(CHR₁₂)N(R₁₂)₂, -OP(=O)(OR₁₂)₂, -P(=O)(OR₁₂)₂, or a 3- to 7-membered cycloaliphatic ring and a 3- to 7-membered heterocyclyl ring each containing 1-3 heteroatoms independently selected from nitrogen, oxygen, sulfur, -C(=N-CN)N(R₁₂)₂, -C(=N-O-CH₃)N(R₁₂)₂, -C(=N-SO₂-NH₂)N(R₁₂)₂, -C(=CH-NO₂)N(R₁₂)₂, or -C(=O)OR₁₂;
wherein each R₁₂ is independently hydrogen, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-Ia), or a pharmaceutically acceptable salt thereof, or a stereoisomer thereof, as described above, wherein:
A₁ and A₂ are each independently optionally substituted with C₃-C₇ cyclohydrocarbyl containing 1-3 heteroatoms independently selected from nitrogen, oxygen, or sulfur, optionally substituted with C₁-C₅ alkyl, =O, -C₀-C₅ alkylene-hydroxyl, -C(=O)N(R₁₅)₂; 8- to 10-membered bicyclic heteroaryl containing 1-4 heteroatoms independently selected from nitrogen, oxygen, or sulfur, optionally substituted with C₁-C₅ alkyl, hydroxyl; 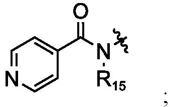 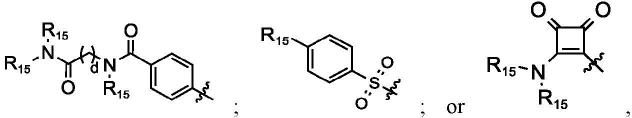 wherein each R₁₅ is independently H or C₁-C₃ alkyl, and d is 0, 1, 2, 3, 4, or 5.

In some embodiments, the present disclosure provides the amino lipid compound of formula (II-Ia), or a pharmaceutically acceptable salt thereof, or a stereoisomer thereof, as described above, wherein:
A₁ and A₂ are each independently

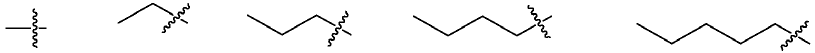

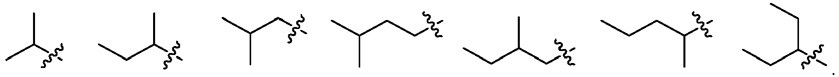

In some embodiments, the present disclosure provides the amino lipid compound of formula (II-Ia), or a pharmaceutically acceptable salt thereof, or a stereoisomer thereof, as described above, wherein:
A₁ and A₂ may also each independently be 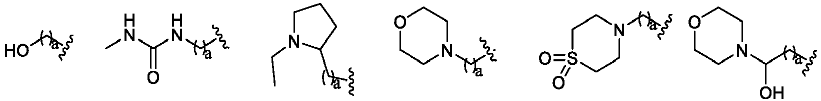 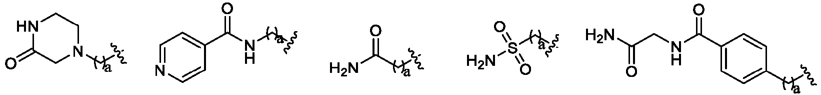 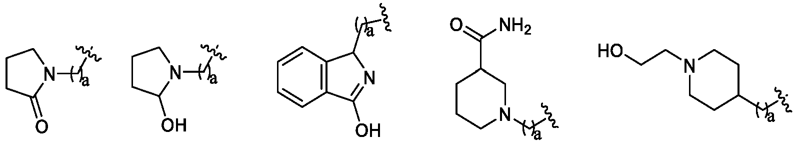 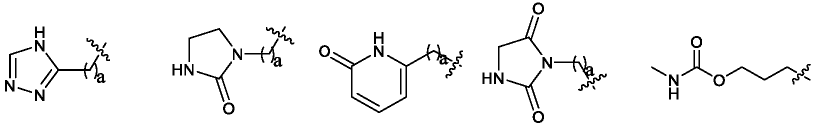 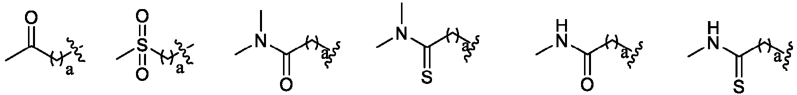 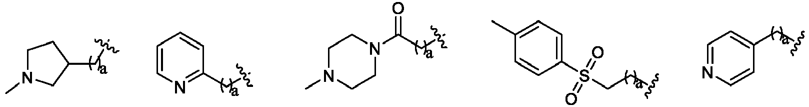 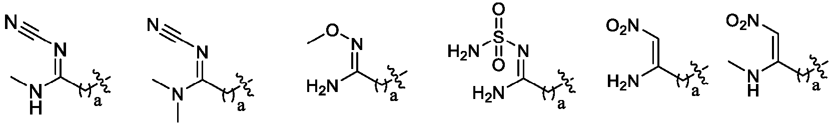 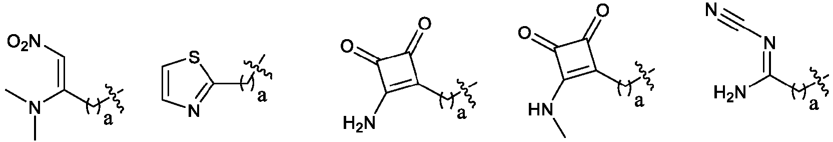
wherein a is 1, 2, 3, 4, or 5;
preferably, the a is 1, 2, or 3.

In some embodiments, the present disclosure provides the amino lipid compound of formula (II-Ia), or a pharmaceutically acceptable salt thereof, or a stereoisomer thereof, as described above, wherein:
A₃ is a bond.

In some embodiments, the present disclosure provides the amino lipid compound of formula (II-Ia), or a pharmaceutically acceptable salt thereof, or a stereoisomer thereof, as described above, wherein:
A₃ is C₁, C₂, C₃, C₄, or C₅ hydrocarbylene.

In some embodiments, the present disclosure provides the amino lipid compound of formula (II-Ia), or a pharmaceutically acceptable salt thereof, or a stereoisomer thereof, as described above, wherein:
A₃ is C₁-C₅ alkylene, or C₂-C₅ alkenylene with 1 or 2 double bonds, or C₂-C₅ alkynylene with 1 or 2 triple bonds.

In some embodiments, the present disclosure provides the amino lipid compound of formula (II-Ia), or a pharmaceutically acceptable salt thereof, or a stereoisomer thereof, as described above, wherein: 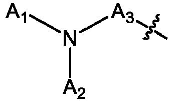 is 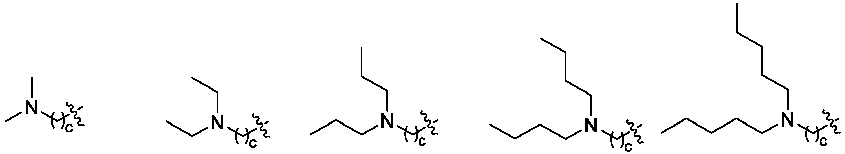 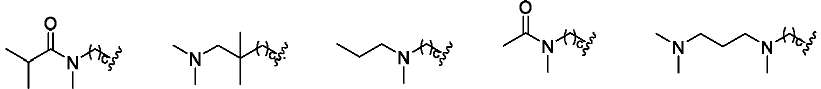 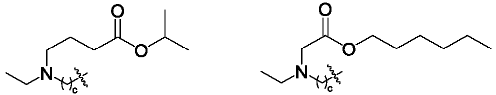
wherein c is 1, 2, 3, 4, or 5;
preferably, the c is 2, 3, or 4;
preferably, the c is 2 or 3.

In some embodiments, the amino lipid compound represented by formula (II-I), or a pharmaceutically acceptable salt thereof, or a stereoisomer thereof, has a structure represented by formula (II-Ib): 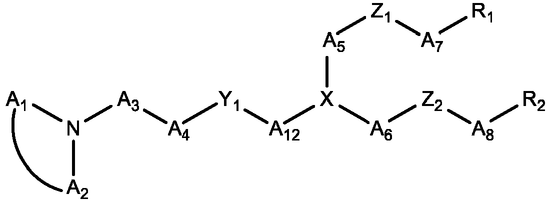 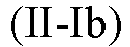 wherein,
A₁ is C₁-C₅ hydrocarbylene or C₁-C₅ heterohydrocarbylene;
A₂ is C₁-C₅ hydrocarbylene or C₁-C₅ heterohydrocarbylene;
A₃ is C₁-C₅ hydrocarbylene or a bond;
A₁ and A₂, together with the nitrogen atom to which they are both attached, form an N-containing heterocyclic ring;
A₄, A₅, A₆, A₇, A₈, Z₁, Z₂, Y₁, X, R₁, R₂, and A₁₂ are as defined for formula (II-I).

The present disclosure provides the amino lipid compound of formula (II-Ib), or a pharmaceutically acceptable salt thereof, or a stereoisomer thereof, as described above, including one or more of the following features where applicable.

In some embodiments, the present disclosure provides the amino lipid compound of formula (II-Ib), or a pharmaceutically acceptable salt thereof, or a stereoisomer thereof, as described above, wherein:
A₁ and A₂ are each independently C₁, C₂, C₃, C₄, or C₅ hydrocarbylene, or C₁, C₂, C₃, C₄, or C₅ heterohydrocarbylene.

In some embodiments, the present disclosure provides the amino lipid compound of formula (II-Ib), or a pharmaceutically acceptable salt thereof, or a stereoisomer thereof, as described above, wherein:
A₁ and A₂ are each independently C₁-C₅ alkylene, or C₂-C₅ alkenylene with 1 or 2 double bonds, or C₂-C₅ alkynylene with 1 or 2 triple bonds, or C₁-C₅ heteroalkylene containing S or O, or C₂-C₅ heteroalkenylene containing S or O with 1 or 2 double bonds, or C₂-C₅ heteroalkynylene containing S or O with 1 or 2 triple bonds.

In some embodiments, the present disclosure provides the amino lipid compound of formula (II-Ib), or a pharmaceutically acceptable salt thereof, or a stereoisomer thereof, as described above, wherein:
A₁ and A₂ are each independently optionally substituted with hydroxyl, cyano, phenyl, C₃-C₇ cyclohydrocarbyl, C₃-C₇ cyclohydrocarbyl containing 1-3 heteroatoms independently selected from nitrogen, oxygen, or sulfur, 5- to 6-membered monocyclic heteroaryl containing 1-4 heteroatoms independently selected from nitrogen, oxygen, and sulfur, 8- to 10-membered bicyclic heteroaryl containing 1-4 heteroatoms independently selected from nitrogen, oxygen, or sulfur, -OR₁₂, -C(=O)R₁₂, -C(=O)SR₁₂, -OC(=O)R₁₂, -OC(=O)OR₁₂, -N(R₁₂)₂, -C(=O)N(R₁₂)₂, C(=S)N(R₁₂)₂, -S(=O)₂R₁₂, -S(=O)₂N(R₁₂)₂, -OC(=O)N(R₁₂)₂, -CH(NR₁₂)N(R₁₂)₂, -C(NR₁₂)₂R₁₂, -C(=O)N(R₁₂)OR₁₂, -CH(R₁₂)N(R₁₂)₂C(=O)OR₁₂, -C(R₁₂)₃, -N(R₁₂)C(=O)R₁₂, -N(R₁₂)C(=O)OR₁₂, -N(R₁₂)S(=O)₂R₁₂, -N(R₁₂)C(=O)N(R₁₂)₂, -N(R₁₂)C(=S)N(R₁₂)₂, -N(R₁₂)C(NR₁₂)N(R₁₂)₂, -N(R₁₂)C(CHR₁₂)N(R₁₂)₂, -N(OR₁₂)C(=O)R₁₂, -N(OR₁₂)S(=O)₂R₁₂, -N(OR₁₂)C(=O)OR₁₂, -N(OR₁₂)C(=O)N(R₁₂)₂, -N(OR₁₂)C(=S)N(R₁₂)₂, -N(OR₁₂)C(NR₁₂)N(R₁₂)₂, -N(OR₁₂)C(CHR₁₂)N(R₁₂)₂, -OP(=O)(OR₁₂)₂, -P(=O)(OR₁₂)₂, or a 3- to 7-membered cycloaliphatic ring and a 3- to 7-membered heterocyclyl ring each containing 1-3 heteroatoms independently selected from nitrogen, oxygen, and sulfur, -C(=N-CN)N(R₁₂)₂, -C(=N-O-CH₃)N(R₁₂)₂, -C(=N-SO₂-NH₂)N(R₁₂)₂, -C(=CH-NO₂)N(R₁₂)₂, or -C(=O)OR₁₂;
wherein each R₁₂ is independently hydrogen, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-Ib), or a pharmaceutically acceptable salt thereof, or a stereoisomer thereof, as described above, wherein:
A₃ is a bond.

In some embodiments, the present disclosure provides the amino lipid compound of formula (II-Ib), or a pharmaceutically acceptable salt thereof, or a stereoisomer thereof, as described above, wherein:
A₃ is C₁, C₂, C₃, C₄, or C₅ hydrocarbylene.

In some embodiments, the present disclosure provides the amino lipid compound of formula (II-Ib), or a pharmaceutically acceptable salt thereof, or a stereoisomer thereof, as described above, wherein:
A₃ is C₁-C₅ alkylene, or C₂-C₅ alkenylene with 1 or 2 double bonds, or C₂-C₅ alkynylene with 1 or 2 triple bonds.

In some embodiments, the present disclosure provides the amino lipid compound of formula (II-Ib), or a pharmaceutically acceptable salt thereof, or a stereoisomer thereof, as described above, wherein:
A₁ and A₂, together with the nitrogen atom to which they are both attached, form an N-containing three-, four-, five-, six-, seven-, or eight-membered heterocyclic ring.

In some embodiments, A₁ and A₂, together with the nitrogen atom to which they are both attached, form an N-containing three-, four-, five-, or six-membered heterocyclic ring.

In some embodiments, A₁ and A₂, together with the nitrogen atom to which they are both attached, form an N-containing four-, five-, or six-membered heterocyclic ring.

In some embodiments, the present disclosure provides the amino lipid compound of formula (II-Ib), or a pharmaceutically acceptable salt thereof, or a stereoisomer thereof, as described above, wherein: 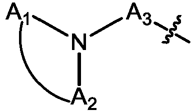 is 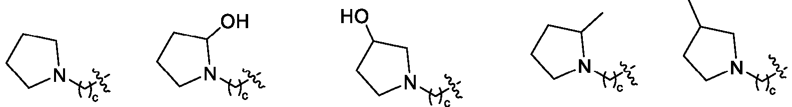 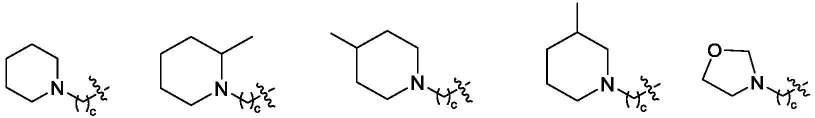 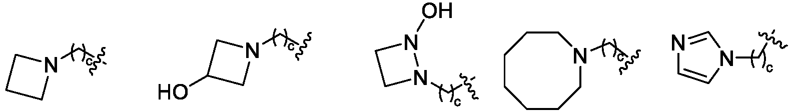 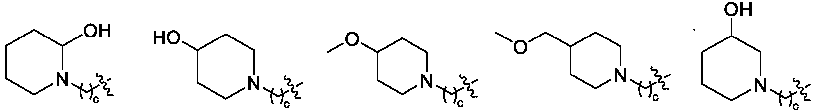 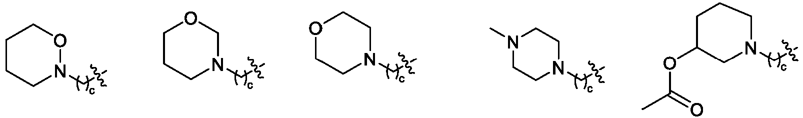 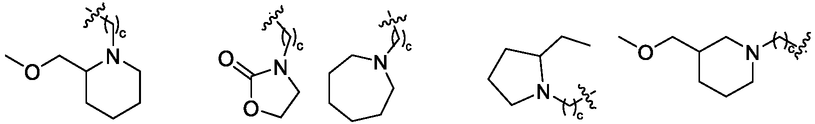 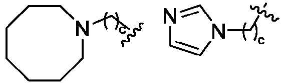
wherein c is 1, 2, 3, 4, or 5;
preferably, the c is 2, 3, or 4;
preferably, the c is 2 or 3.

In some embodiments, the amino lipid compound represented by formula (II-I), or a pharmaceutically acceptable salt thereof, or a stereoisomer thereof, has a structure represented by formula (II-Ic): 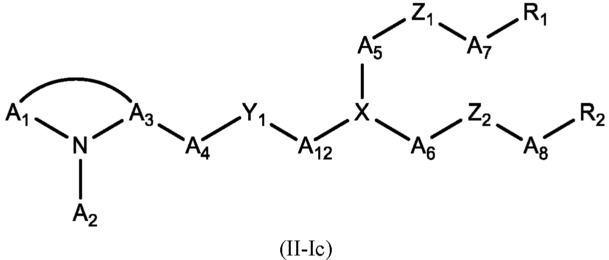 wherein,
A₁ is C₁-C₅ hydrocarbylene, or C₁-C₅ heterohydrocarbylene;
A₂ is H, C₁-C₅ hydrocarbyl, or C₁-C₅ heterohydrocarbyl;
A₃ is C₁-C₅ hydrocarbylene;
A₁ and A₃, together with the nitrogen atom to which they are both attached, form an N-containing heterocyclic ring;
A₄, A₅, A₆, A₇, A₈, Z₁, Z₂, Y₁, X, R₁, R₂, and A₁₂ are as defined for formula (II-I).

The present disclosure provides the amino lipid compound of formula (II-Ic), or a pharmaceutically acceptable salt thereof, or a stereoisomer thereof, as described above, including one or more of the following features where applicable.

In some embodiments, the present disclosure provides the amino lipid compound of formula (II-Ic), or a pharmaceutically acceptable salt thereof, or a stereoisomer thereof, as described above, wherein:
A₂ is H.

In some embodiments, the present disclosure provides the amino lipid compound of formula (II-Ic), or a pharmaceutically acceptable salt thereof, or a stereoisomer thereof, as described above, wherein:
A₁ is C₁, C₂, C₃, C₄, or C₅ hydrocarbylene, or C₁, C₂, C₃, C₄, or C₅ heterohydrocarbylene.

In some embodiments, the present disclosure provides the amino lipid compound of formula (II-Ic), or a pharmaceutically acceptable salt thereof, or a stereoisomer thereof, as described above, wherein:
A₁ is C₁-C₅ alkylene, or C₂-C₅ alkenylene with 1 or 2 double bonds, or C₂-C₅ alkynylene with 1 or 2 triple bonds, or C₁-C₅ heteroalkylene containing S or O, or C₂-C₅ heteroalkenylene containing S or O with 1 or 2 double bonds, or C₂-C₅ heteroalkynylene containing S or O with 1 or 2 triple bonds.

In some embodiments, the present disclosure provides the amino lipid compound of formula (II-Ic), or a pharmaceutically acceptable salt thereof, or a stereoisomer thereof, as described above, wherein:
A₂ is C₁, C₂, C₃, C₄, or C₅ hydrocarbyl, or C₁, C₂, C₃, C₄, or C₅ heterohydrocarbyl.

In some embodiments, the present disclosure provides the amino lipid compound of formula (II-Ic), or a pharmaceutically acceptable salt thereof, or a stereoisomer thereof, as described above, wherein:
A₂ is C₁-C₅ alkyl, or C₂-C₅ alkenyl with 1 or 2 double bonds, or C₂-C₅ alkynyl with 1 or 2 triple bonds, or C₁-C₅ heteroalkyl containing S or O, or C₂-C₅ heteroalkenyl containing S or O with 1 or 2 double bonds, or C₂-C₅ heteroalkynyl containing S or O with 1 or 2 triple bonds.

In some embodiments, the present disclosure provides the amino lipid compound of formula (II-Ic), or a pharmaceutically acceptable salt thereof, or a stereoisomer thereof, as described above, wherein:
A₁ and A₂ are each independently optionally substituted with hydroxyl, cyano, phenyl, C₃-C₇ cyclohydrocarbyl, C₃-C₇ cyclohydrocarbyl containing 1-3 heteroatoms independently selected from nitrogen, oxygen, or sulfur, 5- to 6-membered monocyclic heteroaryl containing 1-4 heteroatoms independently selected from nitrogen, oxygen, and sulfur, 8- to 10-membered bicyclic heteroaryl containing 1-4 heteroatoms independently selected from nitrogen, oxygen, or sulfur, -OR₁₂, -C(=O)R₁₂, -C(=O)SR₁₂, -OC(=O)R₁₂, -OC(=O)OR₁₂, -N(R₁₂)₂, -C(=O)N(R₁₂)₂, C(=S)N(R₁₂)₂, -S(=O)₂R₁₂, -S(=O)₂N(R₁₂)₂, -OC(=O)N(R₁₂)₂, -CH(NR₁₂)N(R₁₂)₂, -C(NR₁₂)₂R₁₂, -C(=O)N(R₁₂)OR₁₂, -CH(R₁₂)N(R₁₂)₂C(=O)OR₁₂, -C(R₁₂)₃, -N(R₁₂)C(=O)R₁₂, -N(R₁₂)C(=O)OR₁₂, -N(R₁₂)S(=O)₂R₁₂, -N(R₁₂)C(=O)N(R₁₂)₂, -N(R₁₂)C(=S)N(R₁₂)₂, -N(R₁₂)C(NR₁₂)N(R₁₂)₂, -N(R₁₂)C(CHR₁₂)N(R₁₂)₂, -N(OR₁₂)C(=O)R₁₂, -N(OR₁₂)S(=O)₂R₁₂, -N(OR₁₂)C(=O)OR₁₂, -N(OR₁₂)C(=O)N(R₁₂)₂, -N(OR₁₂)C(=S)N(R₁₂)₂, -N(OR₁₂)C(NR₁₂)N(R₁₂)₂, -N(OR₁₂)C(CHR₁₂)N(R₁₂)₂, -OP(=O)(OR₁₂)₂, -P(=O)(OR₁₂)₂, or a 3- to 7-membered cycloaliphatic ring and a 3- to 7-membered heterocyclyl ring each containing 1-3 heteroatoms independently selected from nitrogen, oxygen, and sulfur, -C(=N-CN)N(R₁₂)₂, -C(=N-O-CH₃)N(R₁₂)₂, -C(=N-SO₂-NH₂)N(R₁₂)₂, -C(=CH-NO₂)N(R₁₂)₂, or -C(=O)OR₁₂;
wherein each R₁₂ is independently hydrogen, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (II-Ic), or a pharmaceutically acceptable salt thereof, or a stereoisomer thereof, as described above, wherein:
A₁ and A₂ are each independently optionally substituted with C₃-C₇ cyclohydrocarbyl containing 1-3 heteroatoms independently selected from nitrogen, oxygen, or sulfur, optionally substituted with C₁-C₅ alkyl, =O, -C₀-C₅ alkylene-hydroxyl, -C(=O)N(R₁₅)₂; 8- to 10-membered bicyclic heteroaryl containing 1-4 heteroatoms independently selected from nitrogen, oxygen, or sulfur, optionally substituted with C₁-C₅ alkyl, hydroxyl; 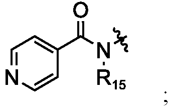 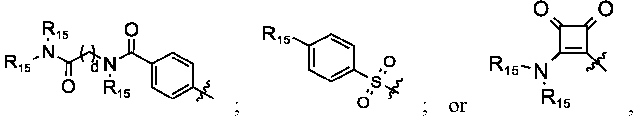 wherein each R₁₅ is independently H or C₁-C₃ alkyl, and d is 0, 1, 2, 3, 4, or 5.

In some embodiments, the present disclosure provides the amino lipid compound of formula (II-Ic), or a pharmaceutically acceptable salt thereof, or a stereoisomer thereof, as described above, wherein:
A₂ is

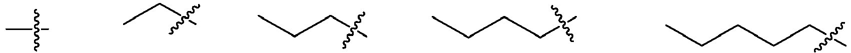

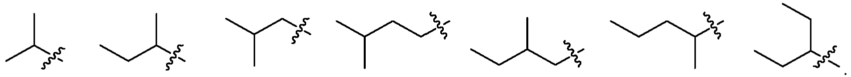

**In** some embodiments, the present disclosure provides the amino lipid compound of formula (II-Ic), or a pharmaceutically acceptable salt thereof, or a stereoisomer thereof, as described above, wherein:
A₂ may also be 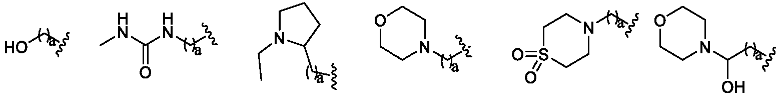 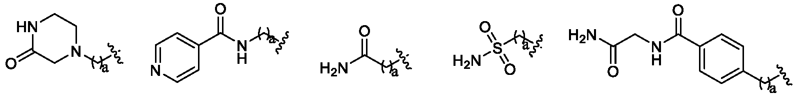 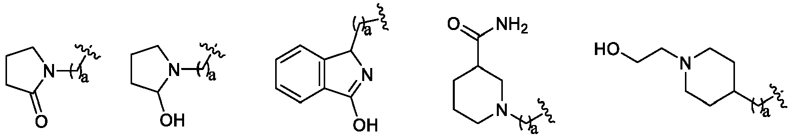 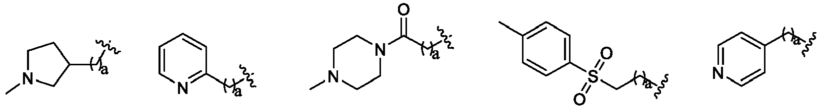 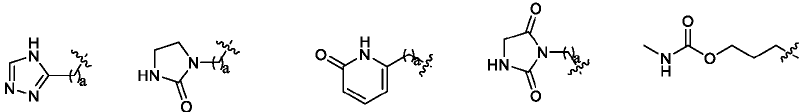 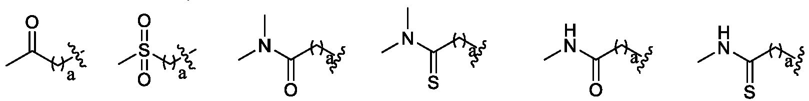 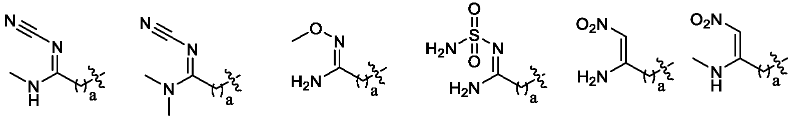 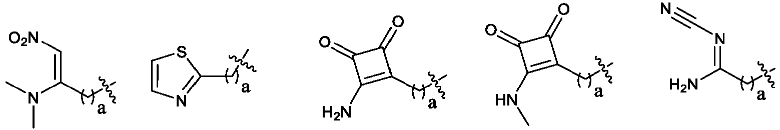
wherein a is 1, 2, 3, 4, or 5;
preferably, the a is 1, 2, or 3.

In some embodiments, the present disclosure provides the amino lipid compound of formula (II-Ic), or a pharmaceutically acceptable salt thereof, or a stereoisomer thereof, as described above, wherein:
A₃ is C₁, C₂, C₃, C₄, or C₅ hydrocarbylene.

In some embodiments, the present disclosure provides the amino lipid compound of formula (II-Ic), or a pharmaceutically acceptable salt thereof, or a stereoisomer thereof, as described above, wherein:
A₃ is C₁-C₅ alkylene, or C₂-C₅ alkenylene with 1 or 2 double bonds, or C₂-C₅ alkynylene with 1 or 2 triple bonds.

In some embodiments, the present disclosure provides the amino lipid compound of formula (II-Ic), or a pharmaceutically acceptable salt thereof, or a stereoisomer thereof, as described above, wherein:
A₁ and A₃, together with the nitrogen atom to which they are both attached, form an N-containing three-, four-, five-, six-, seven-, or eight-membered heterocyclic ring.

In some embodiments, A₁ and A₃, together with the nitrogen atom to which they are both attached, form an N-containing three-, four-, five-, or six-membered heterocyclic ring.

In some embodiments, A₁ and A₃, together with the nitrogen atom to which they are both attached, form an N-containing four-, five-, or six-membered heterocyclic ring.

In some embodiments, the present disclosure provides the amino lipid compound of formula (II-Ic), or a pharmaceutically acceptable salt thereof, or a stereoisomer thereof, as described above, wherein: 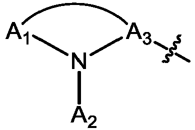 is 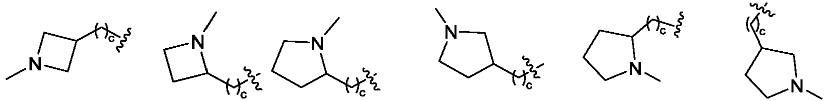 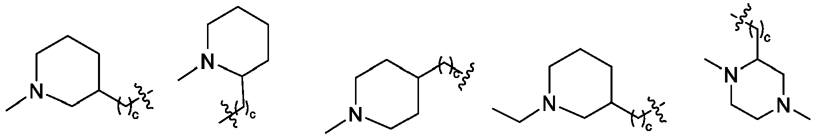 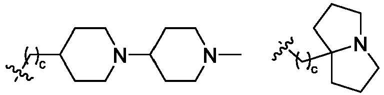
wherein c is 1, 2, 3, 4, or 5;
preferably, the c is 2, 3, or 4;
preferably, the c is 2 or 3.

In some embodiments, the amino lipid compound represented by formula (II-I), or a pharmaceutically acceptable salt thereof, or a stereoisomer thereof, has a structure represented by formula (II-Id): 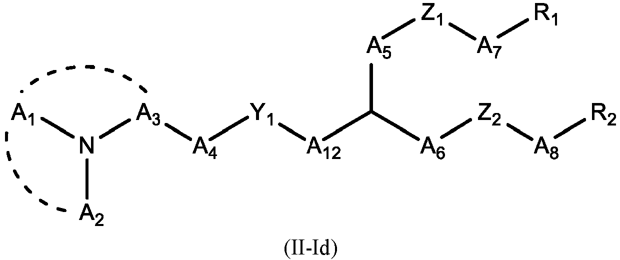 wherein A₁, A₂, A₃, A₄, A₅, A₆, A₇, A₈, Z₁, Z₂, Y₁, R₁, R₂, and A₁₂ are as defined for formula (II-I).

In some embodiments, the amino lipid compound represented by formula (II-I), or a pharmaceutically acceptable salt thereof, or a stereoisomer thereof, has a structure represented by formula (II-Ie): 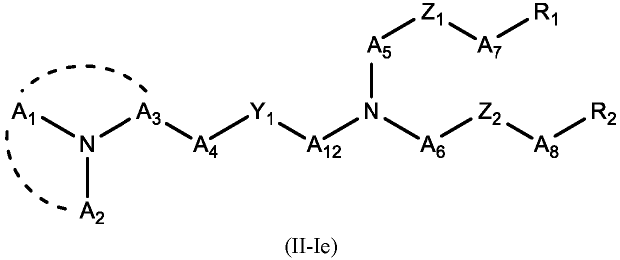 wherein A₁, A₂, A₃, A₄, A₅, A₆, A₇, A₈, Z₁, Z₂, Y₁, R₁, R₂, and A₁₂ are as defined for formula (II-I).

In some embodiments, the amino lipid compound represented by formula (II-I), or a pharmaceutically acceptable salt thereof, or a stereoisomer thereof, has a structure represented by formula (II-II): 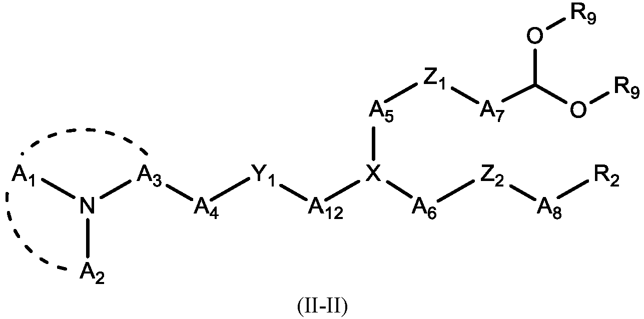 wherein A₁, A₂, A₃, A₄, A₅, A₆, A₇, A₈, Z₁, Z₂, Y₁, X, R₂, R₉, and A₁₂ are as defined for formula (II-I).

In some embodiments, the amino lipid compound represented by formula (II-I), or a pharmaceutically acceptable salt thereof, or a stereoisomer thereof, has a structure represented by formula (II-IIa): 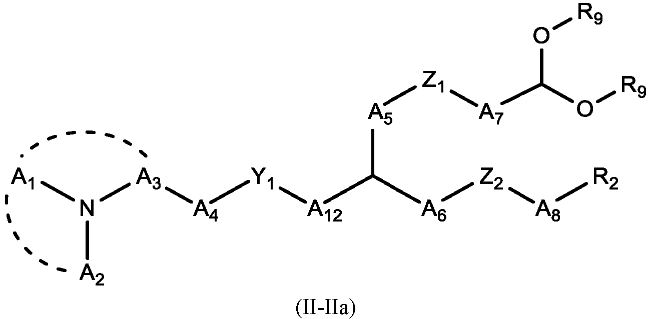 wherein A₁, A₂, A₃, A₄, A₅, A₆, A₇, A₈, Z₁, Z₂, Y₁, R₂, R₉, and A₁₂ are as defined for formula (II-I).

The present disclosure provides the amino lipid compound of formula (II-IIa), or a pharmaceutically acceptable salt thereof, or a stereoisomer thereof, as described above, including one or more of the following features where applicable.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ alkylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₃ alkenylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynylene.

In some embodiments, A₅ and A₆ are not both methylene.

In some embodiments, Z₁ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, Z₂ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, A₅ and A₆ are each independently C₁, C₂, C₃, C₄, C₅, or C₆ hydrocarbylene, or a bond.

In some embodiments, A₅ and A₆ are each independently C₁, C₂, C₃, C₄, C₅, or C₆ alkylene, or a bond.

In some embodiments, A₅ and A₆ are each independently C₂, C₃, C₄, C₅, or C₆ alkenylene, or a bond.

In some embodiments, A₅ and A₆ are each independently C₂, C₃, C₄, C₅, or C₆ alkynylene, or a bond.

In some embodiments, A₅ and A₆ are each independently C₁, or C₂ hydrocarbylene, or a bond. In some embodiments, A₅ and A₆ are each independently C₁, or C₂ alkylene, or a bond.

In some embodiments, A₅ and A₆ are each independently C₂ alkenylene, or a bond.

In some embodiments, A₅ and A₆ are each independently C₂ alkynylene, or a bond.

In some embodiments, the amino lipid compound represented by formula (II-I), or a pharmaceutically acceptable salt thereof, or a stereoisomer thereof, has a structure represented by formula (II-IIb): 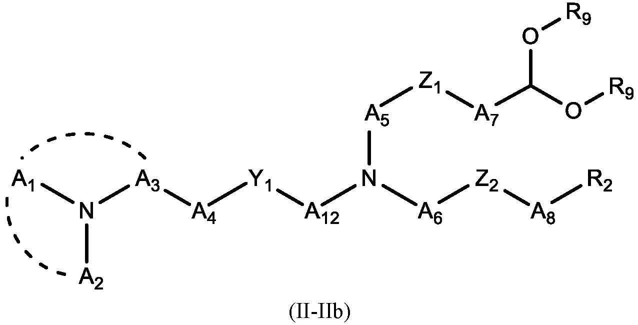 wherein A₁, A₂, A₃, A₄, A₅, A₆, A₇, A₈, Z₁, Z₂, Y₁, R₂, R₉, and A₁₂ are as defined for formula (II-I).

In some embodiments, the amino lipid compound represented by formula (II-I), or a pharmaceutically acceptable salt thereof, or a stereoisomer thereof, has a structure represented by formula (II-III): 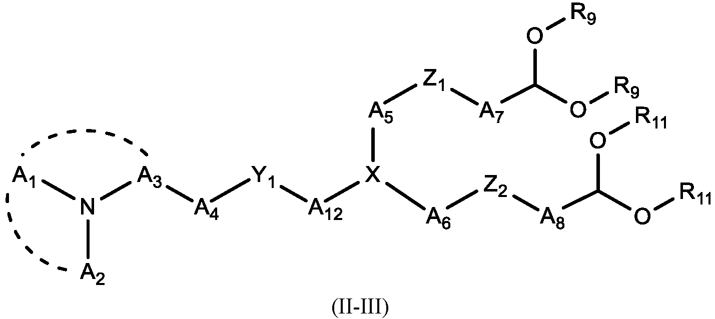 wherein A₁, A₂, A₃, A₄, A₅, A₆, A₇, A₈, Z₁, Z₂, Y₁, X, R₉, R₁₁, and A₁₂ are as defined for formula (II-I).

In some embodiments, the amino lipid compound represented by formula (II-I), or a pharmaceutically acceptable salt thereof, or a stereoisomer thereof, has a structure represented by formula (II-IIIa): 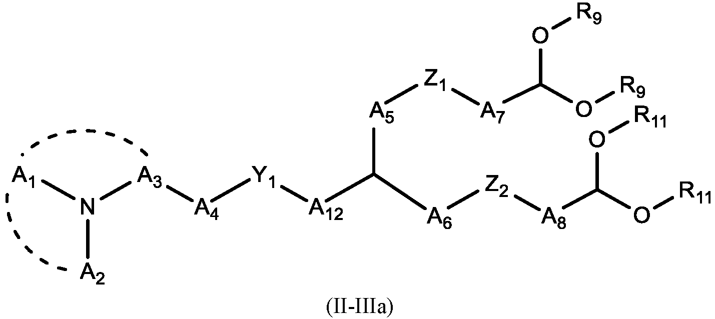 wherein A₁, A₂, A₃, A₄, A₅, A₆, A₇, A₈, Z₁, Z₂, Y₁, R₉, R₁₁, and A₁₂ are as defined for formula (II-I).

The present disclosure provides the amino lipid compound of formula (II-IIIa), or a pharmaceutically acceptable salt thereof, or a stereoisomer thereof, as described above, including one or more of the following features where applicable.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ alkylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenylene.

In some embodiments, A₁₂ is C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynylene.

In some embodiments, A₅ and A₆ are not both methylene.

In some embodiments, Z₁ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, Z₂ is -C(=O)O-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-.

In some embodiments, A₅ and A₆ are each independently C₁, C₂, C₃, C₄, C₅, or C₆ hydrocarbylene, or a bond.

In some embodiments, A₅ and A₆ are each independently C₁, C₂, C₃, C₄, C₅, or C₆ alkylene, or a bond.

In some embodiments, A₅ and A₆ are each independently C₂, C₃, C₄, C₅, or C₆ alkenylene, or a bond.

In some embodiments, A₅ and A₆ are each independently C₂, C₃, C₄, C₅, or C₆ alkynylene, or a bond.

In some embodiments, A₅ and A₆ are each independently C₁, or C₂ hydrocarbylene, or a bond. In some embodiments, A₅ and A₆ are each independently C₁, or C₂ alkylene, or a bond.

In some embodiments, A₅ and A₆ are each independently C₂ alkenylene, or a bond.

In some embodiments, A₅ and A₆ are each independently C₂ alkynylene, or a bond.

In some embodiments, the amino lipid compound represented by formula (II-I), or a pharmaceutically acceptable salt thereof, or a stereoisomer thereof, has a structure represented by formula (II-IIIb): 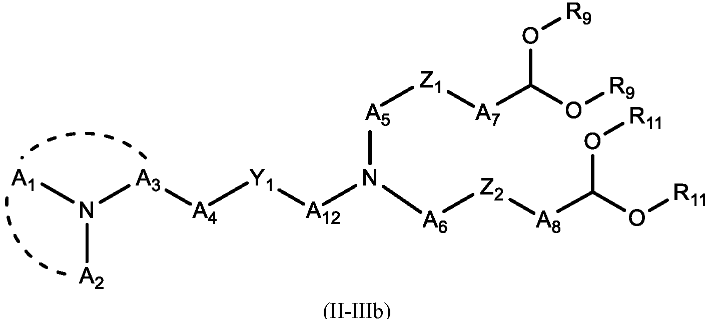 wherein A₁, A₂, A₃, A₄, A₅, A₆, A₇, A₈, Z₁, Z₂, Y₁, R₉, R₁₁, and A₁₂ are as defined for formula (II-I).

In some embodiments, the amino lipid compound represented by formula (II-I), or a pharmaceutically acceptable salt thereof, or a stereoisomer thereof, has a structure represented by formula (II-IVa) or (II-IVb): 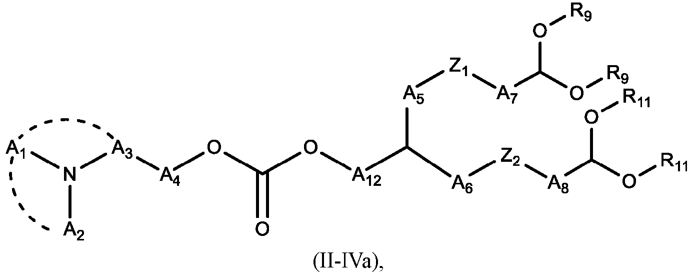 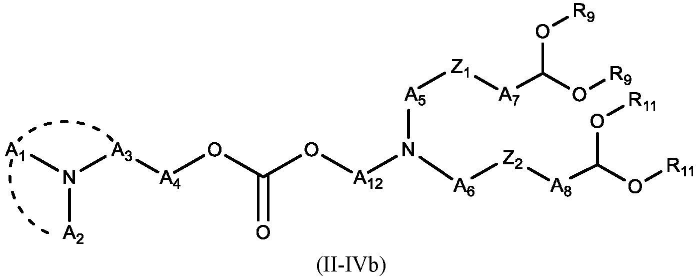 wherein A₁, A₂, A₃, A₄, A₅, A₆, A₇, A₈, Z₁, Z₂, R₉, R₁₁, and A₁₂ are as defined for formula (II-I).

In some embodiments, the present disclosure provides the amino lipid compound of formula (II-IVa), or a pharmaceutically acceptable salt thereof, or a stereoisomer thereof, as described above, wherein
A₁, A₂, and A₃ are one of the following:
   (1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring; or
   (3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
   (4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene, or a bond;
A₅, A₆, A₇, A₈, and A₁₂ are each independently C₁-C₁₈ hydrocarbylene, C₁-C₁₈ heterohydrocarbylene, or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
each R₆ is independently H or C₁-C₈ hydrocarbyl;
each R₉ and each R₁₁ are each independently C₁-C₂₄ hydrocarbyl, or C₁-C₂₄ heterohydrocarbyl containing O or S;
each A₉ is C₁-C₈ hydrocarbylene, or a bond;
each A₁₀ and each A₁₁ are each independently C₁-C₈ hydrocarbyl;
preferably, the hydrocarbyl is alkyl, alkenyl, or alkynyl;
preferably, the hydrocarbylene is alkylene, alkenylene, or alkynylene;
preferably, the heterohydrocarbyl is heteroalkyl, heteroalkenyl, or heteroalkynyl;
preferably, the heterohydrocarbylene is heteroalkylene, heteroalkenylene, or heteroalkynylene.

In some embodiments, the amino lipid compound represented by formula (II-I), or a pharmaceutically acceptable salt thereof, or a stereoisomer thereof, has a structure represented by formula (II-Va), (II-Vb), (II-Vc), (II-Vd), (II-Ve), (II-Vf), (II-Vg), or (II-Vh): 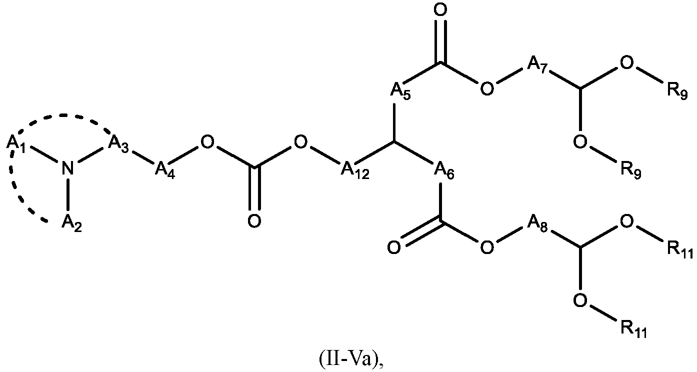 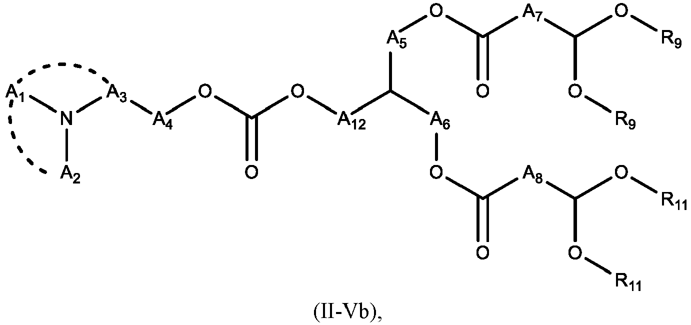 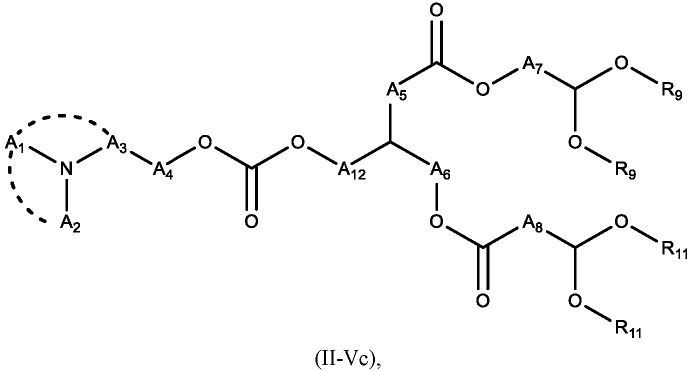 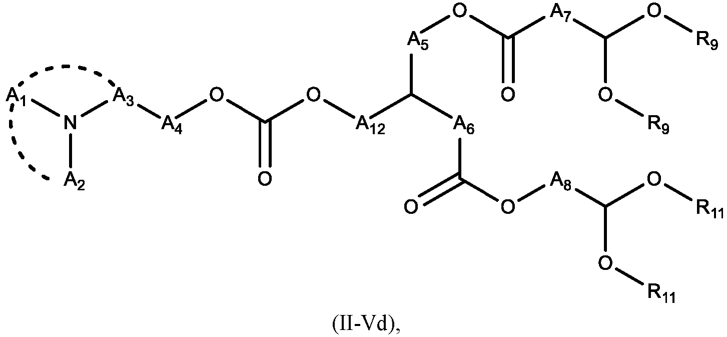 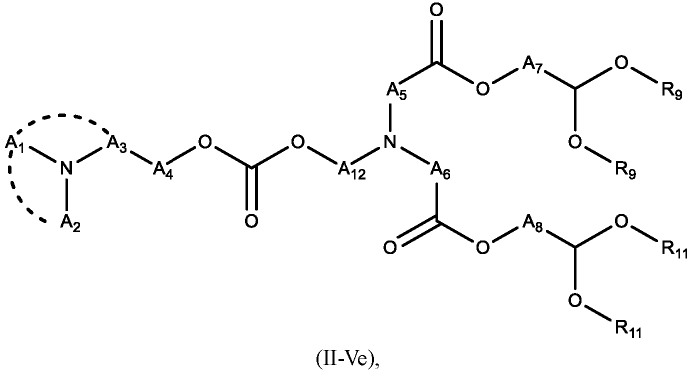 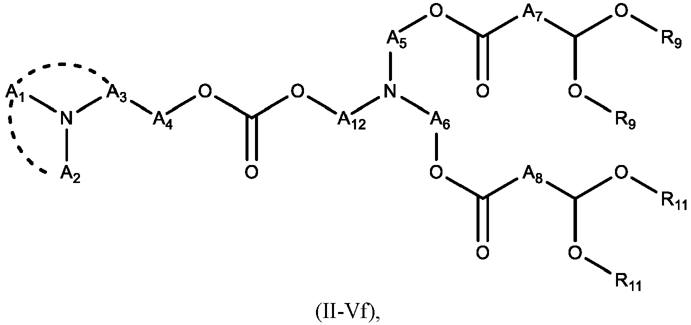 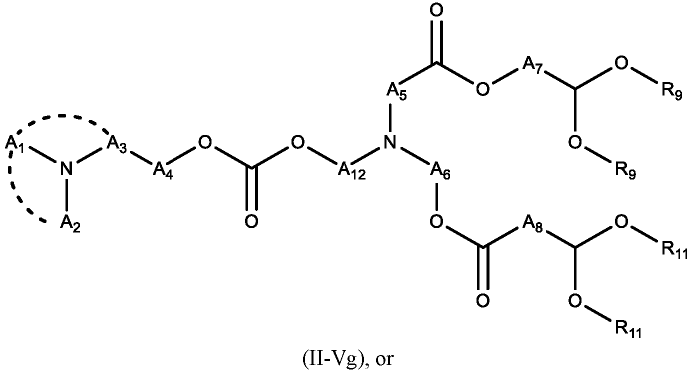 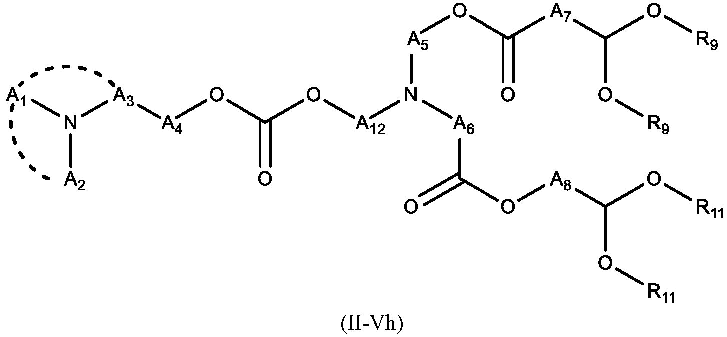 wherein A₁, A₂, A₃, A₄, A₅, A₆, A₇, A₈, R₉, R₁₁, and A₁₂ are as defined for formula (II-I).

In some embodiments, the amino lipid compound represented by formula (II-I), or a pharmaceutically acceptable salt thereof, or a stereoisomer thereof, has a structure represented by formula (II-VIa): 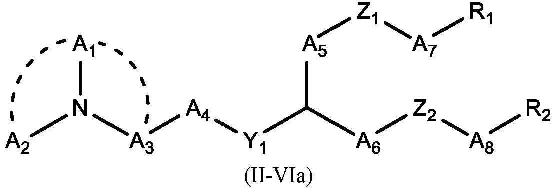 wherein,
A₁, A₂, A₃, A₄, A₅, A₆, A₇, A₈, Z₁, Z₂, Y₁, R₁, and R₂ are as defined for formula (II-I).
In some embodiments, the present disclosure provides the amino lipid compound of formula (II-VIa), or a pharmaceutically acceptable salt thereof, or a stereoisomer thereof, as described above, wherein
A₁, A₂, and A₃ are one of the following:
   (1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring; or
   (3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
   (4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene, or a bond;
A₅, A₆, A₇, and A₈ are each independently C₁-C₁₈ hydrocarbylene, C₁-C₁₈ heterohydrocarbylene, or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
Y₁ is -Z₃C(=O)Z₄-, -N(R₅)-, -Z₃C(=S)Z₄-, -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-, -OP(=O)O-, -OP(=O)(OH)O-, -OP(=O)(H)O-, -O-, -S-, -NHS(=O)₂O-, -NHP(=O)O-, -OP(=O)NH-, -NHP(=O)(OH)O-, -OP(=O)(OH)NH-, -OC(=O)S-, or -SC(=O)O-;
X is C or N;
R₁ is H, C₁-C₂₄ hydrocarbyl, C₁-C₂₄ heterohydrocarbyl, Z₆R₈, -C(R₆)(OA₁₃Z₈R₉)₂, -C(R₆)(SA₁₃Z₈R₉)₂, or -C(R₆)(SA₁₃Z₈R₉)(OA₁₃Z₈R₉);
R₂ is H, C₁-C₂₄ hydrocarbyl, C₁-C₂₄ heterohydrocarbyl, Z₇R₁₀, -C(R₆)(OA₁₄Z₉R₁₁)₂, -C(R₆)(SA₁₄Z₉R₁₁)₂, or -C(R₆)(SA₁₄Z₉R₁₁)(OA₁₄Z₉R₁₁);
Z₃ and Z₄ are each independently -O-, -N(R₆)-, -S-, or a bond;
Z₆ and Z₇ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
R₅ is -C(=O)A₉Z₅A₁₀, -C(=O)A₁₁, H, or C₁-C₅ hydrocarbyl;
Z₅ is -O- or -S-;
Z₈ and Z₉ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, -NHS(=O)₂O-, or a bond;
each R₆ is independently H or C₁-C₅ hydrocarbyl;
R₈ and R₁₀ are each independently H, C₁-C₂₄ hydrocarbyl, or C₁-C₂₄ heterohydrocarbyl containing O or S;
each R₉ and each R₁₁ are each independently C₁-C₂₄ hydrocarbyl, or C₁-C₂₄ heterohydrocarbyl containing O or S;
each A₉ is C₁-C₅ hydrocarbylene, or a bond;
each A₁₀ and each A₁₁ are each independently C₁-C₈ hydrocarbyl;
each A₁₃ and each A₁₄ are each independently C₁-C₈ hydrocarbylene, C₁-C₈ heterohydrocarbylene, or a bond;
preferably, the hydrocarbyl is alkyl, alkenyl, or alkynyl;
preferably, the hydrocarbylene is alkylene, alkenylene, or alkynylene;
preferably, the heterohydrocarbyl is heteroalkyl, heteroalkenyl, or heteroalkynyl;
preferably, the heterohydrocarbylene is heteroalkylene, heteroalkenylene, or heteroalkynylene.

In some embodiments, A₅ and A₆ are each independently C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene, or a bond, and are not both methylene.

In some embodiments, A₅ and A₆ are each independently C₁, or C₂ hydrocarbylene, or a bond, and are not both methylene.

In some embodiments, A₅ and A₆ are each independently C₃, C₄, C₅, or C₆ hydrocarbylene.

In some embodiments, A₅ and A₆ are each independently C₇, C₈, C₉, or C₁₀ hydrocarbylene.

In some embodiments, A₅ and A₆ are each independently C₁₁, C₁₂, C₁₃, or C₁₄ hydrocarbylene.

In some embodiments, A₅ and A₆ are each independently C₁₅, C₁₆, C₁₇, or C₁₈ hydrocarbylene.

In some embodiments, A₅ and A₆ are each independently C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ alkylene, or a bond, and are not both methylene.

In some embodiments, A₅ and A₆ are each independently C₁, or C₂ alkylene, or a bond, and are not both methylene.

In some embodiments, A₅ and A₆ are each independently C₃, C₄, C₅, or C₆ alkylene.

In some embodiments, A₅ and A₆ are each independently C₇, C₈, C₉, or C₁₀ alkylene.

In some embodiments, A₅ and A₆ are each independently C₁₁, C₁₂, C₁₃, or C₁₄ alkylene.

In some embodiments, A₅ and A₆ are each independently C₁₅, C₁₆, C₁₇, or C₁₈ alkylene.

In some embodiments, A₅ and A₆ are each independently C₂, C₃, C₄, C₅, C₆, C₇, C₈, C₉, C₁₀, C₁₁, C₁₂, C₁₃, C₁₄, C₁₅, C₁₆, C₁₇, or C₁₈ alkenylene, or a bond.

In some embodiments, A₅ and A₆ are each independently C₂ alkenylene, or a bond.

In some embodiments, A₅ and A₆ are each independently C₃, C₄, C₅, or C₆ alkenylene.

In some embodiments, A₅ and A₆ are each independently C₇, C₈, C₉, or C₁₀ alkenylene.

In some embodiments, A₅ and A₆ are each independently C₁₁, C₁₂, C₁₃, or C₁₄ alkenylene.

In some embodiments, A₅ and A₆ are each independently C₁₅, C₁₆, C₁₇, or C₁₈ alkenylene.

In some embodiments, A₅ and A₆ are each independently C₂, C₃, C₄, C₅, C₆, C₇, C₈, C₉, C₁₀, C₁₁, C₁₂, C₁₃, C₁₄, C₁₅, C₁₆, C₁₇, or C₁₈ alkynylene, or a bond.

In some embodiments, A₅ and A₆ are each independently C₂ alkynylene, or a bond.

In some embodiments, A₅ and A₆ are each independently C₃, C₄, C₅, or C₆ alkynylene.

In some embodiments, A₅ and A₆ are each independently C₇, C₈, C₉, or C₁₀ alkynylene.

In some embodiments, A₅ and A₆ are each independently C₁₁, C₁₂, C₁₃, or C₁₄ alkynylene.

In some embodiments, A₅ and A₆ are each independently C₁₅, C₁₆, C₁₇, or C₁₈ alkynylene.

In some embodiments, A₁ and A₂ are each independently C₁-C₅ alkyl, preferably C₁-C₃ alkyl, such as methyl, ethyl, n-propyl, or isopropyl; A₃ is C₁-C₅ alkylene, preferably C₂-C₄ alkylene, such as -(CH₂)₂-, -(CH₂)₃-, -(CH₂)₄-; Y₁ is -Z₃C(=O)Z₄-, preferably -N(R₆)C(=O)N(R₆)-, more preferably -N(H)C(=O)N(H)-; Z₁ and Z₂ are each independently -C(=O)O- or -OC(=O)-; A₇ and A₈ are each independently a bond; R₁ and R₂ are each independently C₁-C₂₄ hydrocarbyl, preferably C₁-C₂₄ alkyl, more preferably a straight C₃-C₁₂ alkyl or a branched C₇-C₂₁ alkyl, such as -C₄H₉, -C₅H₁₁, -C₆H₁₃, -C₇H₁₅, -C₈H₁₇, -C₉H₁₉, -CH(C₄H₉)₂, -CH(C₅H₁₁)₂, -CH(C₆H₁₃)₂, -CH(C₇H₁₅)₂, -CH(C₈H₁₇)₂, -CH(C₉H₁₉)₂, -CH₂CH(C₆H₁₃)(C₄H₉).

In some embodiments, the amino lipid compound represented by formula (II-VIa), or a pharmaceutically acceptable salt thereof, or a stereoisomer thereof, has a structure represented by formula (II-VIa-a): 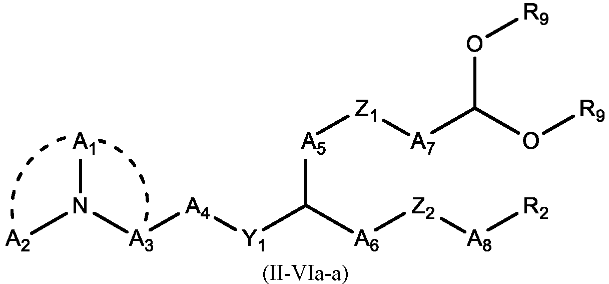 wherein,
A₁, A₂, A₃, A₄, A₅, A₆, A₇, A₈, Z₁, Z₂, Y₁, R₉, and R₂ are as defined for formula (II-VIa).

In some embodiments, the amino lipid compound represented by formula (II-VIa), or a pharmaceutically acceptable salt thereof, or a stereoisomer thereof, has a structure represented by formula (II-VIa-b): 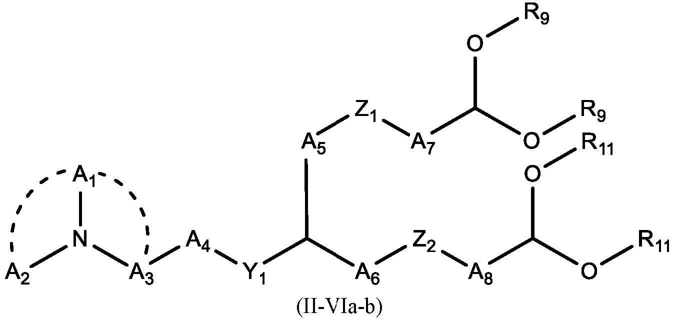 wherein,
A₁, A₂, A₃, A₄, A₅, A₆, A₇, A₈, Z₁, Z₂, Y₁, R₉, and R₁₁ are as defined for formula (II-VIa).

In some embodiments, the amino lipid compound represented by formula (II-VIa), or a pharmaceutically acceptable salt thereof, or a stereoisomer thereof, has a structure represented by formula (II-VIa-c): 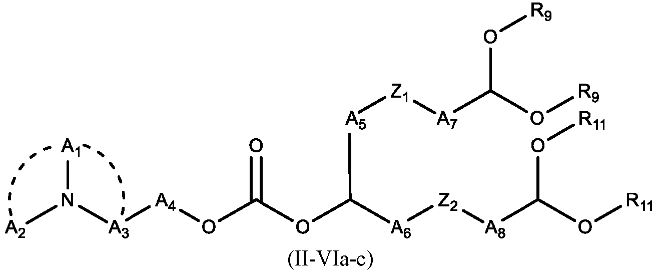 wherein,
A₁, A₂, A₃, A₄, A₅, A₆, A₇, A₈, Z₁, Z₂, R₉, and R₁₁ are as defined for formula (II-VIa).

In some embodiments, the present disclosure provides the amino lipid compound of formula (II-VIa-c), or a pharmaceutically acceptable salt thereof, or a stereoisomer thereof, as described above, wherein
A₁, A₂, and A₃ are one of the following:
   (1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
   (2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring; or
   (3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
   (4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene, or a bond;
A₅, A₆, A₇, and A₈ are each independently C₁-C₁₈ hydrocarbylene, C₁-C₁₈ heterohydrocarbylene, or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O-, -OP(=O)O-, -OP(=O)(OH)O-, -OP(=O)(H)O-, -OS(=O)₂NH-, or -NHS(=O)₂O-;
each R₆ is independently H or C₁-C₅ hydrocarbyl;
each R₉ and each R₁₁ are each independently C₁-C₂₄ hydrocarbyl, or C₁-C₂₄ heterohydrocarbyl containing O or S;
each A₉ is C₁-C₅ hydrocarbylene, or a bond;
each A₁₀ and each A₁₁ are each independently C₁-C₈ hydrocarbyl;
preferably, the hydrocarbyl is alkyl, alkenyl, or alkynyl;
preferably, the hydrocarbylene is alkylene, alkenylene, or alkynylene;
preferably, the heterohydrocarbyl is heteroalkyl, heteroalkenyl, or heteroalkynyl;
preferably, the heterohydrocarbylene is heteroalkylene, heteroalkenylene, or heteroalkynylene.

The amino lipid compound represented by formula (II-IVa), (II-VIa), or (II-VIa-c), or a pharmaceutically acceptable salt thereof, or a stereoisomer thereof, includes one or more of the following features where applicable.

In some embodiments, A₄ is a bond.

In some embodiments, Y₁ is -Z₃C(=O)Z₄- or -N(R₅)-.

In some embodiments, Y₁ is -N(R₆)C(=O)O-, -OC(=O)N(R₆)-, or -OC(=O)O-.

In some embodiments, Y₁ is -OC(=O)O-.

In some embodiments, Y₁ is -N(R₆)C(=O)O- or -OC(=O)N(R₆)-.

In some embodiments, each R₆ is independently H, or C₁-C₅ alkyl substituted with -OC(=O)R₁₄, -C(=O)OR₁₄, or -OR₁₄.

In some embodiments, Y₁ is -N(R₆)C(=O)O- or -OC(=O)N(R₆)-, wherein each R₆ is independently C₁-C₈ alkyl (e.g., C₁-C₅ alkyl, C₁-C₃ alkyl) substituted with -OC(=O)R₁₄, -C(=O)OR₁₄, or -OR₁₄.

In some embodiments, Y₁ is -N(H)C(=O)O-.

In some embodiments, Y₁ is -OC(=O)N(H)-.

In some embodiments, Y₁ is -N(R₅)-.

In some embodiments, Y₁ is -N(C(=O)A₉OA₁₀)-.

In some embodiments, Y₁ is -N(C(=O)A₁₀)-.

In some embodiments, Z₁ and Z₂ are each independently -C(=O)O-, -OC(=O)-, -O-, -C(=O)-, -C(=O)N(R₆)-, -N(R₆)C(=O)-, -N(R₆)C(=O)O-, -OC(=O)N(R₆)-, -N(R₆)C(=O)N(R₆)-, -C(=O)S-, -SC(=O)-, -OC(=O)O-, -OC(=O)S-, -SC(=O)O-, -SC(=O)S-, -OS(=O)O-, -OS(=O)₂O-, -OP(=O)O-, -OP(=O)(OH)O-, or -OP(=O)(H)O-.

In some embodiments, Z₁ and Z₂ are each independently -C(=O)O-, -OC(=O)-, -OC(=O)O-, -N(H)C(=O)O-, or -OC(=O)N(H)-.

In some embodiments, Z₁ and Z₂ are each independently -(C=O)O- or -O(C=O)-;

In some embodiments, Z₁ is -C(=O)O-.

In some embodiments, Z₁ is -OC(=O)-.

In some embodiments, Z₁ is -OC(=O)O-.

In some embodiments, Z₁ is -N(H)C(=O)O-.

In some embodiments, Z₁ is -OC(=O)N(H)-.

In some embodiments, Z₂ is -C(=O)O-.

In some embodiments, Z₂ is -OC(=O)-.

In some embodiments, Z₂ is -OC(=O)O-.

In some embodiments, Z₂ is -N(H)C(=O)O-.

In some embodiments, Z₂ is -OC(=O)N(H)-.

In some embodiments, Z₁ and Z₂ are -C(=O)O-.

In some embodiments, Z₁ and Z₂ are -OC(=O)-.

In some embodiments, Z₁ and Z₂ are -OC(=O)O-.

In some embodiments, Z₁ and Z₂ are -N(H)C(=O)O-.

In some embodiments, Z₁ and Z₂ are -OC(=O)N(H)-.

In some embodiments, Z₁ is -C(=O)O-, and Z₂ is -OC(=O)-.

In some embodiments, A₄ is a bond, and Z₁ and Z₂ are C(=O)O-.

In some embodiments, A₄ is a bond, and Z₁ and Z₂ are -OC(=O)-.

In some embodiments, A₄ is a bond, and Z₁ and Z₂ are -OC(=O)O-.

In some embodiments, A₄ is a bond, and Z₁ and Z₂ are -N(H)C(=O)O-.

In some embodiments, A₄ is a bond, and Z₁ and Z₂ are -OC(=O)N(H)-.

In some embodiments, A₄ is a bond, Z₁ is -C(=O)O-, and Z₂ is -OC(=O)-.

In some embodiments, A₁₂ is C₁-C₃ alkylene, such as C₂-C₃ alkylene.

In some embodiments, A₁₂ is C₁-C₃ alkylene (e.g., C₁ alkylene), and A₅ and A₆ are each independently C₃-C₉ alkylene (e.g., C₄-C₉ alkylene, C₅-C₉ alkylene, C₆ alkylene, C₇ alkylene, C₈ alkylene).

In some embodiments, R₁ is -C(R₆)(OR₉)₂.

In some embodiments, R₁ is -C(H)(OR₉)₂.

In some embodiments, R₂ is -C(R₆)(OR₁₁)₂.

In some embodiments, R₂ is -C(H)(OR₁₁)₂.

In some embodiments, R₂ is Z₇R₁₀.

In some embodiments, R₂ is -C(=O)OR₁₀.

In some embodiments, R₂ is -OC(=O)R₁₀.

In some embodiments, R₂ is -OR₁₀.

In some embodiments, R₂ is -N(H)C(=O)R₁₀.

In some embodiments, R₂ is -C(=O)N(R₆)R₁₀.

In some embodiments, R₂ is substituted C₁-C₂₄ alkyl, such as substituted C₅-C₁₆ alkyl, substituted C₆-C₁₄ alkyl, substituted C₆-C₁₂ alkyl. In some embodiments, R₂ is C₁-C₂₄ alkyl substituted with hydroxyl, e.g., C₅-C₁₆ alkyl substituted with hydroxyl, such as C₆-C₁₄ alkyl substituted with hydroxyl, C₆-C₁₂ alkyl substituted with hydroxyl, C₁₀ alkyl substituted with hydroxyl.

In some embodiments, R₁ is -C(R₆)(OR₉)₂, and R₂ is -C(R₆)(OR₁₁)₂.

In some embodiments, R₁ is -C(H)(OR₉)₂, and R₂ is -C(H)(OR₁₁)₂.

In some embodiments, R₁ is -C(H)(OR₉)₂, and R₂ is -C(=O)OR₁₀.

In some embodiments, R₁ is -C(H)(OR₉)₂, and R₂ is -OC(=O)R₁₀.

In some embodiments, R₁ is -C(H)(OR₉)₂, and R₂ is -OR₁₀.

In some embodiments, R₁ is -C(H)(OR₉)₂, and R₂ is C₁-C₂₄ alkyl substituted with hydroxyl (e.g., R₂ is C₅-C₁₆ alkyl substituted with hydroxyl, C₆-C₁₄ alkyl substituted with hydroxyl, C₆-C₁₂ alkyl substituted with hydroxyl, or C₁₀ alkyl substituted with hydroxyl).

In some embodiments, Y₁ is -OC(=O)O-, R₁ is -C(H)(OR₉)₂, and R₂ is -C(=O)OR₁₀.

In some embodiments, Y₁ is -OC(=O)O-, R₁ is -C(H)(OR₉)₂, and R₂ is -OC(=O)R₁₀.

In some embodiments, Y₁ is -OC(=O)O-, R₁ is -C(H)(OR₉)₂, and R₂ is -OR₁₀.

In some embodiments, Y₁ is -OC(=O)O-, R₁ is -C(H)(OR₉)₂, and R₂ is substituted C₁-C₂₄ alkyl (e.g., R₂ is substituted C₅-C₁₆ alkyl, substituted C₆-C₁₄ alkyl, substituted C₆-C₁₂ alkyl, or substituted C₁₀ alkyl).

In some embodiments, Y₁ is -OC(=O)O-, R₁ is -C(H)(OR₉)₂, and R₂ is C₁-C₂₄ alkyl substituted with hydroxyl (e.g., R₂ is C₅-C₁₆ alkyl substituted with hydroxyl, C₆-C₁₄ alkyl substituted with hydroxyl, C₆-C₁₂ alkyl substituted with hydroxyl, or C₁₀ alkyl substituted with hydroxyl).

In some embodiments, Y₁ is -OC(=O)O-, R₁ is -C(H)(OR₉)₂, R₂ is C₁-C₂₄ alkyl substituted with hydroxyl (e.g., R₂ is C₅-C₁₆ alkyl substituted with hydroxyl, C₆-C₁₄ alkyl substituted with hydroxyl, C₆-C₁₂ alkyl substituted with hydroxyl, or C₁₀ alkyl substituted with hydroxyl), and A₅ and A₆ are each independently C₂-C₁₀ alkylene (e.g., C₅-C₉ alkylene, C₈ alkylene).

In some embodiments, Y₁ is -OC(=O)O-, A₇ and A₈ are unsubstituted C₁-C₅ alkylene (e.g., unsubstituted C₂-C₄ alkylene), R₁ is -C(H)(OR₉)₂, and R₂ is -C(=O)OR₁₀.

In some embodiments, Y₁ is -OC(=O)O-, A₇ and A₈ are unsubstituted C₁-C₅ alkylene (e.g., unsubstituted C₂-C₄ alkylene), R₁ is -C(H)(OR₉)₂, and R₂ is -OC(=O)R₁₀.

In some embodiments, Y₁ is -N(H)C(=O)O-, R₁ is -C(H)(OR₉)₂, and R₂ is -C(H)(OR₁₁)₂.

In some embodiments, Y₁ is -OC(=O)N(H)-, R₁ is -C(H)(OR₉)₂, and R₂ is -C(H)(OR₁₁)₂.

In some embodiments, Y₁ is -N(C(=O)A₉OA₁₀)-, R₁ is -C(H)(OR₉)₂, and R₂ is -C(H)(OR₁₁)₂. In some embodiments, Y₁ is -N(C(=O)A₁₀)-, R₁ is -C(H)(OR₉)₂, and R₂ is -C(H)(OR₁₁)₂.

In some embodiments, Y₁ is -N(C(=O)A₁₀)-, R₁ is -C(H)(OR₉)₂, and R₂ is C₁-C₂₄ alkyl substituted with hydroxyl (e.g., R₂ is C₅-C₁₆ alkyl substituted with hydroxyl, C₆-C₁₄ alkyl substituted with hydroxyl, C₆-C₁₂ alkyl substituted with hydroxyl, or C₁₀ alkyl substituted with hydroxyl).

In some embodiments, A₉ is C₁-C₈ alkylene.

In some embodiments, A₉ is C₁-C₅ alkylene.

In some embodiments, A₉ is C₁-C₃ alkylene.

In some embodiments, A₁₀ is C₁-C₅ alkyl, such as C₁, C₂, C₃, C₅, or C₈ alkyl. In some embodiments, R₁₀ is C₁-C₂₄ alkyl, C₂-C₂₄ alkenyl, or C₂-C₂₄ alkynyl. In some embodiments, R₁₀ is a straight C₂-C₁₀ alkyl.

In some embodiments, R₁₀ is a straight C₂-C₈ alkyl.

In some embodiments, R₁₀ is a branched C₇-C₁₈ alkyl, such as:

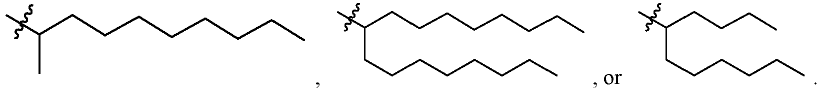

In some embodiments, R₁₀ is C₅-C₁₆ alkenyl.

In some embodiments, R₁₀ is C₅-C₁₂ alkenyl.

In some embodiments, R₁₀ is C₅-C₈ alkenyl, such as C₅, or C₆ alkenyl.

In some embodiments, R₁₀ is C₅-C₁₆ alkynyl.

In some embodiments, R₁₀ is C₅-C₁₂ alkynyl, such as C₁₀ alkynyl.

In some embodiments, R₉ is C₉-C₁₁ alkynyl, such as C₁₀ alkynyl.

In some embodiments, R₁₄ is C₁-C₁₂ alkyl.

In some embodiments, R₁₄ is C₁-C₅ alkyl.

In some embodiments, R₁₄ is C₂-C₁₂ alkenyl with 1, 2, 3, or more double bonds.

In some embodiments, R₁₄ is C₄-C₈ alkenyl with one double bond, such as C₆ alkenyl with one double bond.

In some embodiments, A₅ and A₆ are each independently C₁-C₁₁ alkylene.

In some embodiments, A₅ and A₆ are each independently C₃-C₁₀ alkylene.

In some embodiments, A₅ and A₆ are each independently C₄-C₉ alkylene.

In some embodiments, A₅ and A₆ are each independently C₅-C₉ alkylene, such as C₆, C₇, or C₈ alkylene.

In some embodiments, A₇ and A₈ are each independently C₁-C₁₂ alkylene.

In some embodiments, A₇ and A₈ are each independently C₅-C₁₀ alkylene.

In some embodiments, A₇ and A₈ are each independently C₁-C₅ alkylene.

In some embodiments, A₇ and A₈ are each independently C₂-C₄ alkylene.

In some embodiments, A₇ and A₈ are C₃ alkylene.

In some embodiments, A₇ or A₈ is a bond.

In some embodiments, A₇ is a bond.

In some embodiments, A₈ is a bond.

In some embodiments, each R₉ is independently C₁-C₁₂ alkyl, C₅-C₁₆ alkenyl, or C₅-C₁₆ alkynyl.

In some embodiments, each R₉ is independently C₃-C₉ alkyl.

In some embodiments, each R₉ is independently C₅-C₈ alkyl, such as C₆, C₇, or C₈ alkyl. In some embodiments, each R₉ is independently C₅-C₁₂ alkenyl.

In some embodiments, each R₉ is independently C₅-C₈ alkenyl, such as C₆ alkenyl.

In some embodiments, each R₉ is independently C₉-C₁₁ alkynyl, such as C₁₀ alkynyl.

In some embodiments, each R₁₁ is independently C₁-C₁₂ alkyl.

In some embodiments, each R₁₁ is independently C₃-C₉ alkyl.

In some embodiments, each R₁₁ is independently C₅-C₈ alkyl, such as C₆, C₇, or C₈ alkyl. In some embodiments, each R₁₁ is independently C₅-C₁₂ alkenyl.

In some embodiments, each R₁₁ is independently C₅-C₈ alkenyl, such as C₆ alkenyl.

In some embodiments, each R₁₁ is independently C₉-C₁₁ alkynyl, such as C₁₀ alkynyl.

In some embodiments, the dashed line connecting A₁ and A₂ and the dashed line connecting A₁ and A₃ are absent.

In some embodiments, the dashed line connecting A₁ and A₂ is a bond, and the dashed line connecting A₁ and A₃ is absent.

In some embodiments, the dashed line connecting A₁ and A₃ is a bond, and the dashed line connecting A₁ and A₂ is absent.

In some embodiments, A₁ and A₂ are each independently C₁-C₅ alkyl or C₁-C₅ alkylene.

In some embodiments, A₁ and A₂ are each independently C₁-C₃ alkyl.

In some embodiments, A₁ and A₂ are each independently C₁-C₂ alkyl.

In some embodiments, A₂ is C₁-C₃ alkyl, such as C₁-C₂ alkyl.

In some embodiments, A₃ is C₁-C₅ alkylene.

In some embodiments, A₃ is C₂-C₄ alkylene, such as C₃ alkylene.

In some embodiments, the dashed line connecting A₁ and A₂ and the dashed line connecting A₁ and A₃ are absent, and A₁ and A₂ are each independently C₁-C₃ alkyl, such as C₁-C₂ alkyl.

In some embodiments, the dashed line connecting A₁ and A₂ and the dashed line connecting A₁ and A₃ are absent, and A₁ and/or A₂ is unsubstituted C₁-C₅ alkyl, such as unsubstituted C₁-C₄ alkyl, unsubstituted C₁-C₃ alkyl, unsubstituted C₁-C₂ alkyl.

In some embodiments, the dashed line connecting A₁ and A₂ and the dashed line connecting A₁ and A₃ are absent, and A₁ and A₂ are each independently unsubstituted C₁-C₅ alkyl, such as unsubstituted C₁-C₄ alkyl, unsubstituted C₁-C₃ alkyl, unsubstituted C₁-C₂ alkyl.

In some embodiments, the dashed line connecting A₁ and A₂ and the dashed line connecting A₁ and A₃ are absent, and A₁ and/or A₂ is substituted C₁-C₅ alkyl, such as substituted C₁-C₄ alkyl, substituted C₁-C₃ alkyl, substituted C₁-C₂ alkyl.

In some embodiments, when the dashed line connecting A₁ and A₂ is a bond and the dashed line connecting A₁ and A₃ is absent, A₁ and/or A₂ is C₁-C₅ alkylene.

In some embodiments, when the dashed line connecting A₁ and A₂ is a bond and the dashed line connecting A₁ and A₃ is absent, A₁ and A₂, together with the nitrogen atom to which they are both attached, form an N-containing four-, five-, or six-membered heterocyclic ring.

In some embodiments, when the dashed line connecting A₁ and A₂ is a bond and the dashed line connecting A₁ and A₃ is absent, A₁ and/or A₂ is unsubstituted C₁-C₅ alkylene.

In some embodiments, when the dashed line connecting A₁ and A₂ is a bond and the dashed line connecting A₁ and A₃ is absent, A₁ and A₂ are each independently unsubstituted C₁-C₅ alkylene.

In some embodiments, when the dashed line connecting A₁ and A₂ is a bond and the dashed line connecting A₁ and A₃ is absent, A₁ and/or A₂ is substituted C₁-C₅ alkylene.

In some embodiments, when the dashed line connecting A₁ and A₃ is a bond and the dashed line connecting A₁ and A₂ is absent, A₂ is C₁-C₃ alkyl, and A₁ and/or A₃ is C₁-C₅ alkylene.

In some embodiments, when the dashed line connecting A₁ and A₃ is a bond and the dashed line connecting A₁ and A₂ is absent, A₁ and A₃, together with the nitrogen atom to which they are both attached, form an N-containing four-, five-, or six-membered heterocyclic ring.

In some embodiments, when the dashed line connecting A₁ and A₃ is a bond and the dashed line connecting A₁ and A₂ is absent, A₁ is unsubstituted C₁-C₅ alkylene, and A₂ is unsubstituted C₁-C₅ alkyl (e.g., unsubstituted C₁-C₄ alkyl, unsubstituted C₁-C₃ alkyl, unsubstituted C₁-C₂ alkyl).

In some embodiments, when the dashed line connecting A₁ and A₃ is a bond and the dashed line connecting A₁ and A₂ is absent, A₁ is unsubstituted C₁-C₅ alkylene, and A₂ is substituted C₁-C₅ alkyl (e.g., substituted C₁-C₄ alkyl, substituted C₁-C₃ alkyl, substituted C₁-C₂ alkyl).

In some embodiments, when the dashed line connecting A₁ and A₃ is a bond and the dashed line connecting A₁ and A₂ is absent, A₁ is substituted C₁-C₅ alkylene, and A₂ is unsubstituted C₁-C₅ alkyl (e.g., unsubstituted C₁-C₄ alkyl, unsubstituted C₁-C₃ alkyl, unsubstituted C₁-C₂ alkyl).

In some embodiments, when the dashed line connecting A₁ and A₃ is a bond and the dashed line connecting A₁ and A₂ is absent, A₁ is substituted C₁-C₅ alkylene, and A₂ is substituted C₁-C₅ alkyl (e.g., substituted C₁-C₄ alkyl, substituted C₁-C₃ alkyl, substituted C₁-C₂ alkyl).

In some embodiments, A₁ or A₂ is C₁-C₅ alkyl, C₁-C₂ alkyl, or C₁-C₅ alkylene, which is substituted with hydroxyl, -OR₁₂, -C(=O)OR₁₂, -OC(=O)R₁₂, or C₁-C₅ alkyl, wherein each R₁₂ is independently C₁-C₁₂ alkyl, for example, A₁ or A₂ is

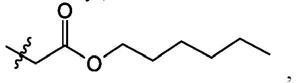

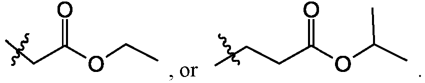

In some embodiments, A₁ and A₂ are each independently C₁-C₅ alkyl, C₁-C₂ alkyl, or C₁-C₅ alkylene, which is substituted with C₁-C₃ alkyl, such as 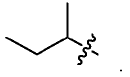 In some embodiments, A₃ is unsubstituted C₁-C₅ alkylene.

In some embodiments, A₃ is unsubstituted C₂-C₄ alkylene.

In some embodiments, A₃ is unsubstituted C₃ alkylene.

In some embodiments, A₃ is substituted C₁-C₅ alkylene, such as substituted C₂-C₄ alkylene, substituted C₃ alkylene.

In some embodiments, A₃ is C₃ alkylene substituted with one or two R₁₄, wherein R₁₄ is C₁-C₁₂ alkyl (e.g., C₁-C₅ alkyl, C₁-C₃ alkyl, C₁ alkyl).

In some embodiments, A₃ is C₃ alkylene substituted with two -R₁₄, wherein R₁₄ is C₁-C₁₂ alkyl (e.g., C₁-C₅ alkyl, C₁-C₃ alkyl, C₁ alkyl). For example, A₃ is

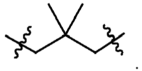

In some embodiments, 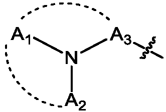 has one of the following structures: 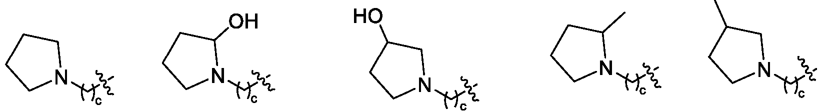 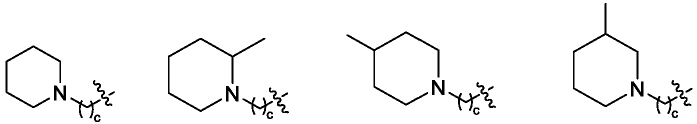 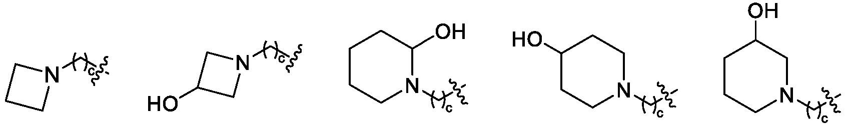 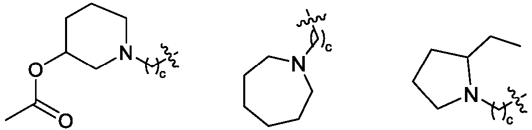 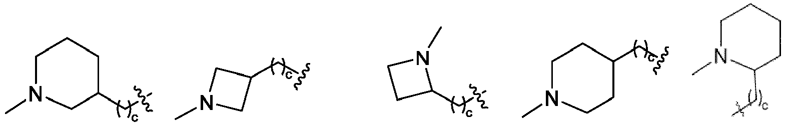 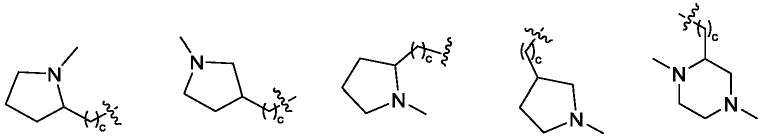 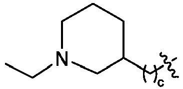 wherein c is 1, 2, 3, 4, or 5.

In some embodiments, each R₉ and/or each R₁₁ is C₁-C₁₂ alkyl (e.g., C₃-C₉ alkyl, C₅-C₈ alkyl, C₆ alkyl) substituted with -R₁₃OC(=O)R₁₄, -R₁₃C(=O)OR₁₄, or -R₁₃OR₁₄, wherein R₁₄ is C₁-C₁₂ alkyl (e.g., C₁-C₅ alkyl, C₁-C₃ alkyl, C₁ alkyl).

In some embodiments, R₉ and R₁₁ are C₂-C₅ alkyl (e.g., C₃-C₄ alkyl) substituted with -R₁₃OC(=O)R₁₄, -R₁₃C(=O)OR₁₄, or -R₁₃OR₁₄, wherein R₁₄ is C₁-C₁₂ alkyl (e.g., C₁-C₅ alkyl, C₁-C₃ alkyl, C₁ alkyl). For example, R₉ and R₁₁ are

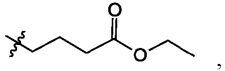

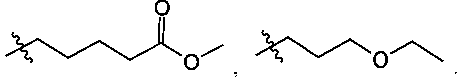

In some embodiments, the present disclosure provides the amino lipid compound of formula (II-VIa-c), or a pharmaceutically acceptable salt thereof, or a stereoisomer thereof, as described above, wherein
Z₁ and Z₂ are each independently -C(=O)O- and -OC(=O)-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₂ and the dashed line connecting A₁ and A₃ are absent, A₁ and A₂ are each independently C₁-C₃ alkyl, and A₃ is C₂-C₄ alkylene.

In some embodiments, the present disclosure provides the amino lipid compound of formula (II-VIa-c), or a pharmaceutically acceptable salt thereof, or a stereoisomer thereof, as described above, wherein Z₁ and Z₂ are -C(=O)O-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₂ and the dashed line connecting A₁ and A₃ are absent, A₁ and A₂ are each independently C₁-C₃ alkyl, and A₃ is C₂-C₄ alkylene.

In some embodiments, the present disclosure provides the amino lipid compound of formula (II-VIa-c), or a pharmaceutically acceptable salt thereof, or a stereoisomer thereof, as described above, wherein Z₁ and Z₂ are each independently -C(=O)O- and -OC(=O)-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₂ is a bond and the dashed line connecting A₁ and A₃ is absent, A₁ and A₂ are each independently C₁-C₅ alkylene, and A₃ is C₂-C₄ alkylene.

In some embodiments, the present disclosure provides the amino lipid compound of formula (II-VIa-c), or a pharmaceutically acceptable salt thereof, or a stereoisomer thereof, as described above, wherein Z₁ and Z₂ are each independently -C(=O)O- and -OC(=O)-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₂ is a bond and the dashed line connecting A₁ and A₃ is absent, A₁ and A₂, together with the nitrogen atom to which they are both attached, form an N-containing four-, five-, or six-membered heterocyclic ring, and A₃ is C₂-C₄ alkylene.

In some embodiments, the present disclosure provides the amino lipid compound of formula (II-VIa-c), or a pharmaceutically acceptable salt thereof, or a stereoisomer thereof, as described above, wherein Z₁ and Z₂ are each independently -C(=O)O- and -OC(=O)-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₃ is a bond and the dashed line connecting A₁ and A₂ is absent, A₂ is C₁-C₃ alkyl, and A₁ and A₃ are each independently C₁-C₅ alkylene.

In some embodiments, the present disclosure provides the amino lipid compound of formula (II-VIa-c), or a pharmaceutically acceptable salt thereof, or a stereoisomer thereof, as described above, wherein Z₁ and Z₂ are each independently -C(=O)O- and -OC(=O)-, A₄ is a bond, A₅ and A₆ are each independently C₃-C₁₀ alkylene, A₇ and A₈ are each independently C₂-C₄ alkylene, each R₉ is independently C₃-C₉ alkyl, each R₁₁ is independently C₃-C₉ alkyl, the dashed line connecting A₁ and A₃ is a bond and the dashed line connecting A₁ and A₂ is absent, A₂ is C₁-C₃ alkyl, and A₁ and A₃, together with the nitrogen atom to which they are both attached, form an N-containing four-, five-, or six-membered heterocyclic ring.

In some embodiments, the amino lipid compound represented by formula (II-I), or a pharmaceutically acceptable salt thereof, or a stereoisomer thereof, has formula (II-VIb): 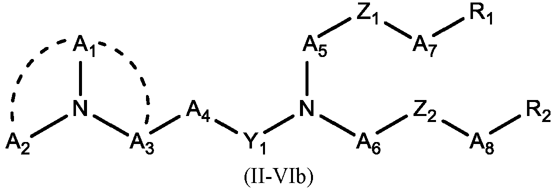 wherein,
A₁, A₂, A₃, A₄, A₅, A₆, A₇, A₈, Z₁, Z₂, Y₁, R₁, and R₂ are as defined for formula (II-I).

In some embodiments, A₅ and A₆ are each independently C₁, or C₂ hydrocarbylene, or a bond.

In some embodiments, A₅ and A₆ are each independently C₃, C₄, C₅, or C₆ hydrocarbylene.

In some embodiments, A₅ and A₆ are each independently C₇, C₈, C₉, or C₁₀ hydrocarbylene.

In some embodiments, A₅ and A₆ are each independently C₁₁, C₁₂, C₁₃, or C₁₄ hydrocarbylene.

In some embodiments, A₅ and A₆ are each independently C₁₅, C₁₆, C₁₇, or C₁₈ hydrocarbylene.

In some embodiments, A₅ and A₆ are each independently C₁, or C₂ alkylene, or a bond.

In some embodiments, A₅ and A₆ are each independently C₃, C₄, C₅, or C₆ alkylene.

In some embodiments, A₅ and A₆ are each independently C₇, C₈, C₉, or C₁₀ alkylene.

In some embodiments, A₅ and A₆ are each independently C₁₁, C₁₂, C₁₃, or C₁₄ alkylene.

In some embodiments, A₅ and A₆ are each independently C₁₅, C₁₆, C₁₇, or C₁₈ alkylene.

In some embodiments, A₅ and A₆ are each independently C₂ alkenylene, or a bond.

In some embodiments, A₅ and A₆ are each independently C₃, C₄, C₅, or C₆ alkenylene.

In some embodiments, A₅ and A₆ are each independently C₇, C₈, C₉, or C₁₀ alkenylene.

In some embodiments, A₅ and A₆ are each independently C₁₁, C₁₂, C₁₃, or C₁₄ alkenylene.

In some embodiments, A₅ and A₆ are each independently C₁₅, C₁₆, C₁₇, or C₁₈ alkenylene.

In some embodiments, A₅ and A₆ are each independently C₂ alkynylene, or a bond.

In some embodiments, A₅ and A₆ are each independently C₃, C₄, C₅, or C₆ alkynylene.

In some embodiments, A₅ and A₆ are each independently C₇, C₈, C₉, or C₁₀ alkynylene.

In some embodiments, A₅ and A₆ are each independently C₁₁, C₁₂, C₁₃, or C₁₄ alkynylene.

In some embodiments, A₅ and A₆ are each independently C₁₅, C₁₆, C₁₇, or C₁₈ alkynylene.

In some embodiments, the amino lipid compound represented by formula (II-VIb), or a pharmaceutically acceptable salt thereof, or a stereoisomer thereof, has a structure represented by formula (II-VIb-a): 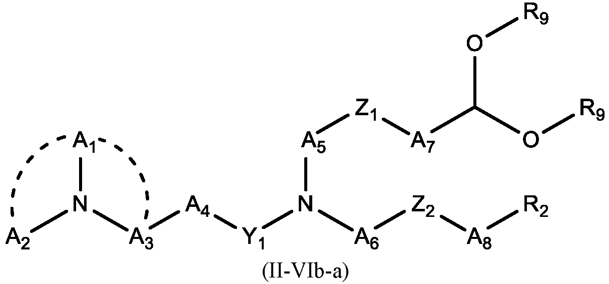 wherein,
A₁, A₂, A₃, A₄, A₅, A₆, A₇, A₈, Z₁, Z₂, Y₁, R₉, and R₂ are as defined for formula (II-VIb).

In some embodiments, the amino lipid compound represented by formula (II-VIb), or a pharmaceutically acceptable salt thereof, or a stereoisomer thereof, has a structure represented by formula (II-VIb-b): 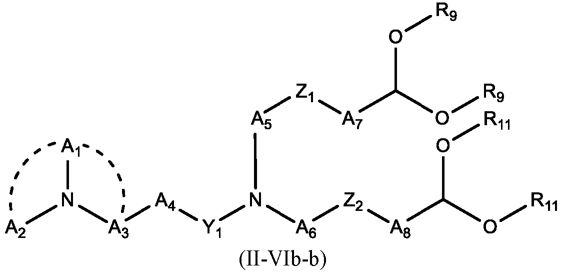 wherein,
A₁, A₂, A₃, A₄, A₅, A₆, A₇, A₈, Z₁, Z₂, Y₁, R₉, and R₁₁ are as defined for formula (II-VIb).

In some embodiments, the amino lipid compound represented by formula (II-VIb), or a pharmaceutically acceptable salt thereof, or a stereoisomer thereof, has a structure represented by formula (II-VIb-c): 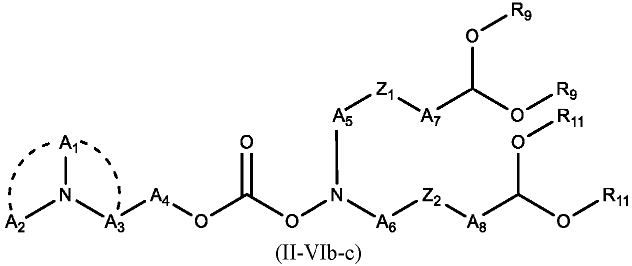 wherein,
A₁, A₂, A₃, A₄, A₅, A₆, A₇, A₈, Z₁, Z₂, R₉, and R₁₁ are as defined for formula (II-VIb).

In some embodiments, the present disclosure provides the amino lipid compound of formula (II-I), (II-la), (II-lb), (II-Ic), (II-Id), (II-Ie), (II-II), (II-IIa), (II-IIb), (II-III), (II-IIIa), (II-IIIb), (II-IVa), (II-IVb), (II-Va), (II-Vb), (II-Vc), (II-Vd), (II-Ve), (II-Vf), (II-Vg), (II-Vh), (II-VIa), (II-VIa-a), (II-VIa-b), (II-VIa-c) (II-VIb), (II-VIb-a), (II-VIb-b), or (II-VIb-c), or a pharmaceutically acceptable salt thereof, or a stereoisomer thereof, as described above, wherein the hydrocarbyl, hydrocarbylene, alkyl, alkylene, alkenyl, alkenylene, alkynyl, alkynylene, heterohydrocarbyl, or heterohydrocarbylene is optionally substituted with hydroxyl, an ester group, hydrocarbyloxy, hydrocarbyl, halogen, oxygen, sulfur, amino, or an amide group.

In some embodiments, the present disclosure provides the amino lipid compound of formula (II-I), (II-Ia), (II-Ib), (II-Ic), (II-Id), (II-Ie), (II-II), (II-IIa), (II-IIb), (II-III), (II-IIIa), (II-IIIb), (II-IVa), (II-IVb), (II-Va), (II-Vb), (II-Vc), (II-Vd), (II-Ve), (II-Vf), (II-Vg), (II-Vh), (II-VIa), (II-VIa-a), (II-VIa-b), (II-VIa-c) (II-VIb), (II-VIb-a), (II-VIb-b), or (II-VIb-c), or a pharmaceutically acceptable salt thereof, or a stereoisomer thereof, as described above, wherein the amino lipid compound has one of the structures shown in Table 1 below.

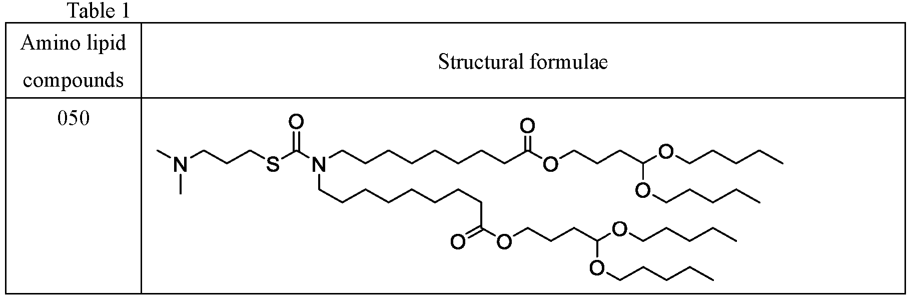

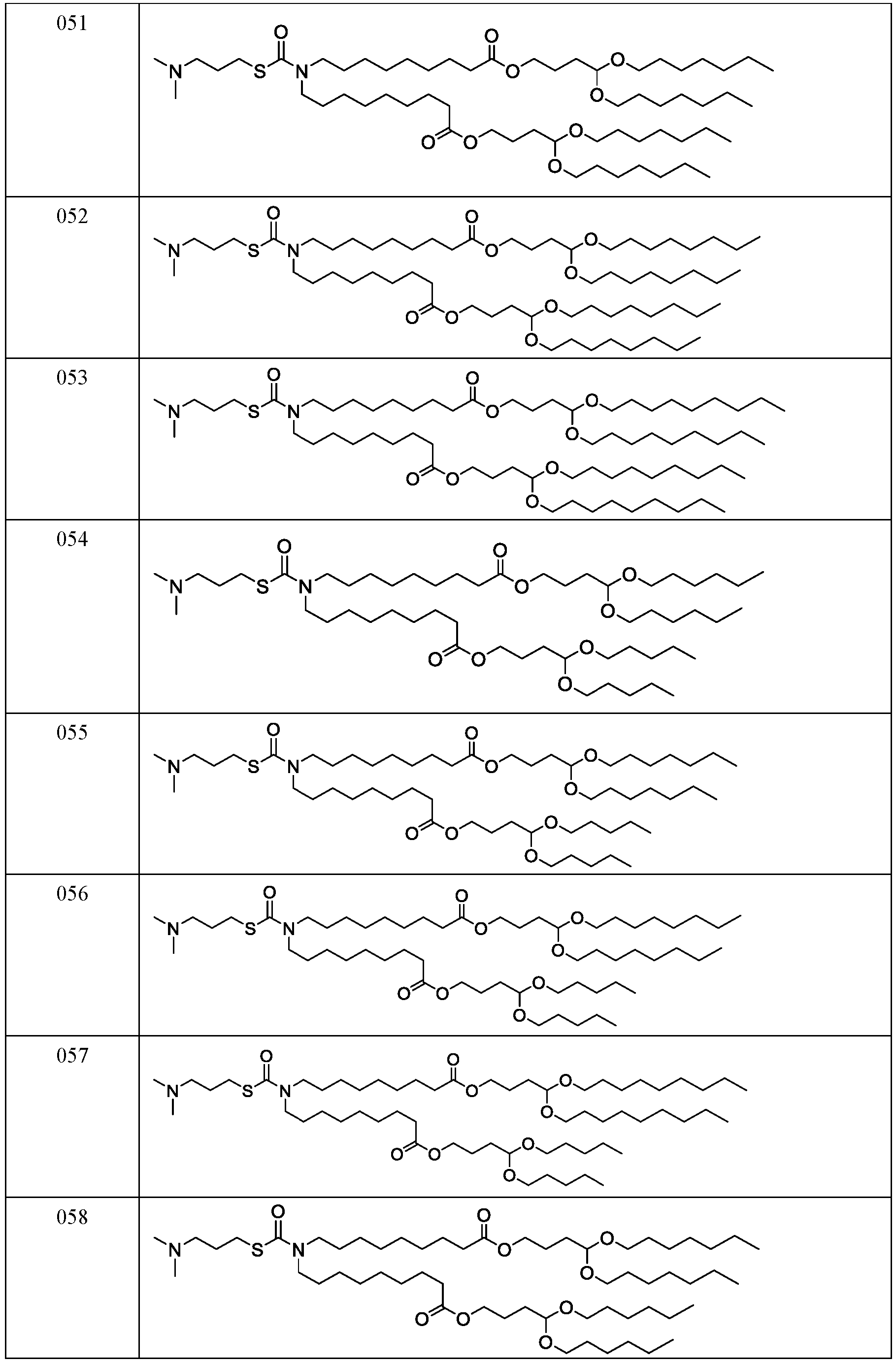

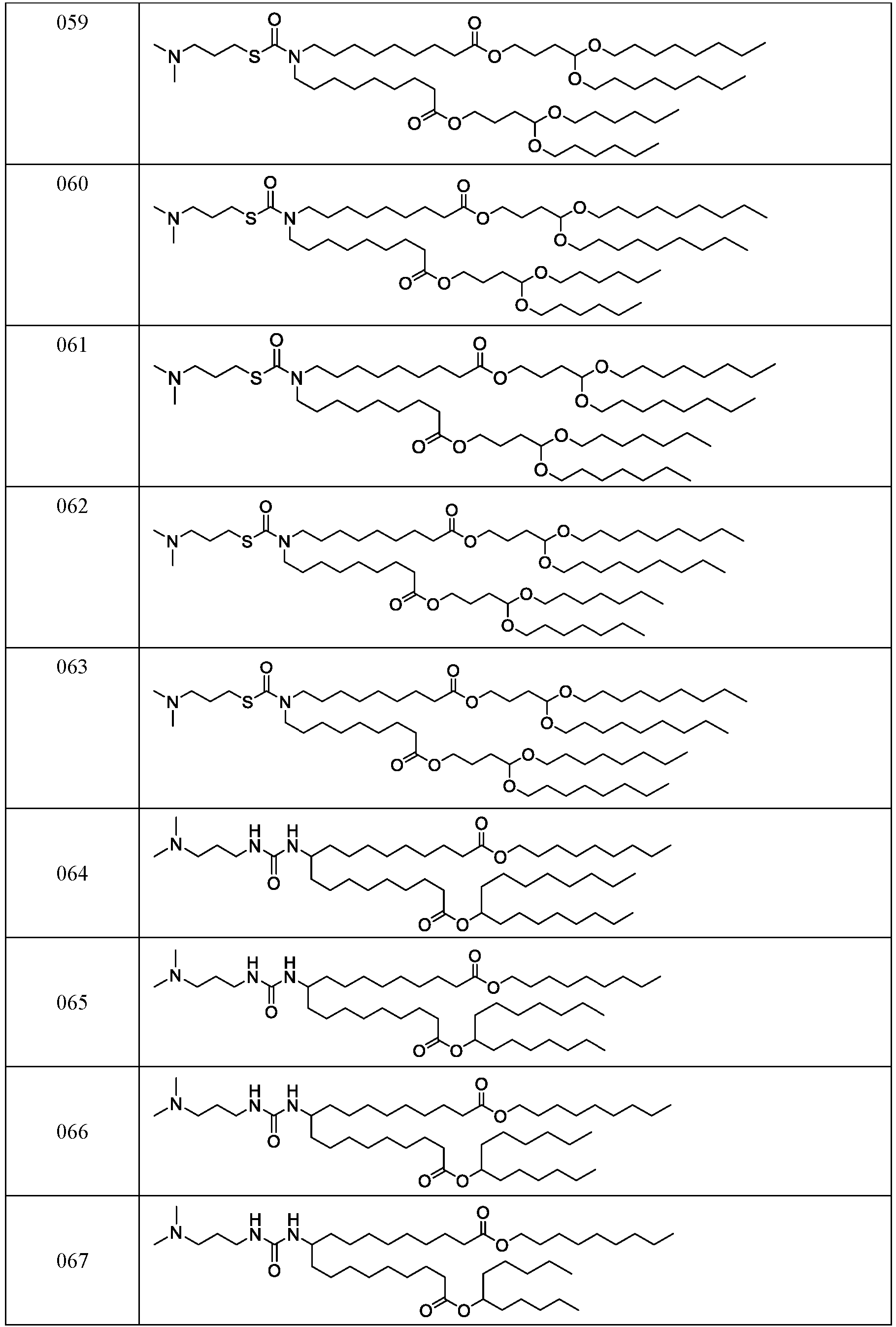

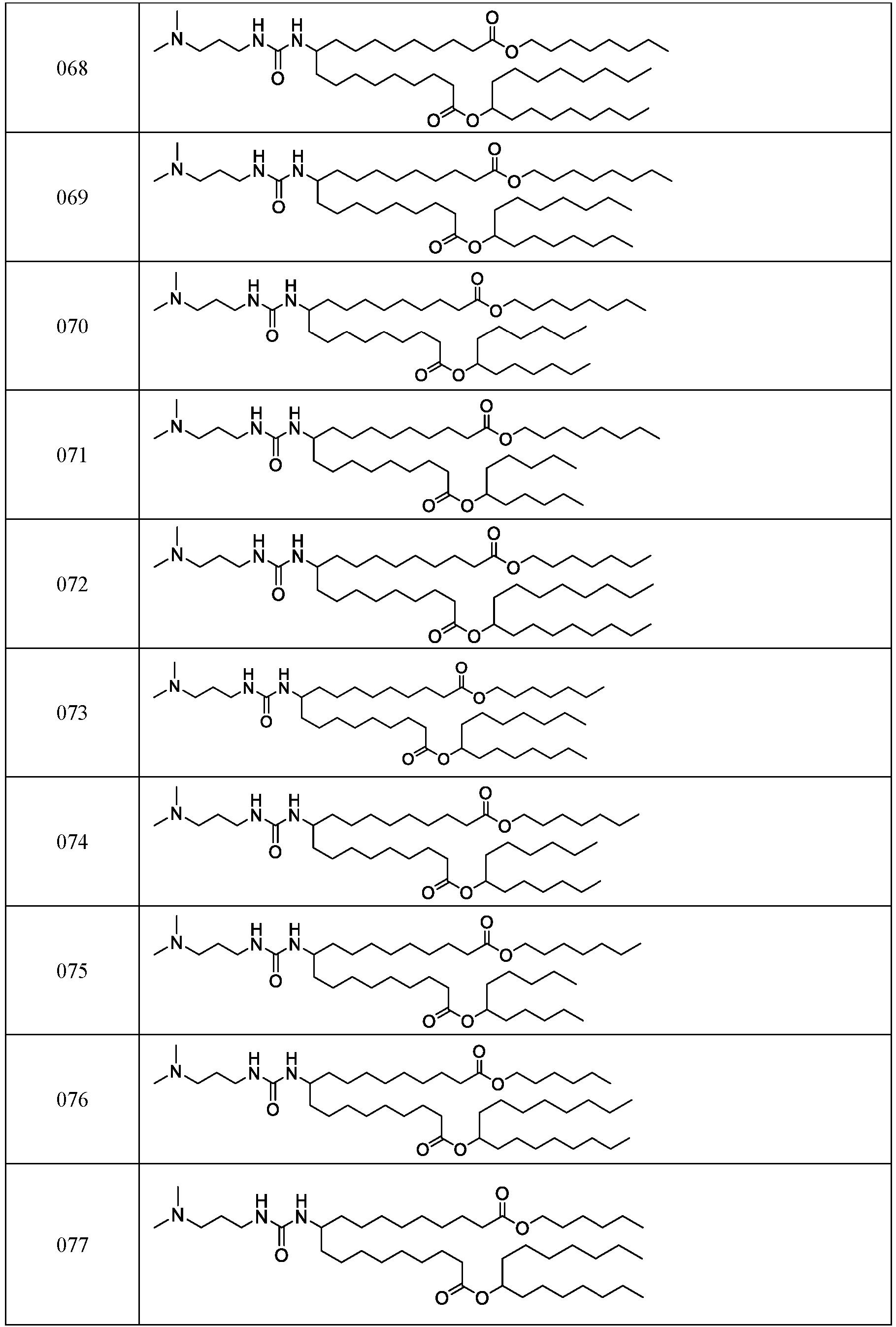

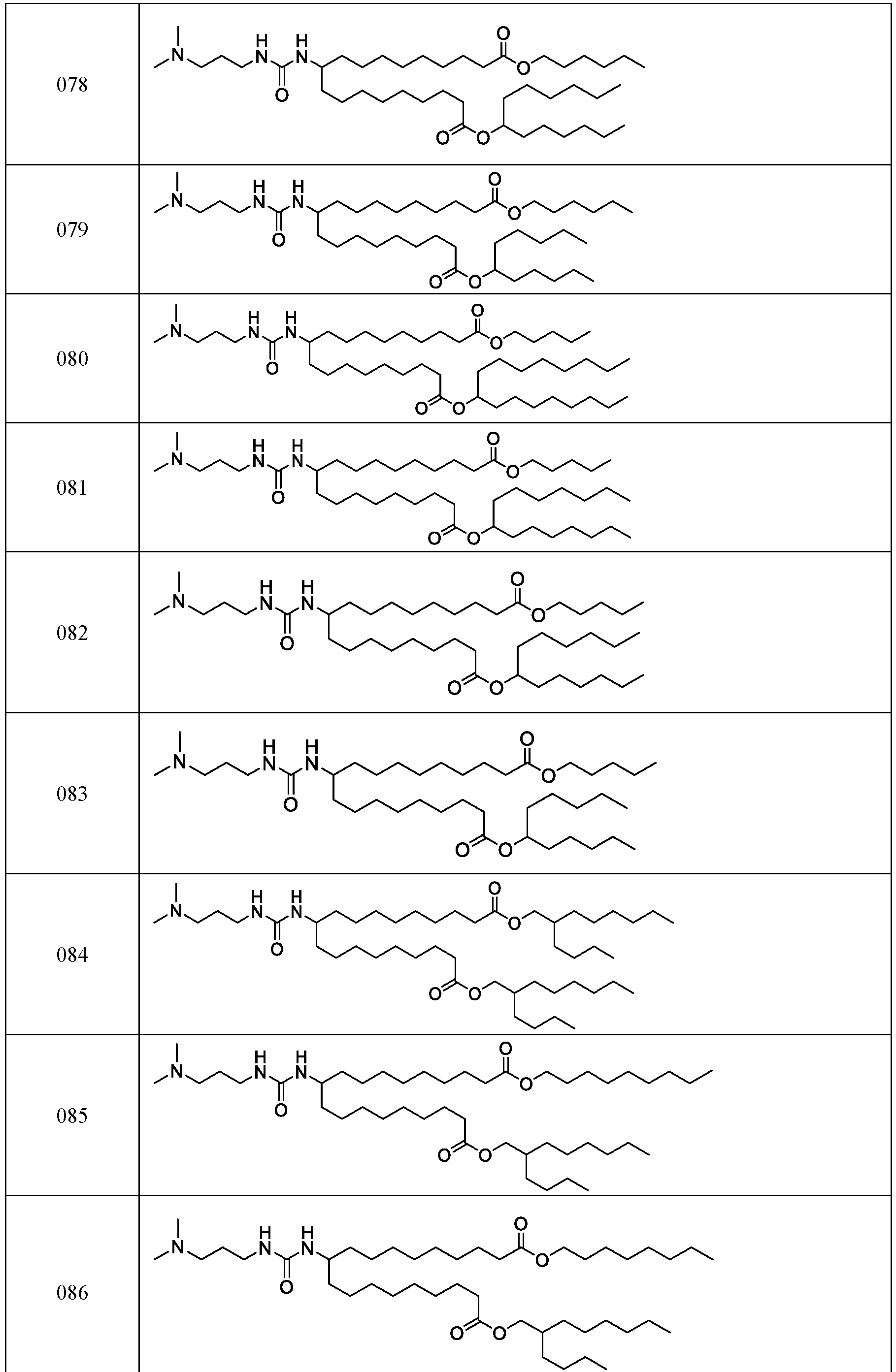

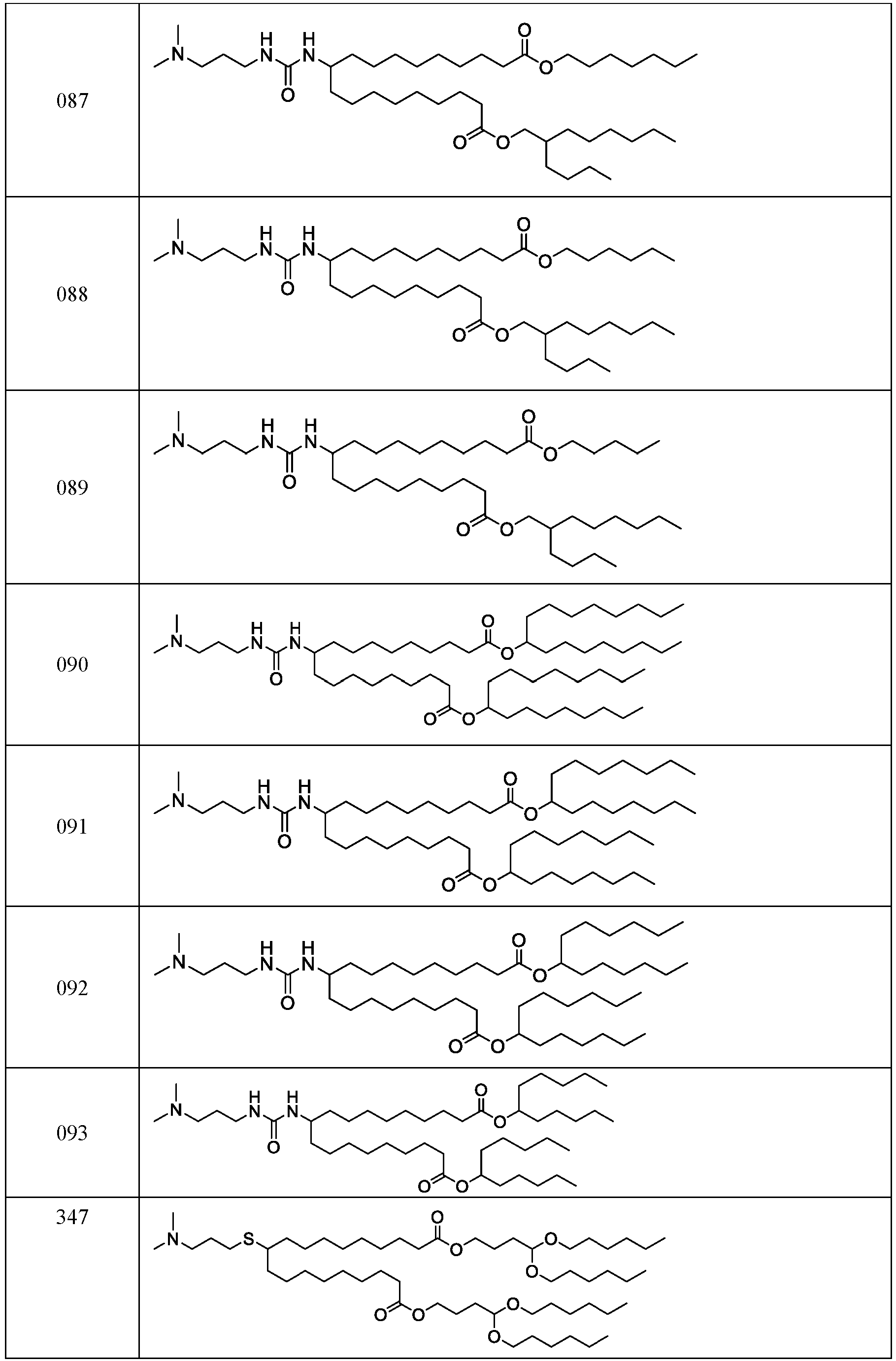

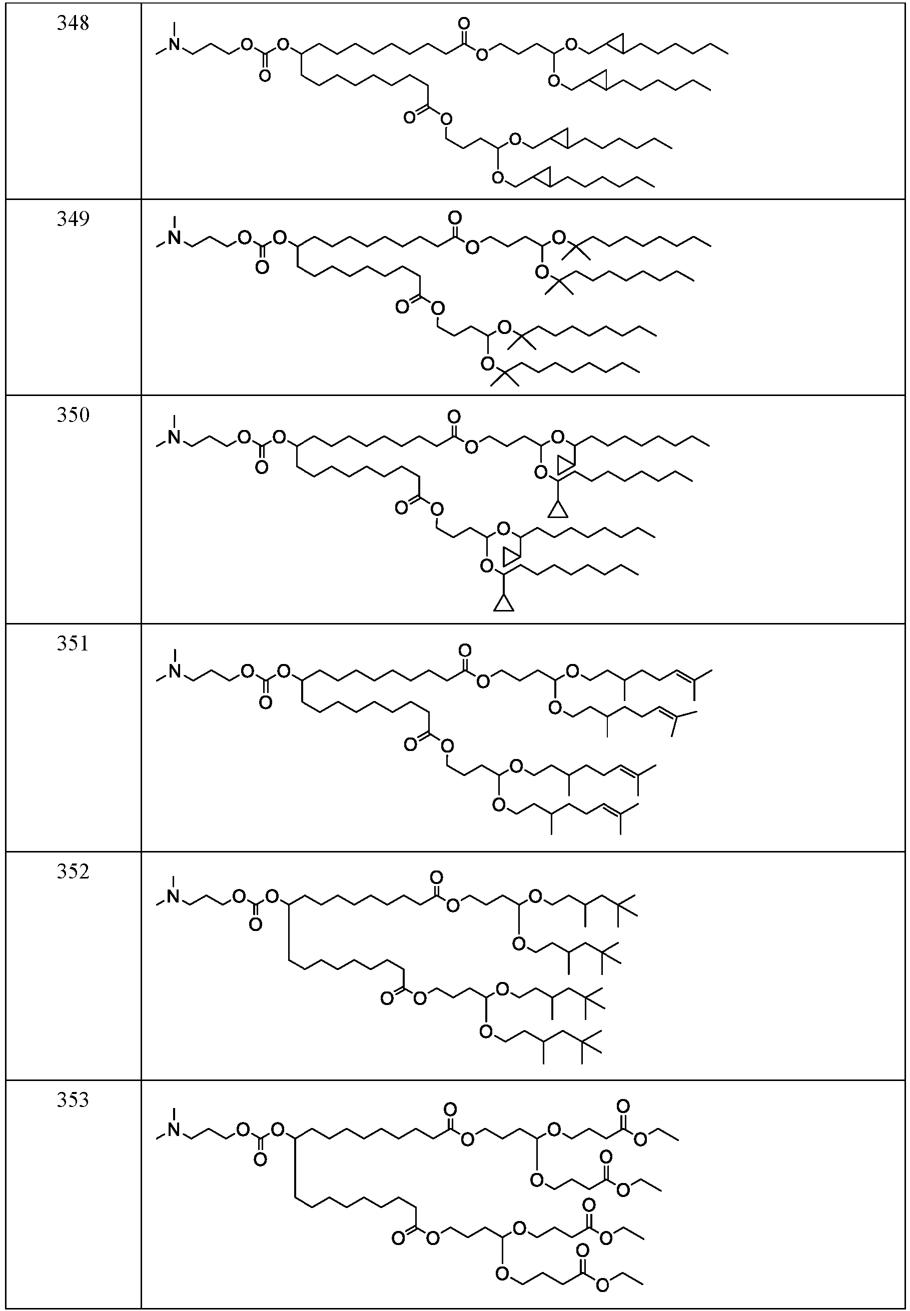

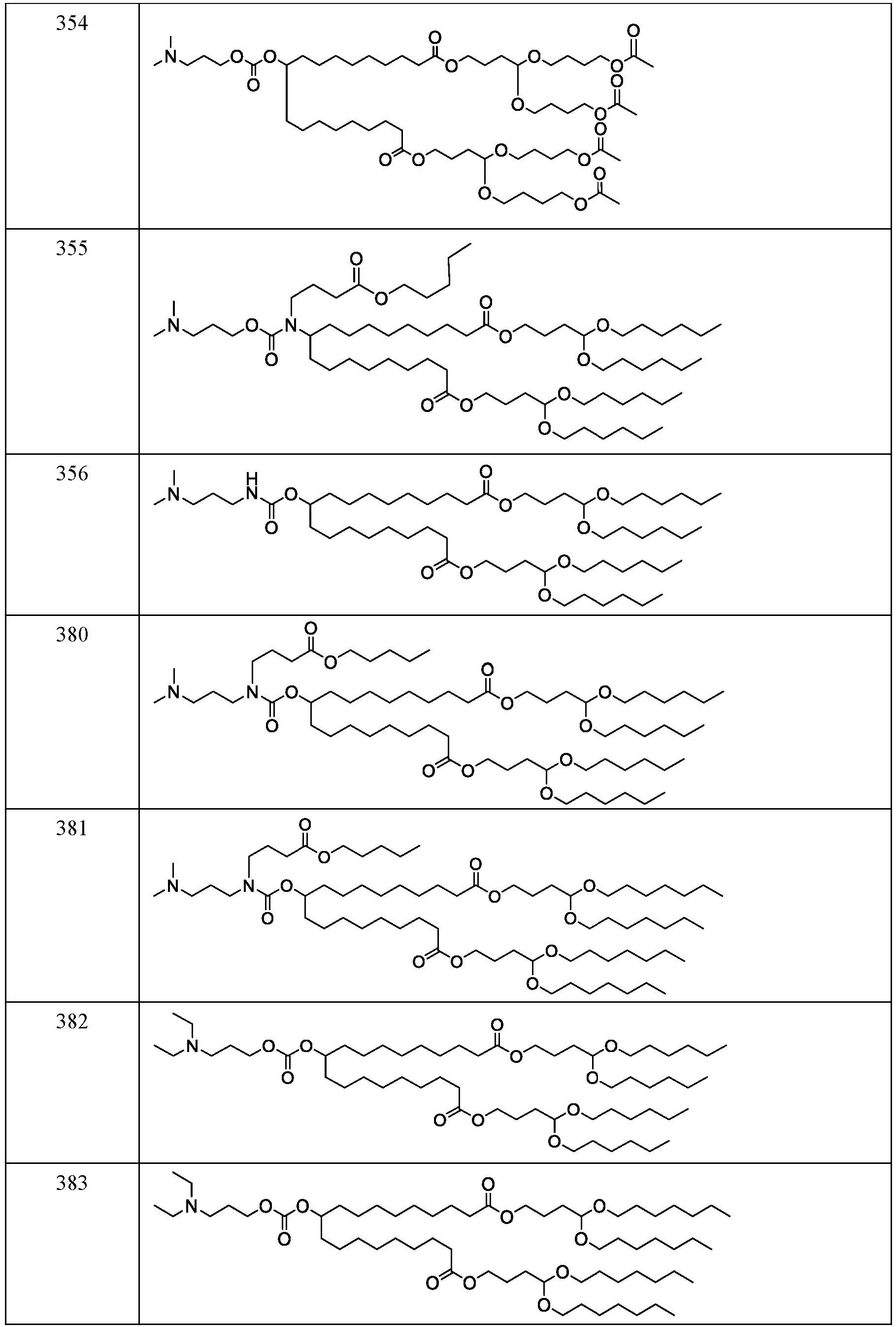

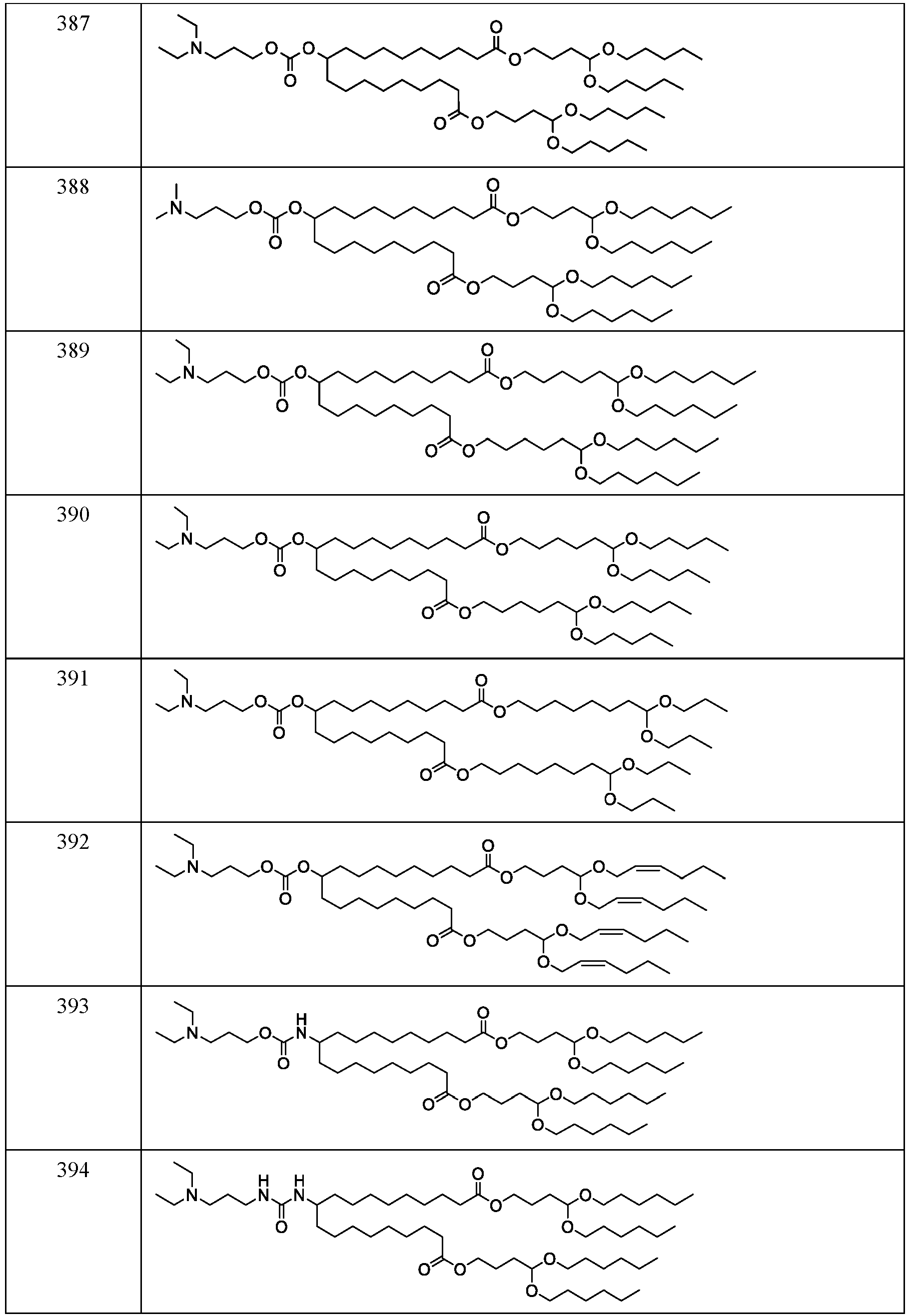

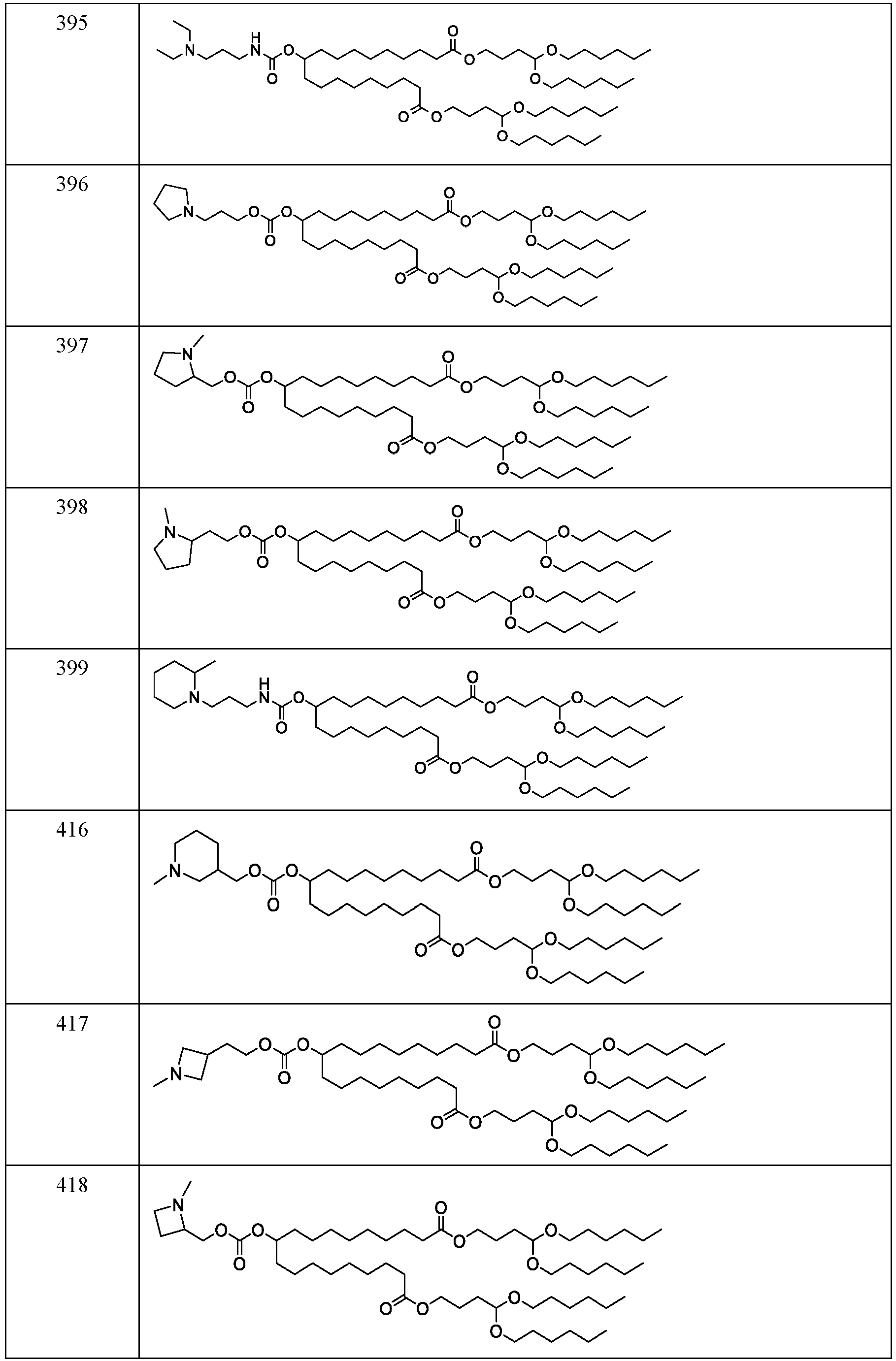

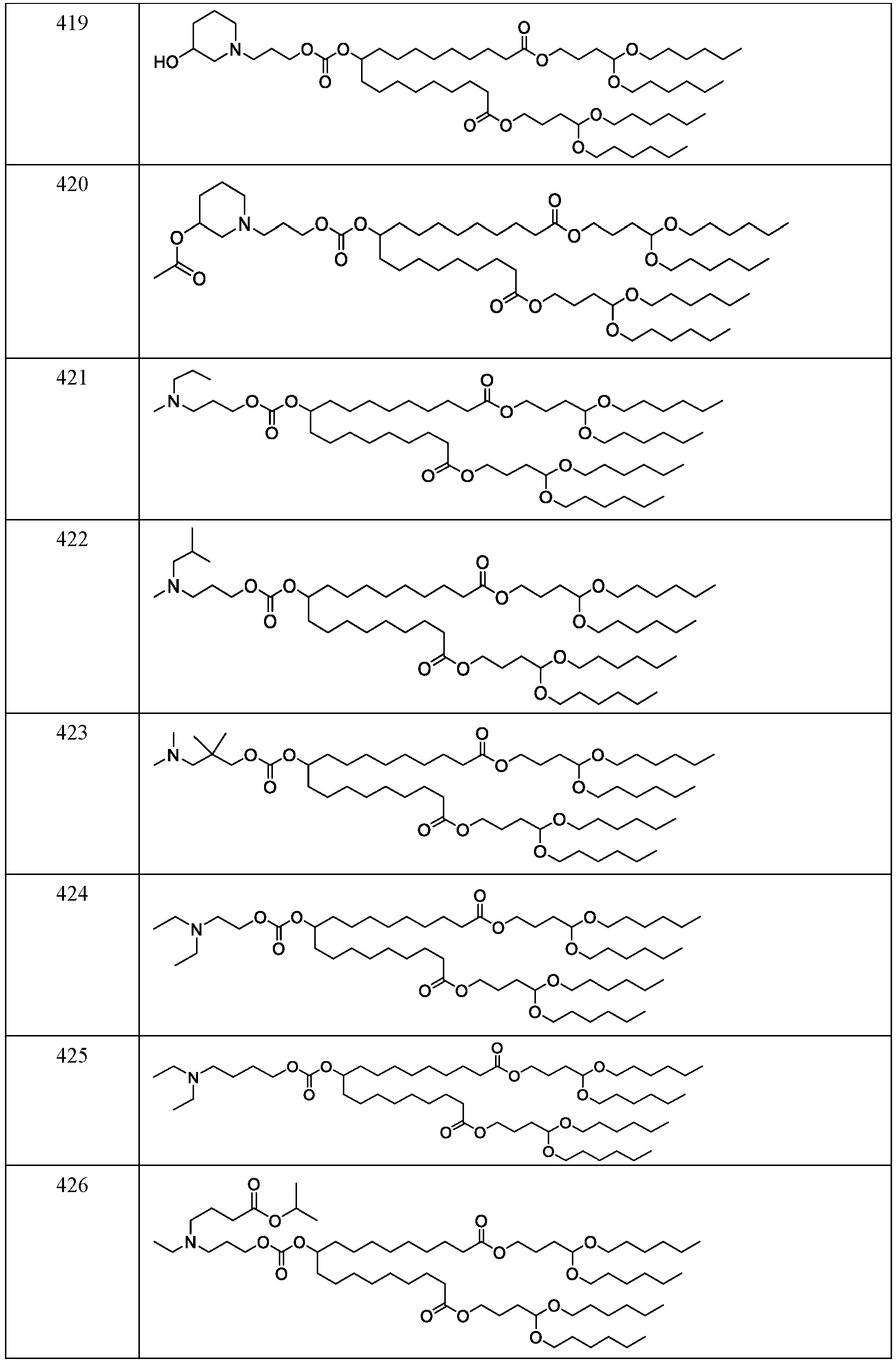

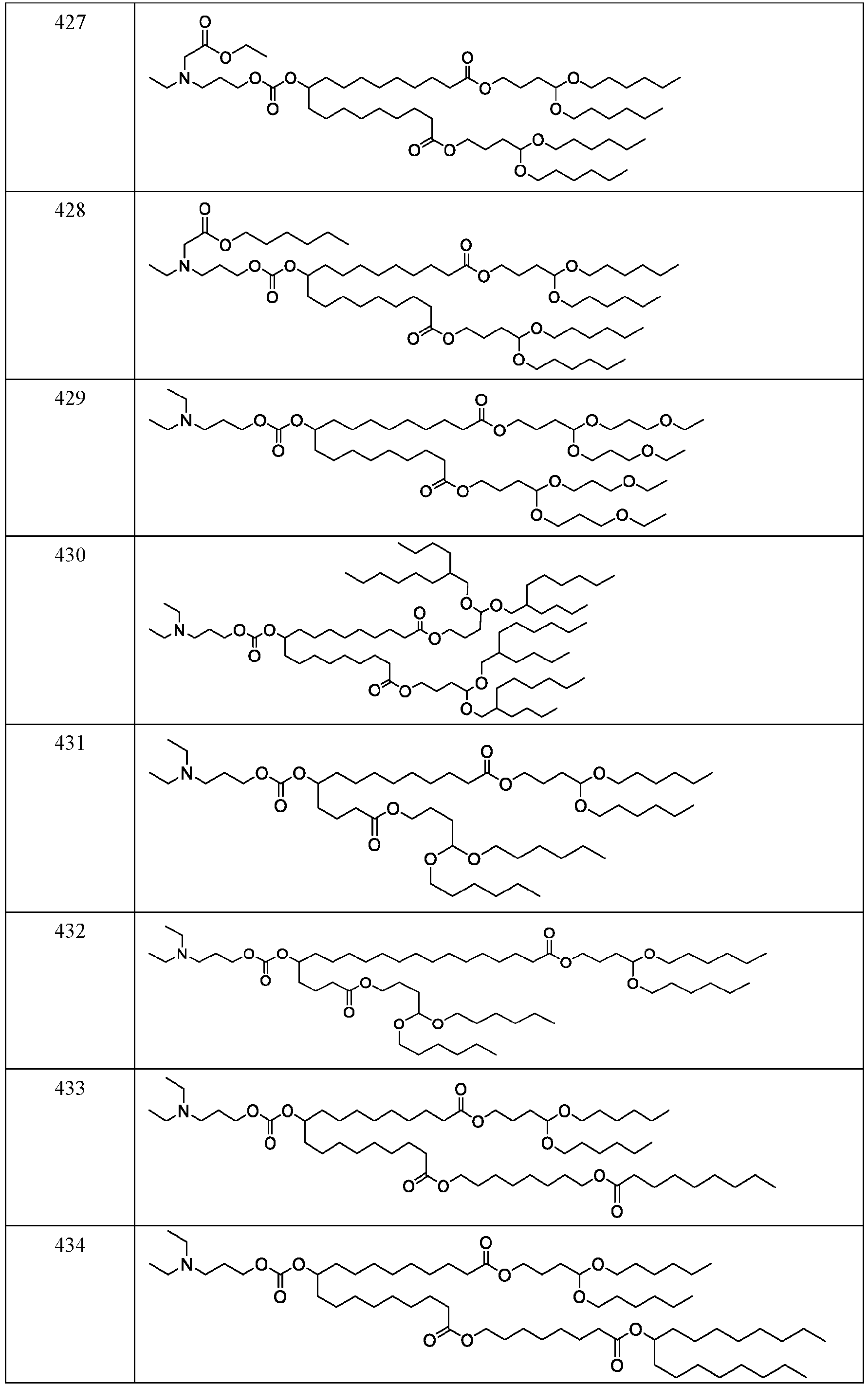

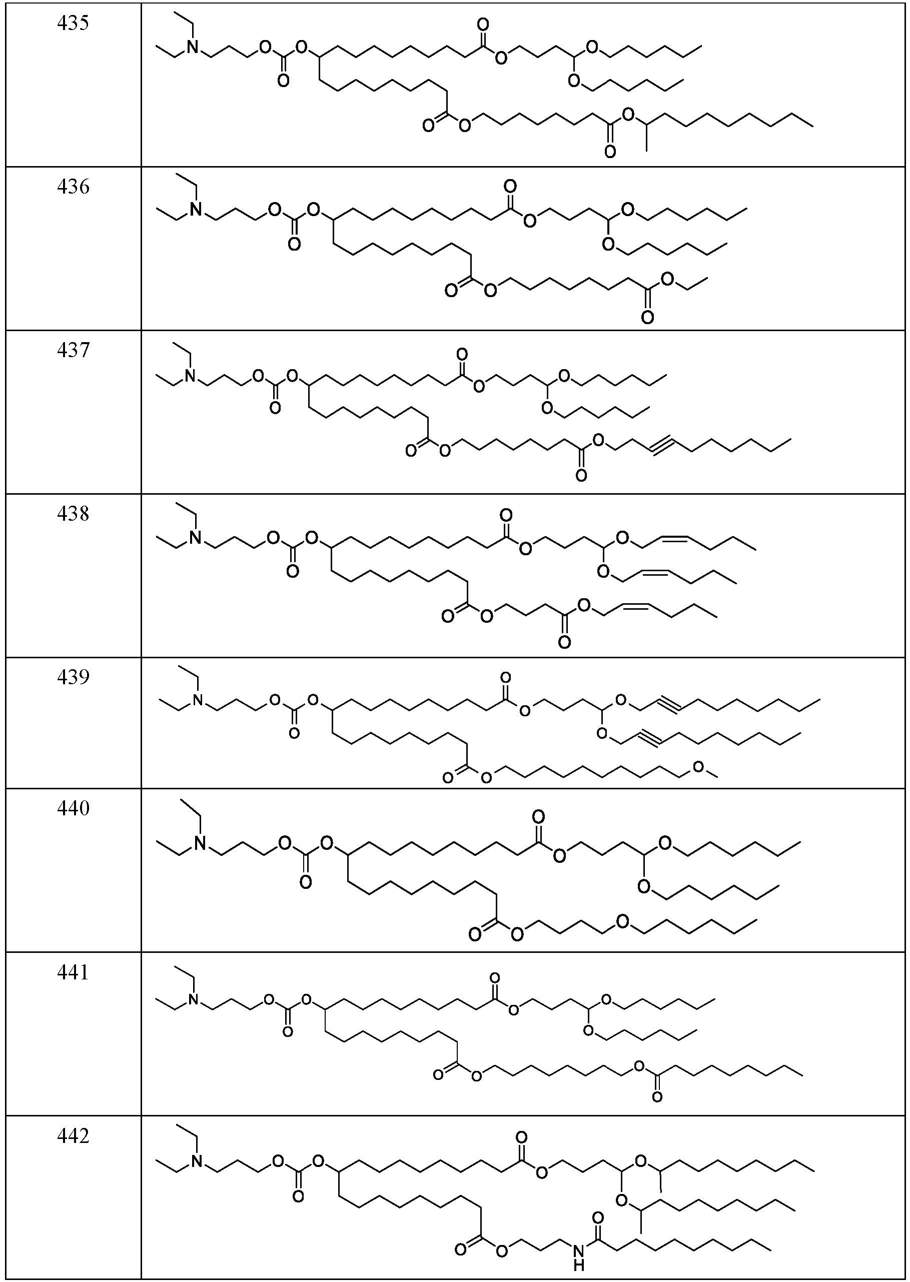

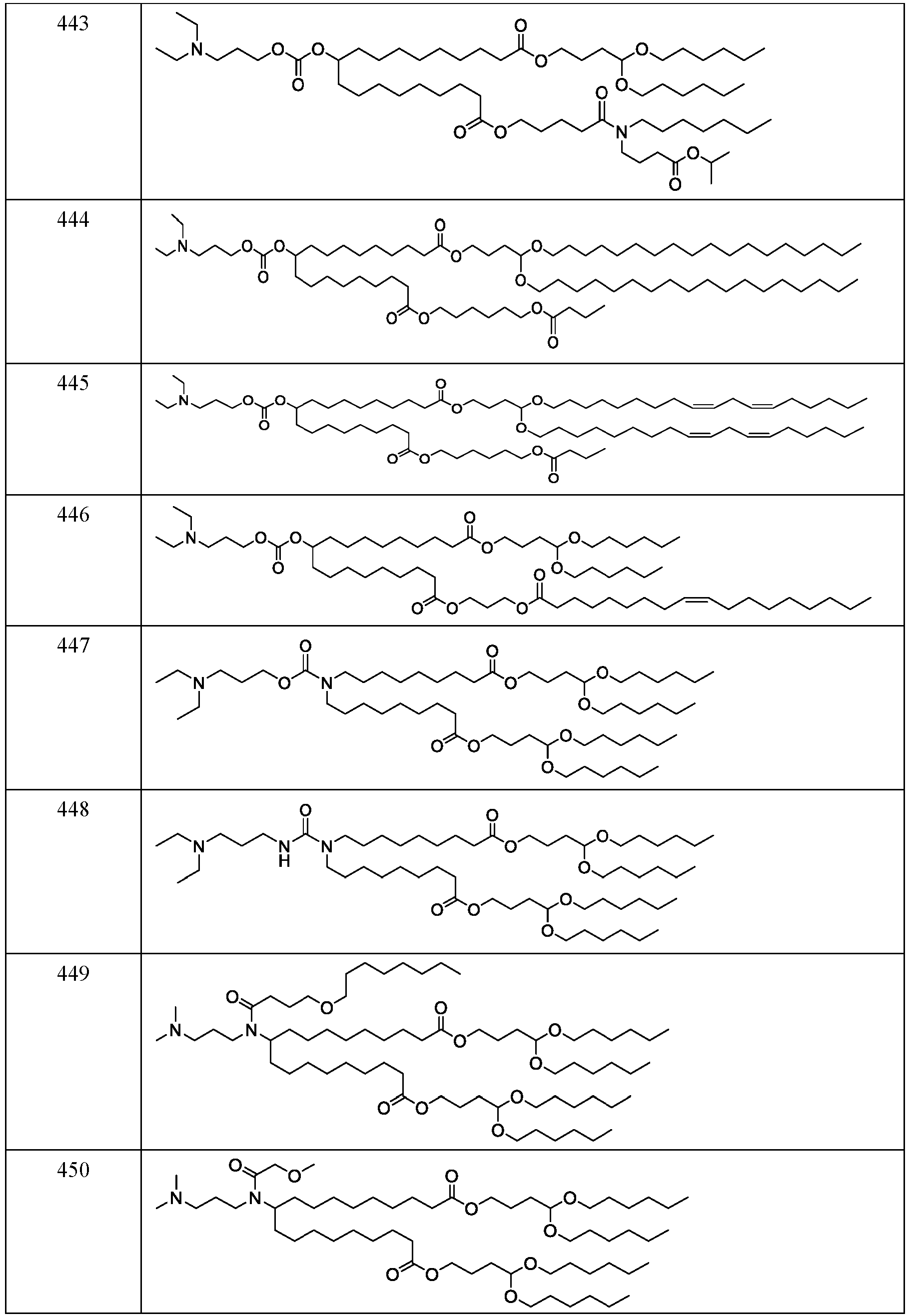

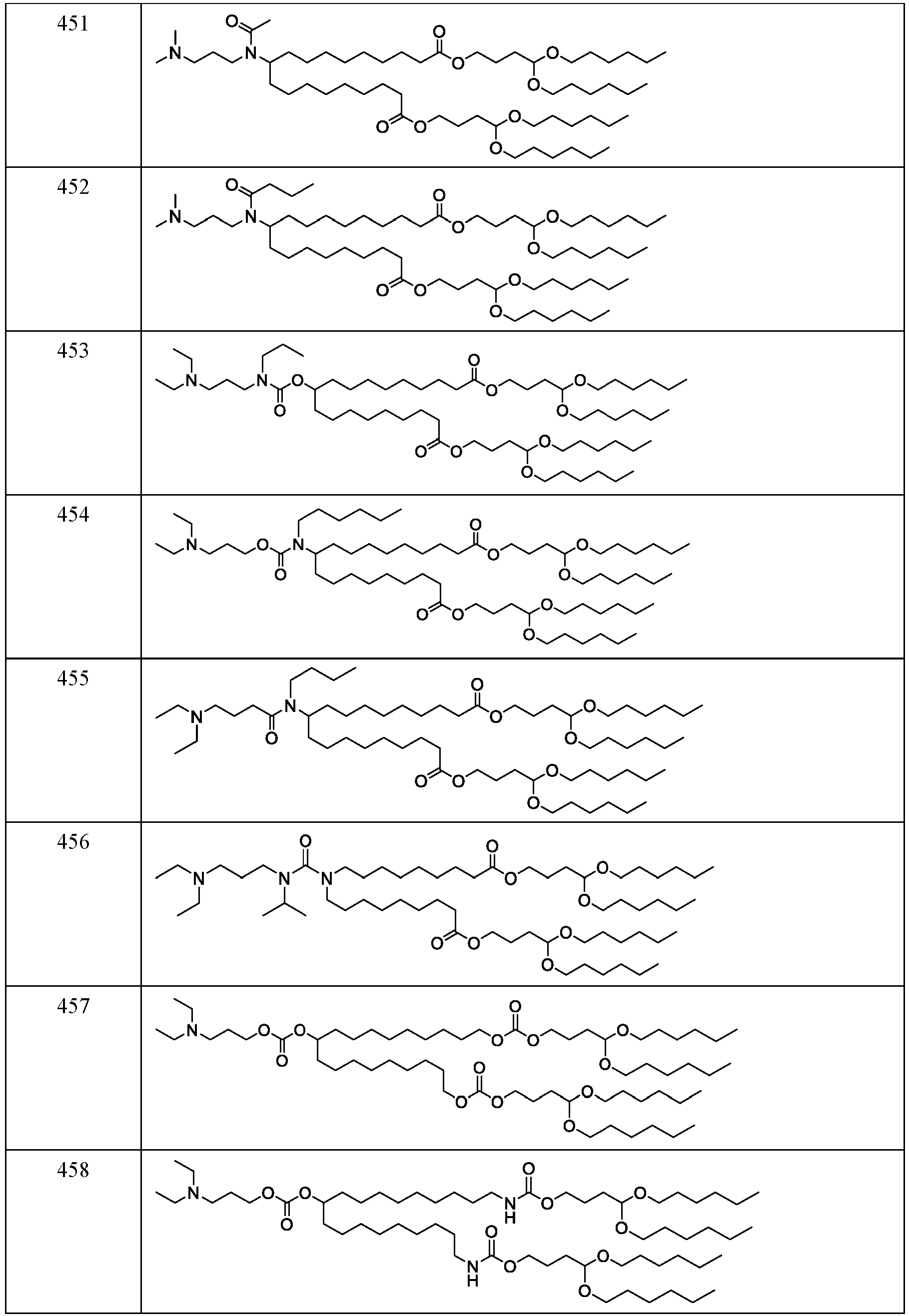

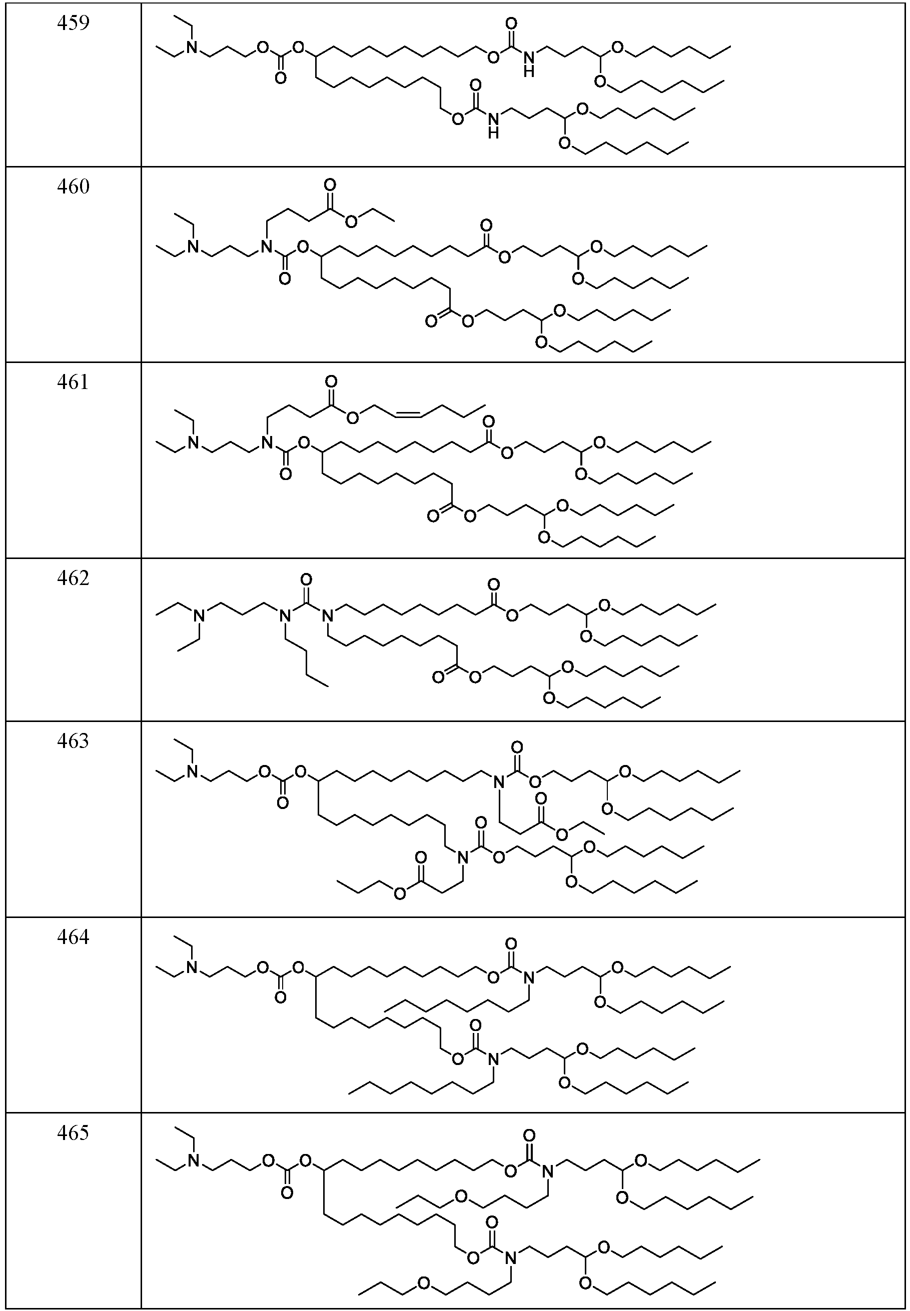

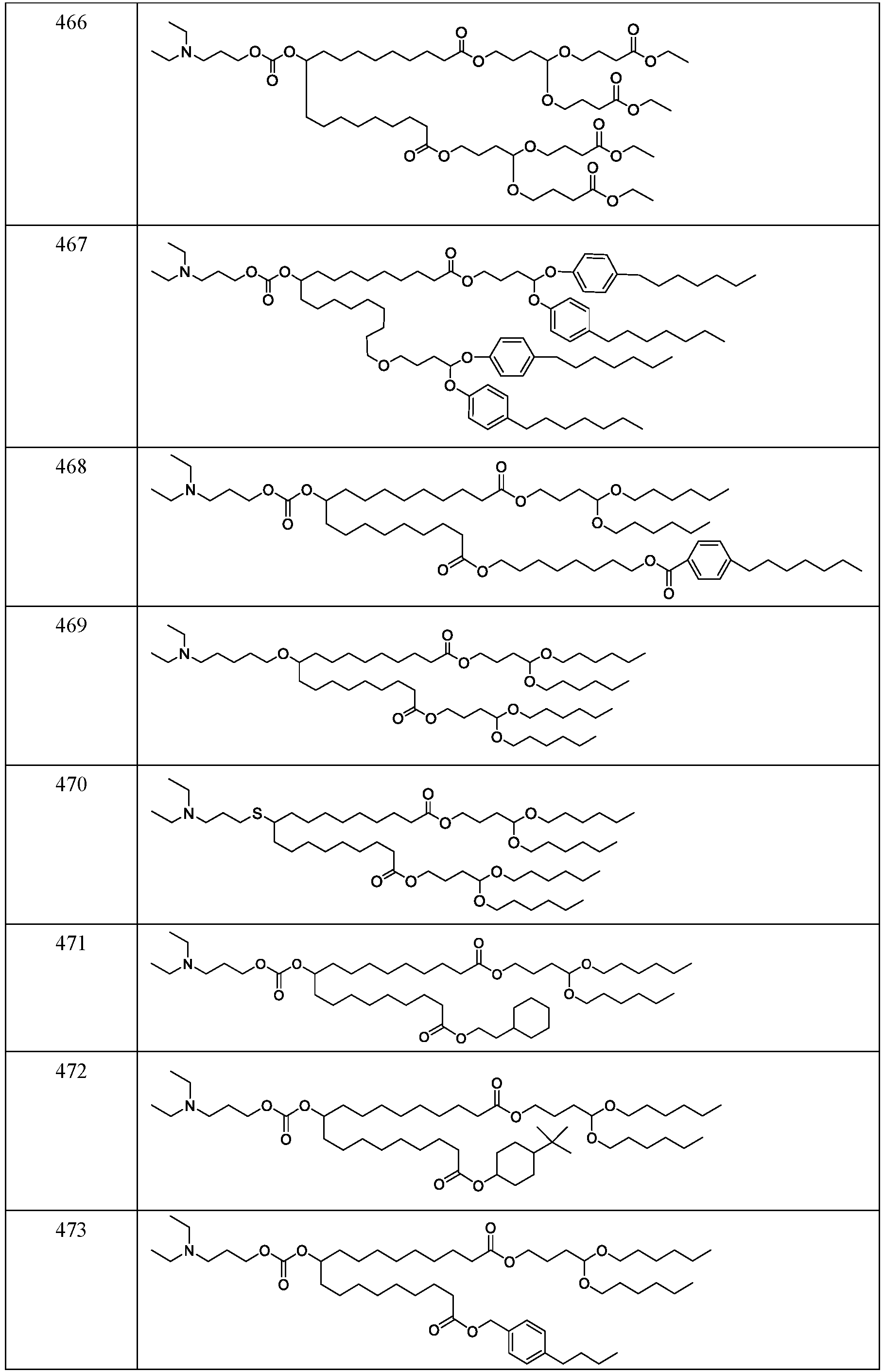

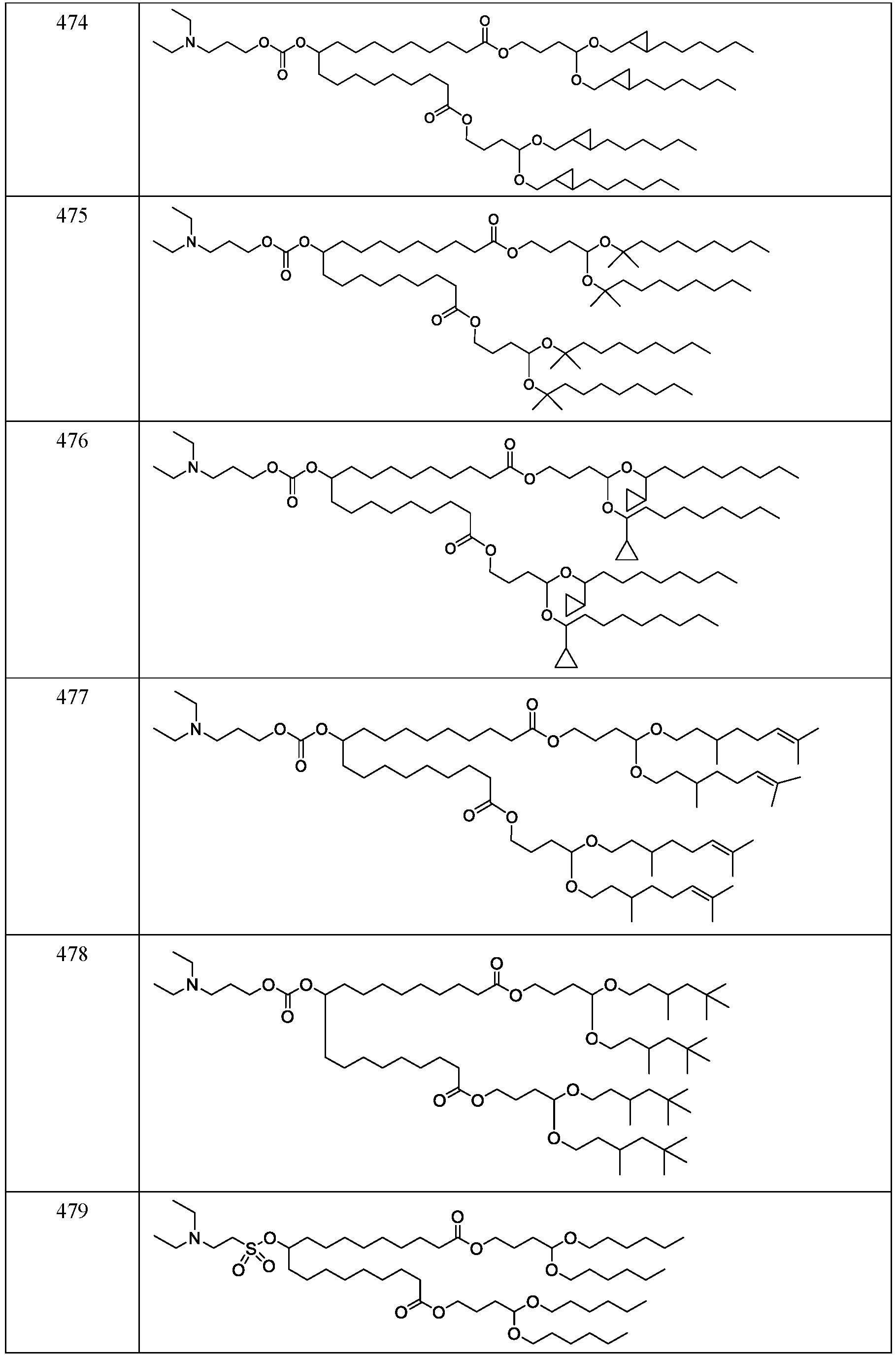

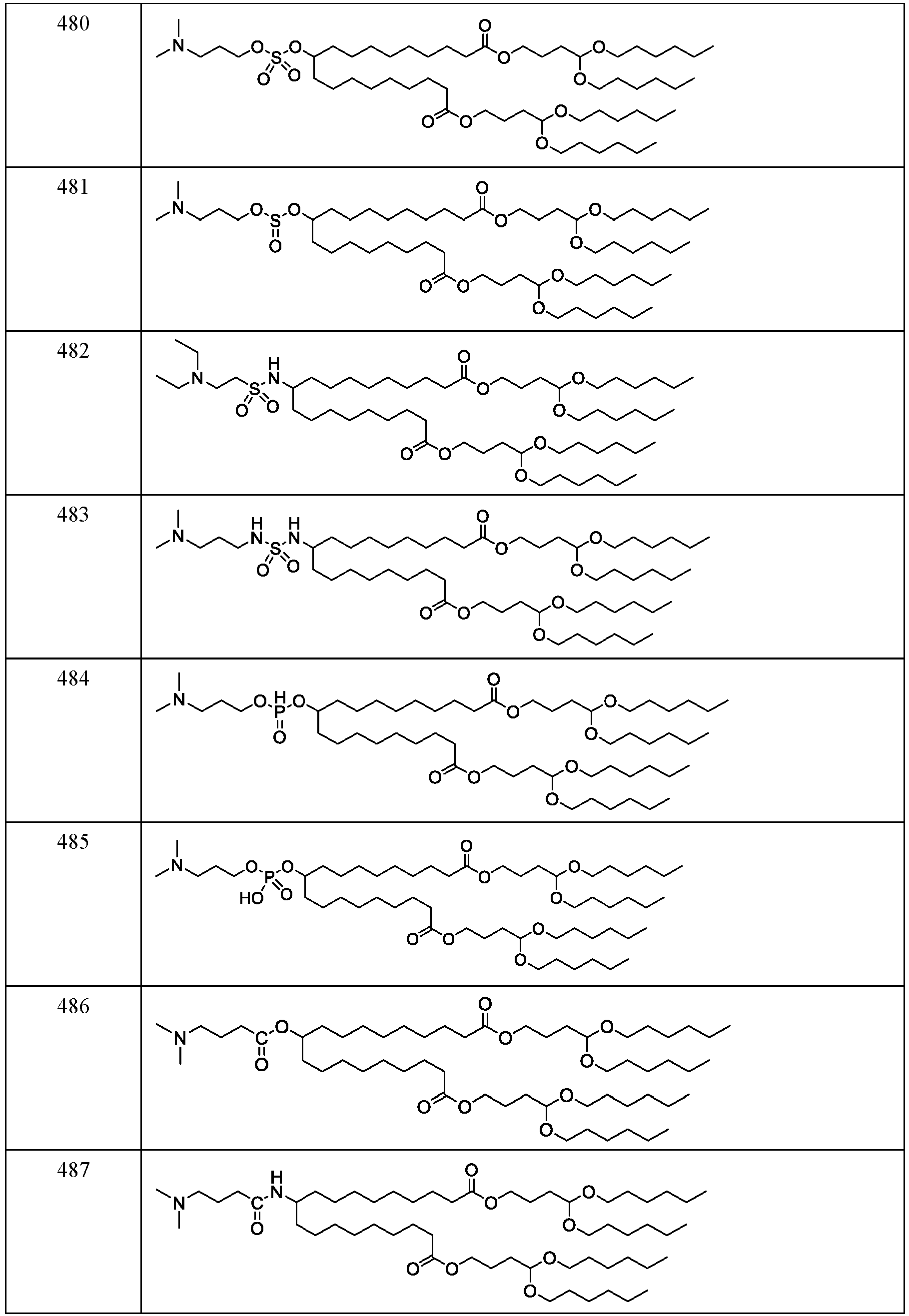

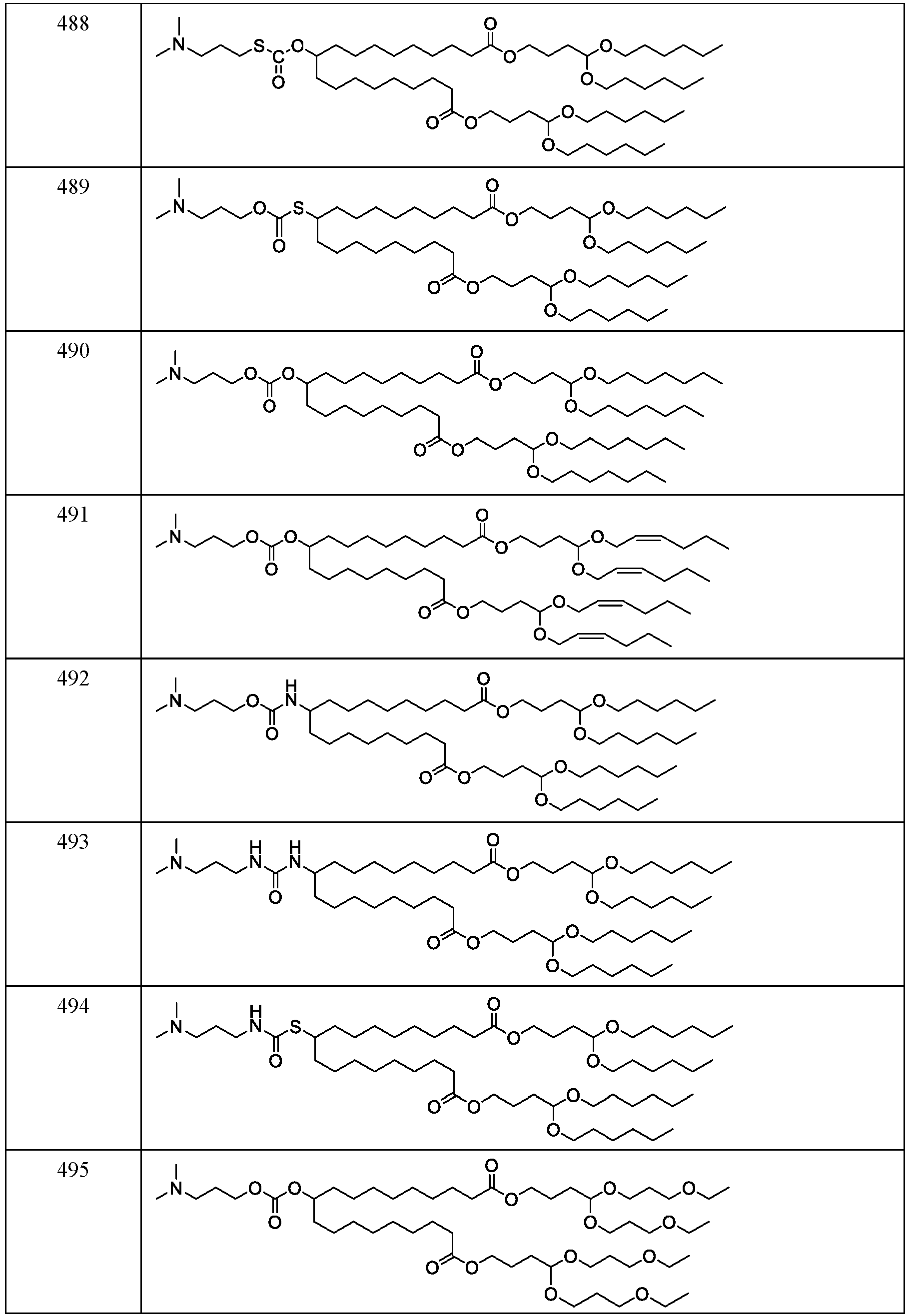

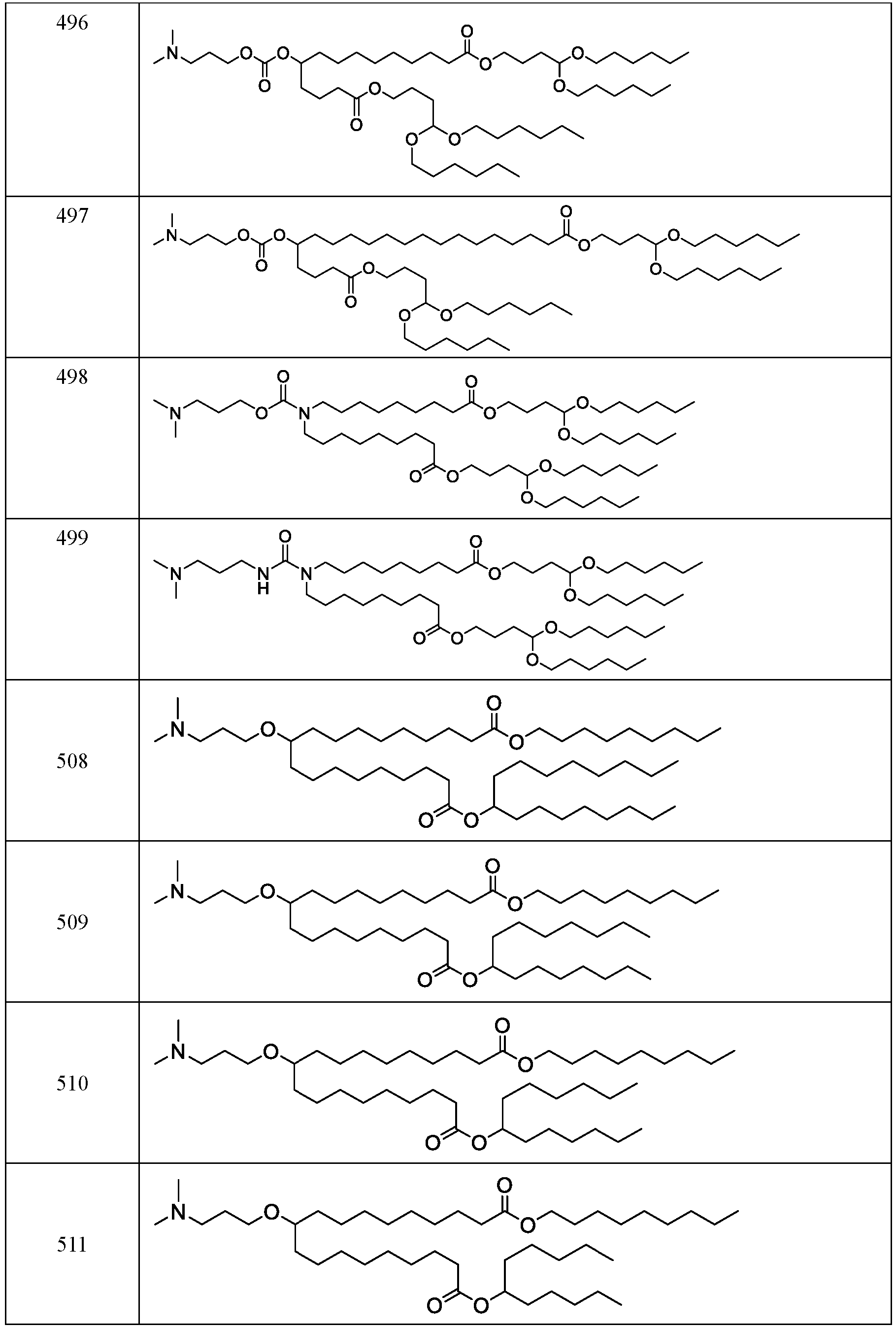

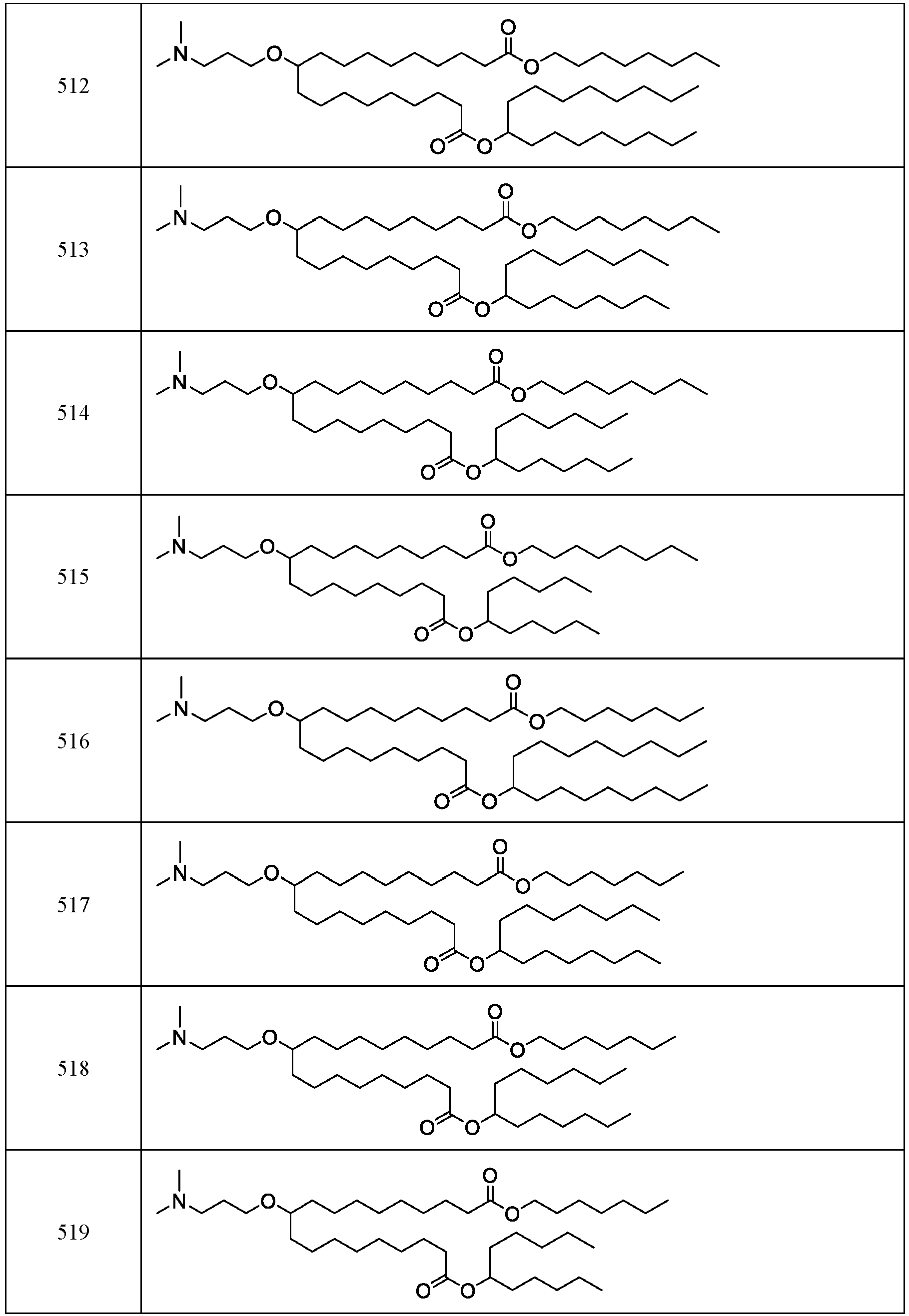

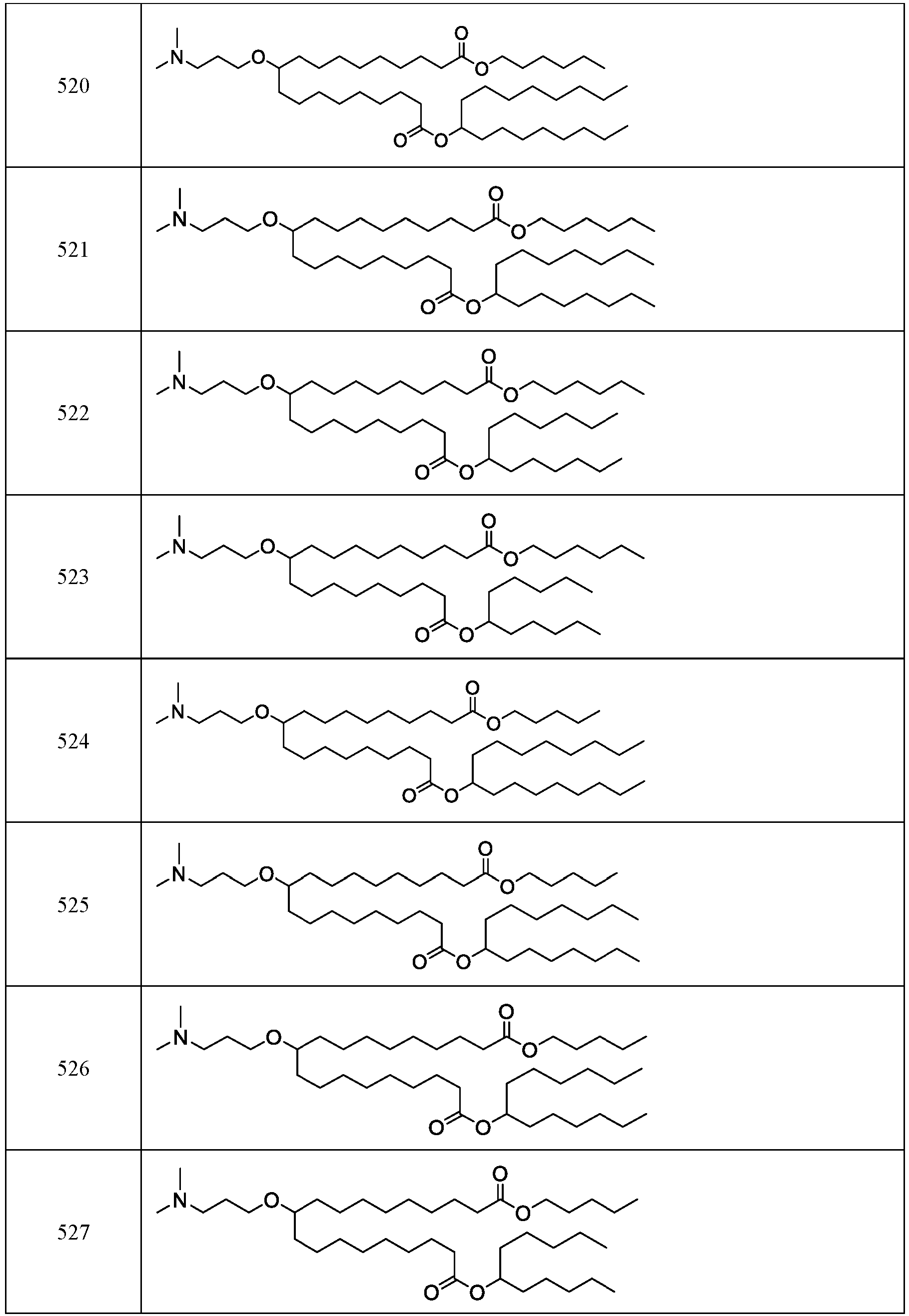

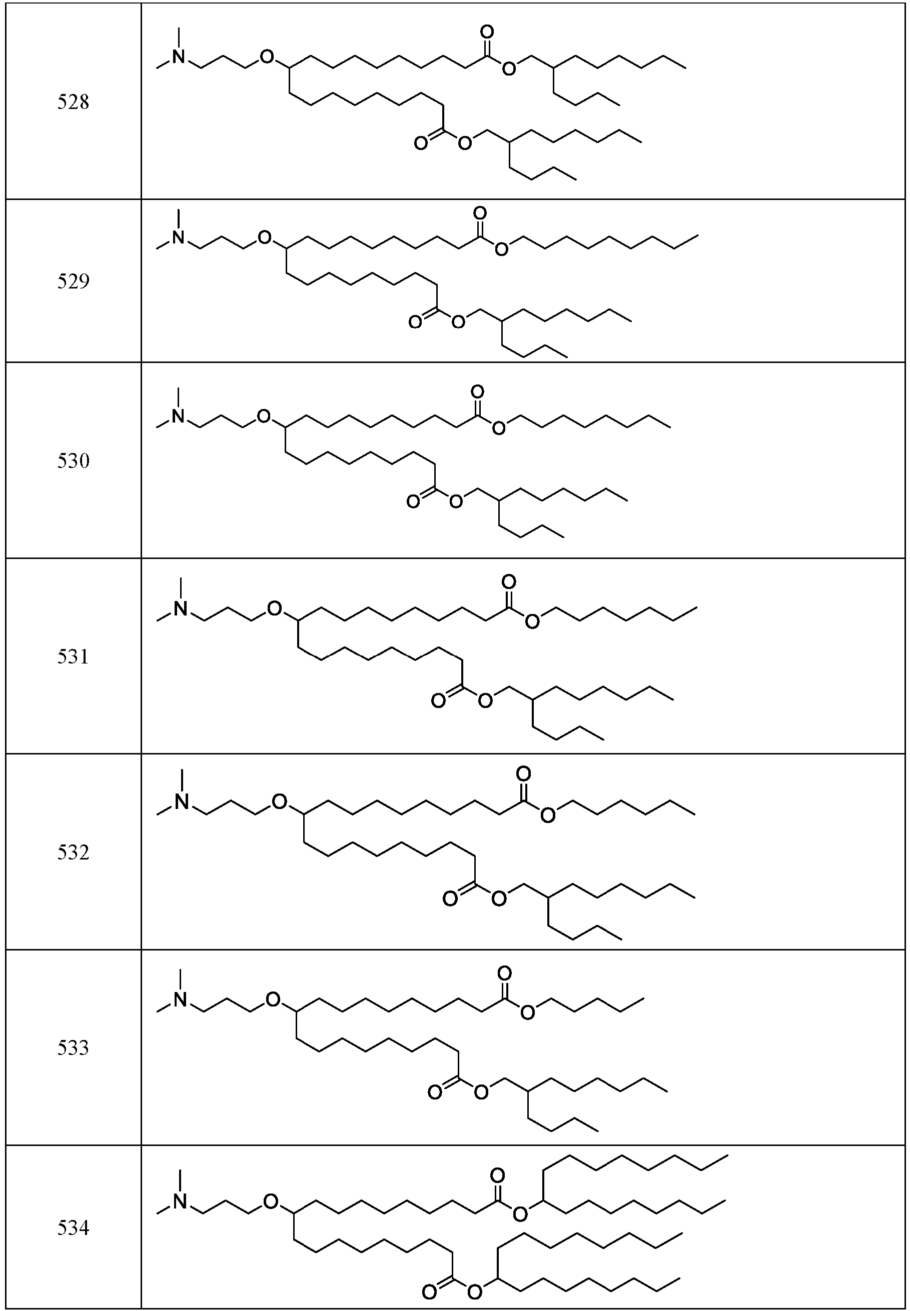

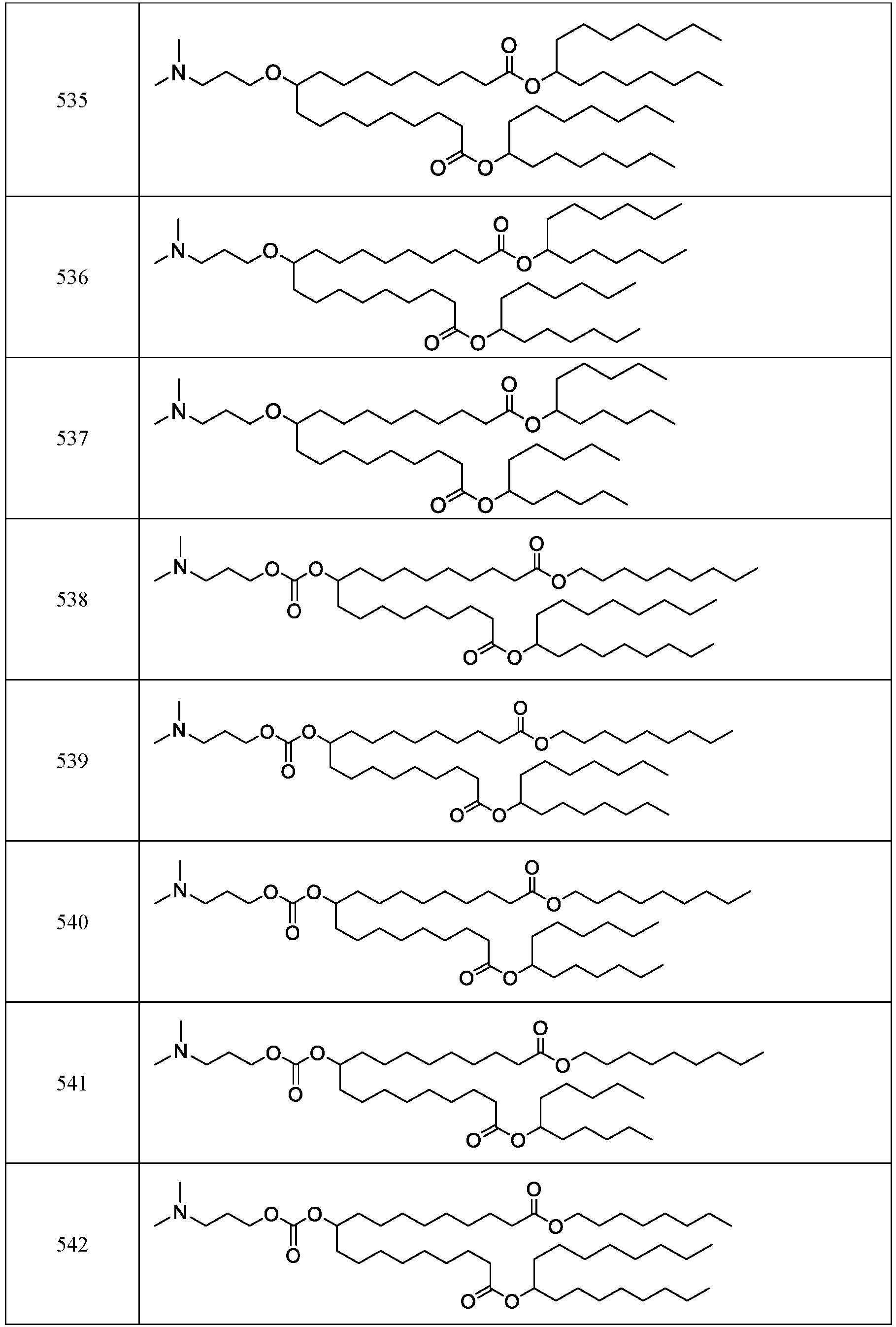

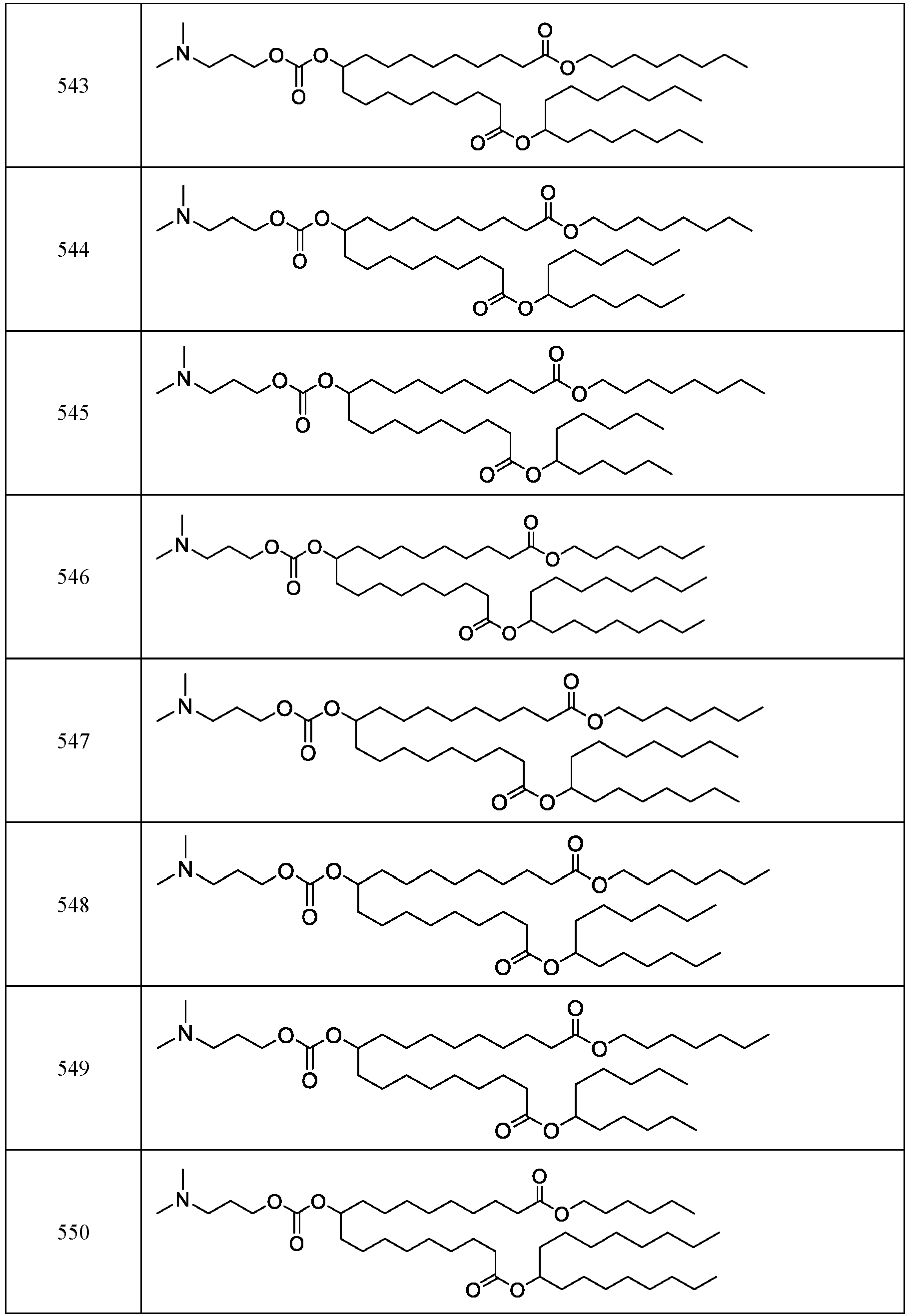

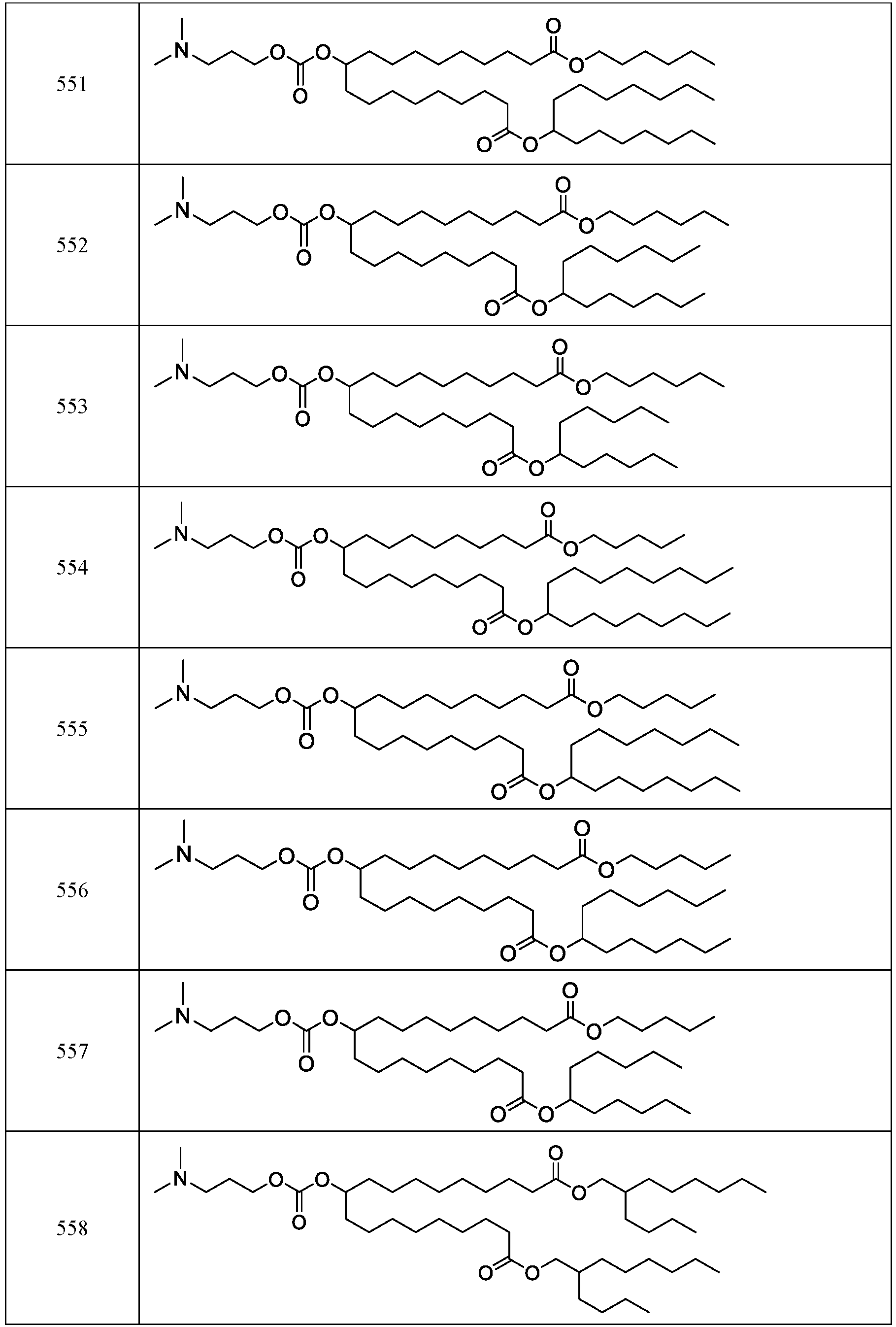

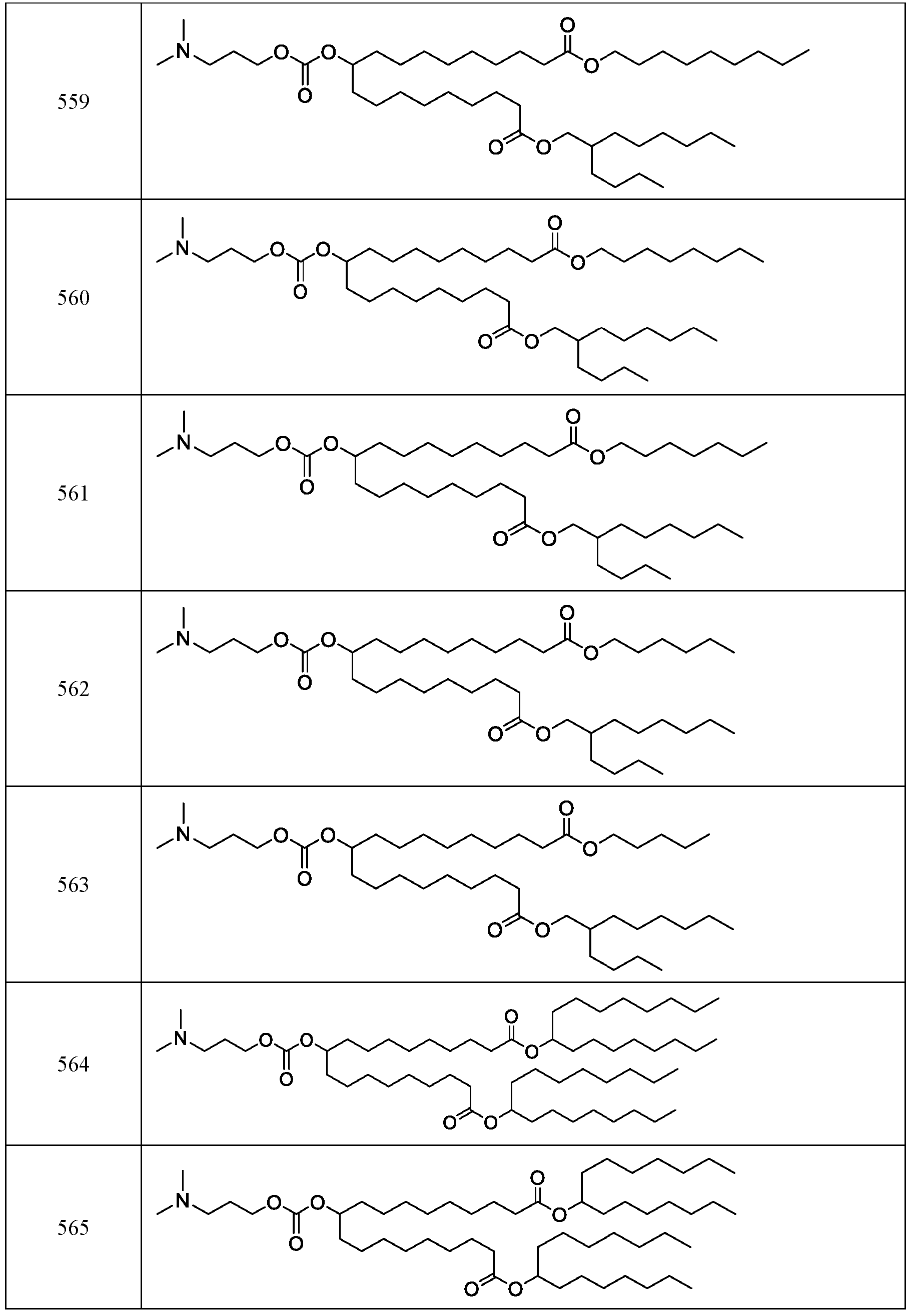

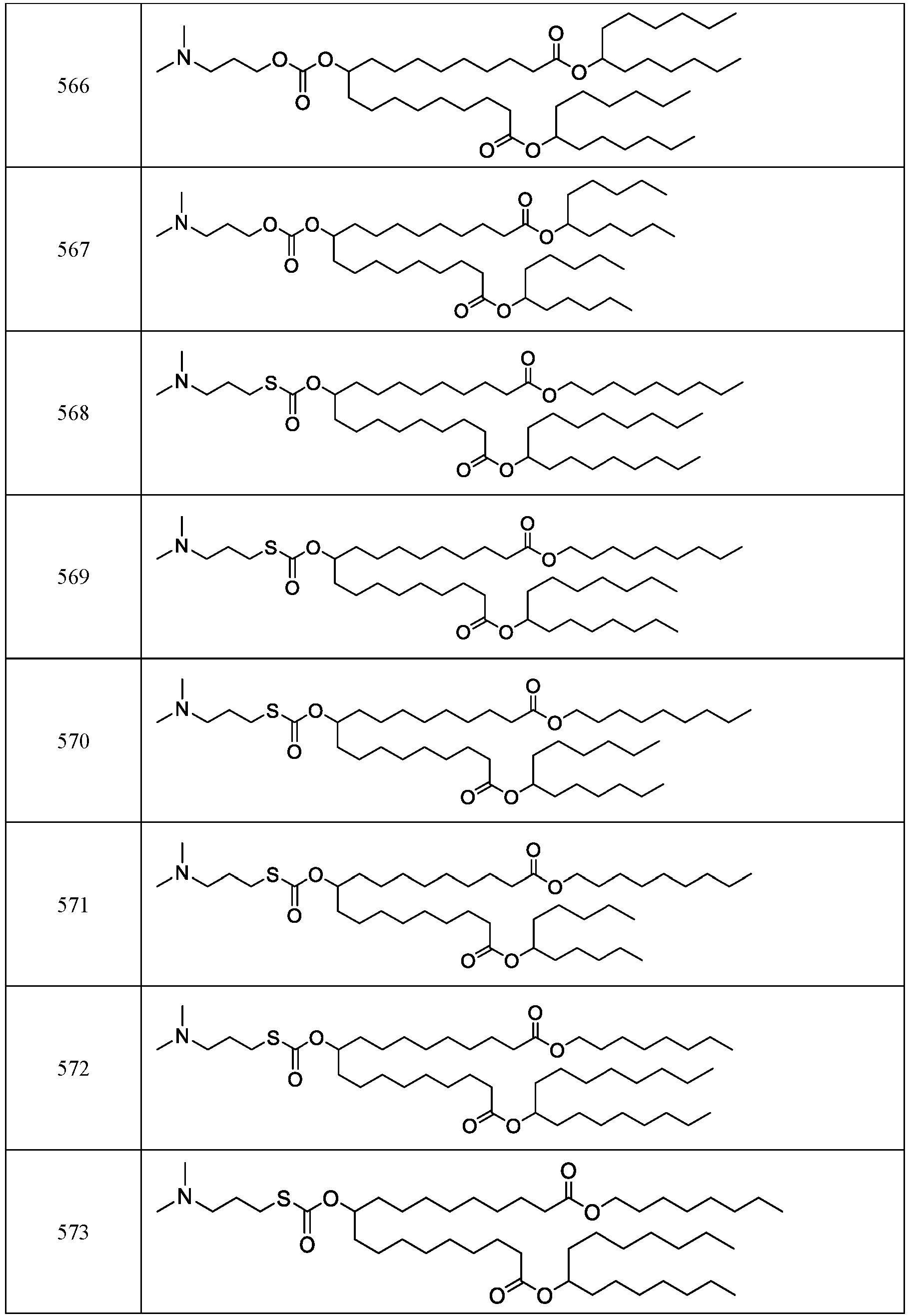

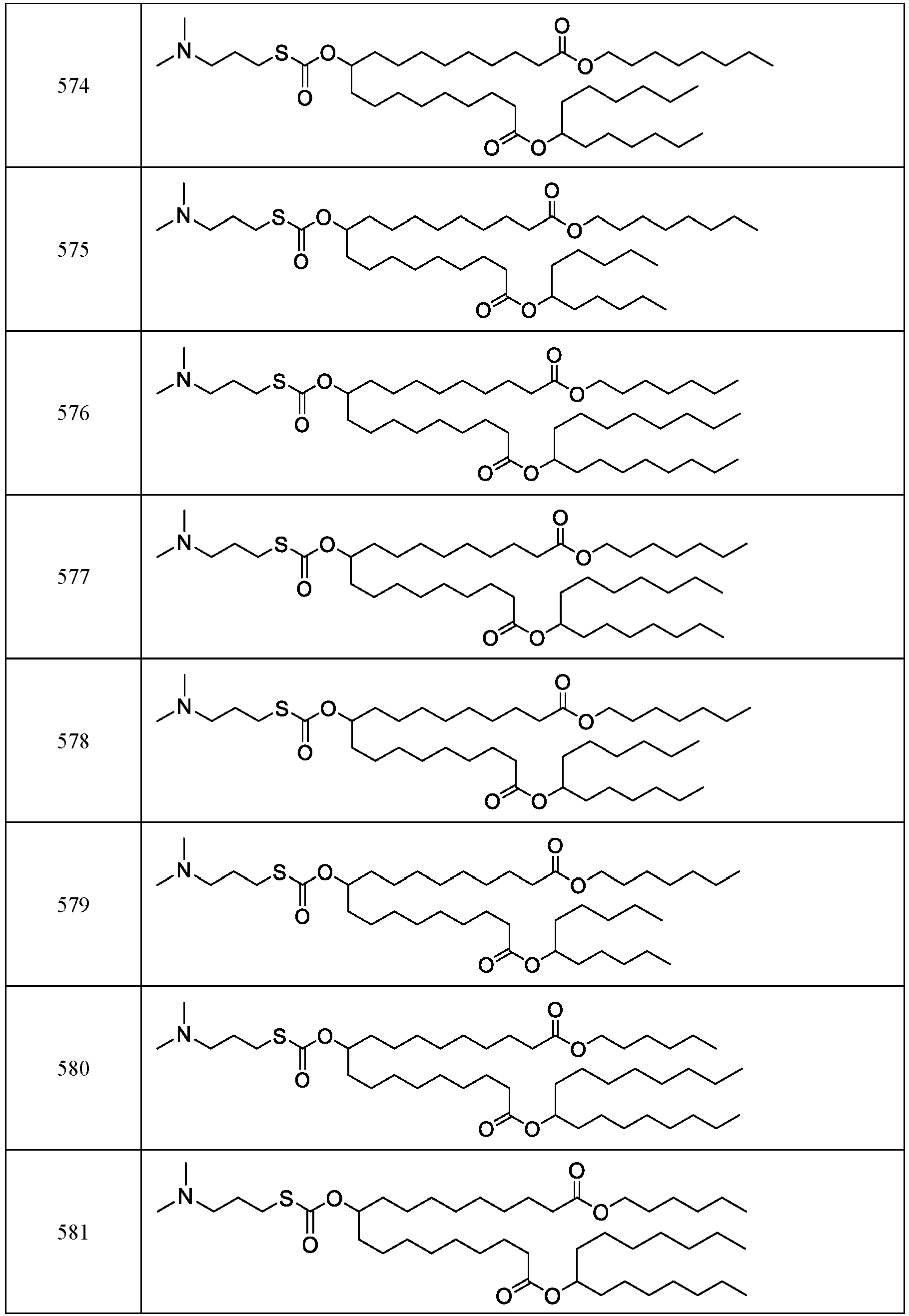

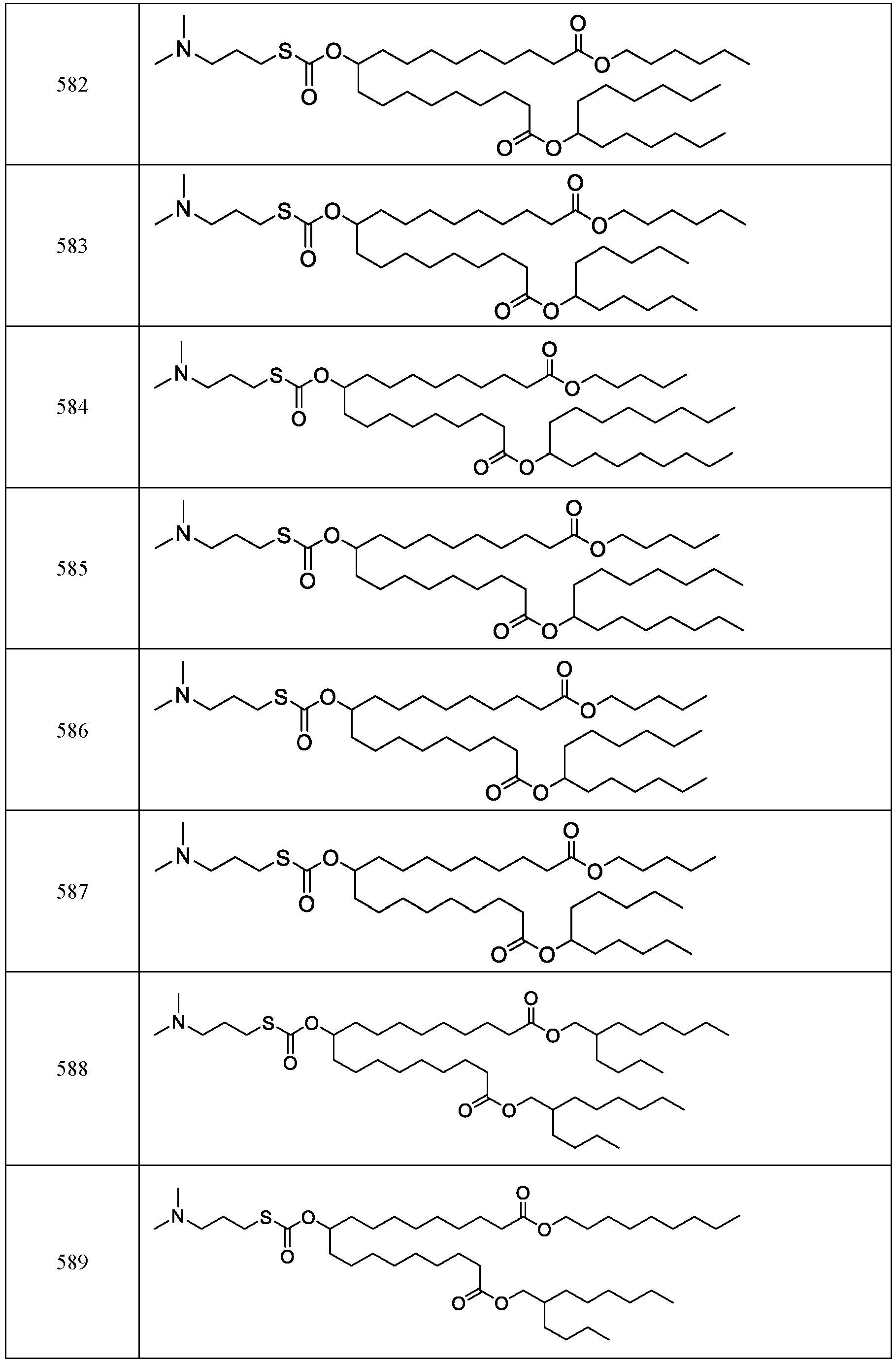

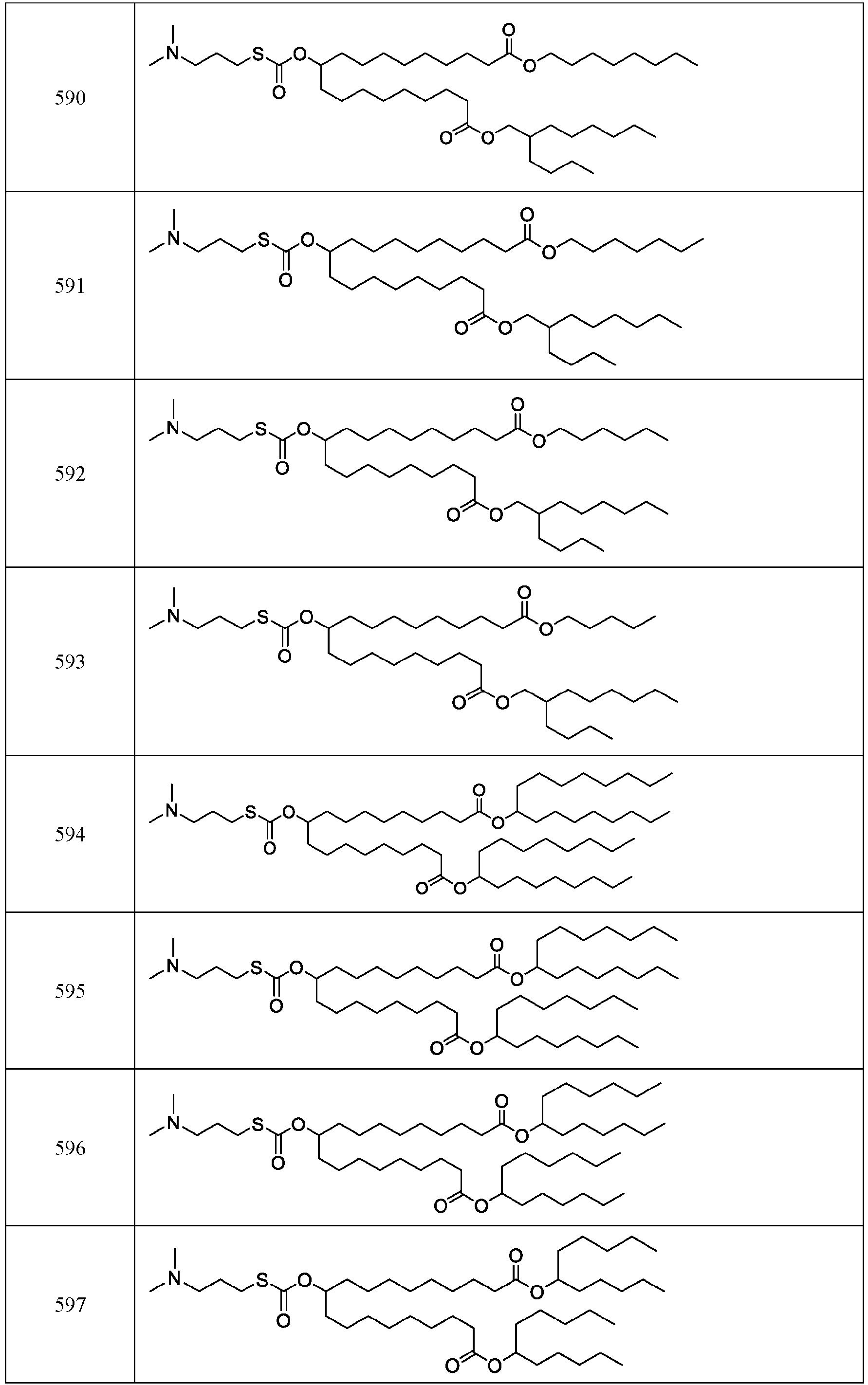

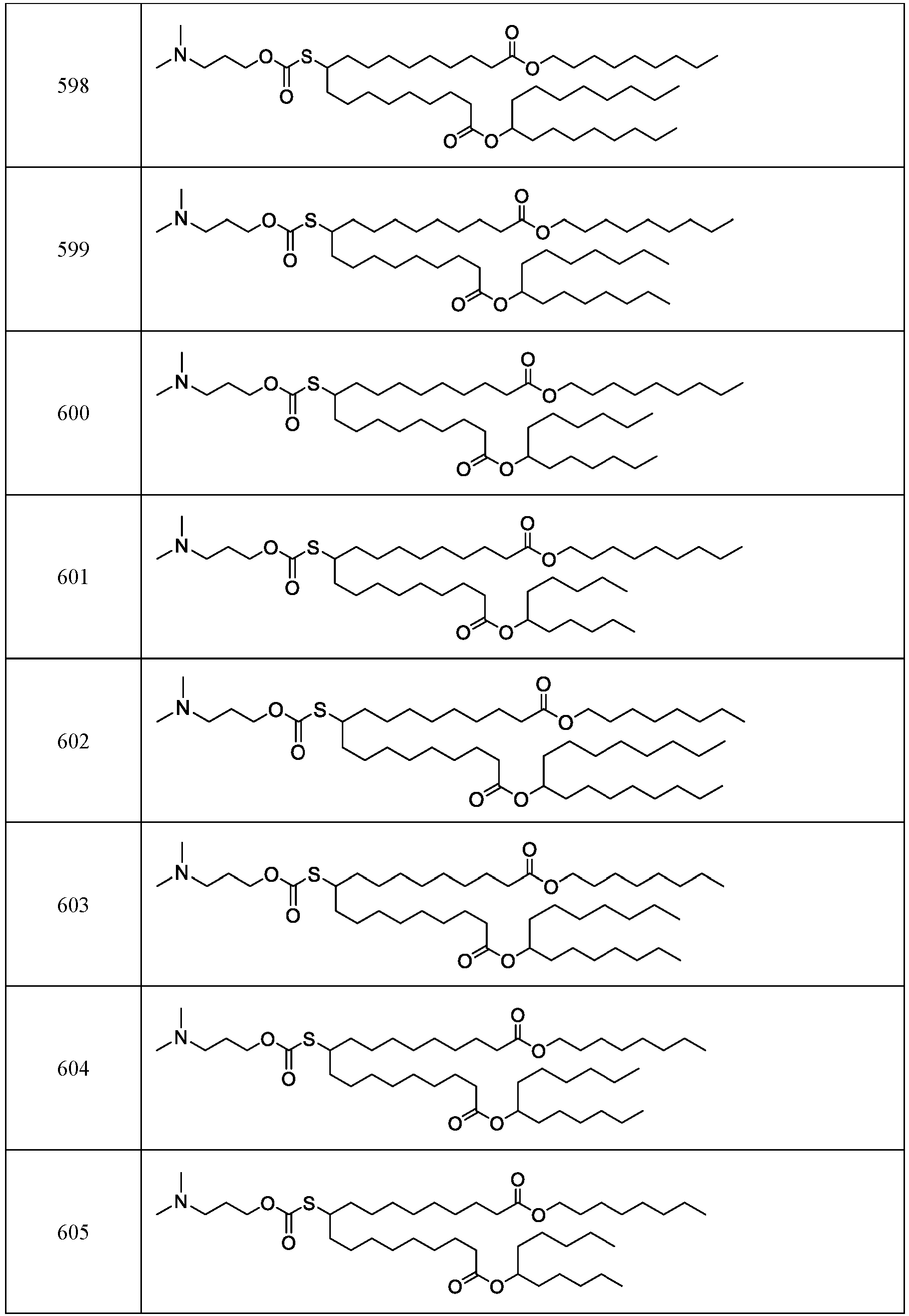

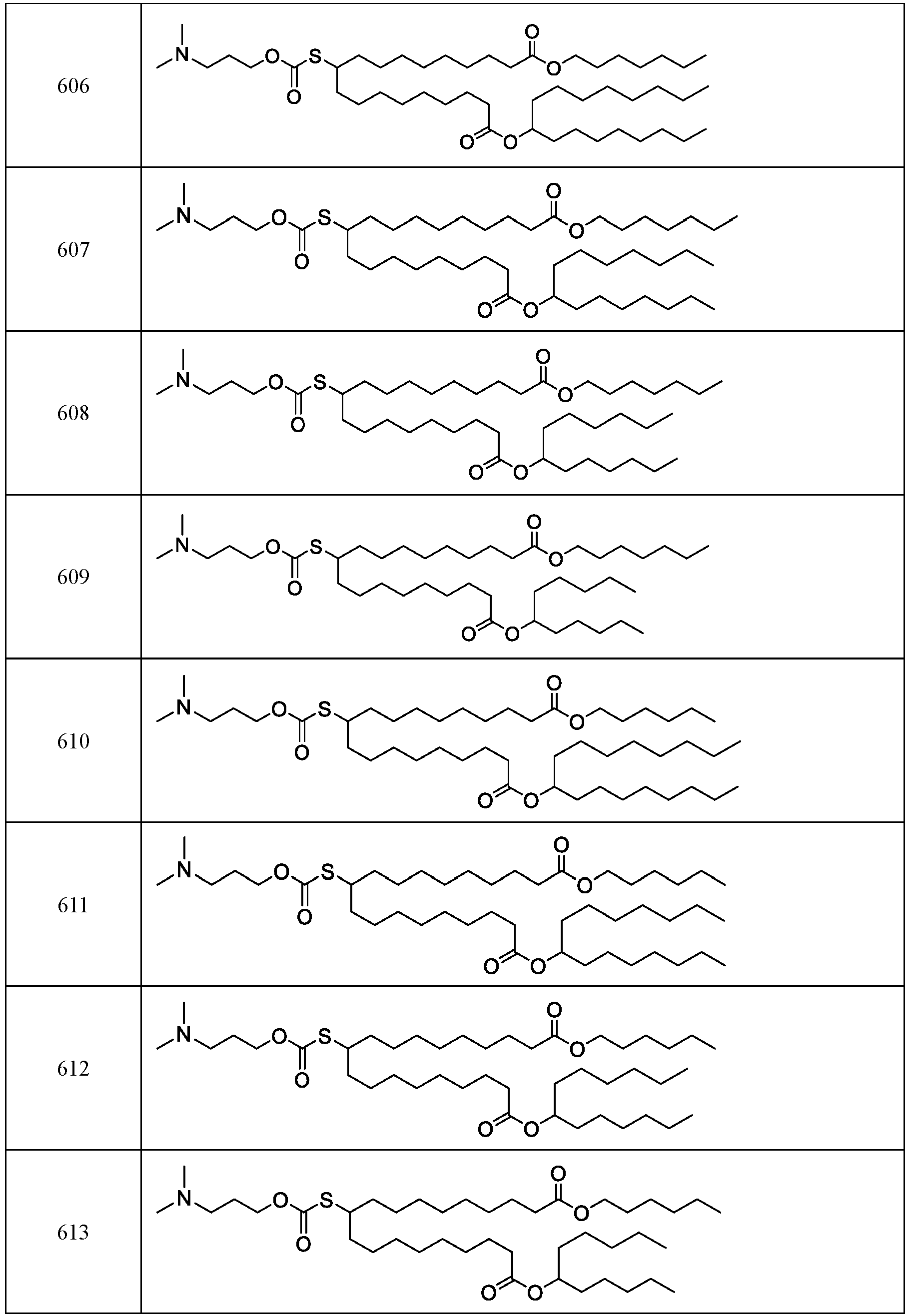

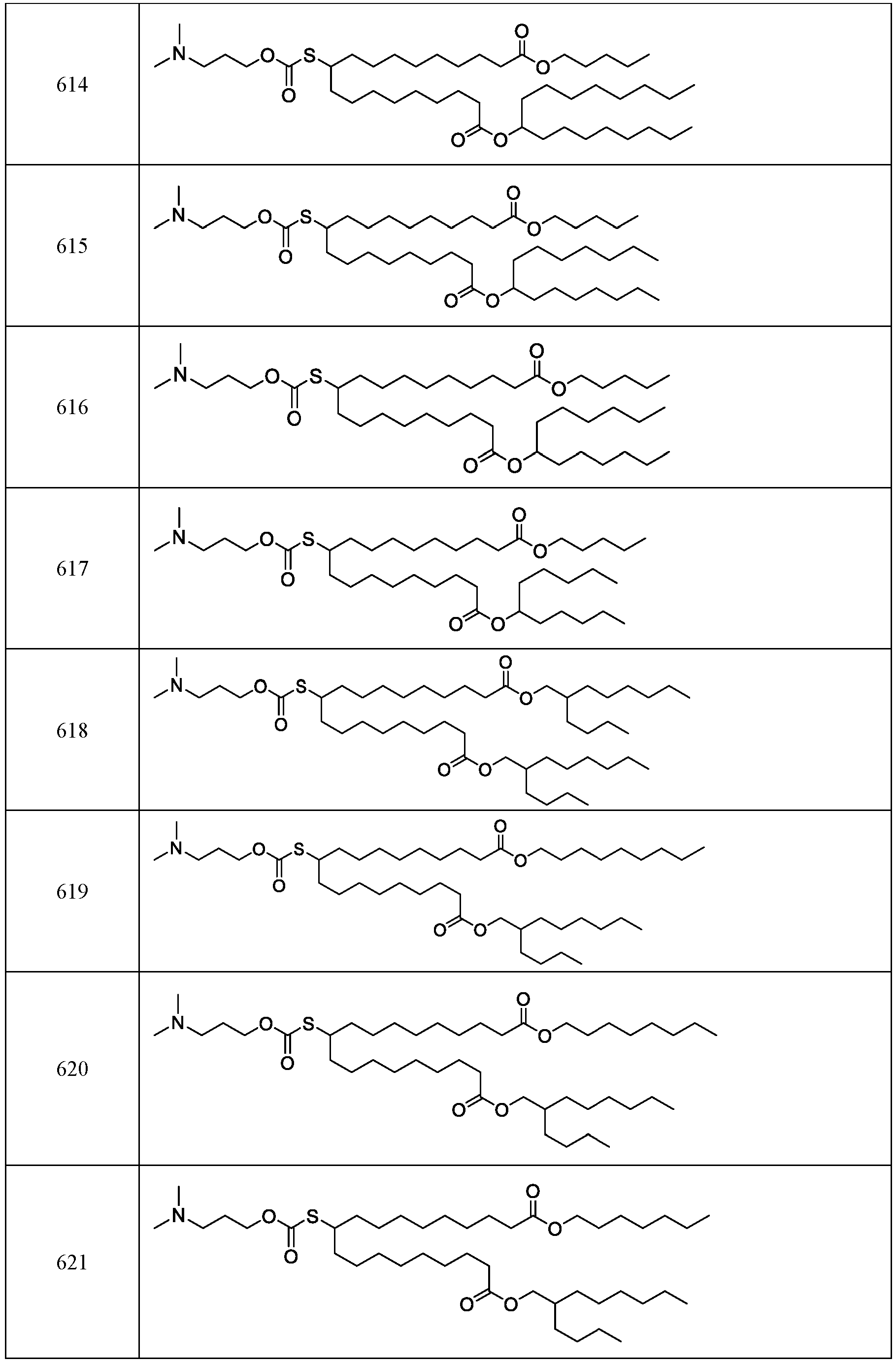

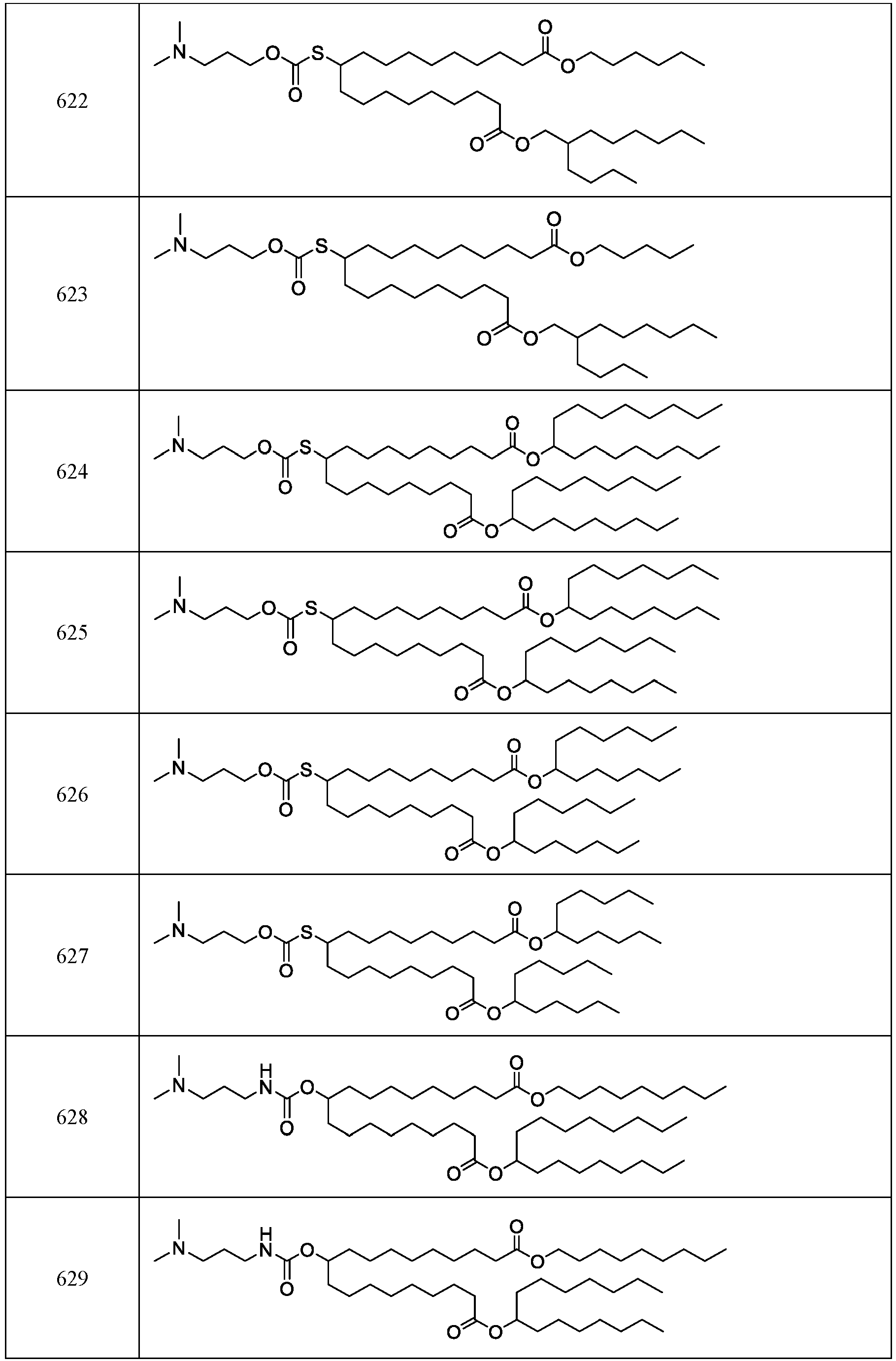

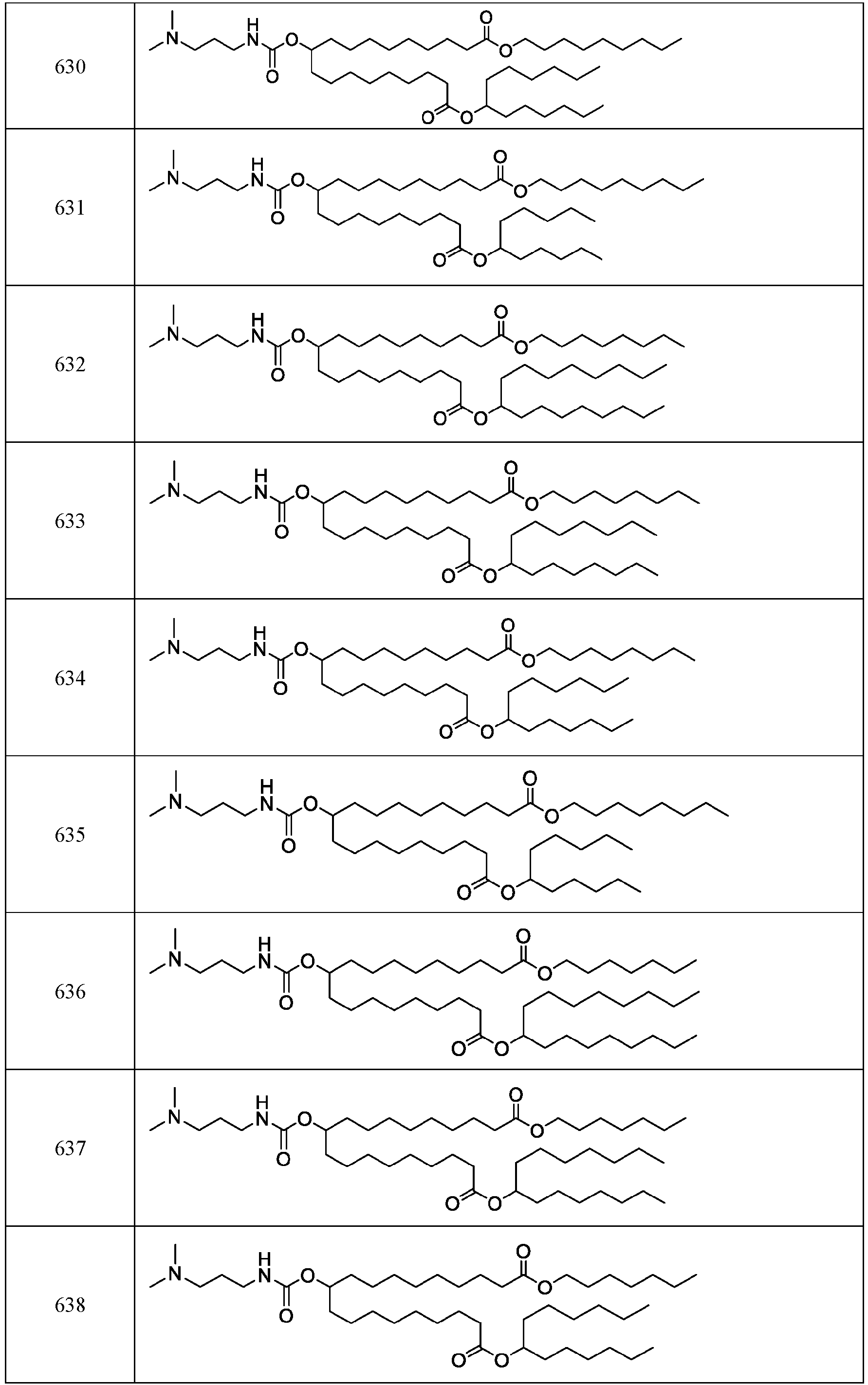

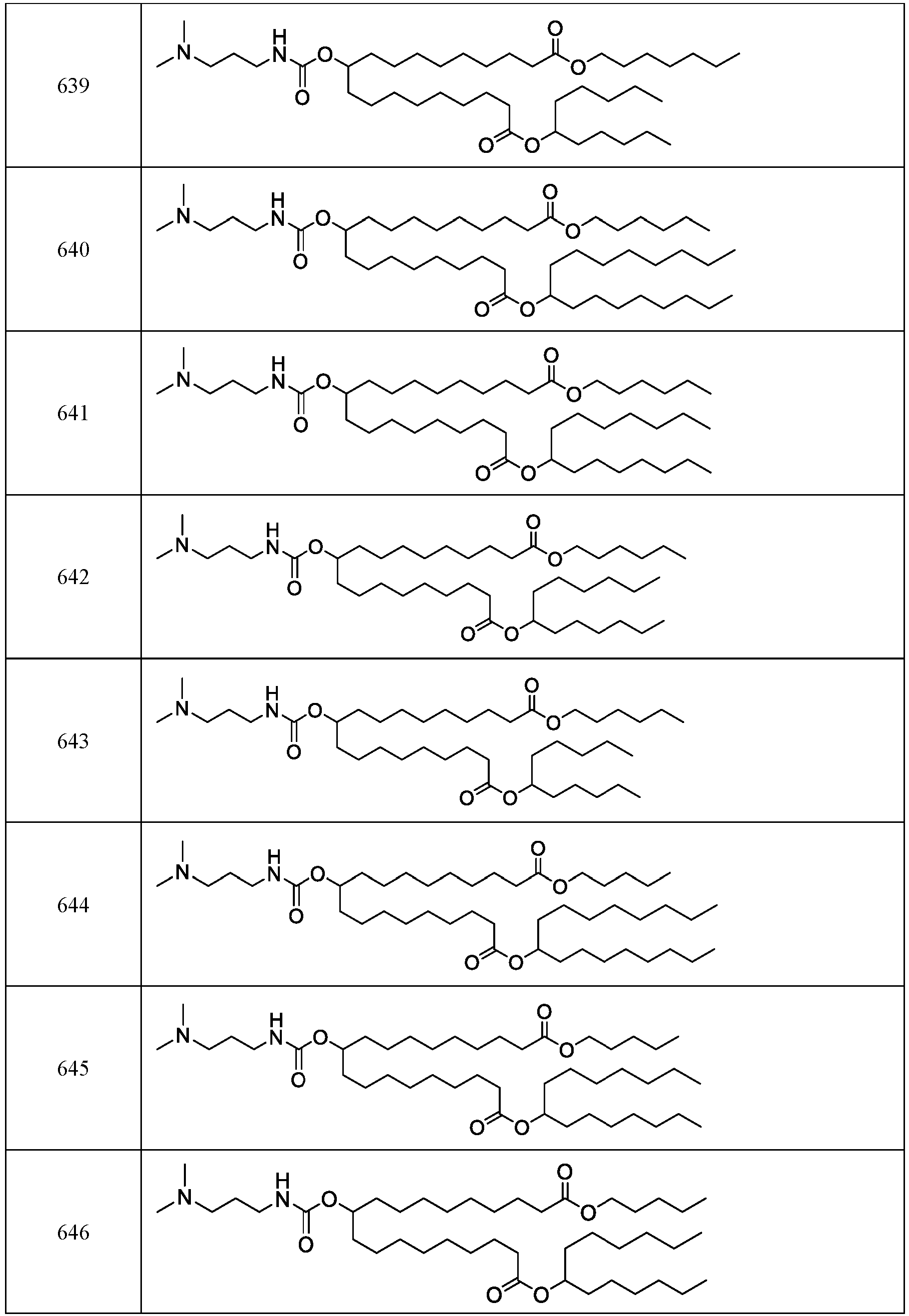

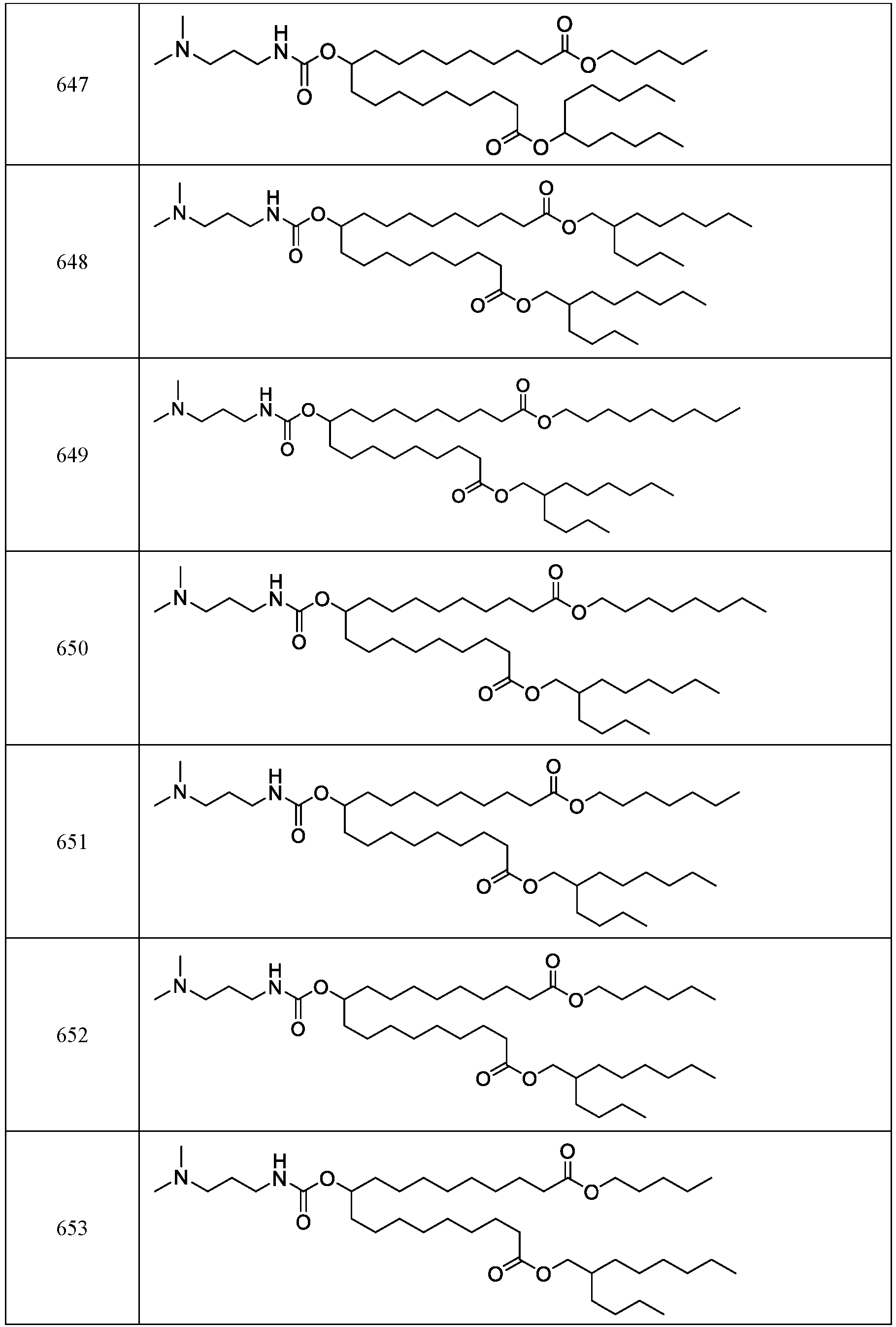

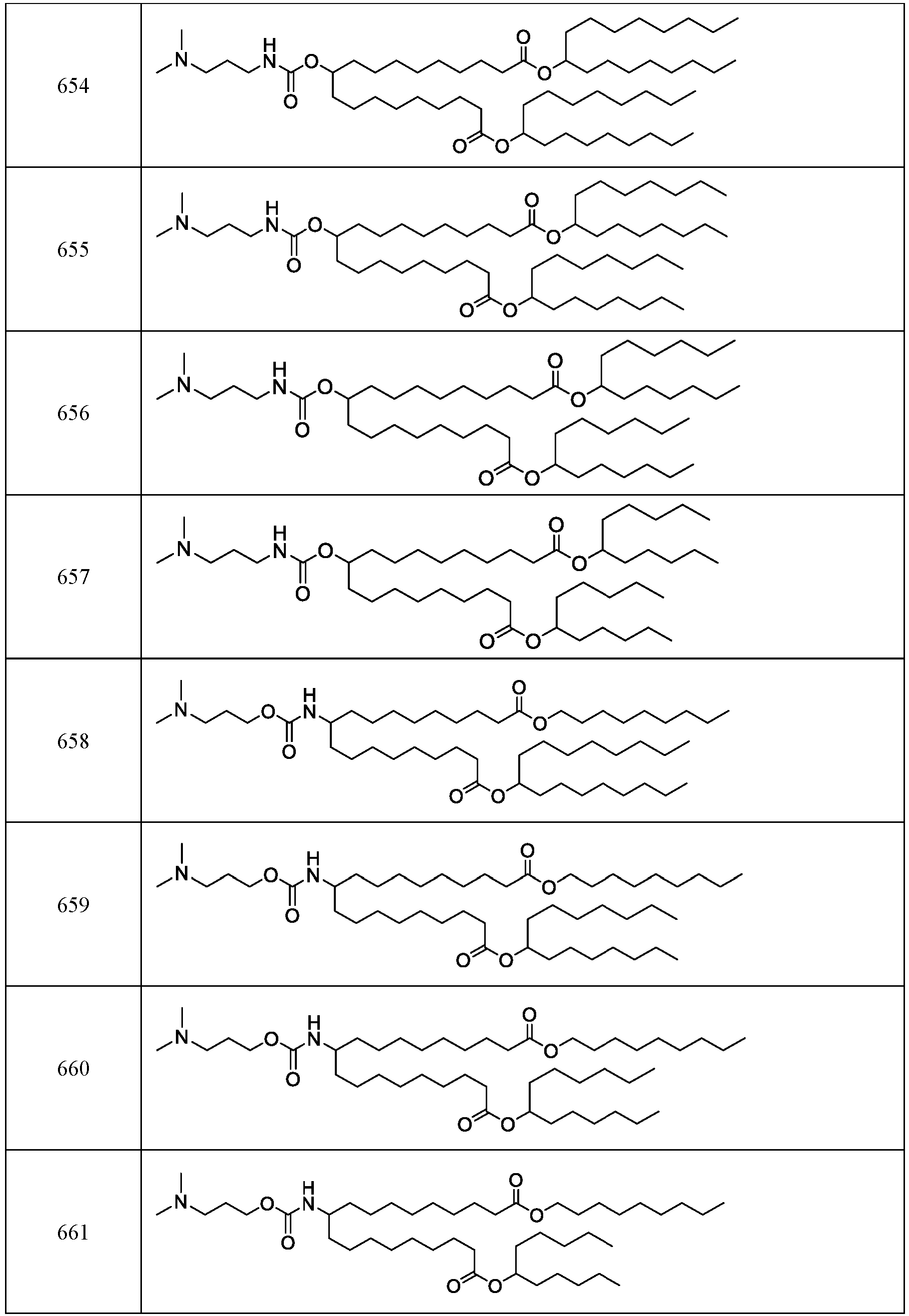

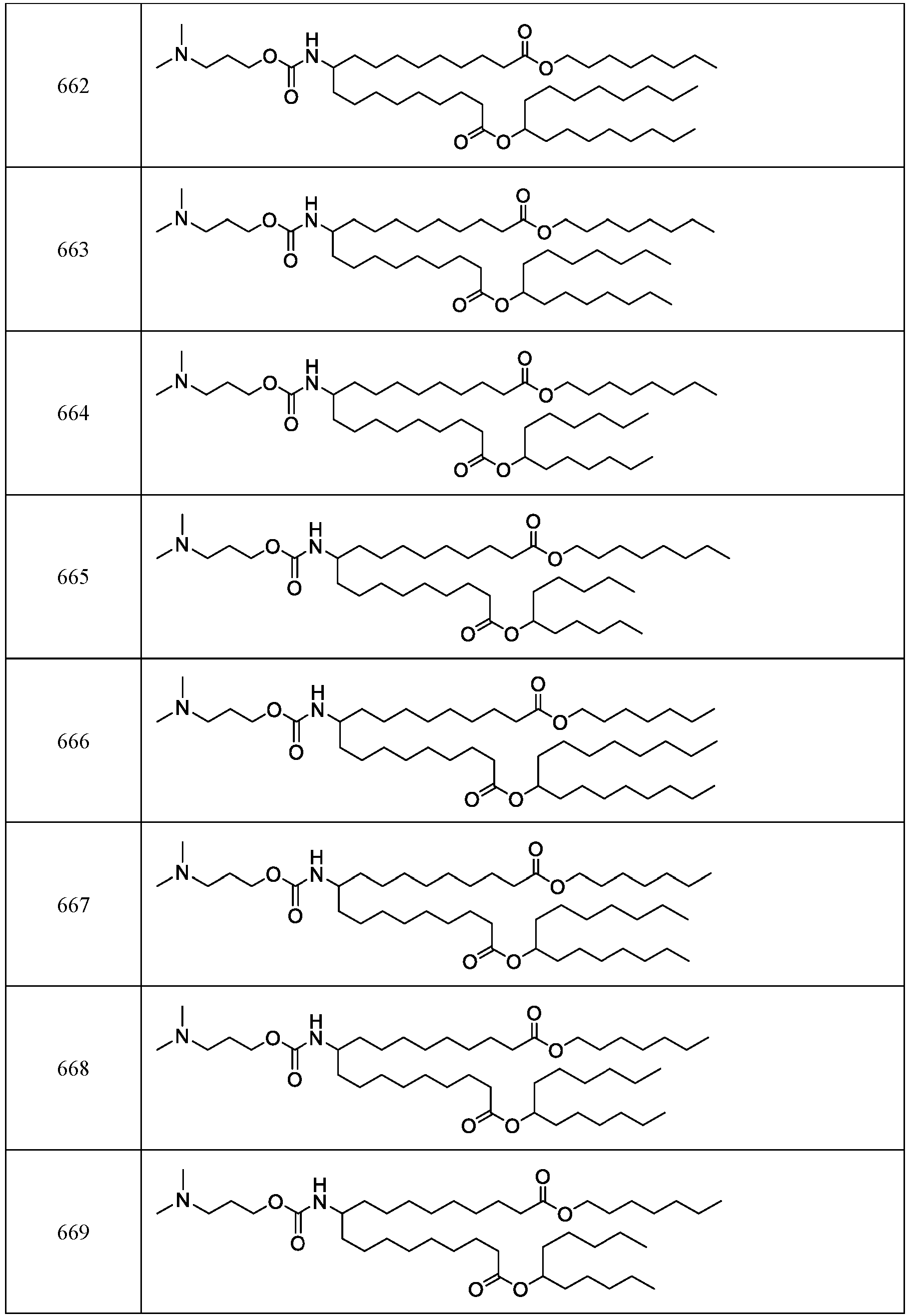

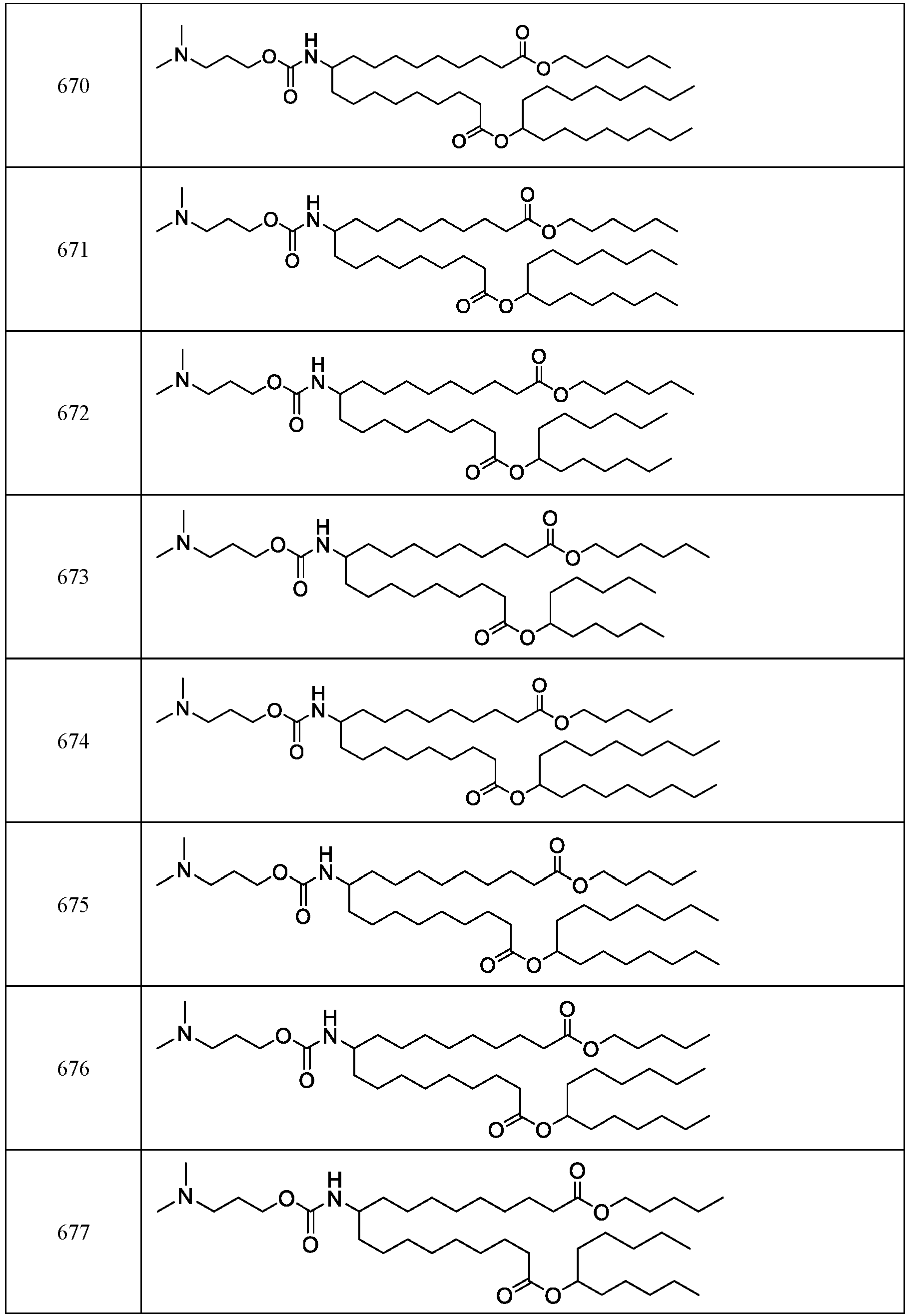

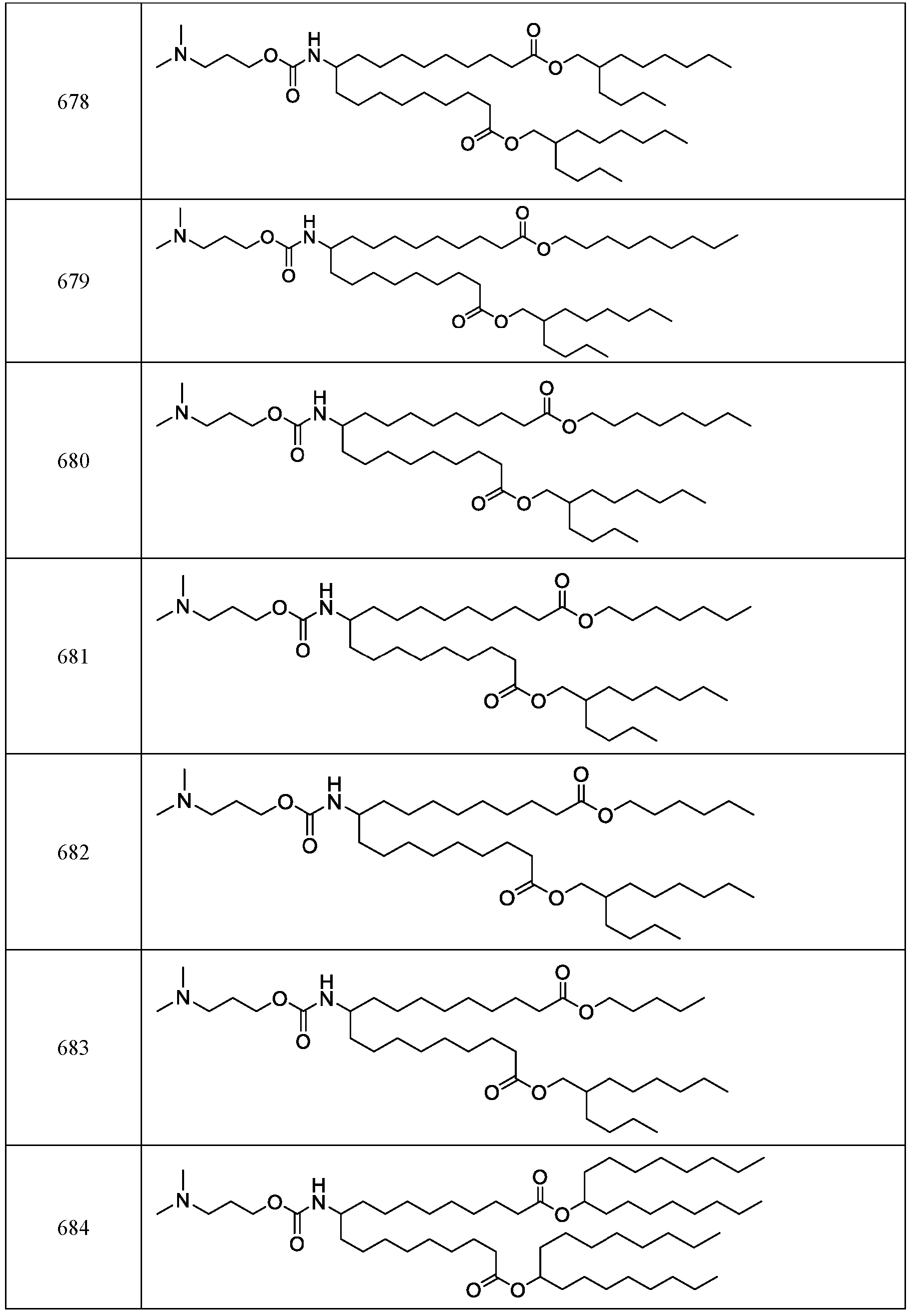

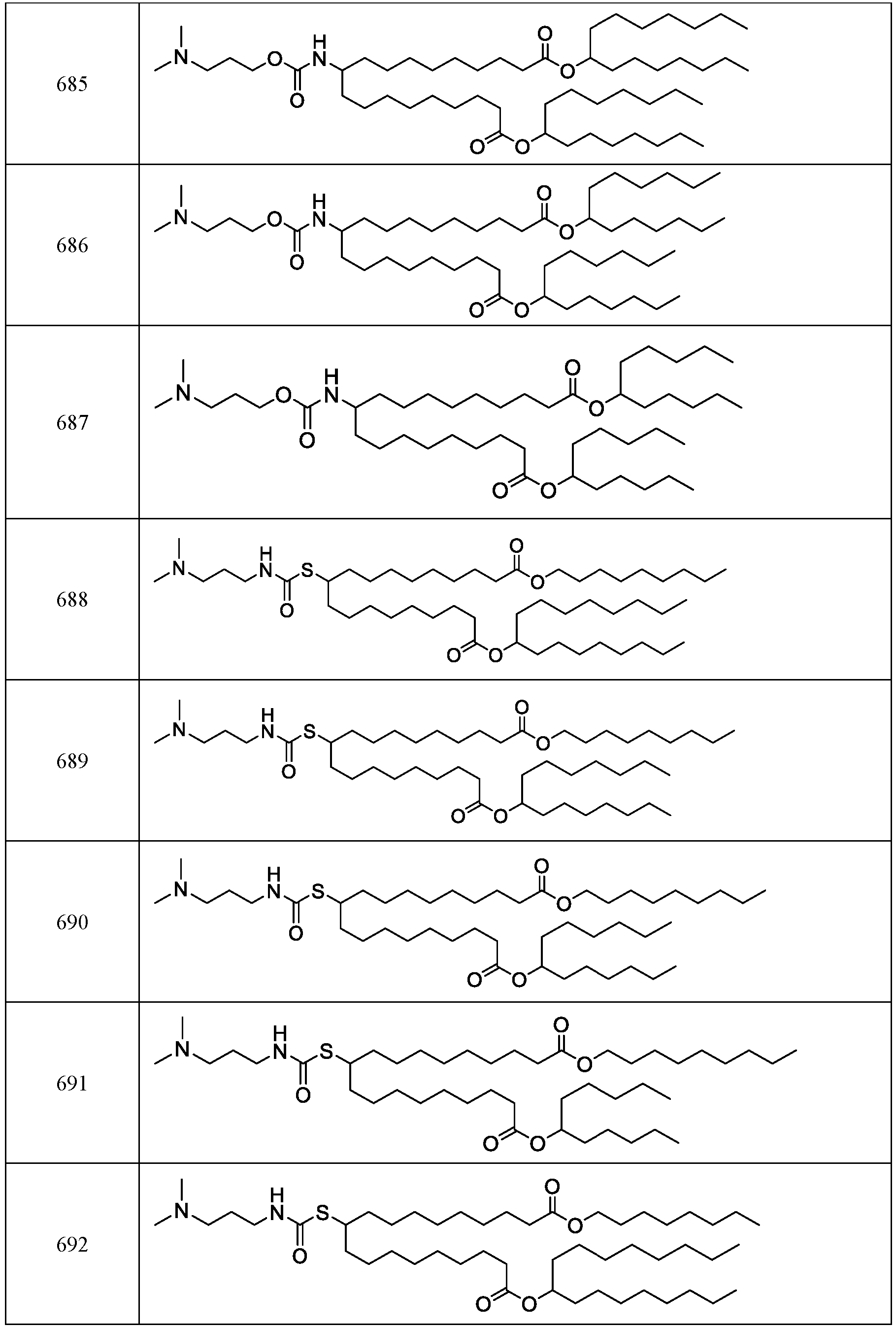

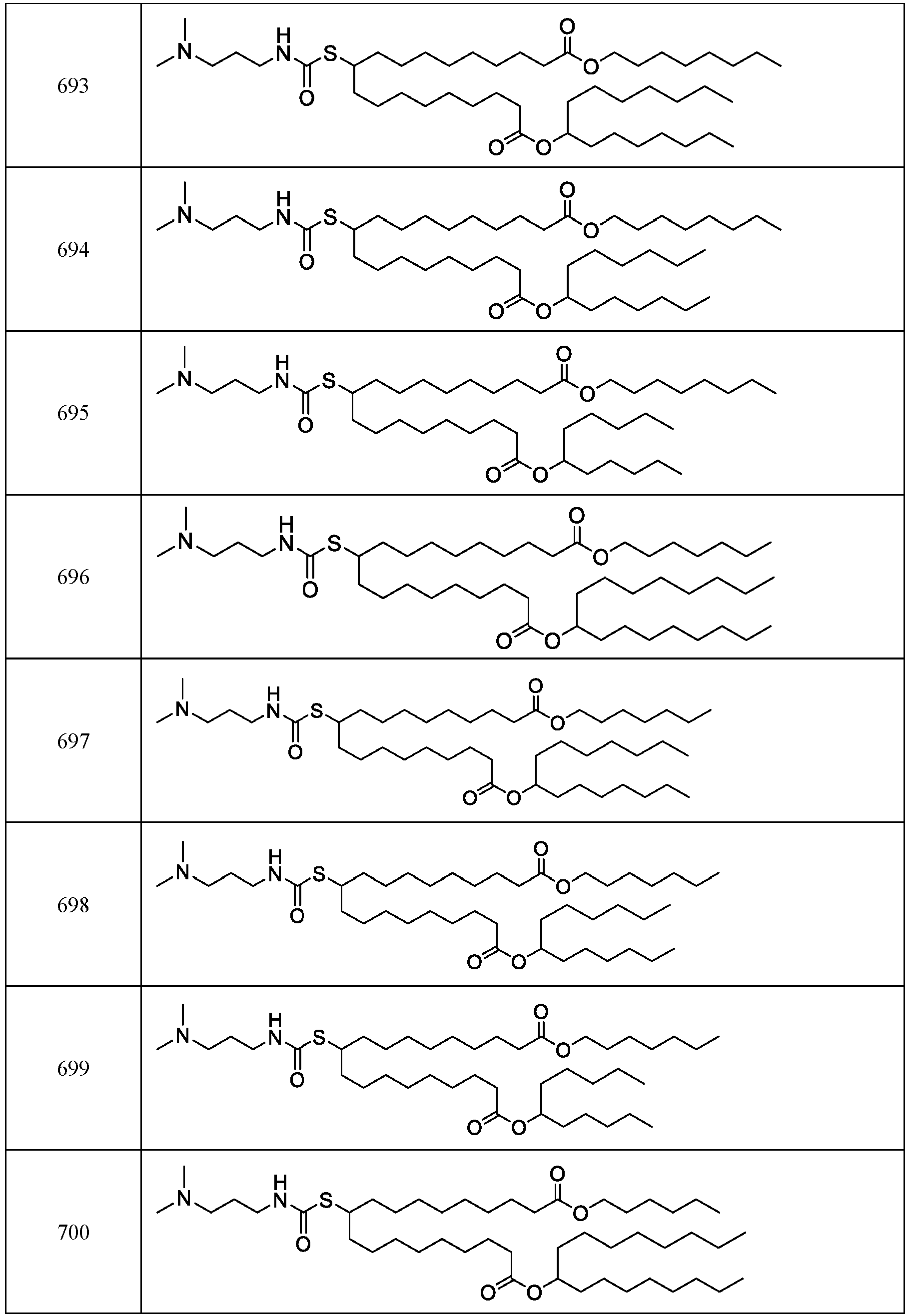

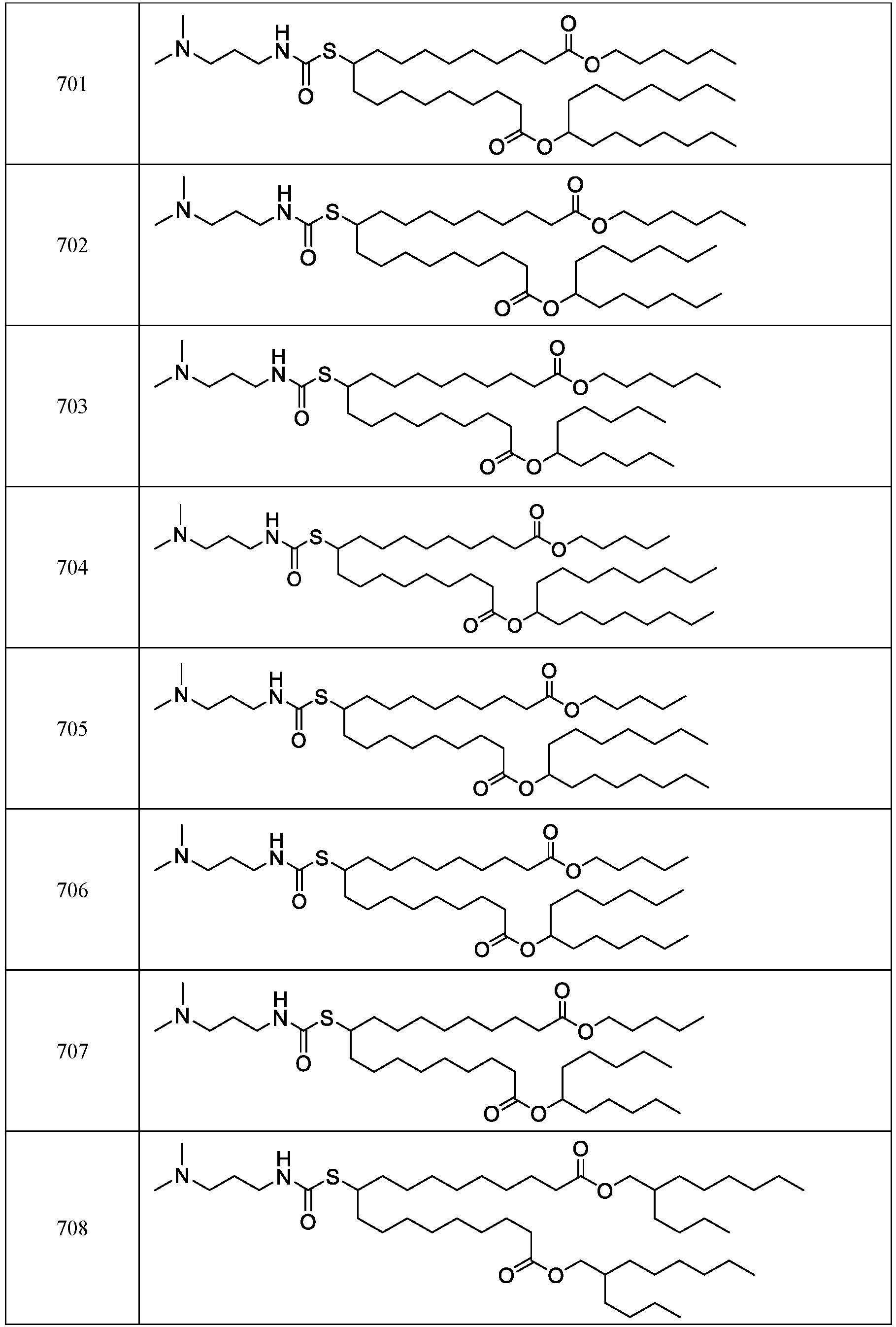

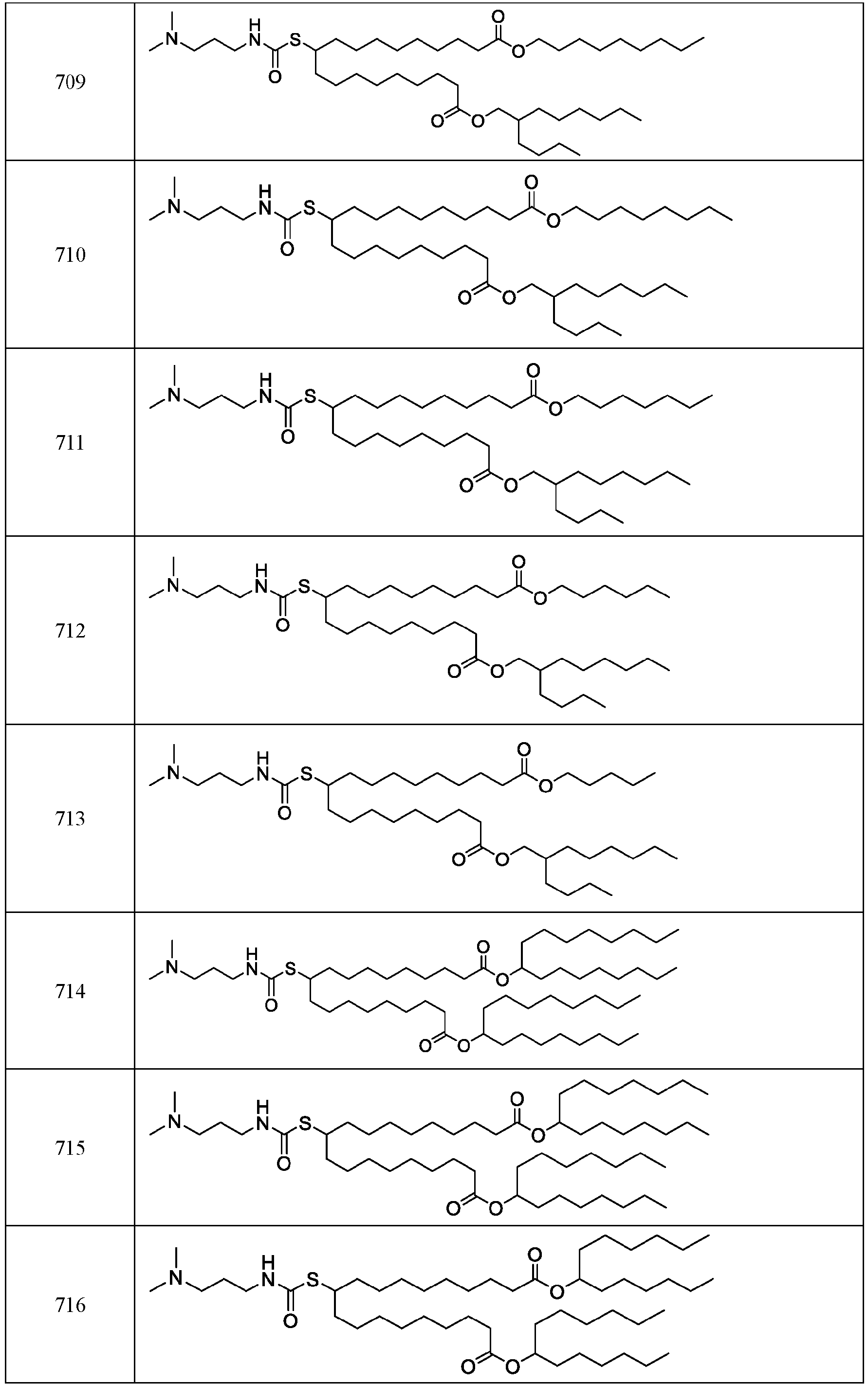

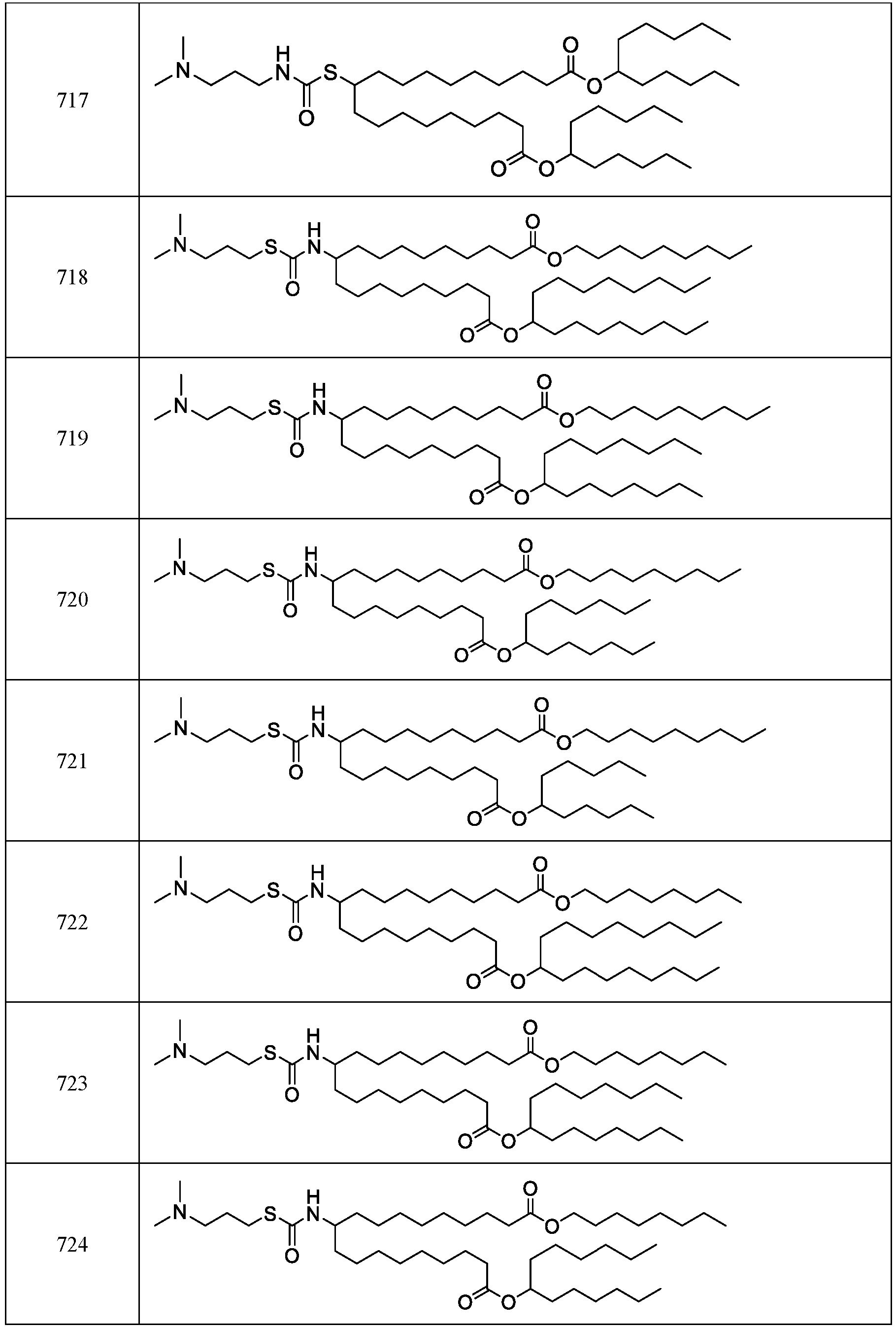

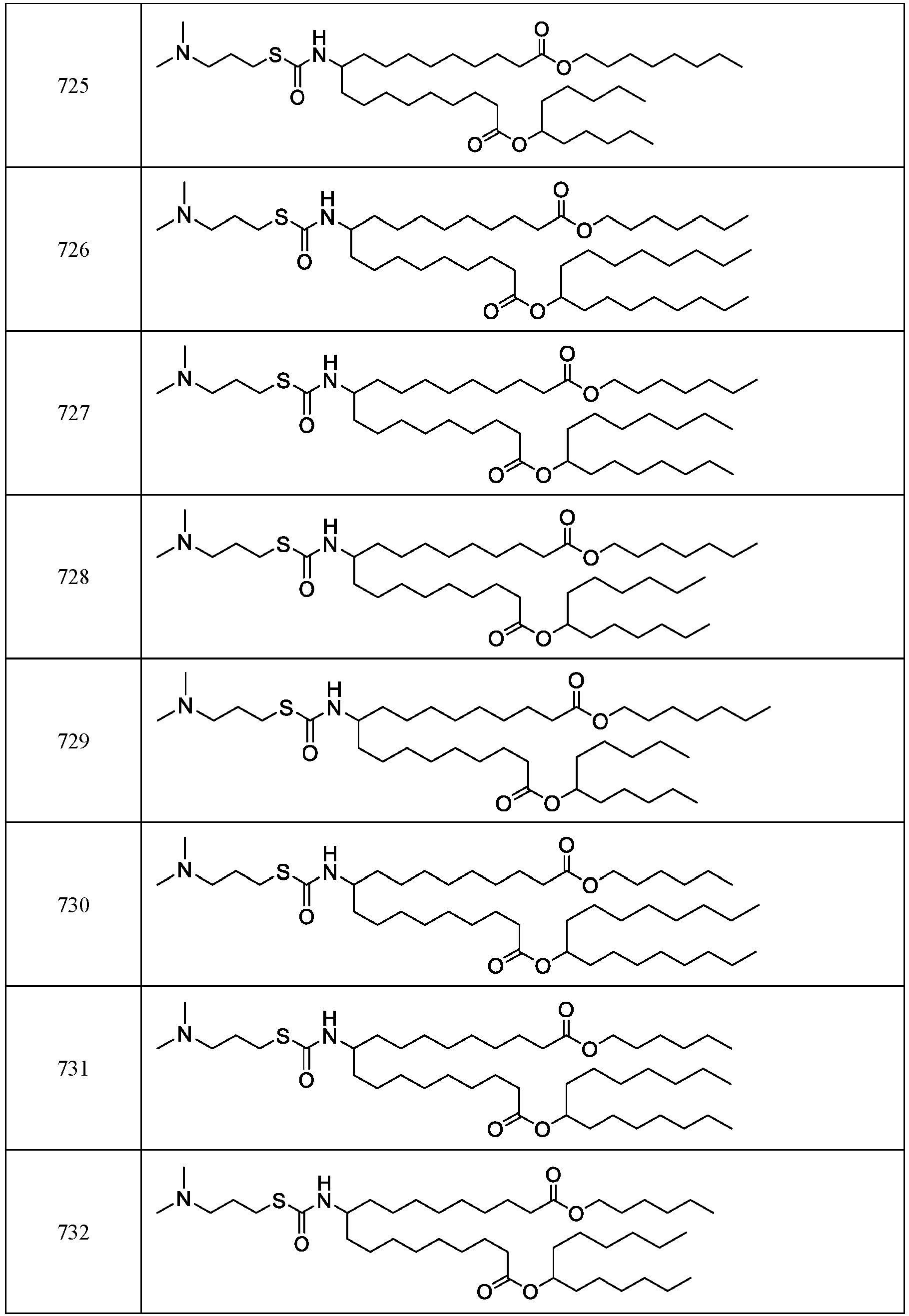

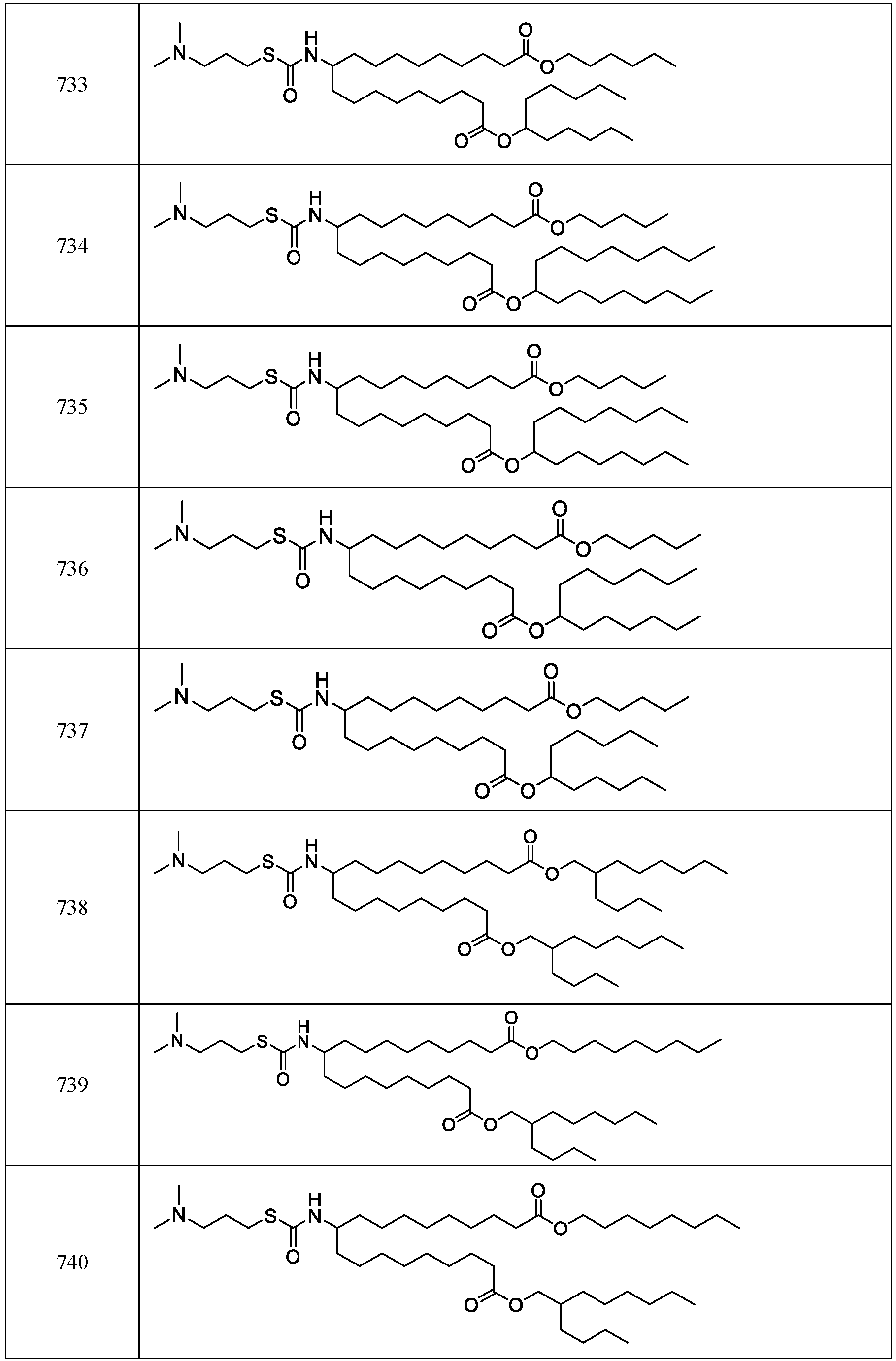

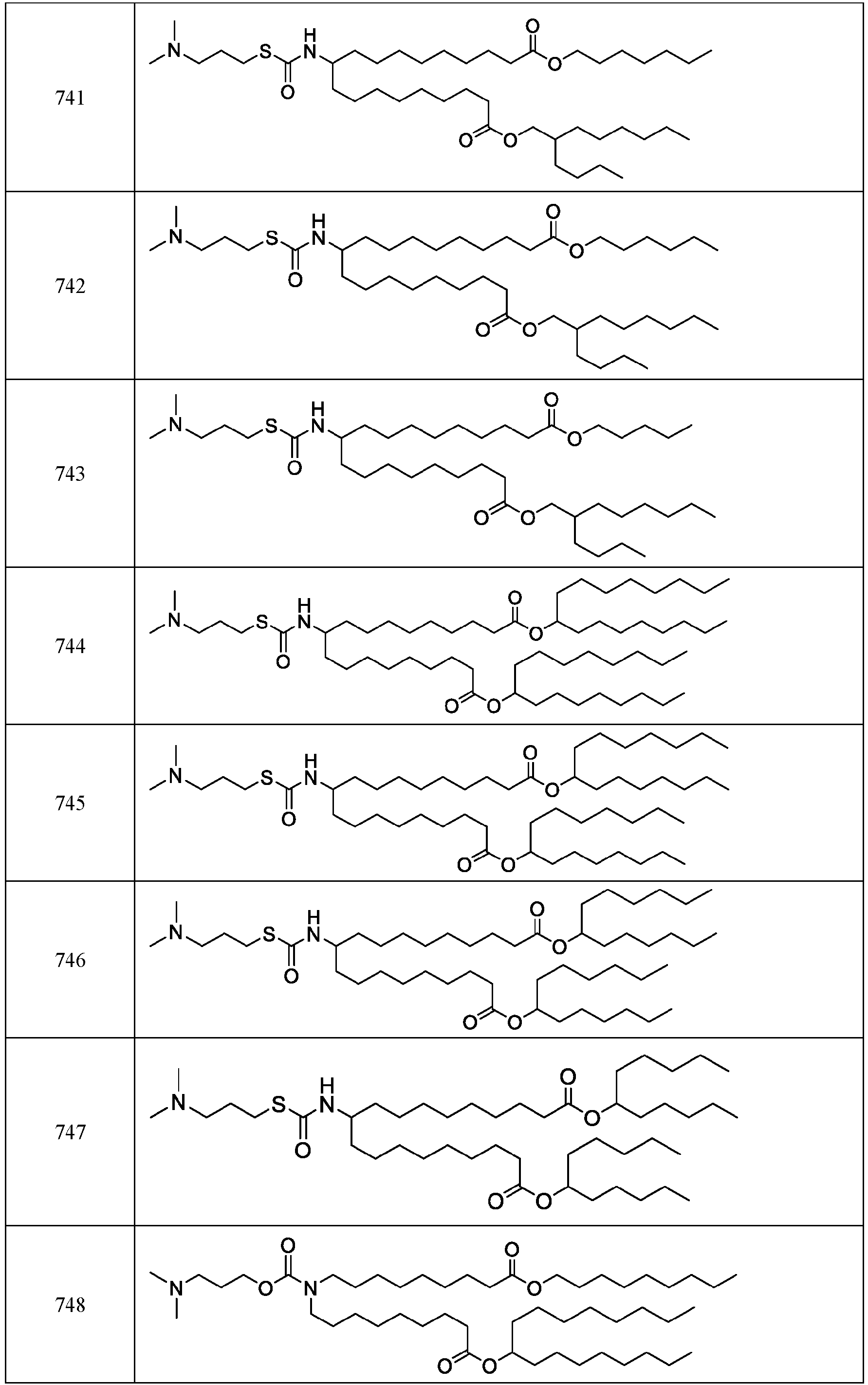

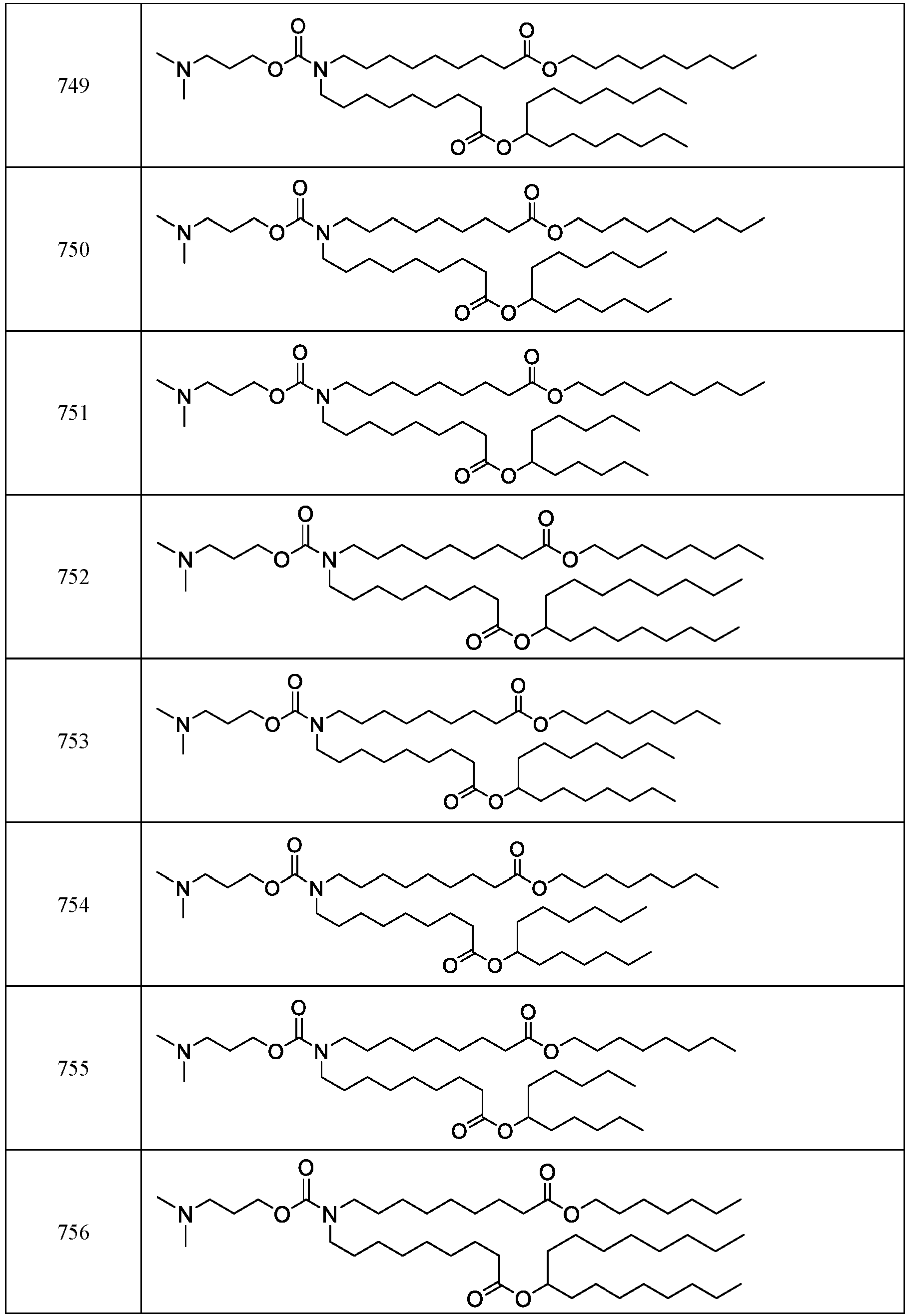

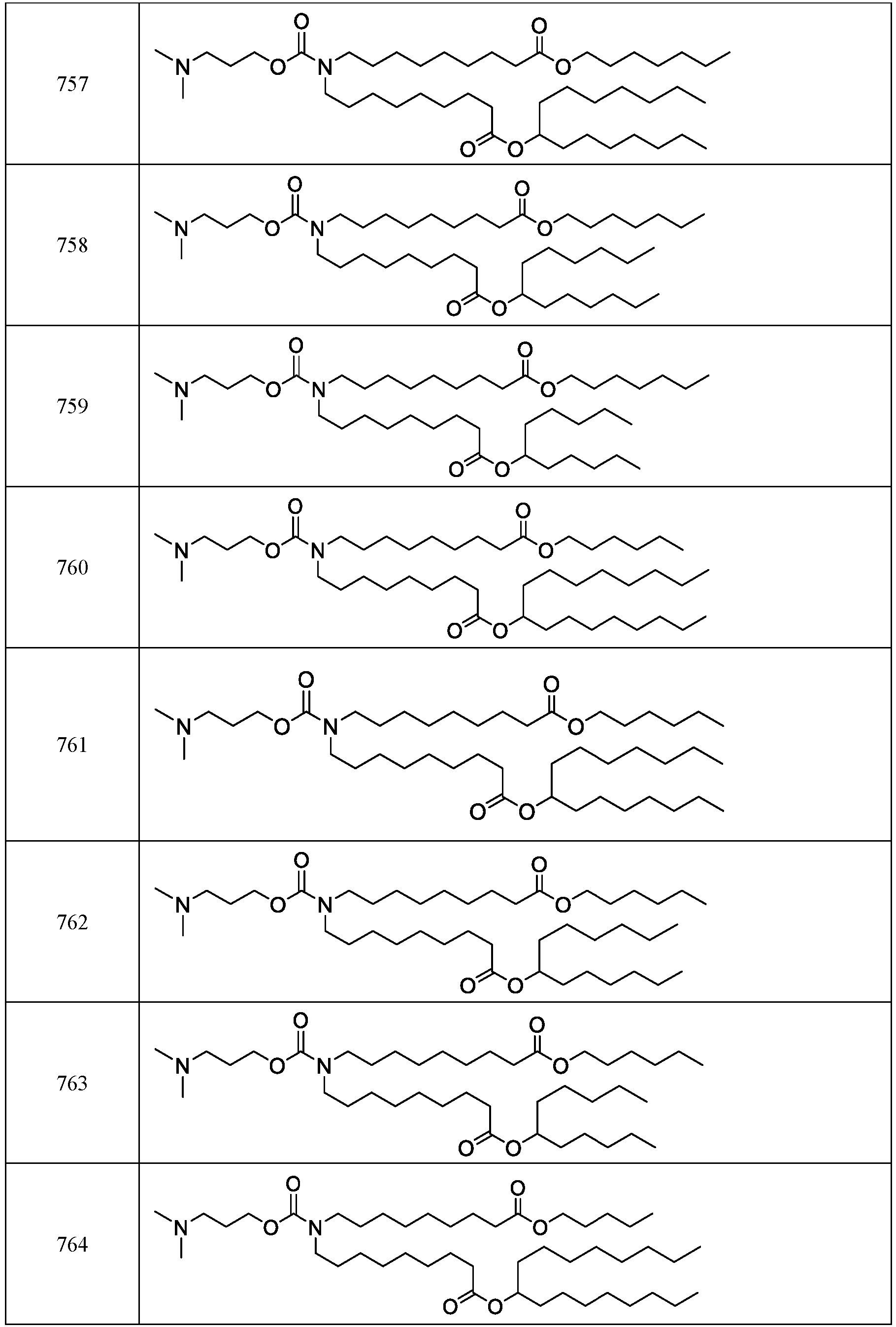

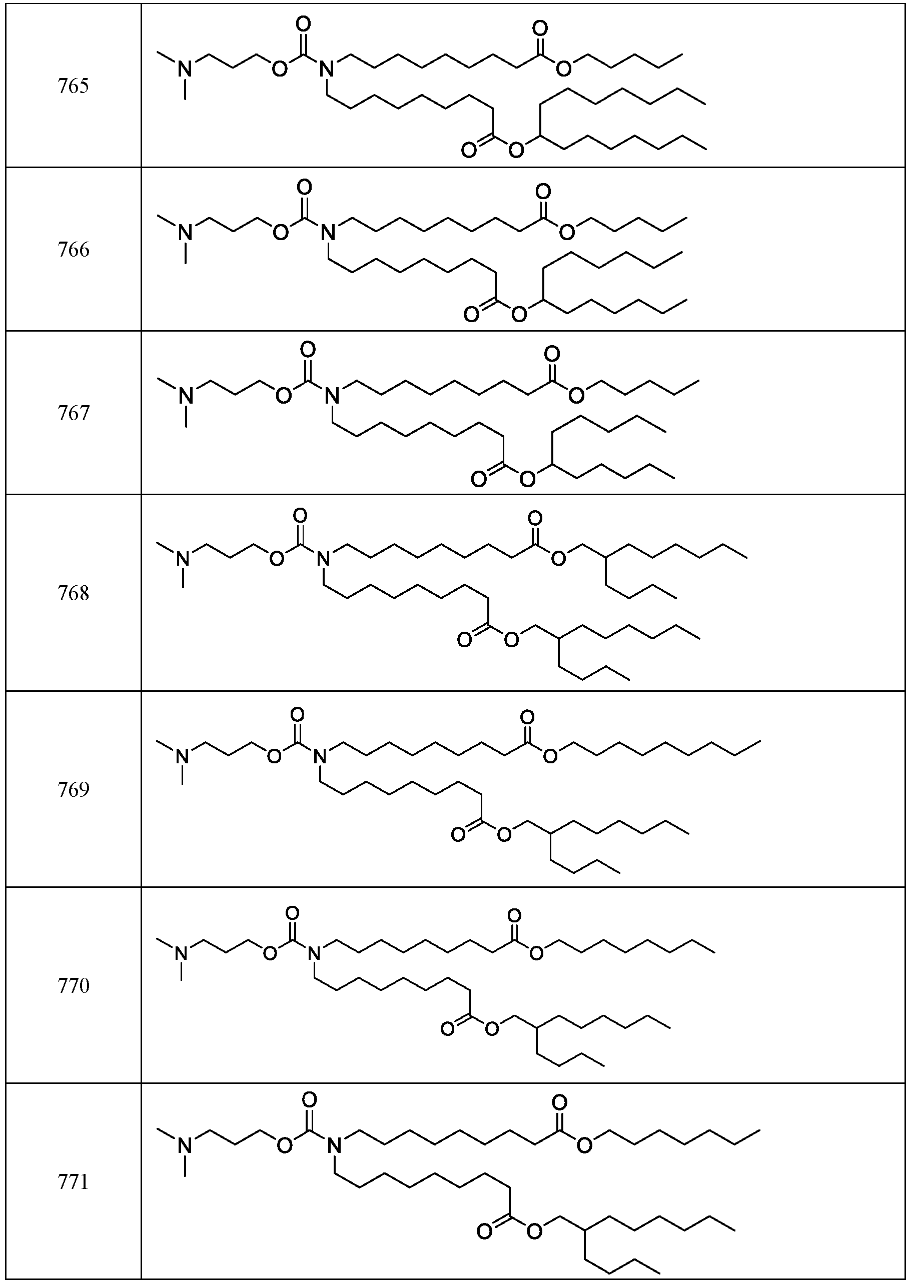

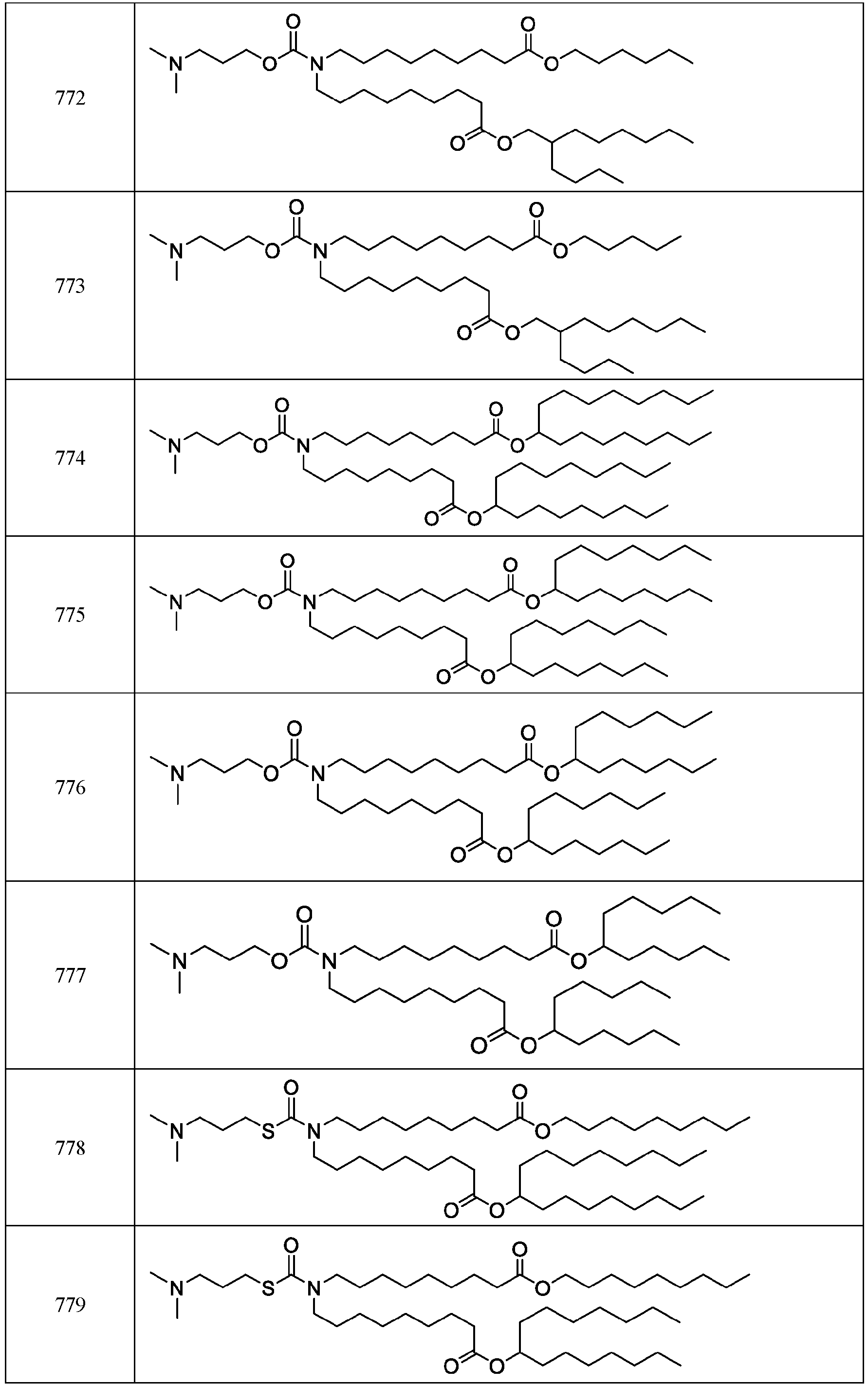

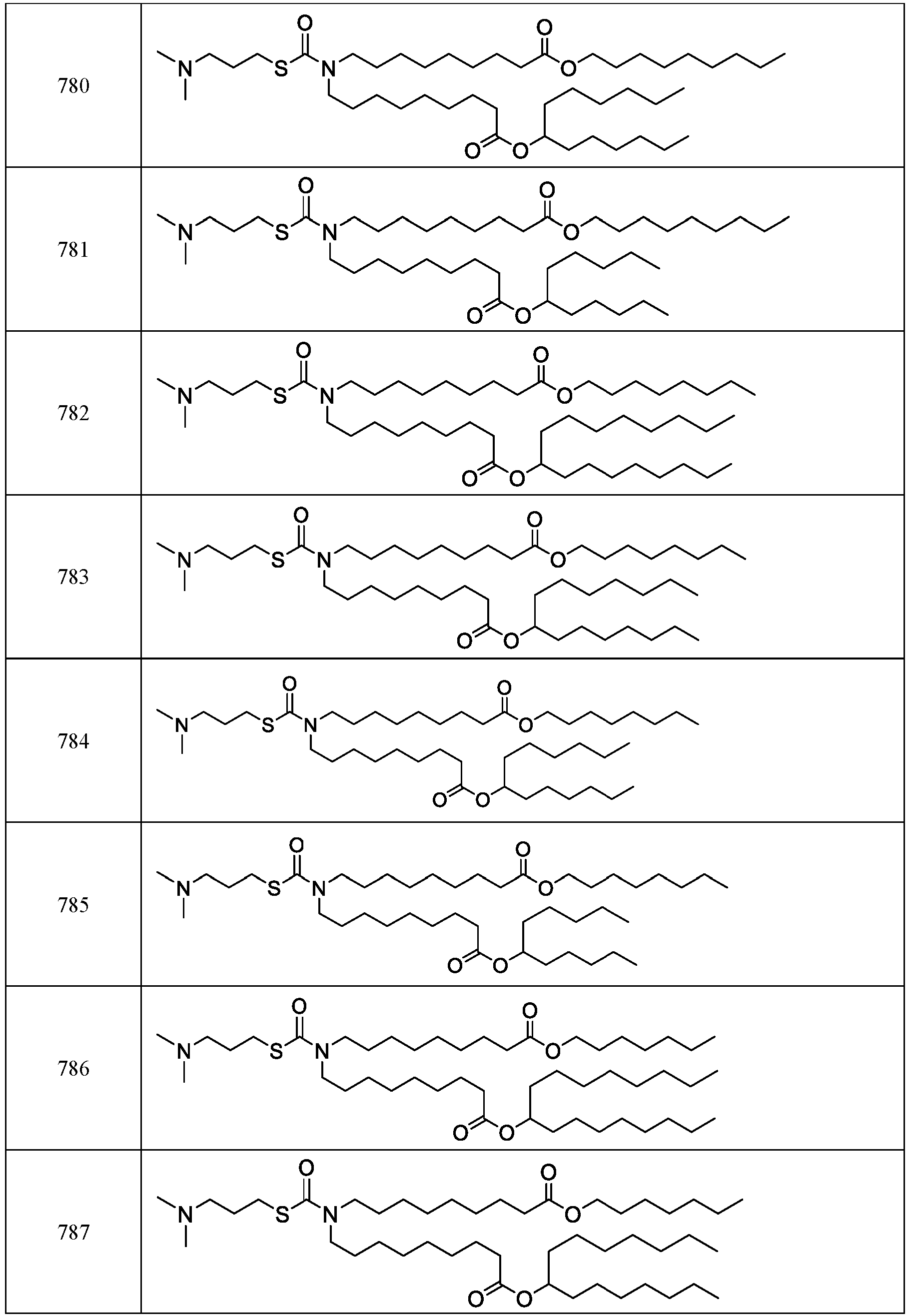

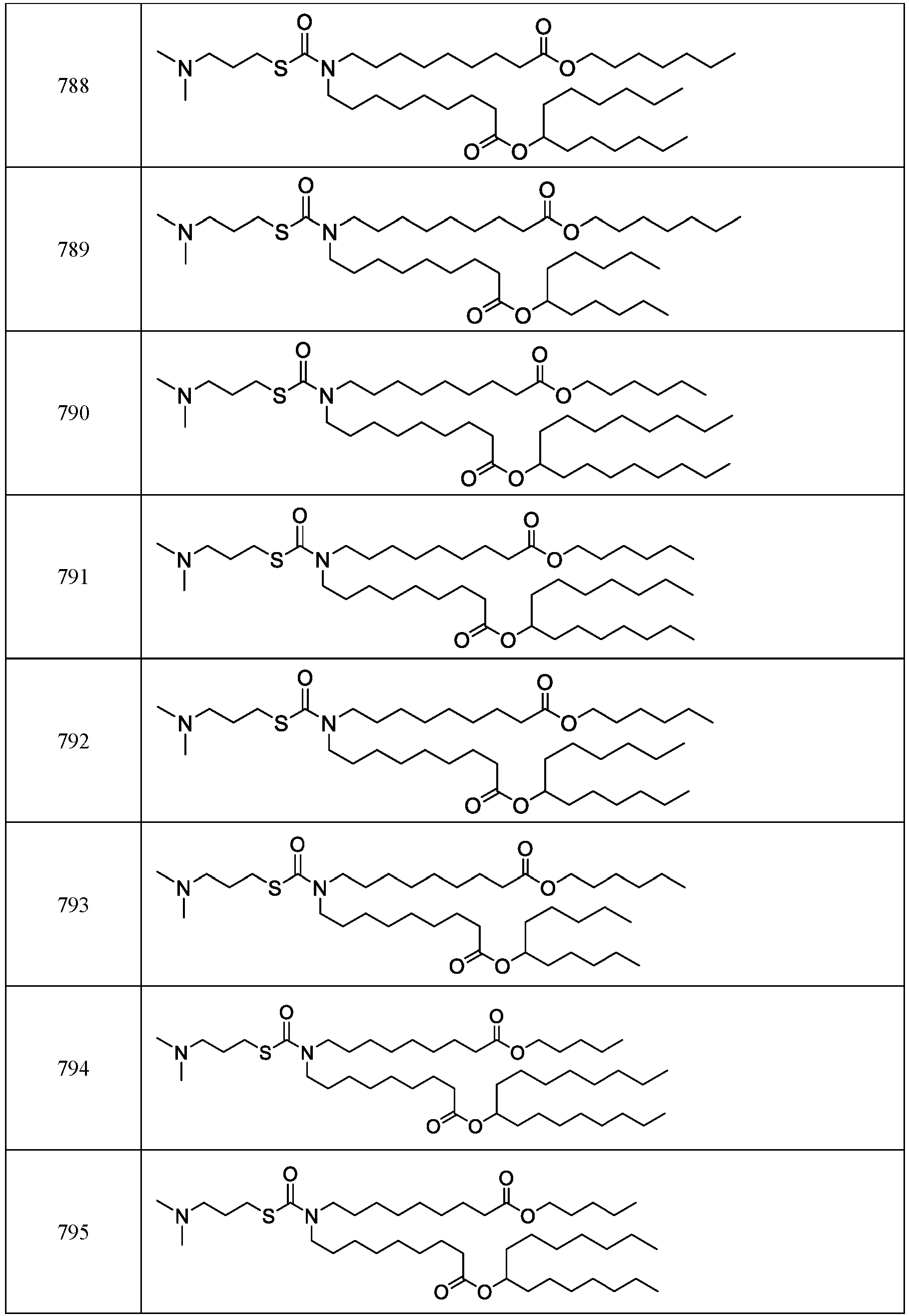

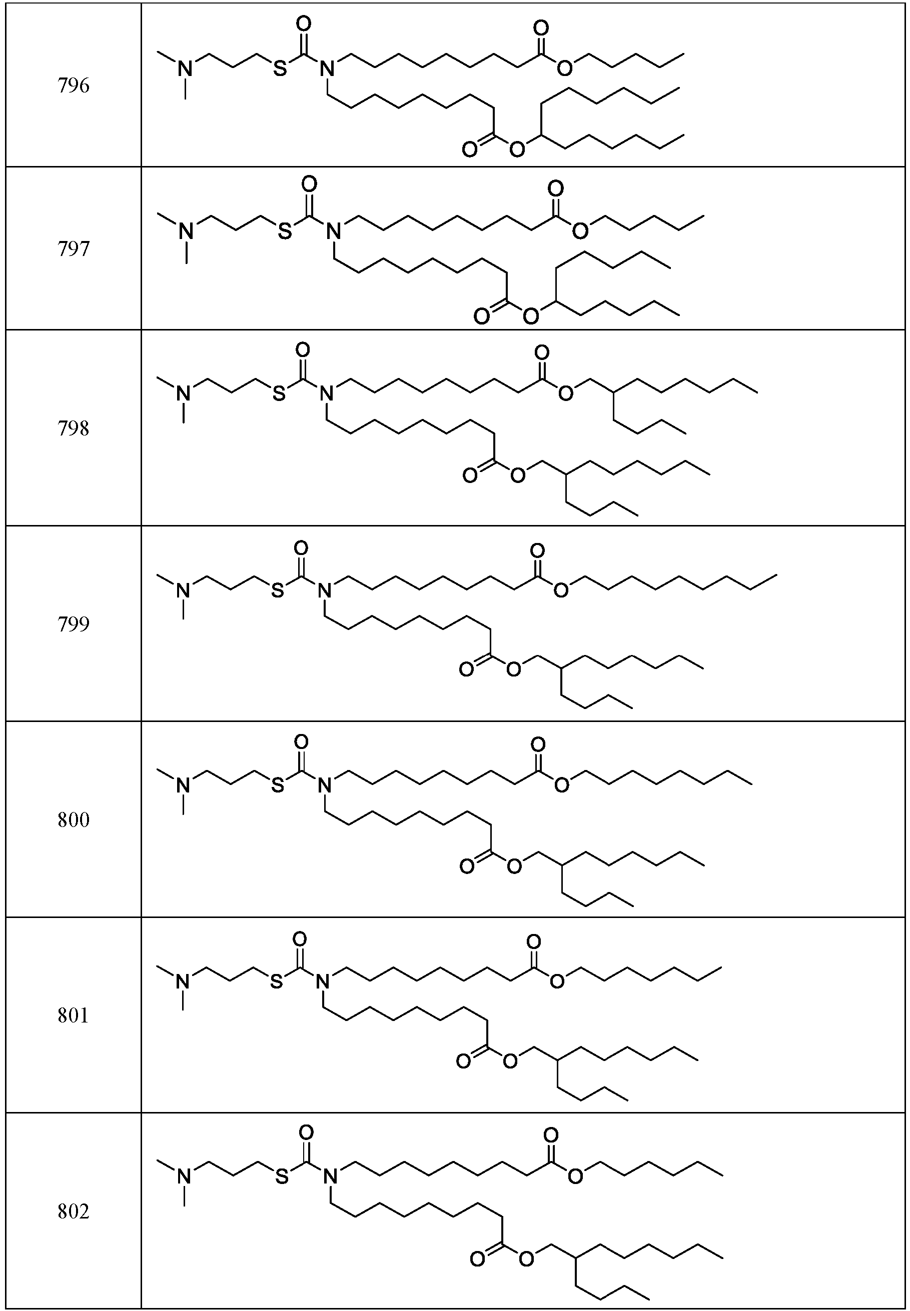

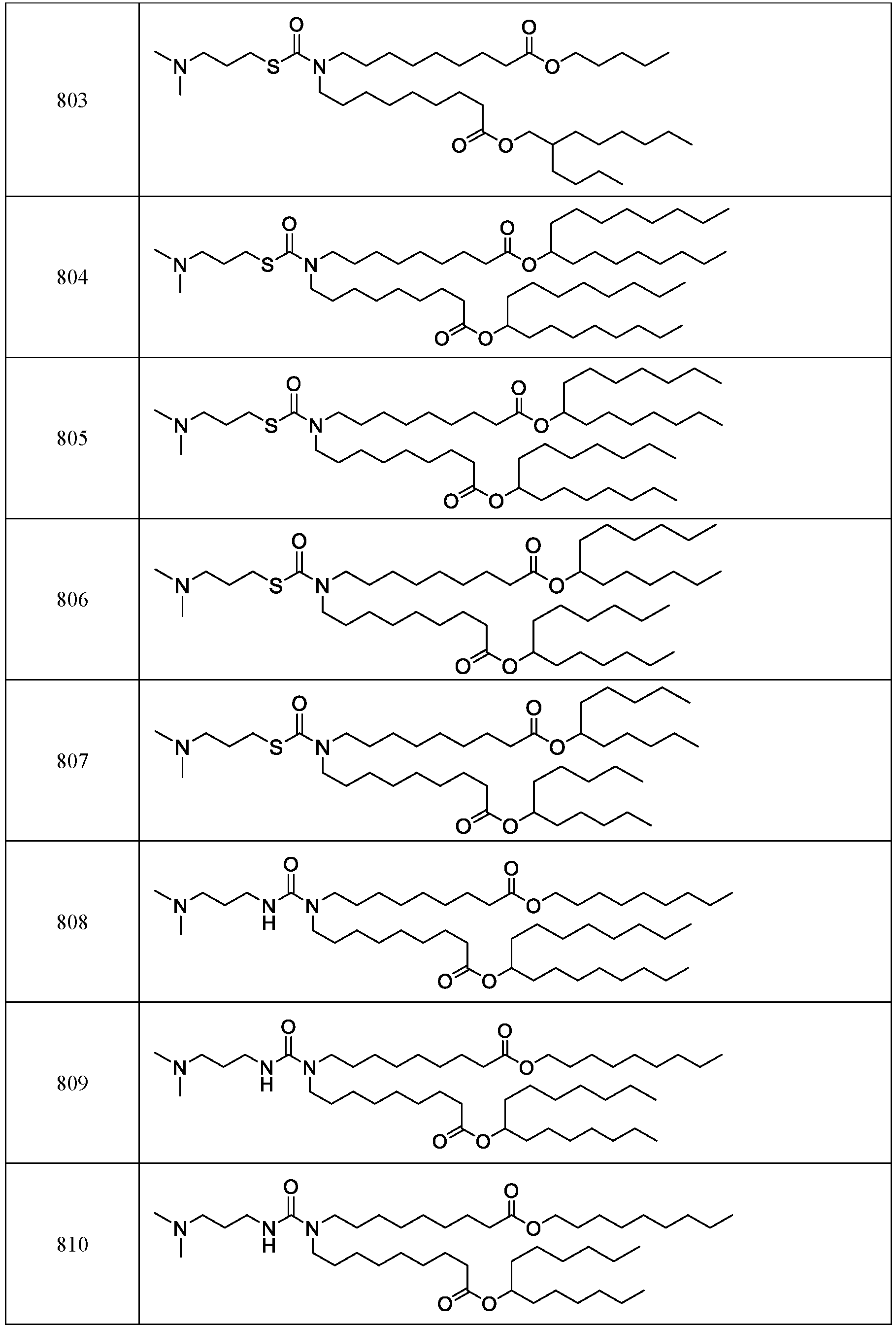

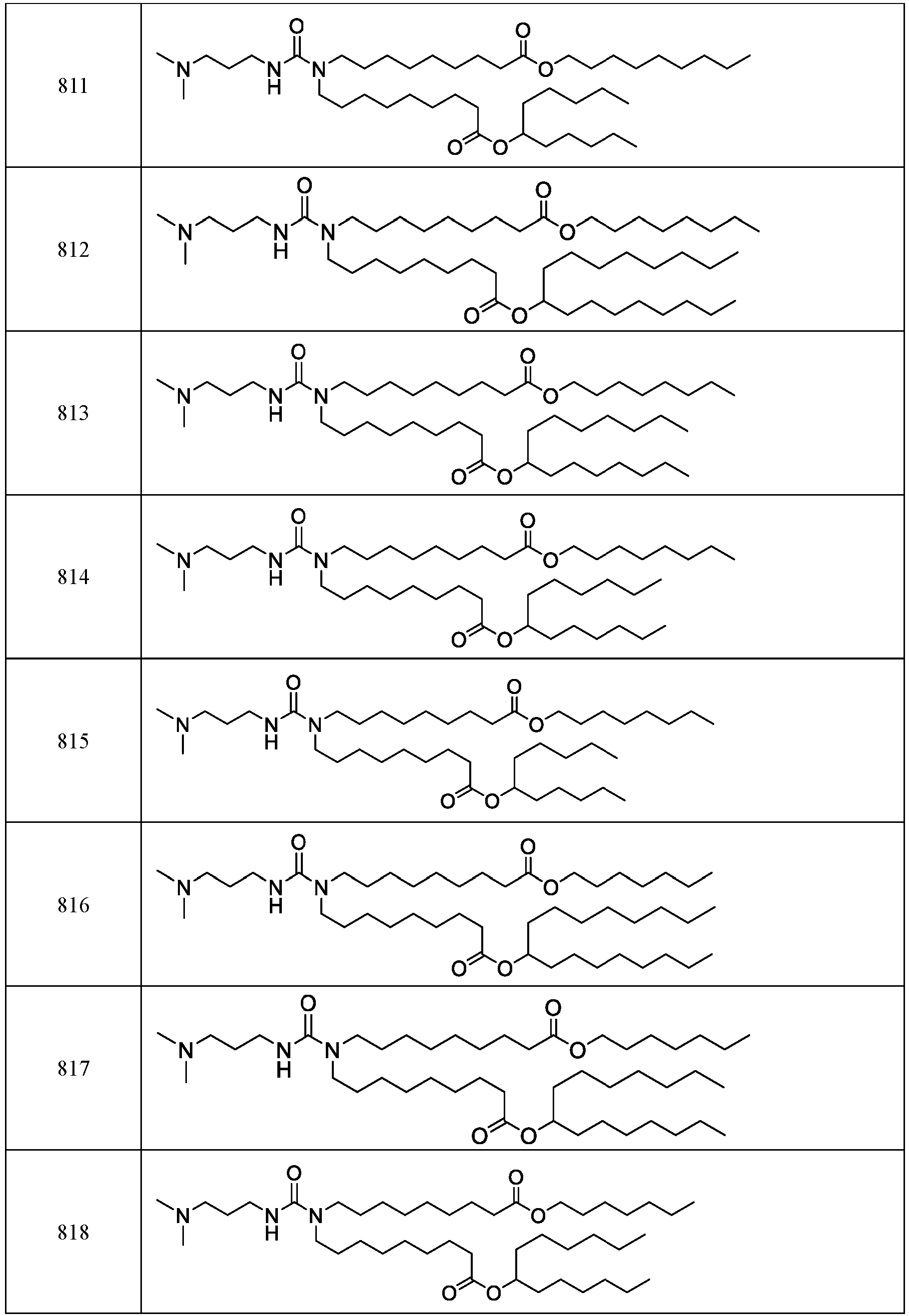

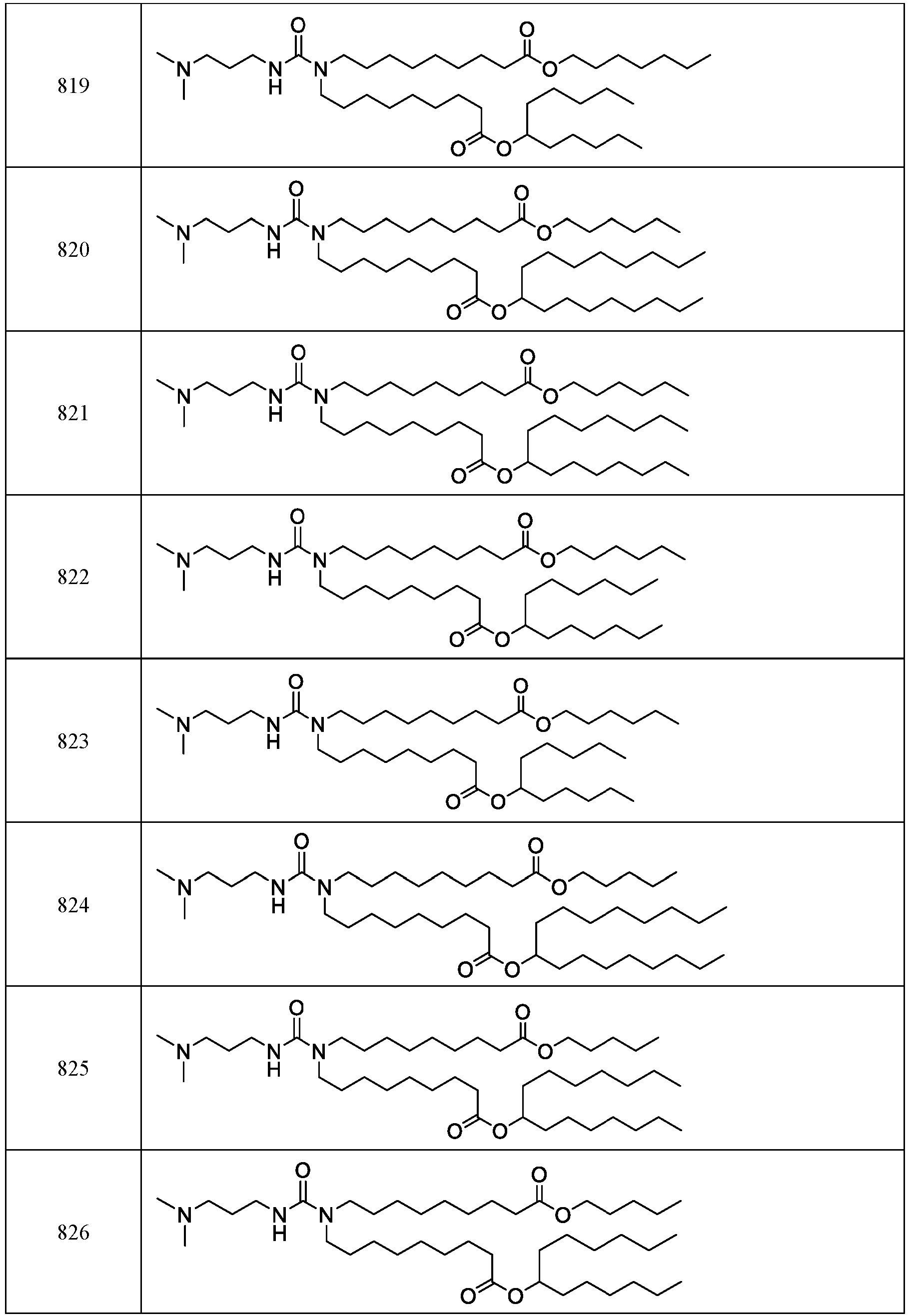

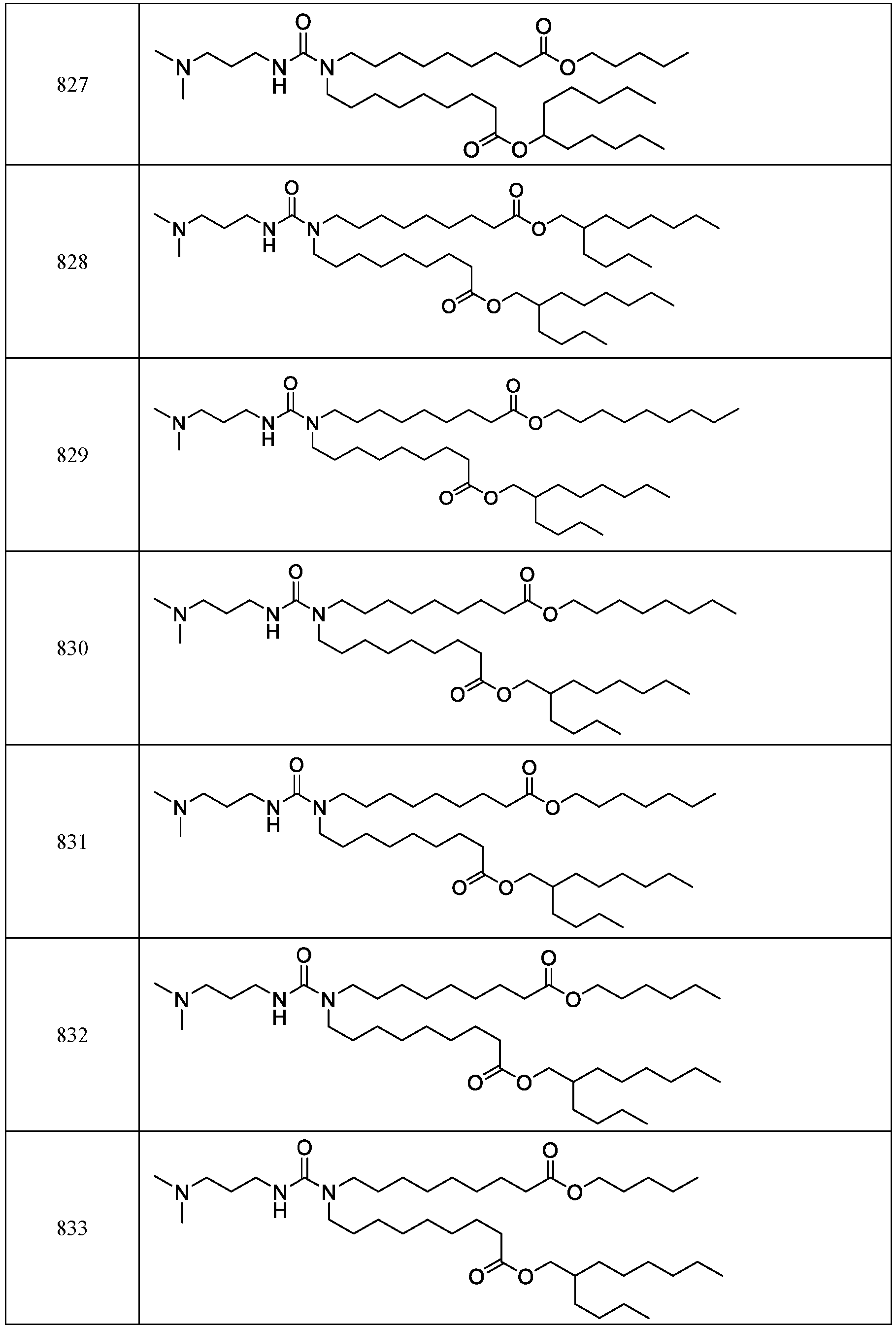

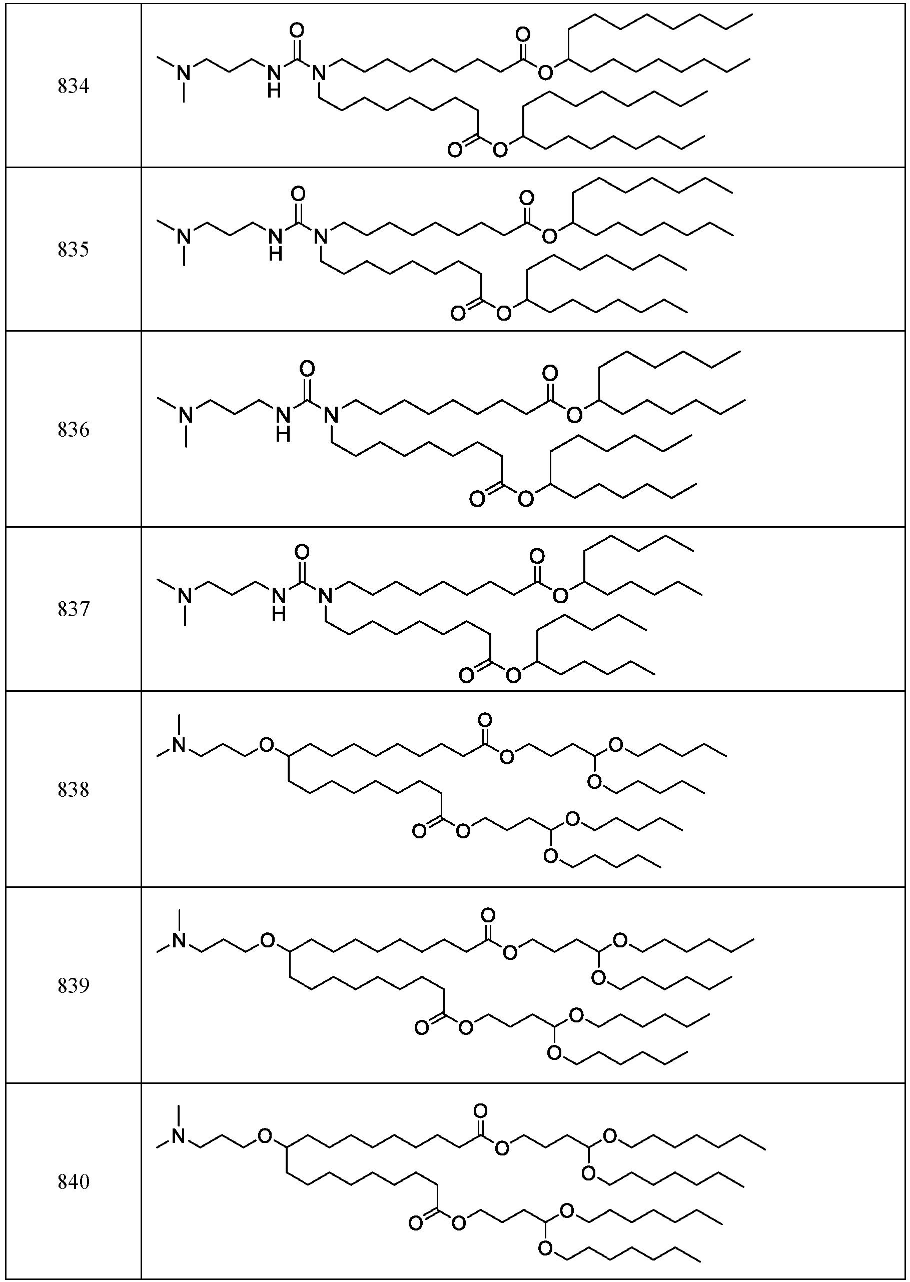

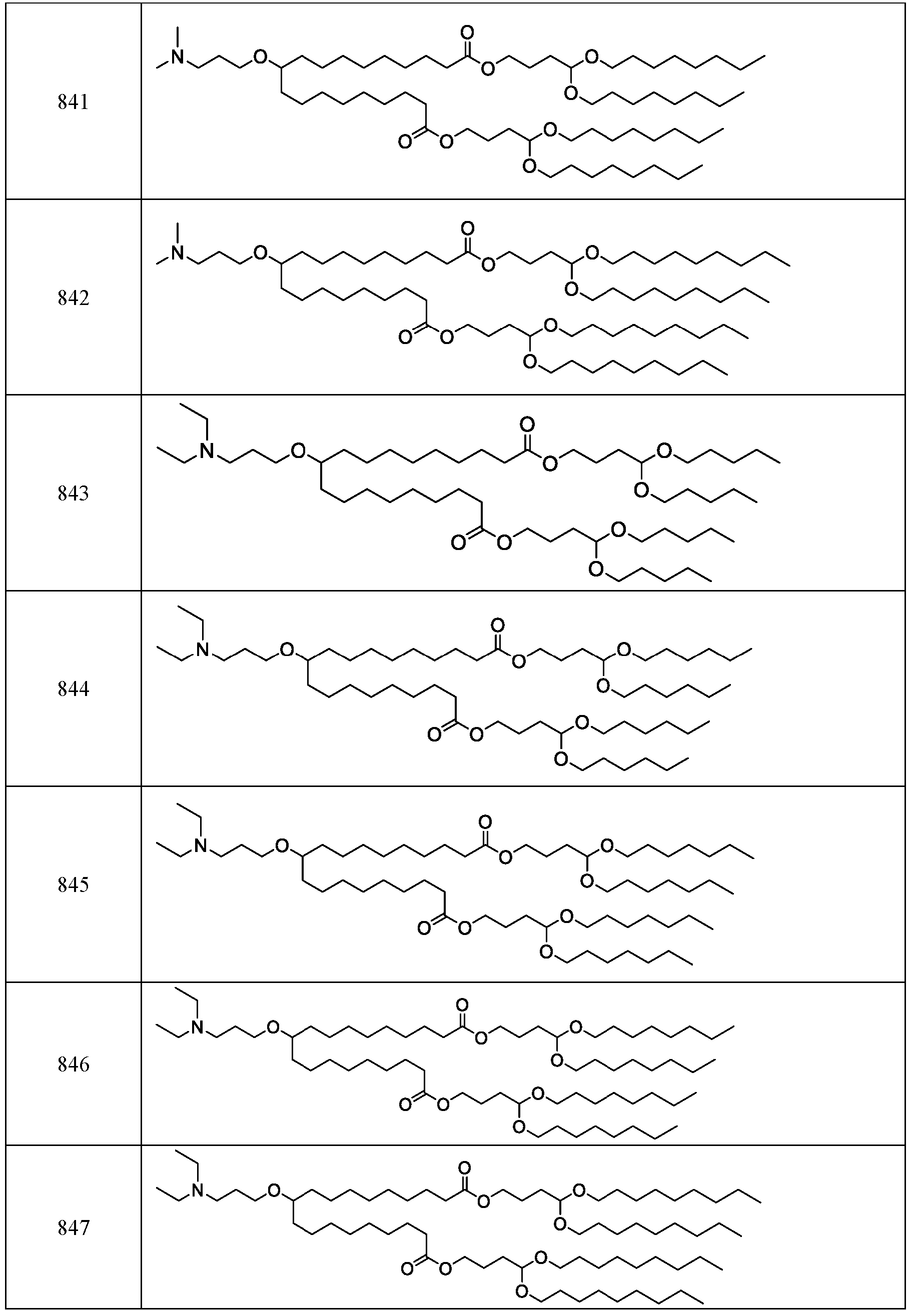

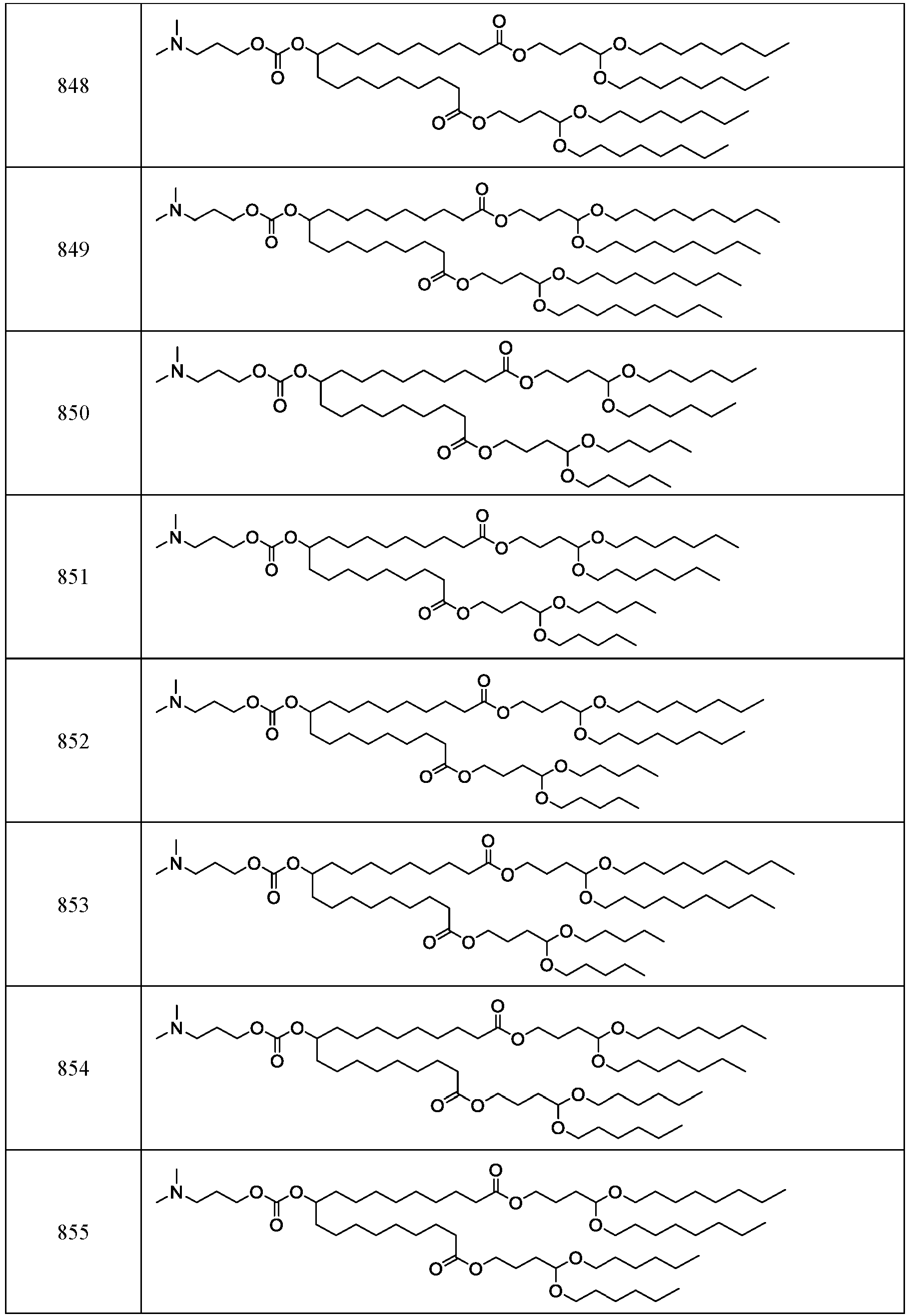

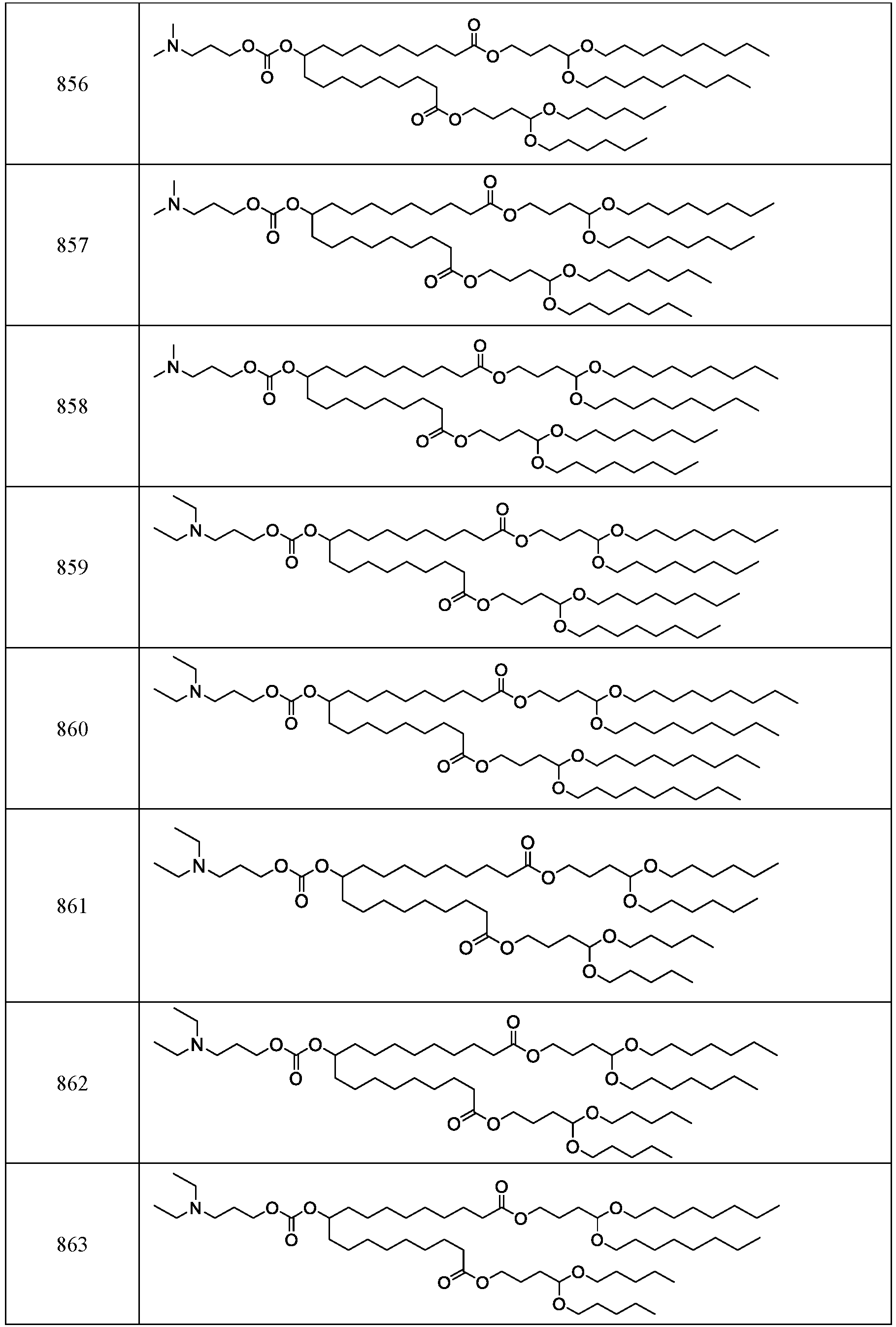

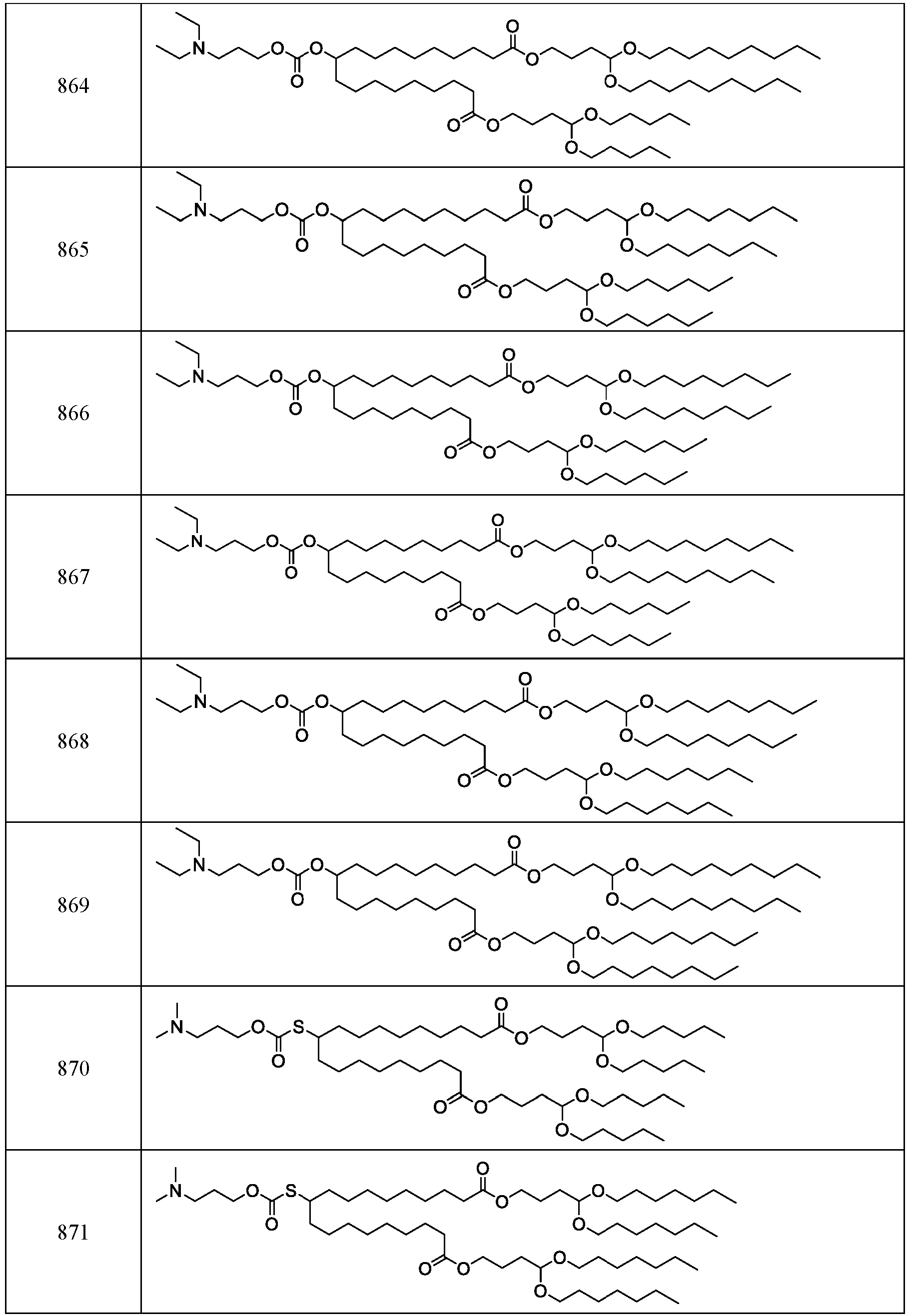

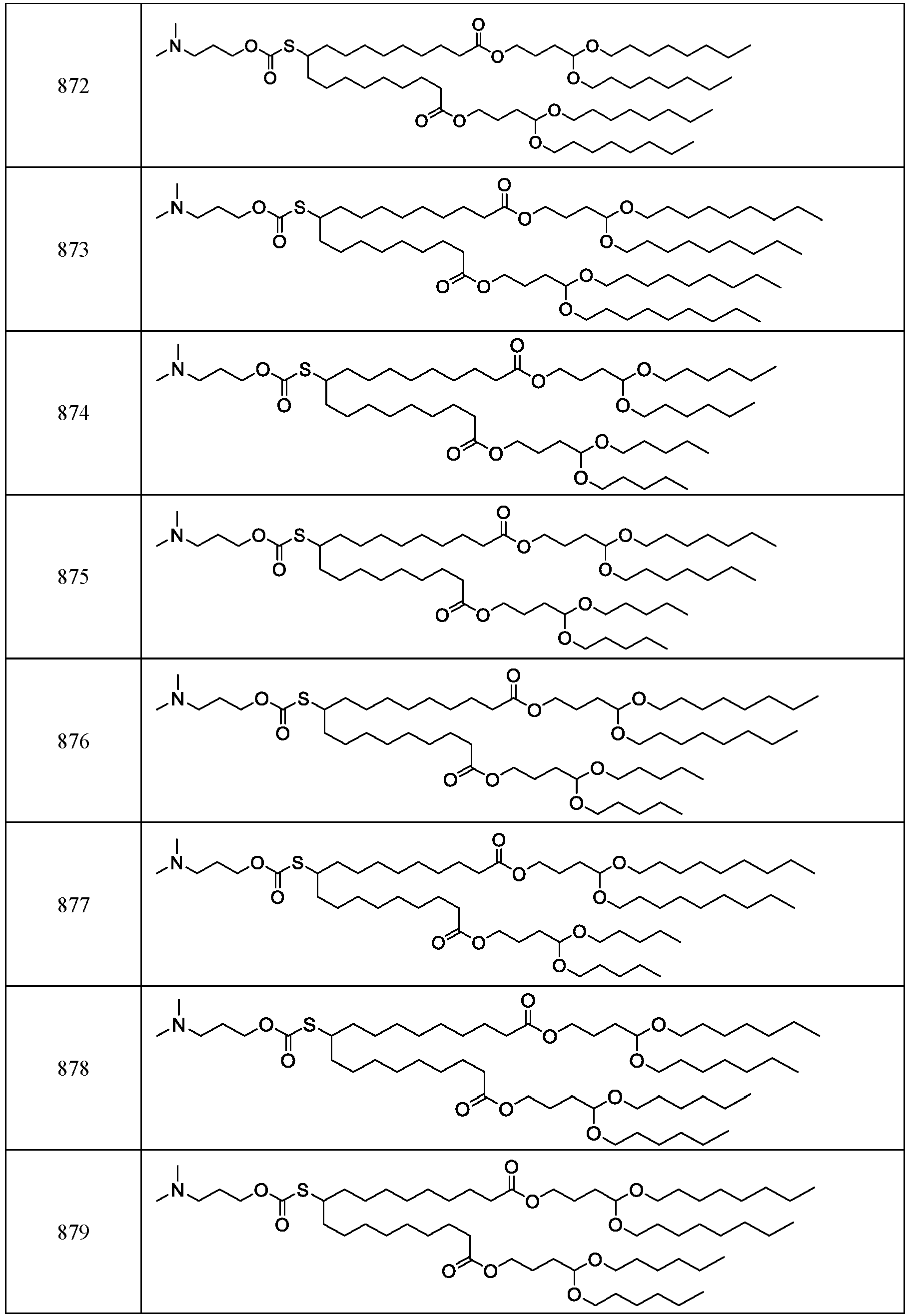

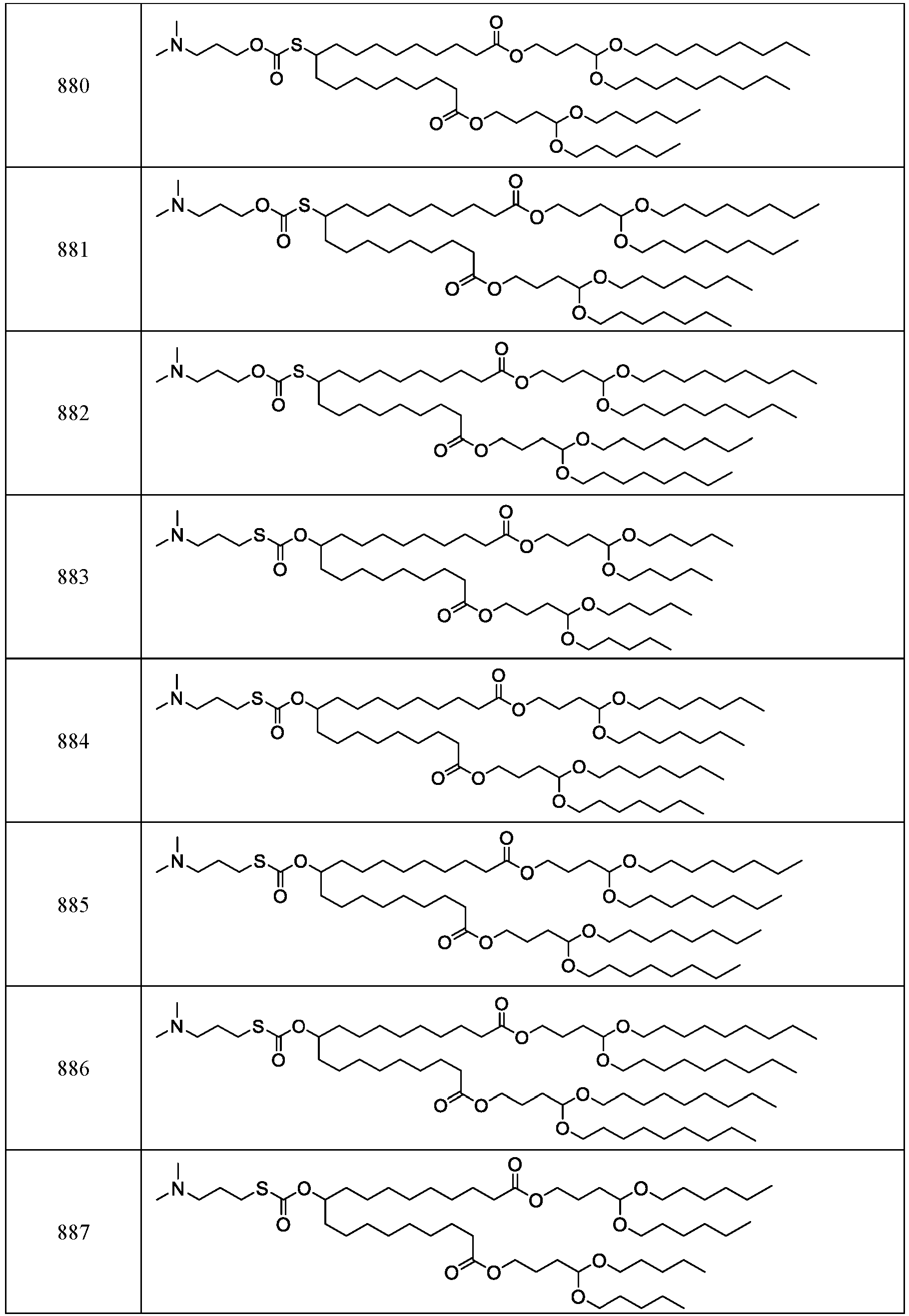

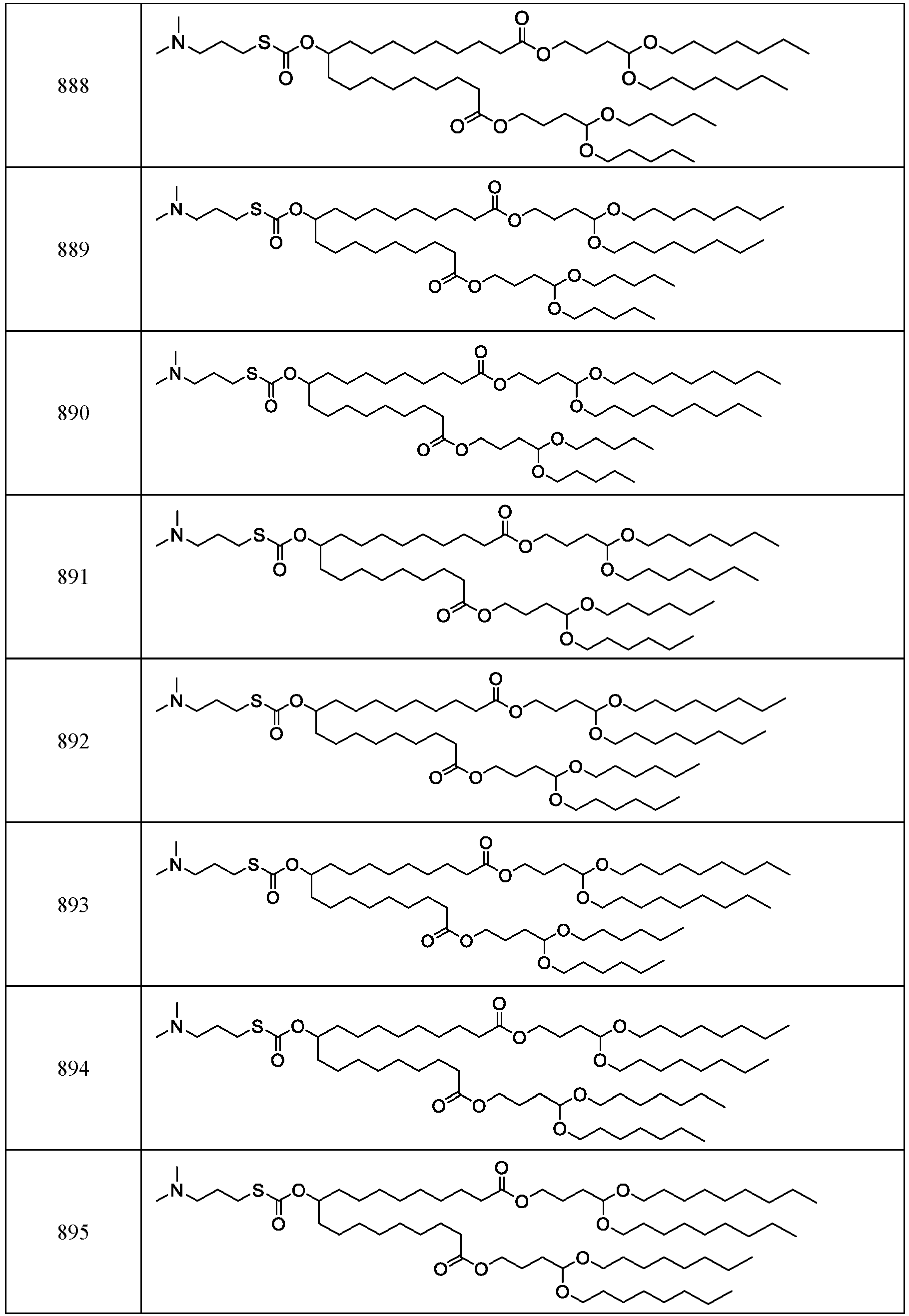

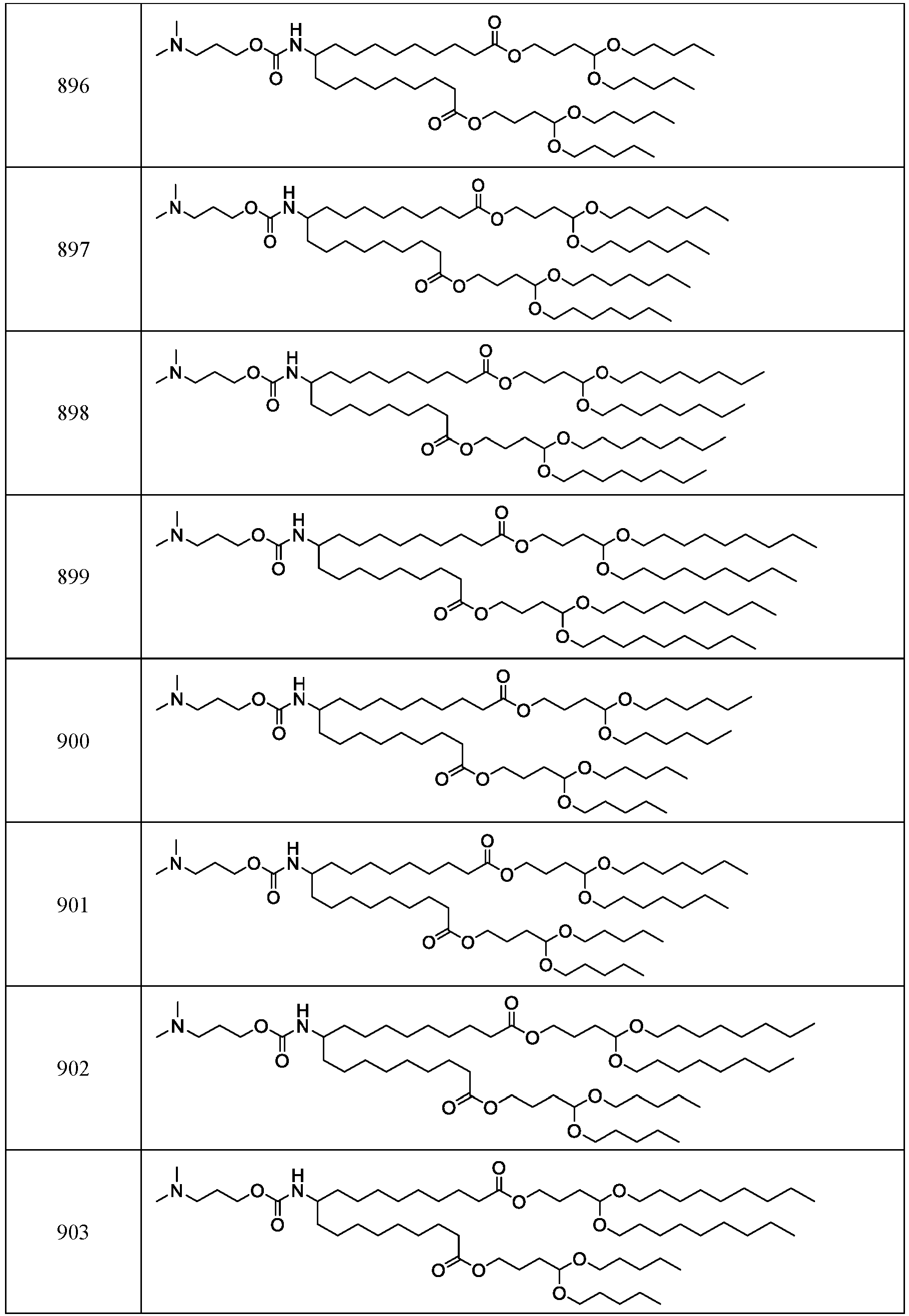

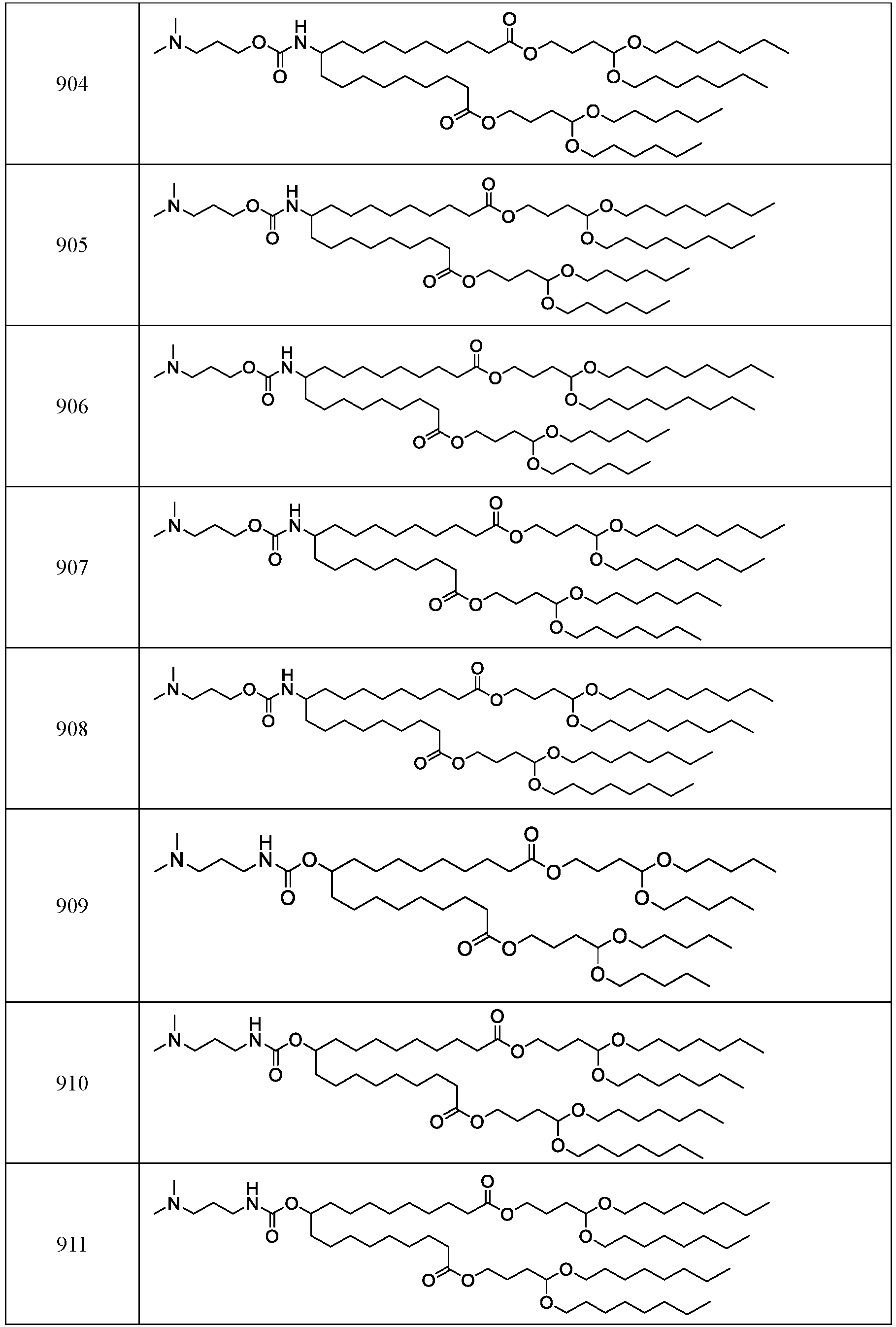

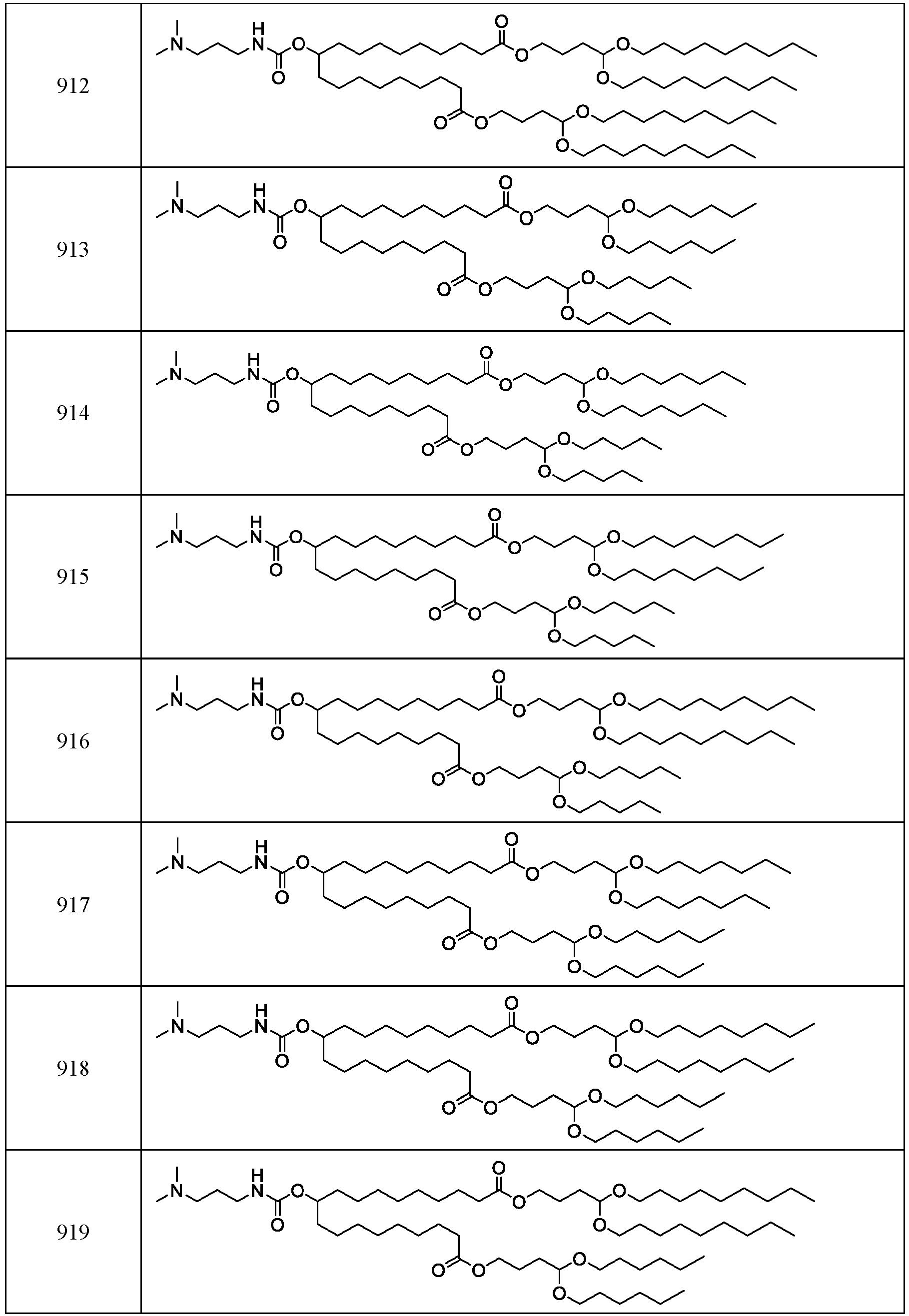

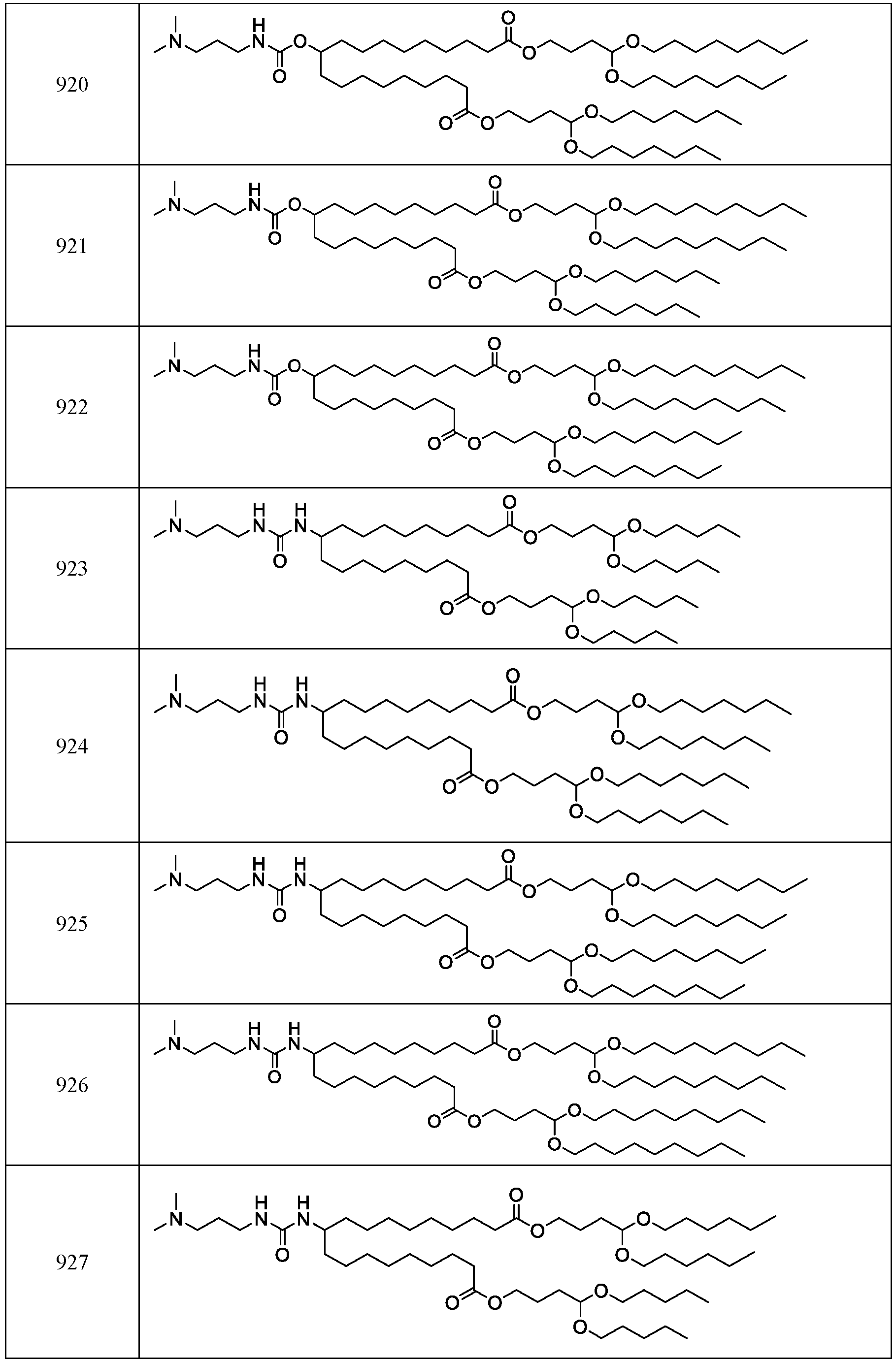

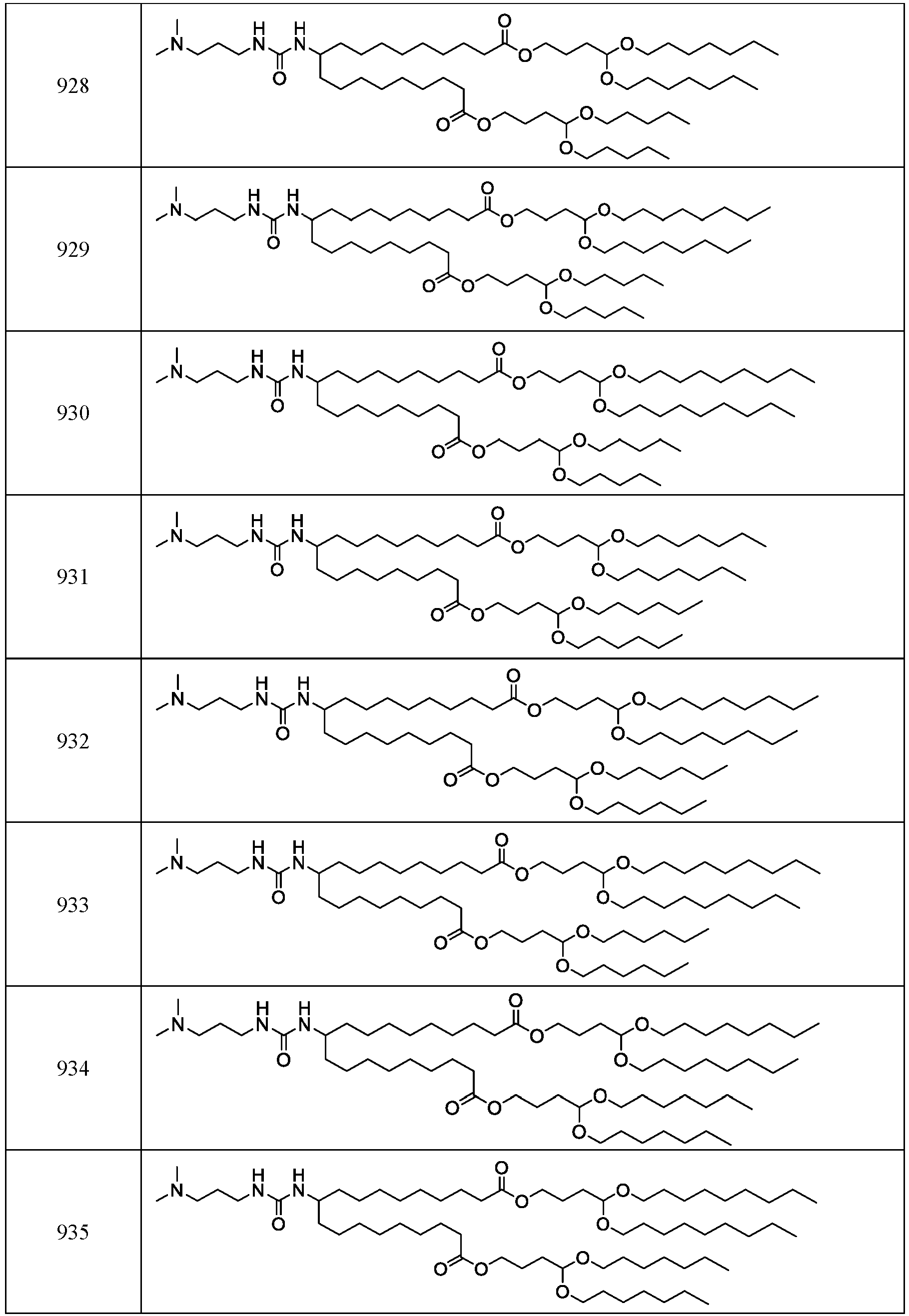

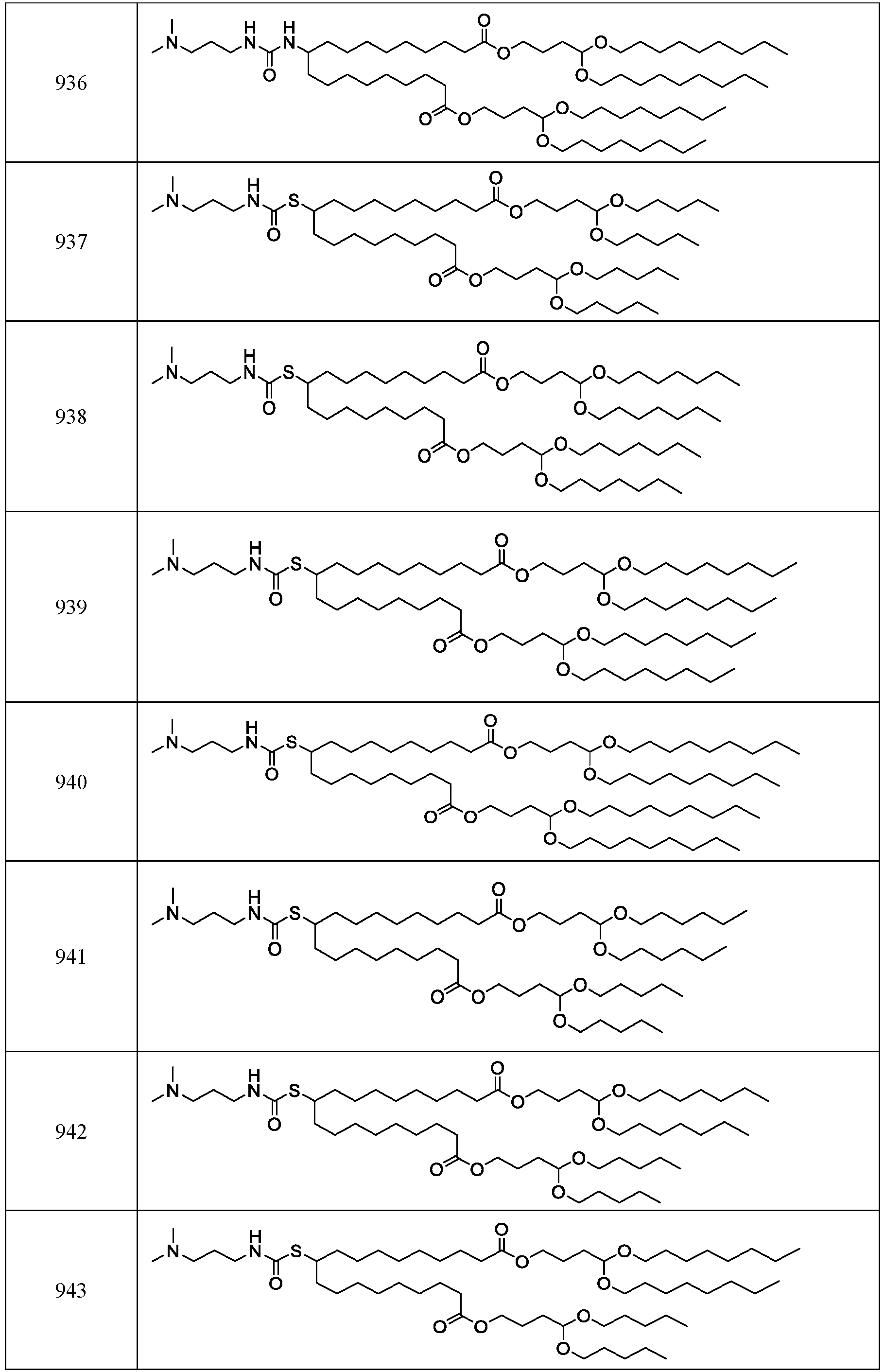

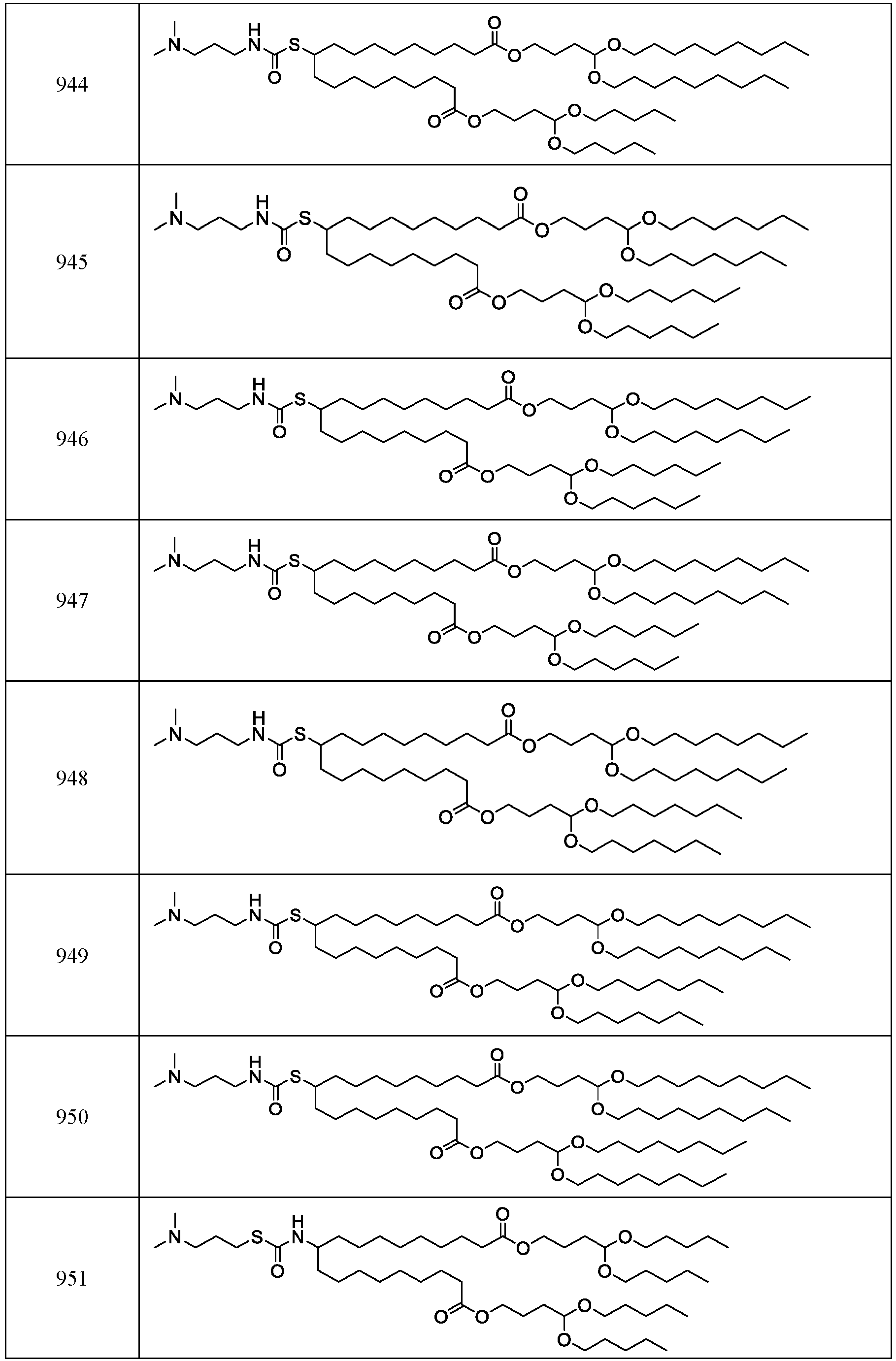

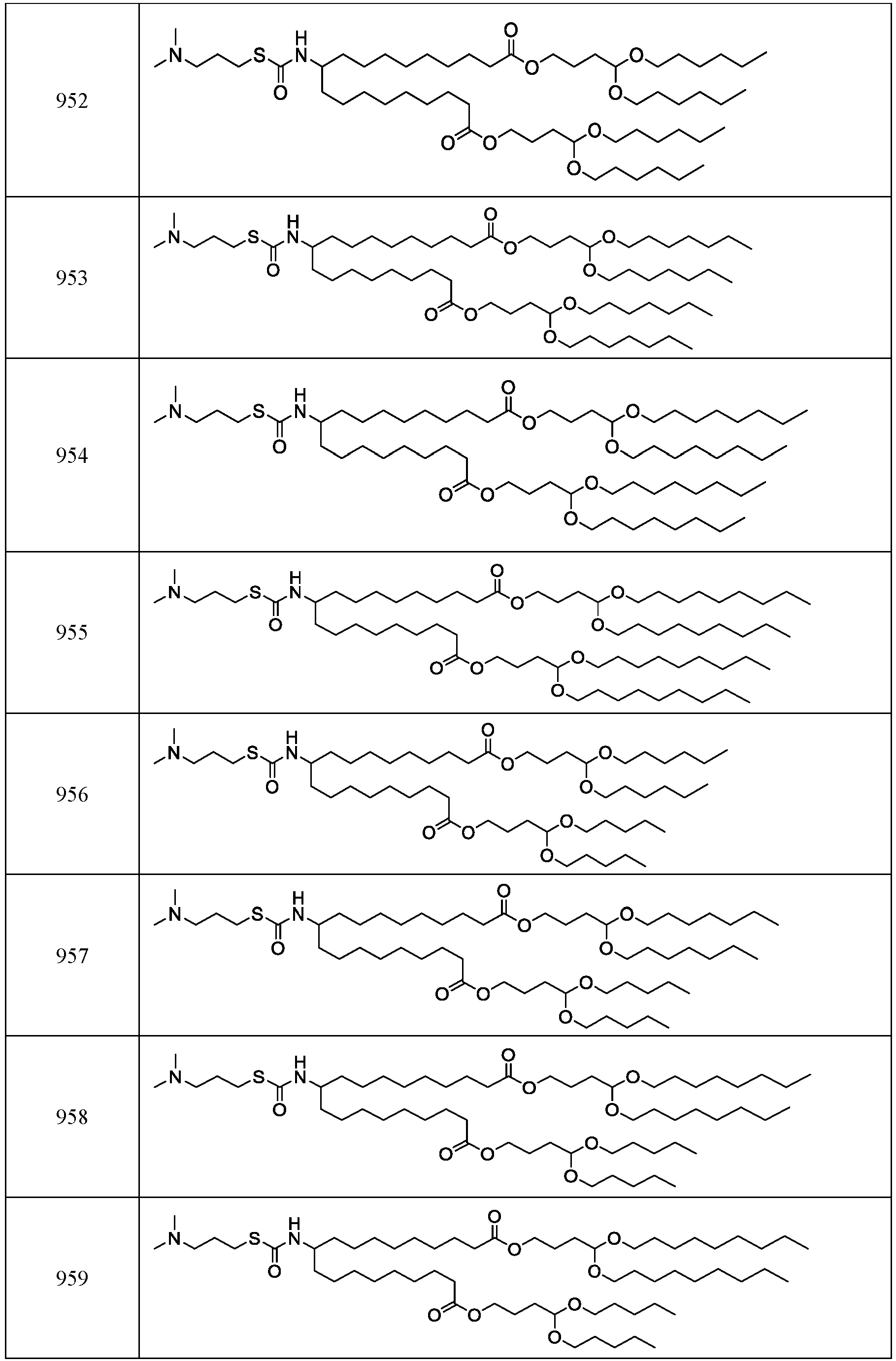

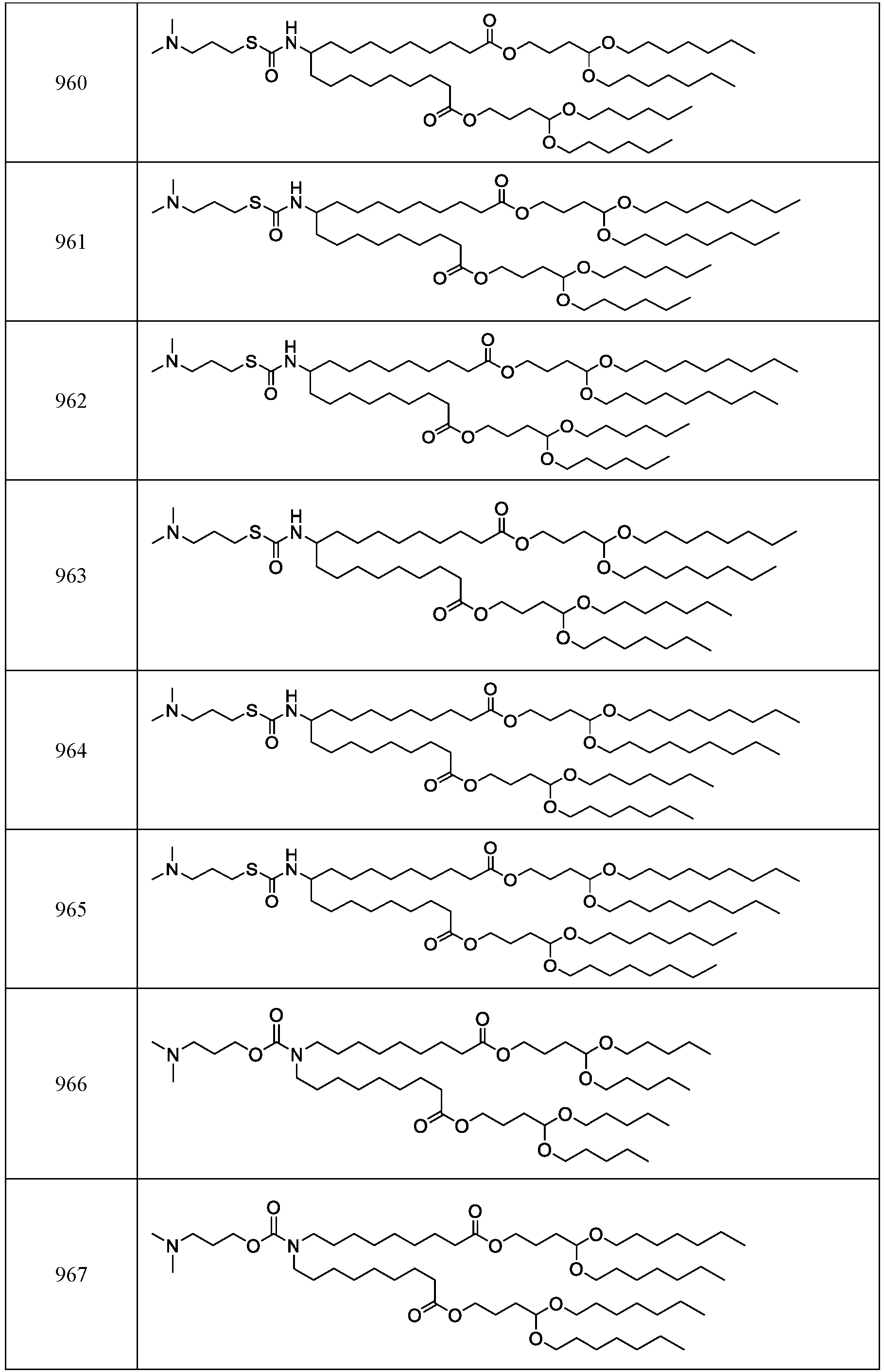

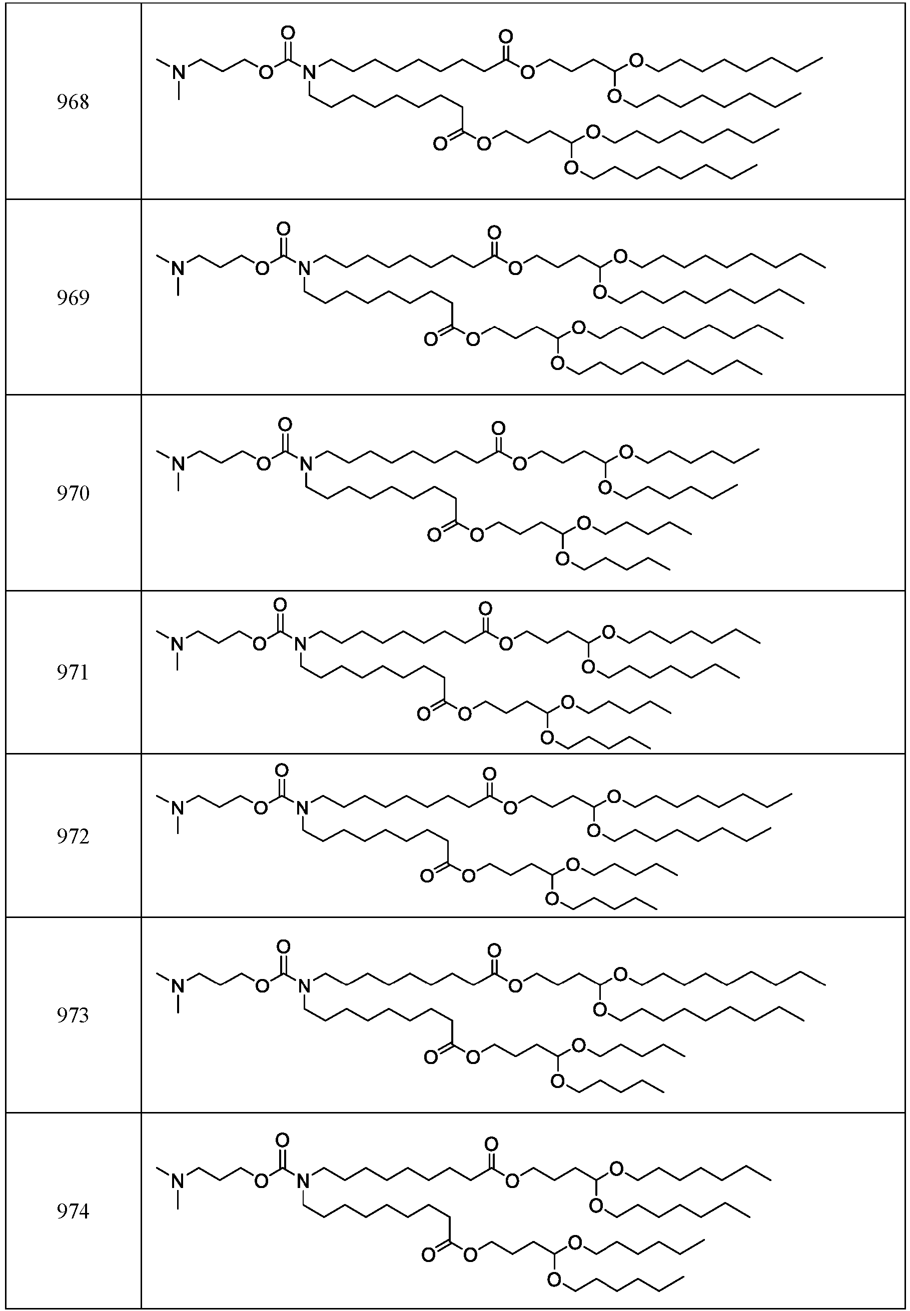

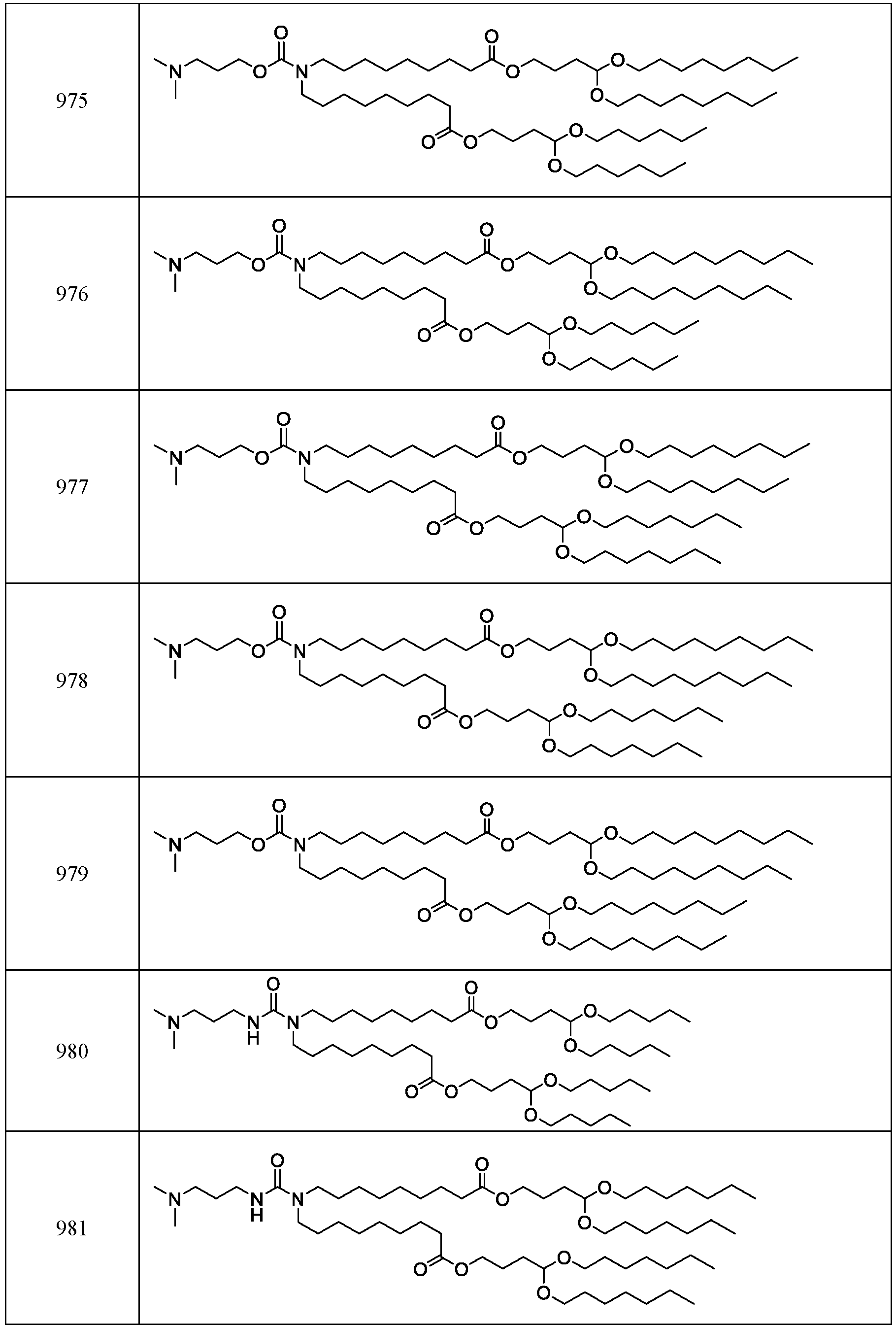

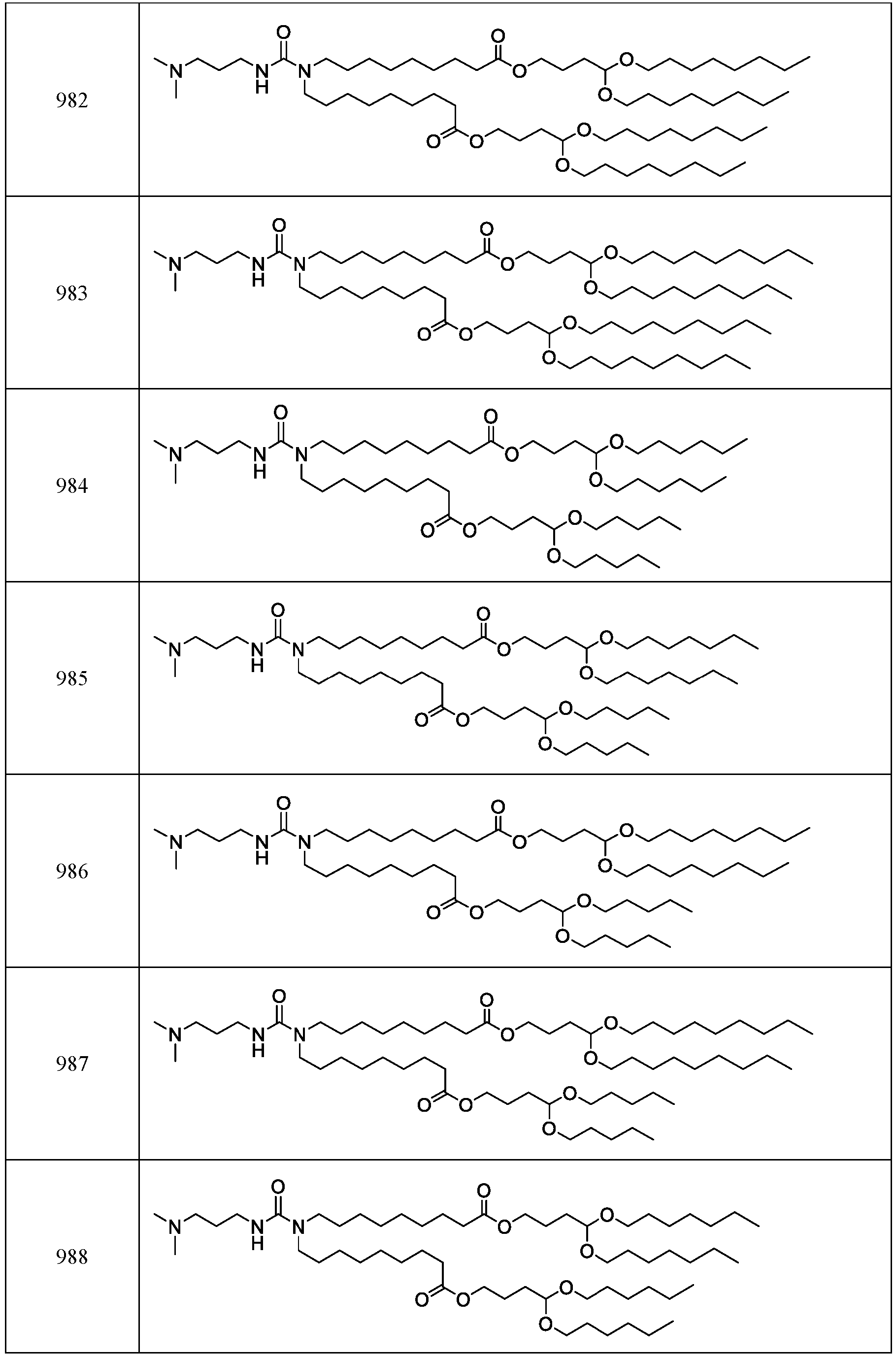

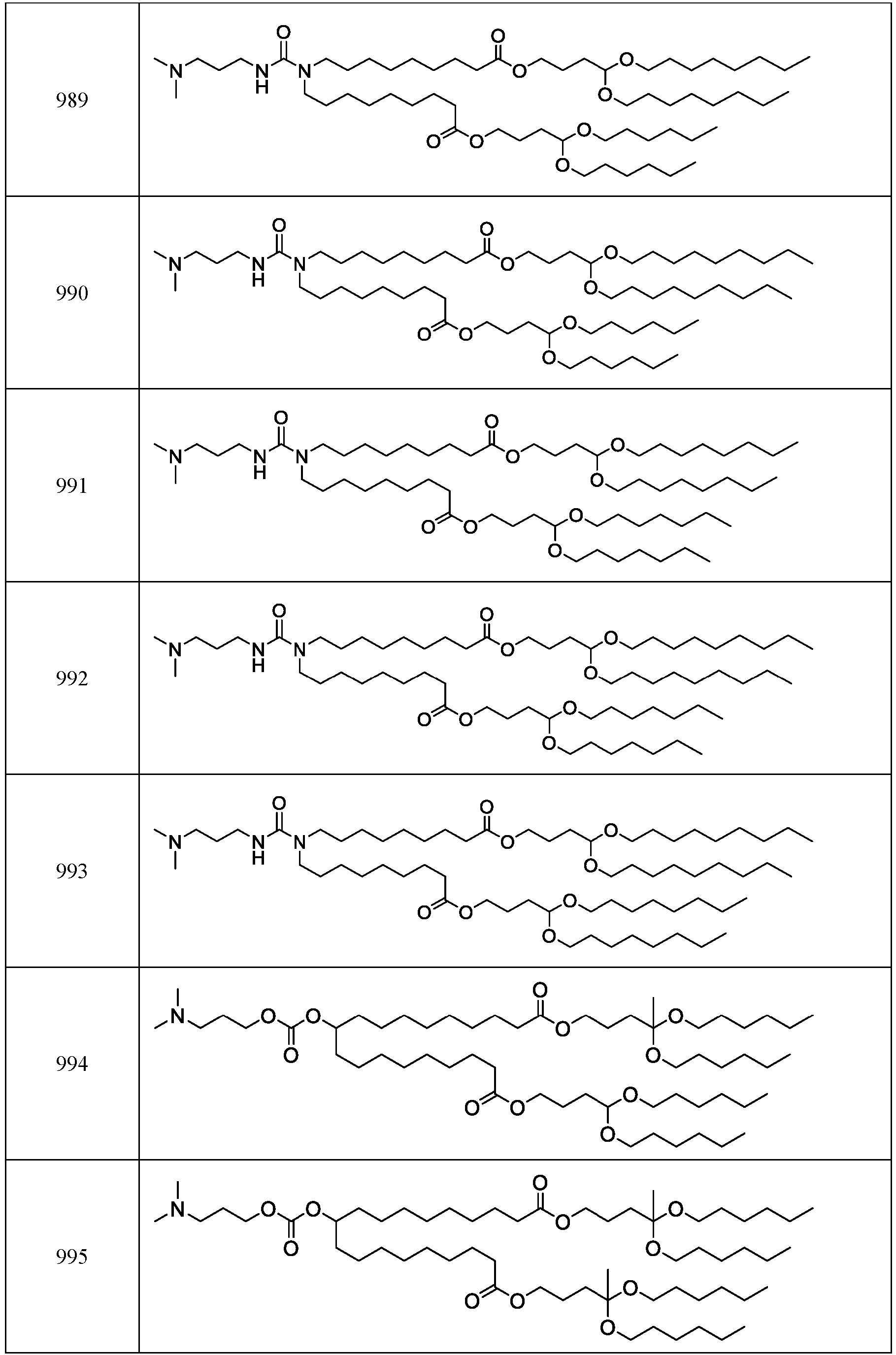

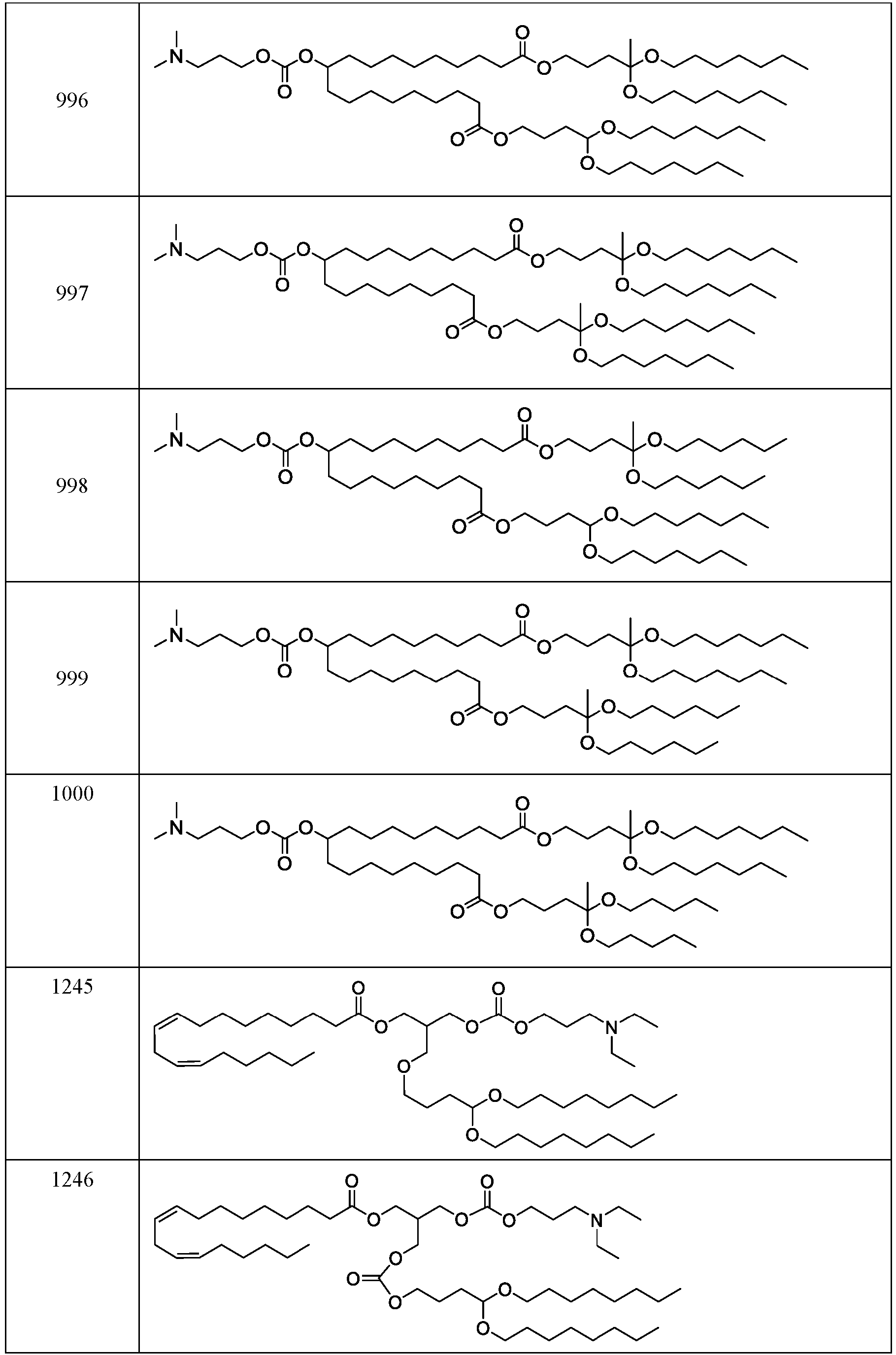

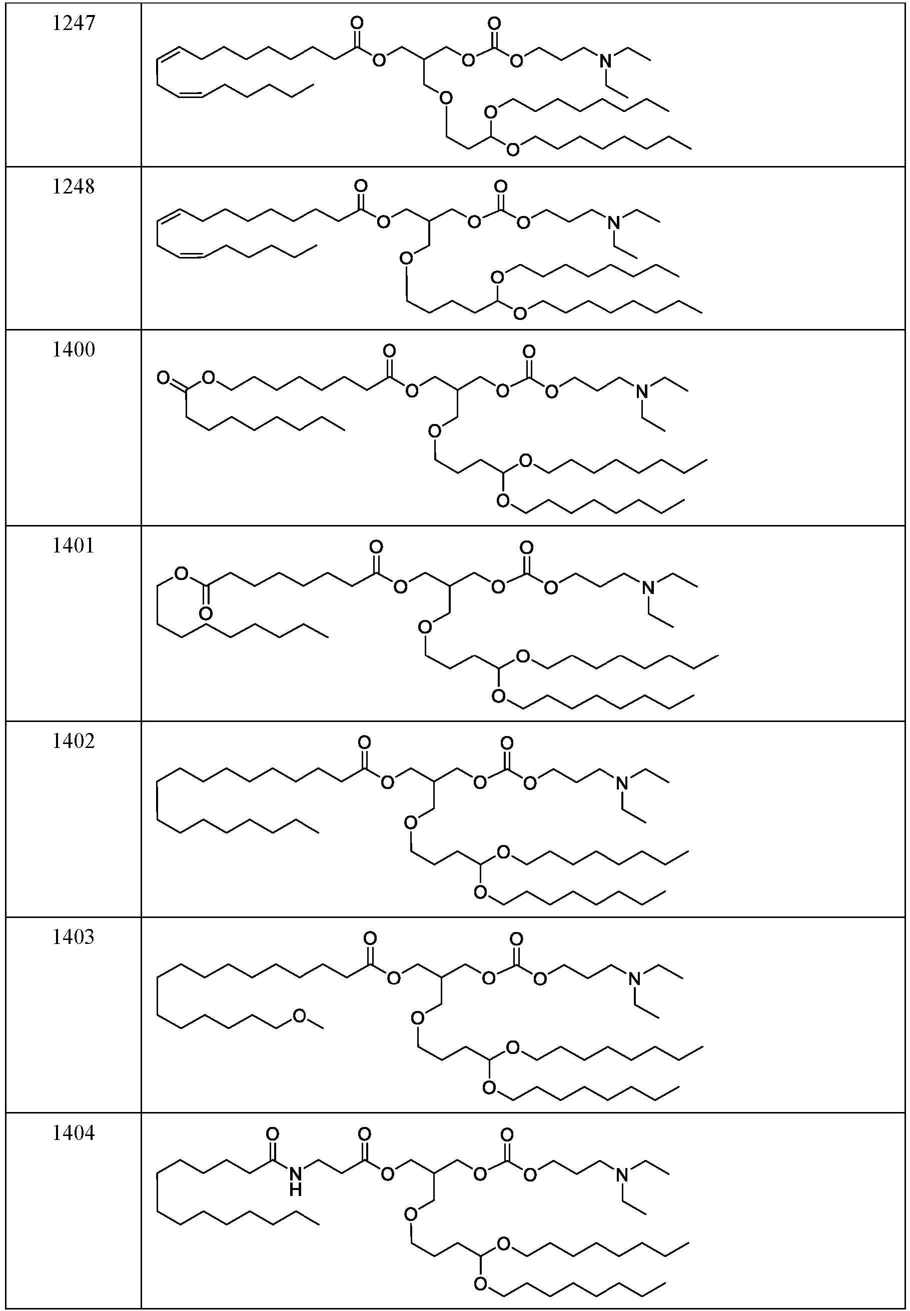

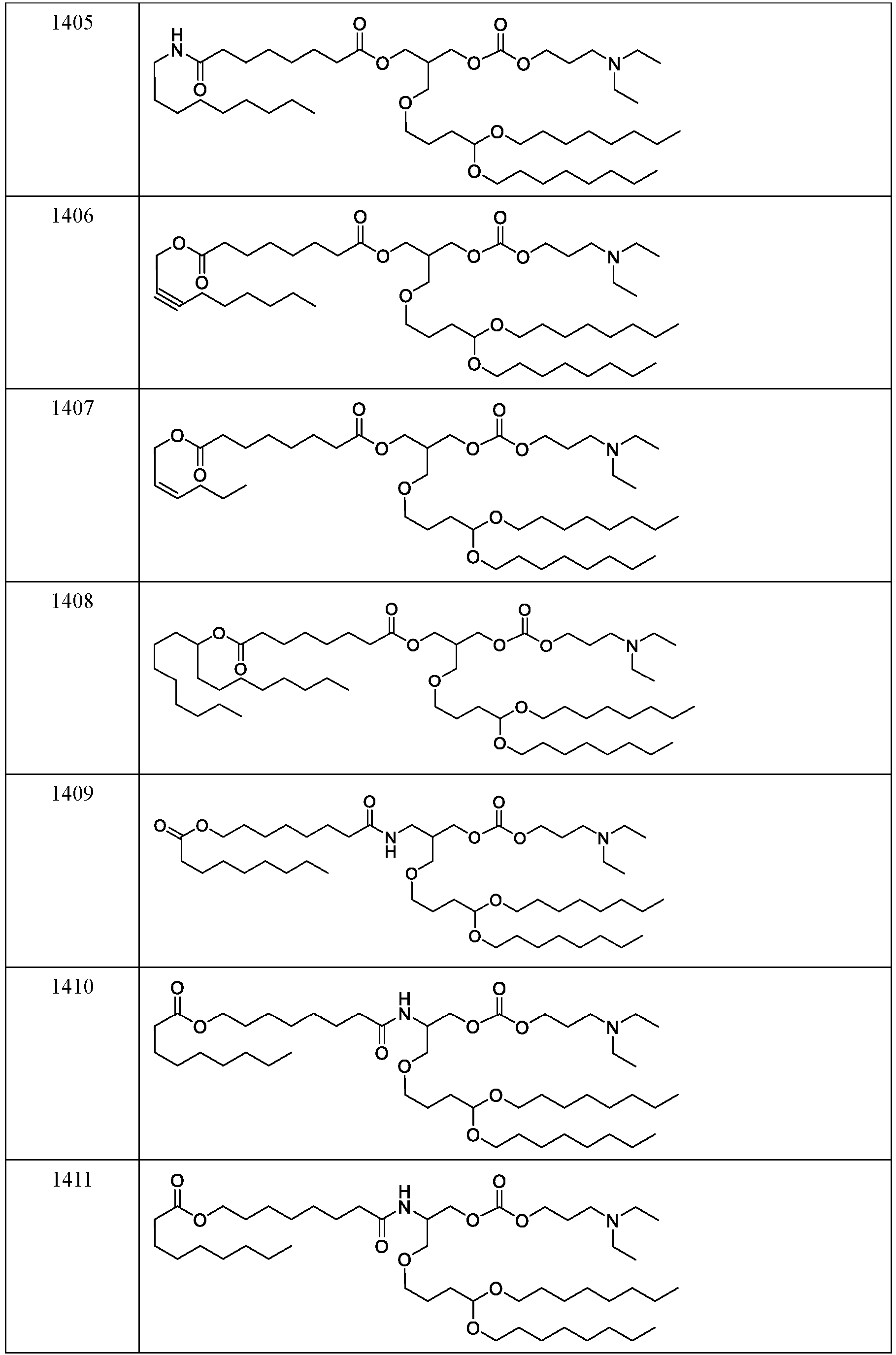

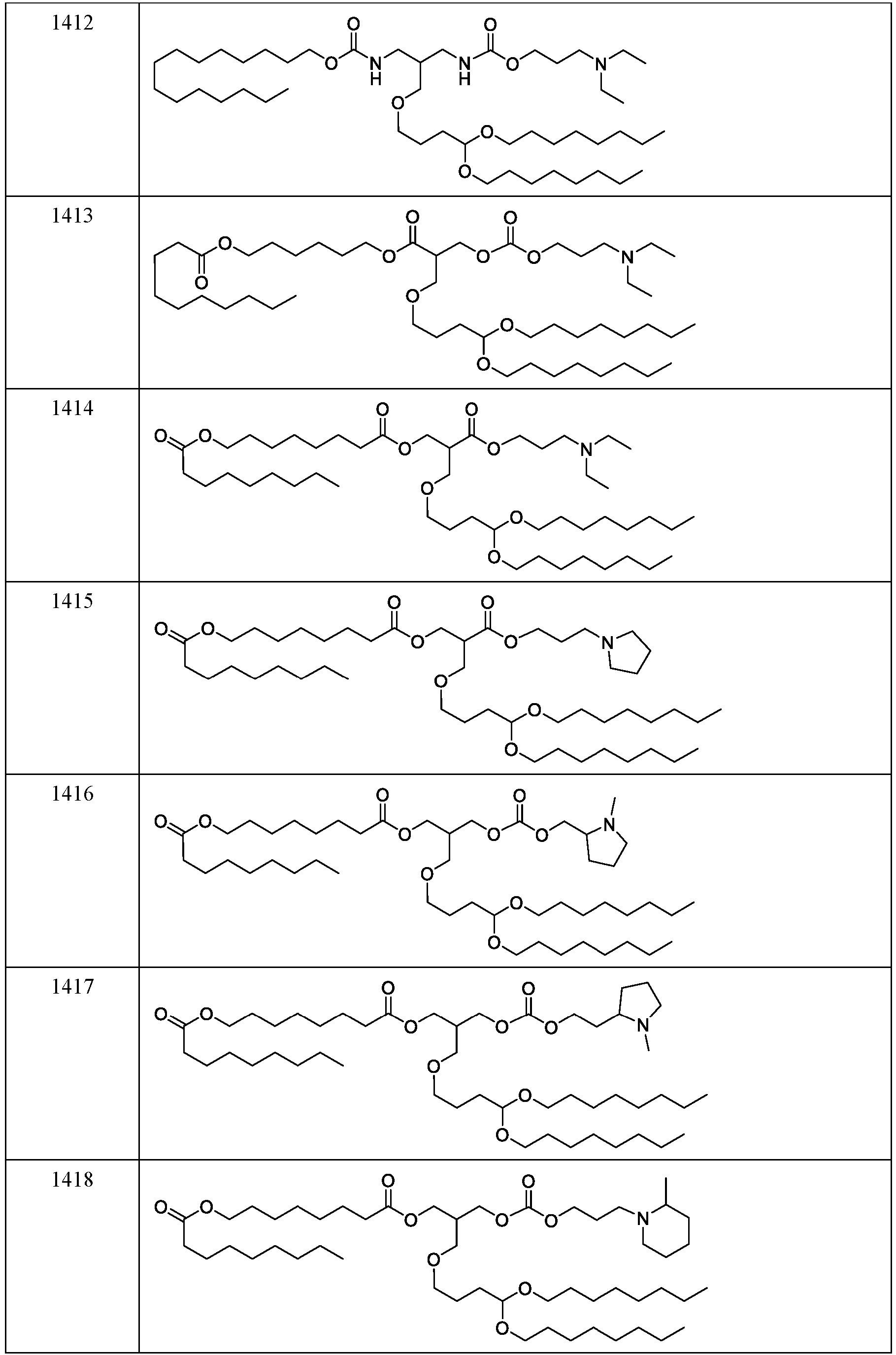

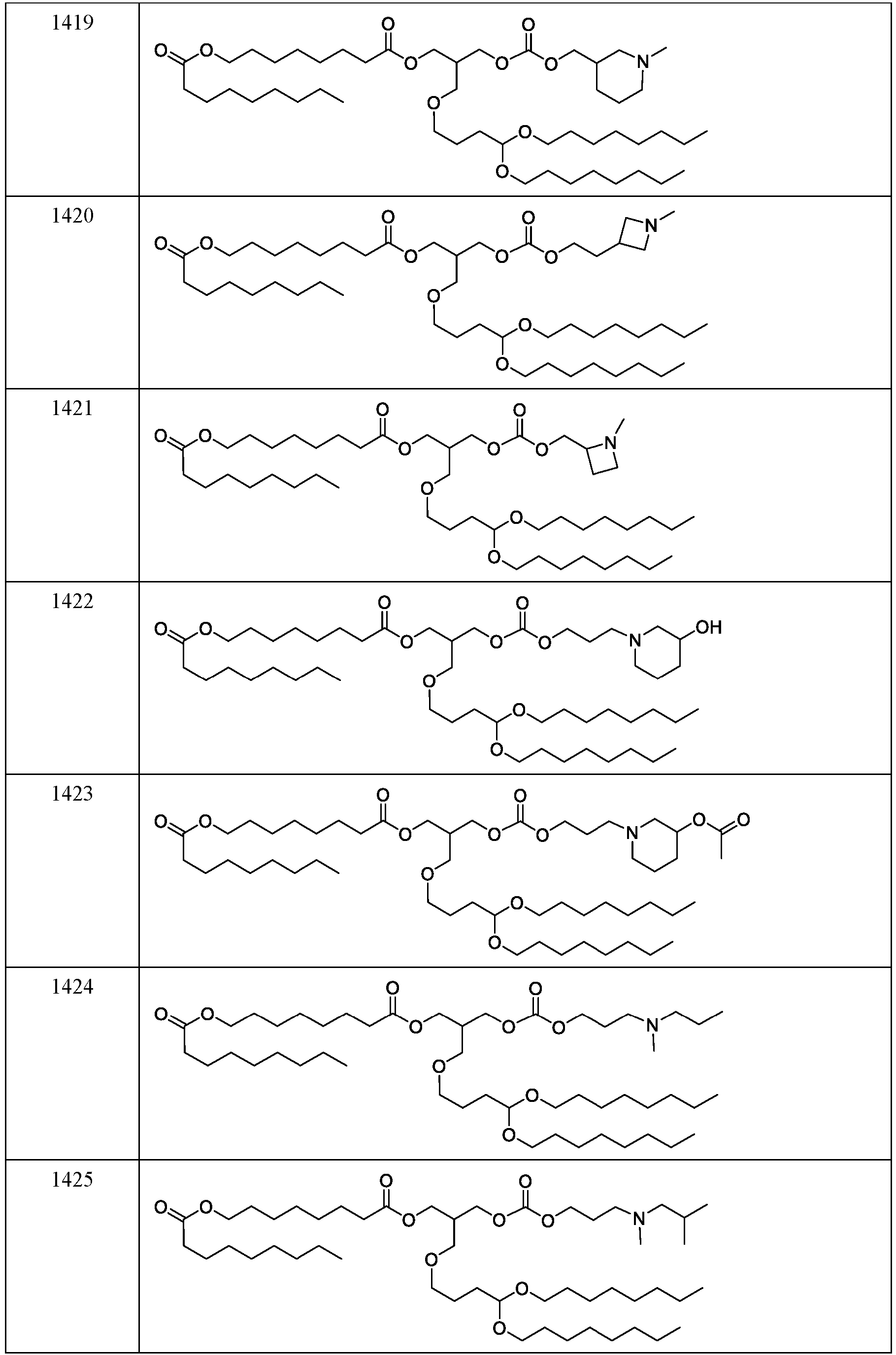

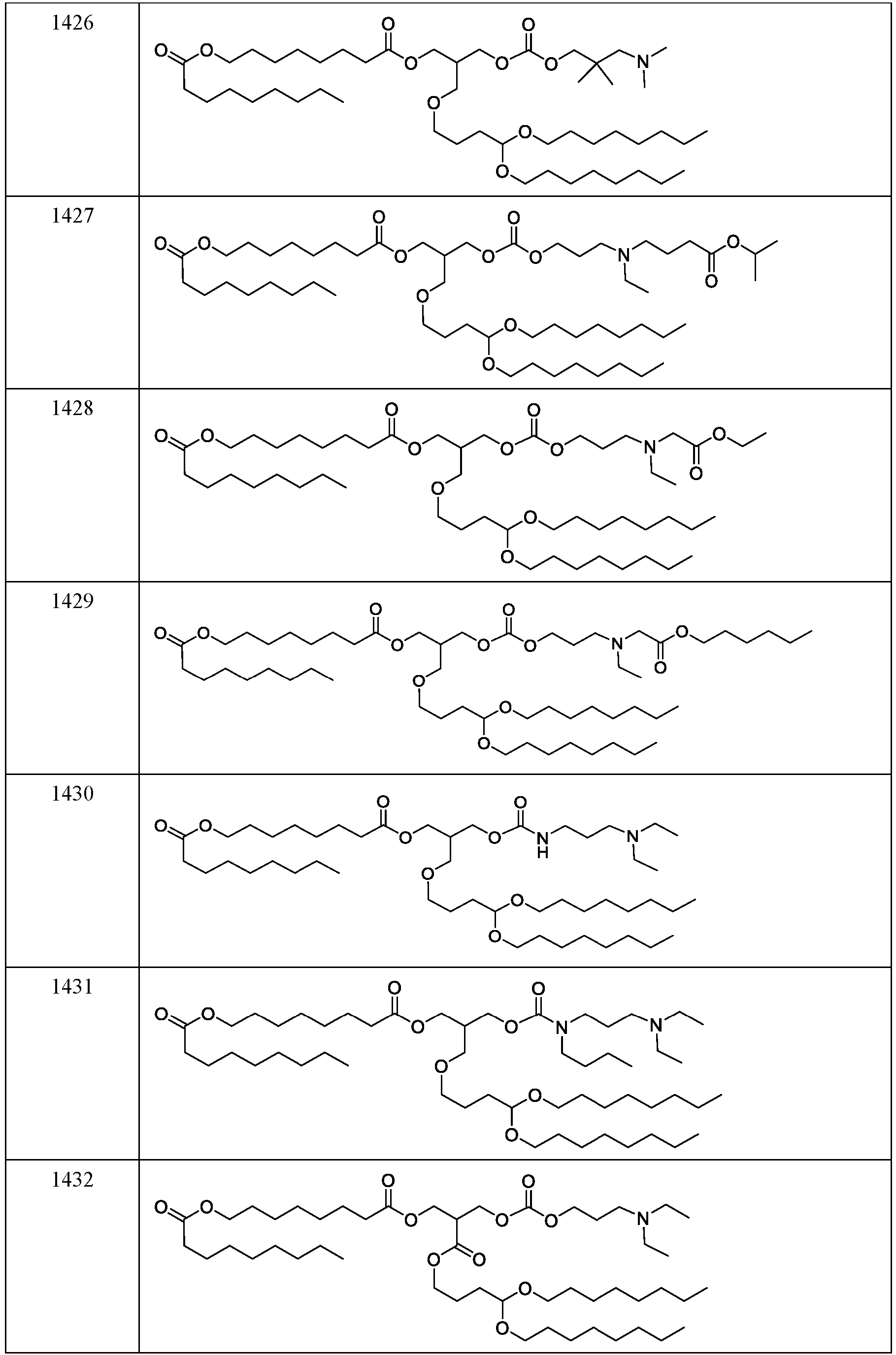

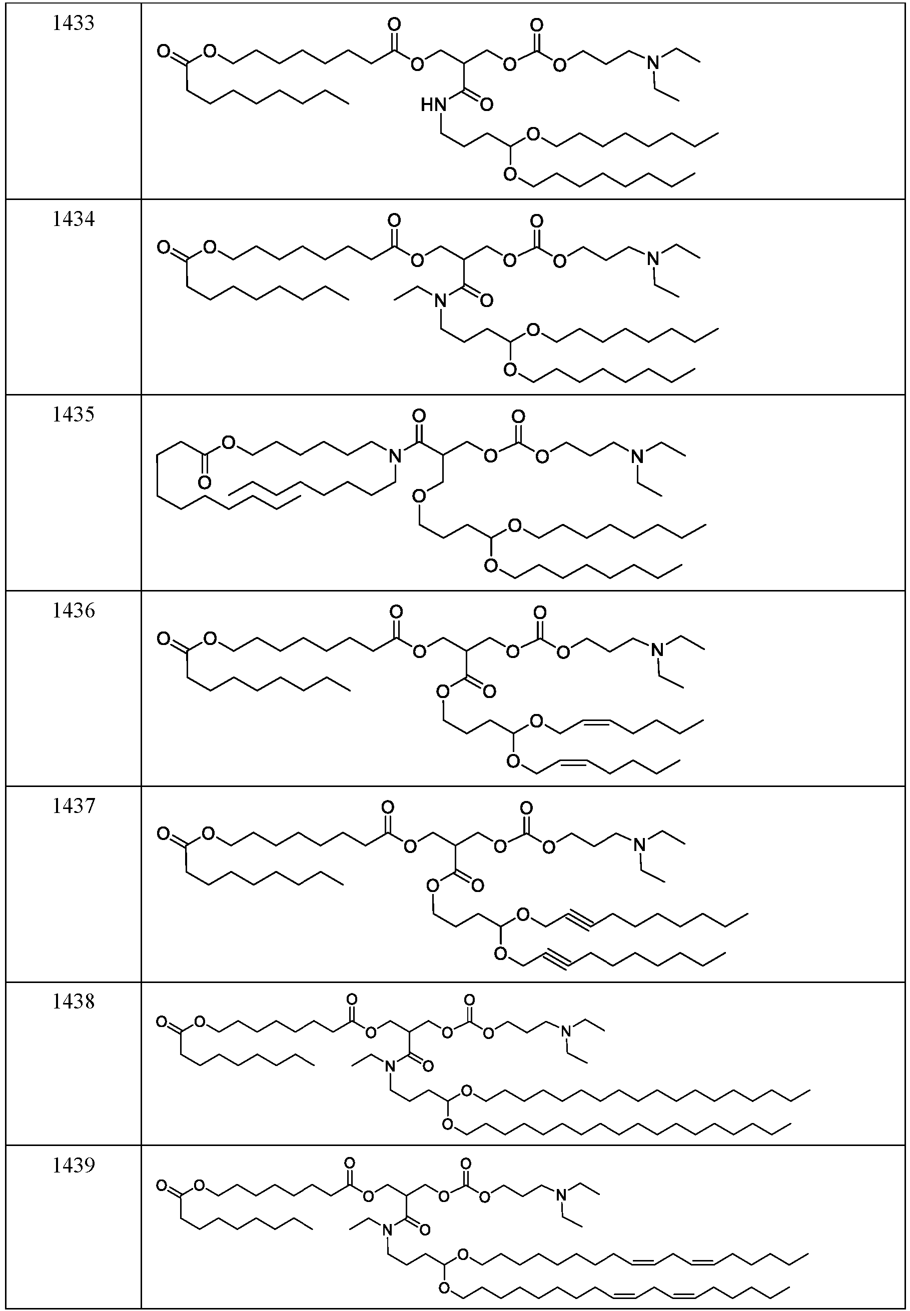

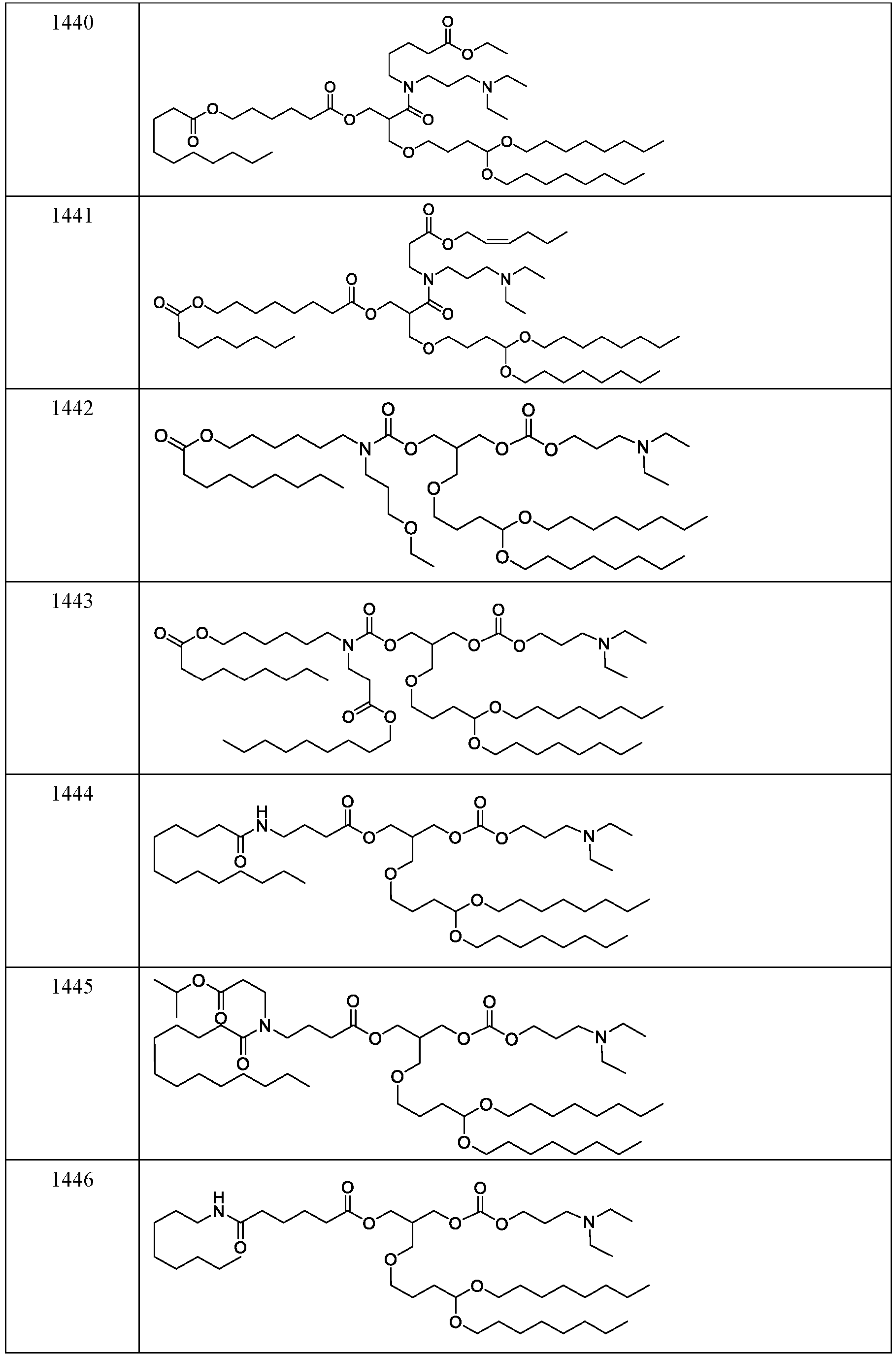

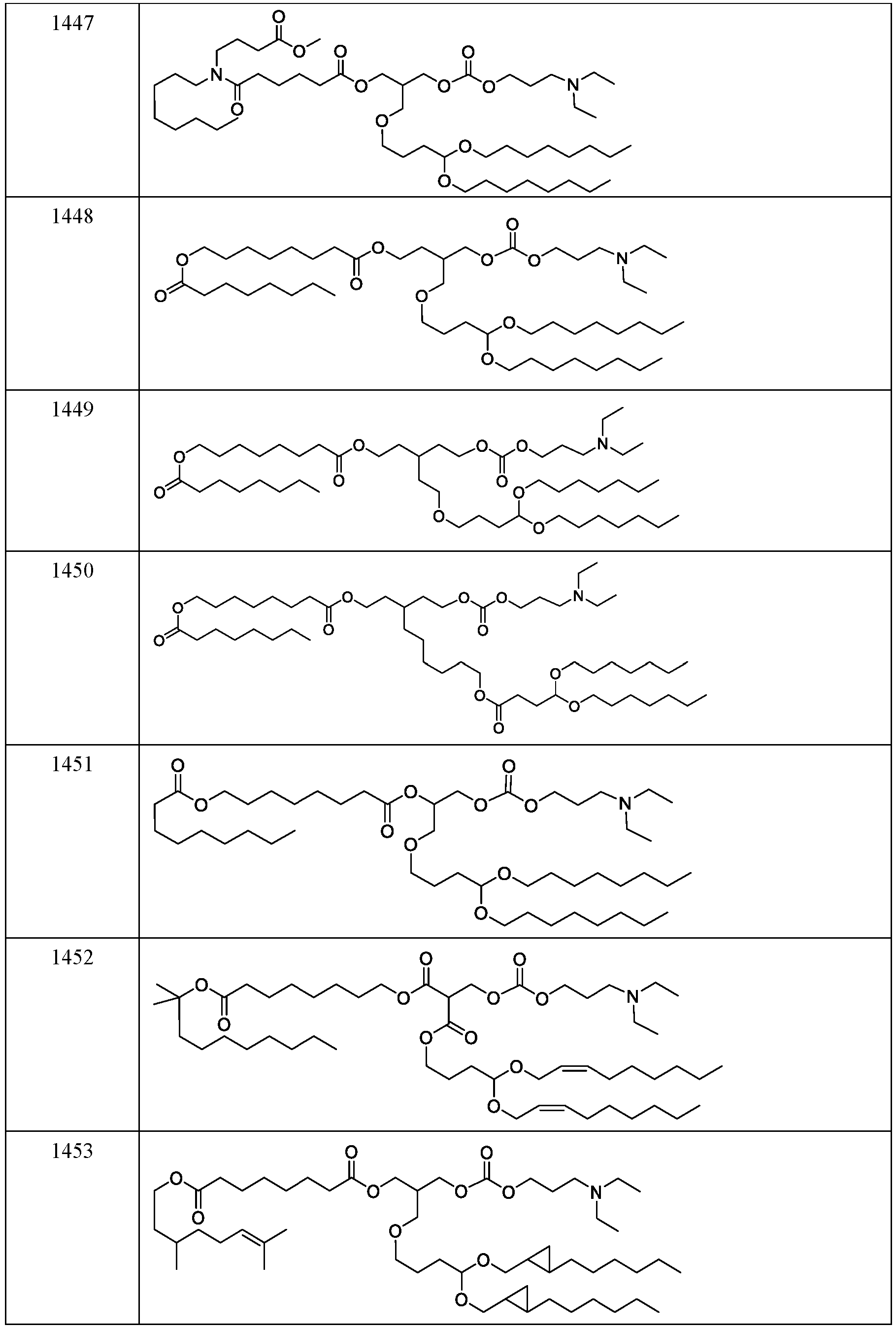

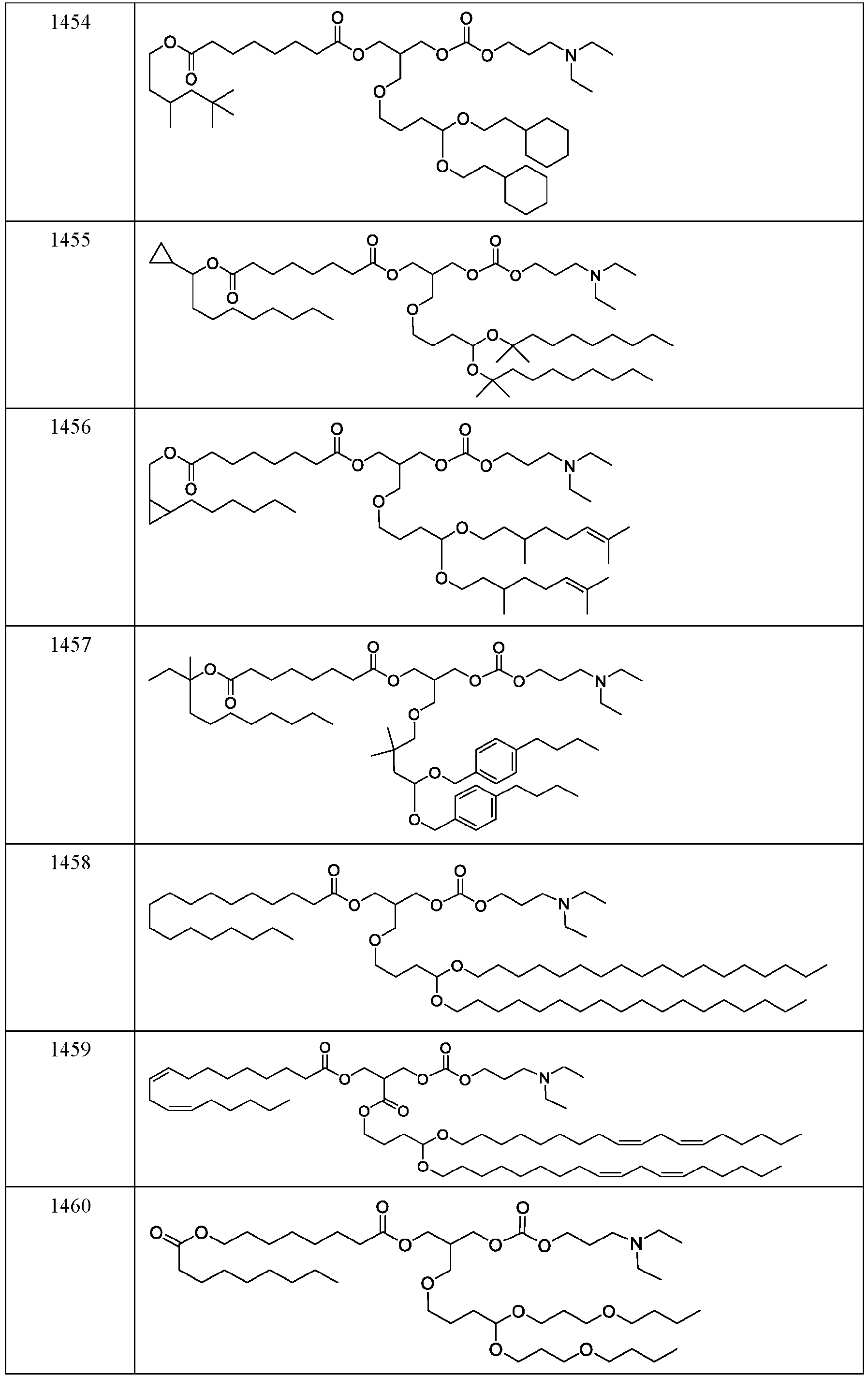

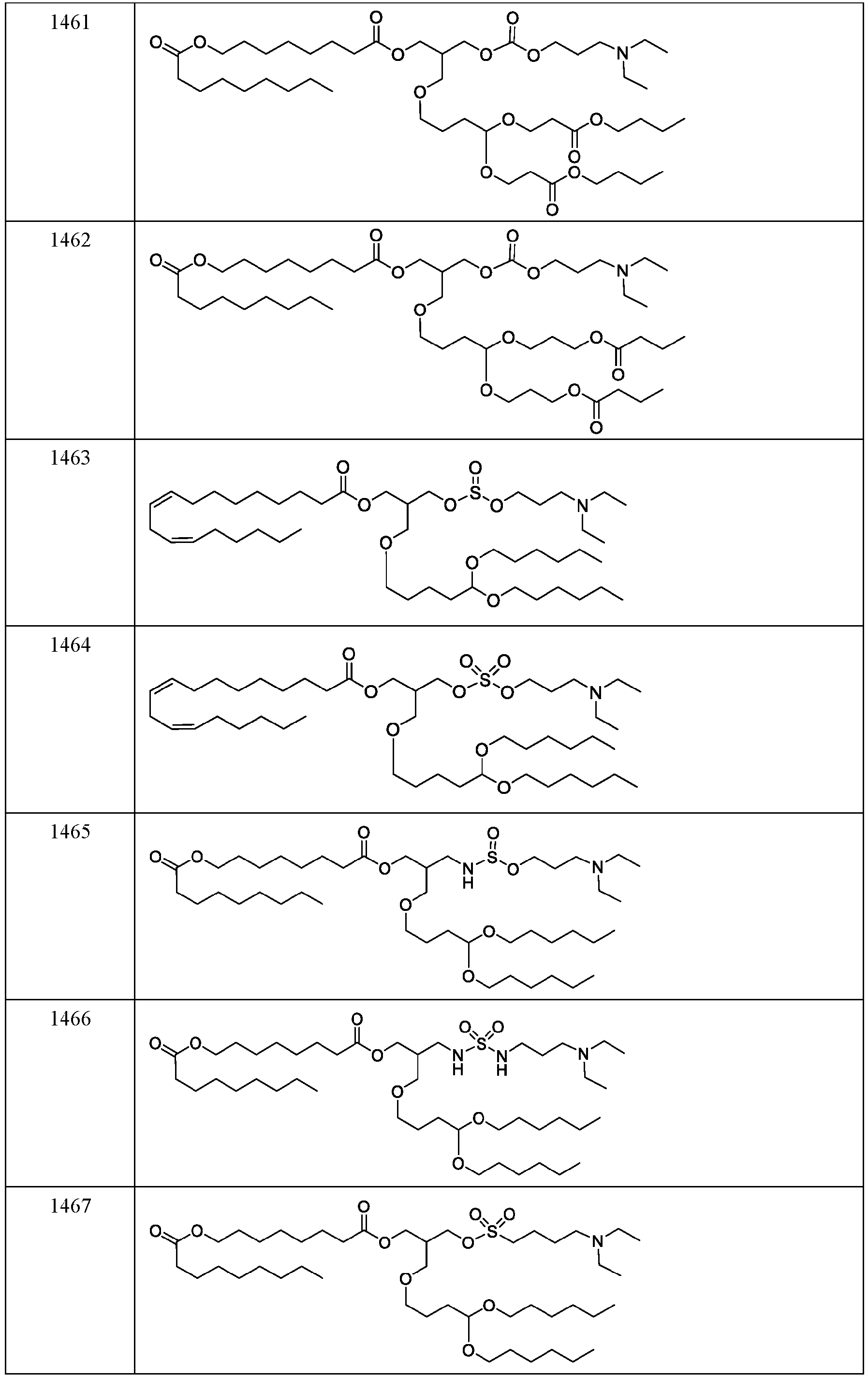

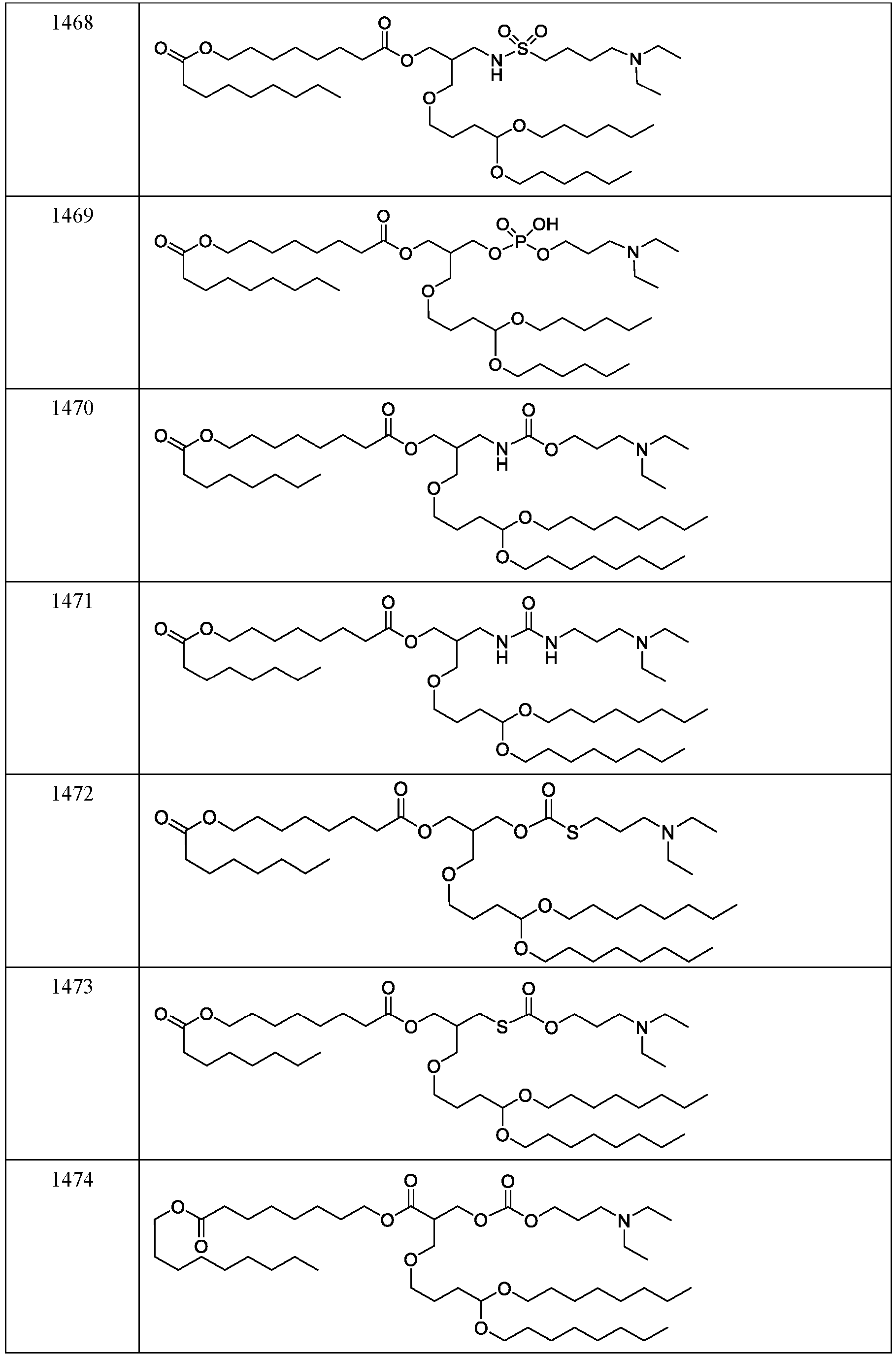

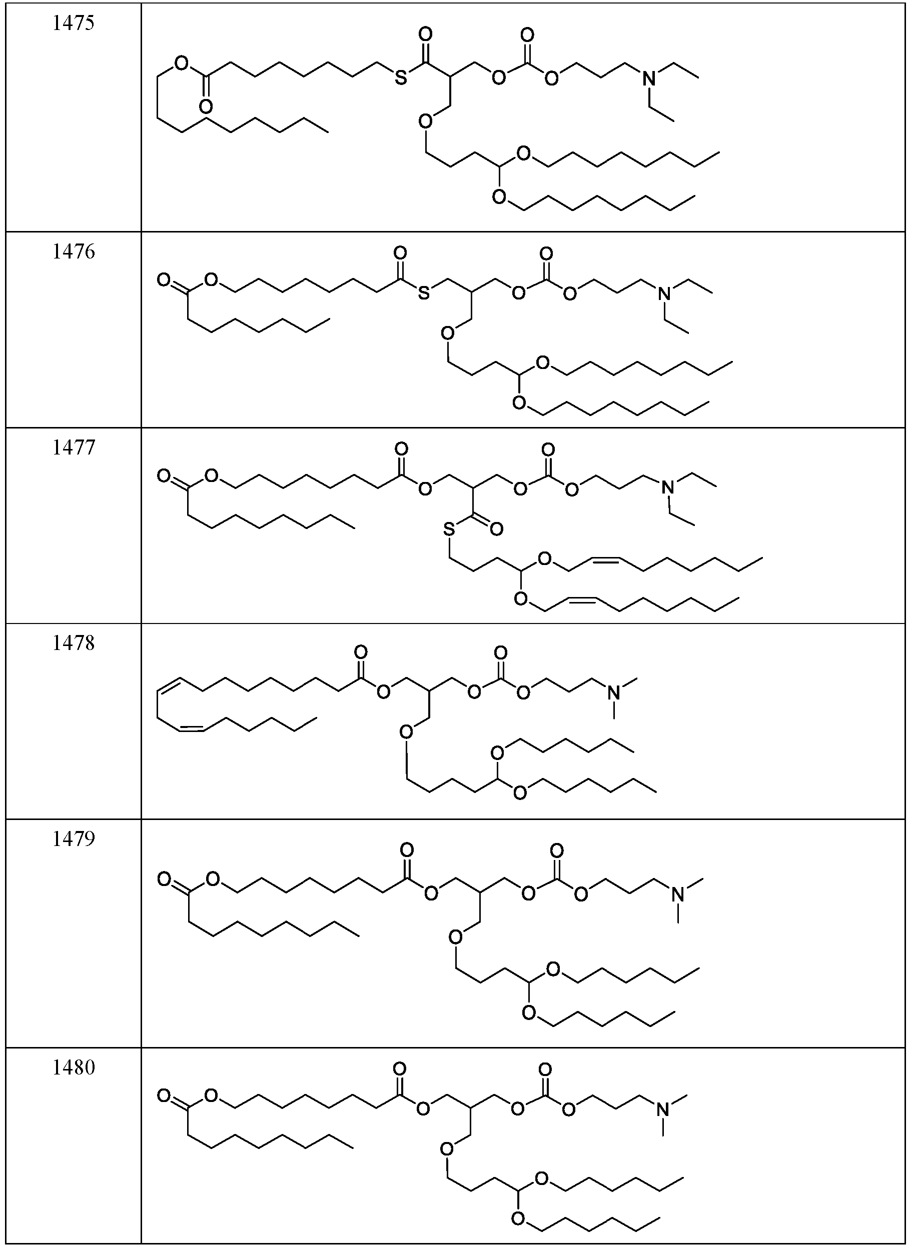

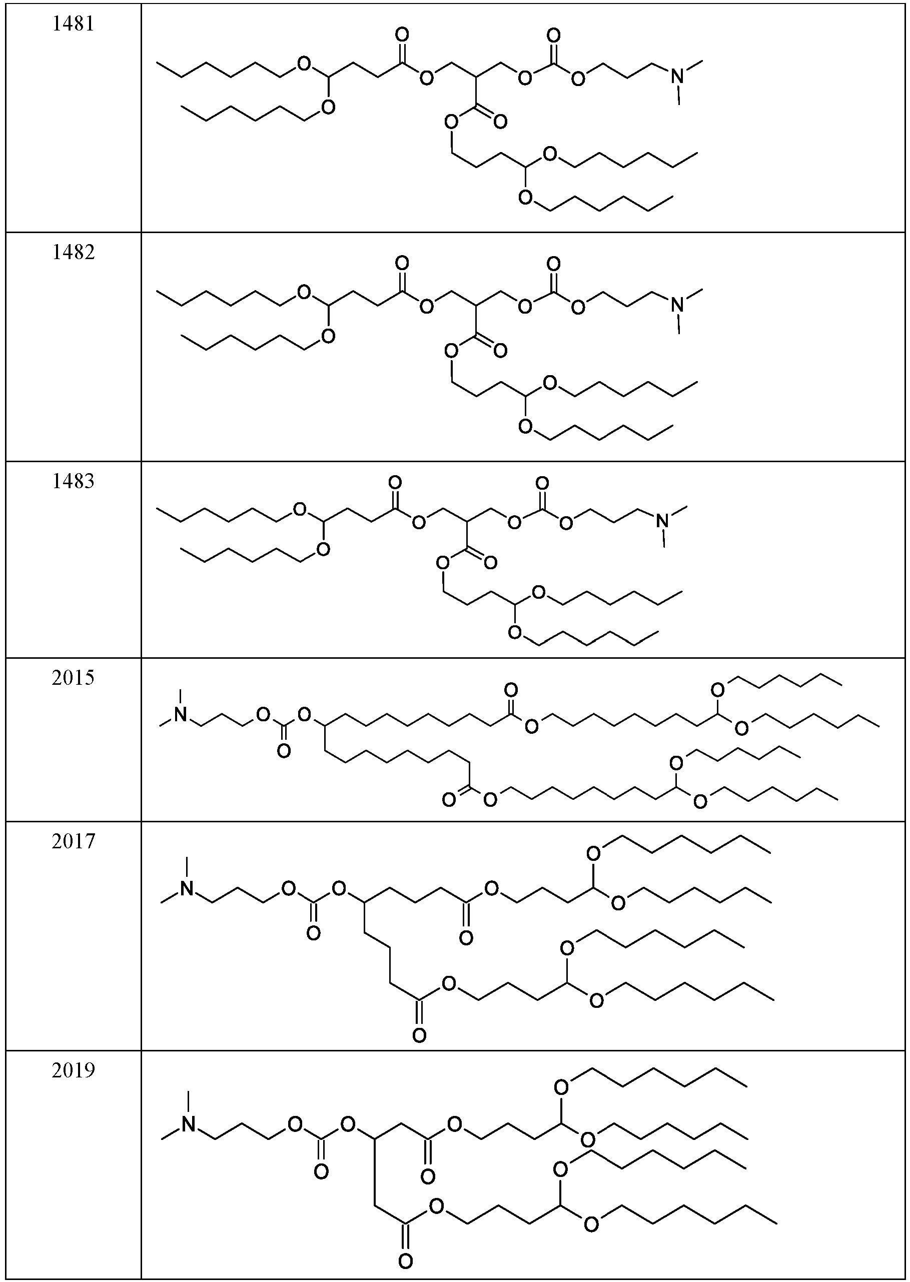

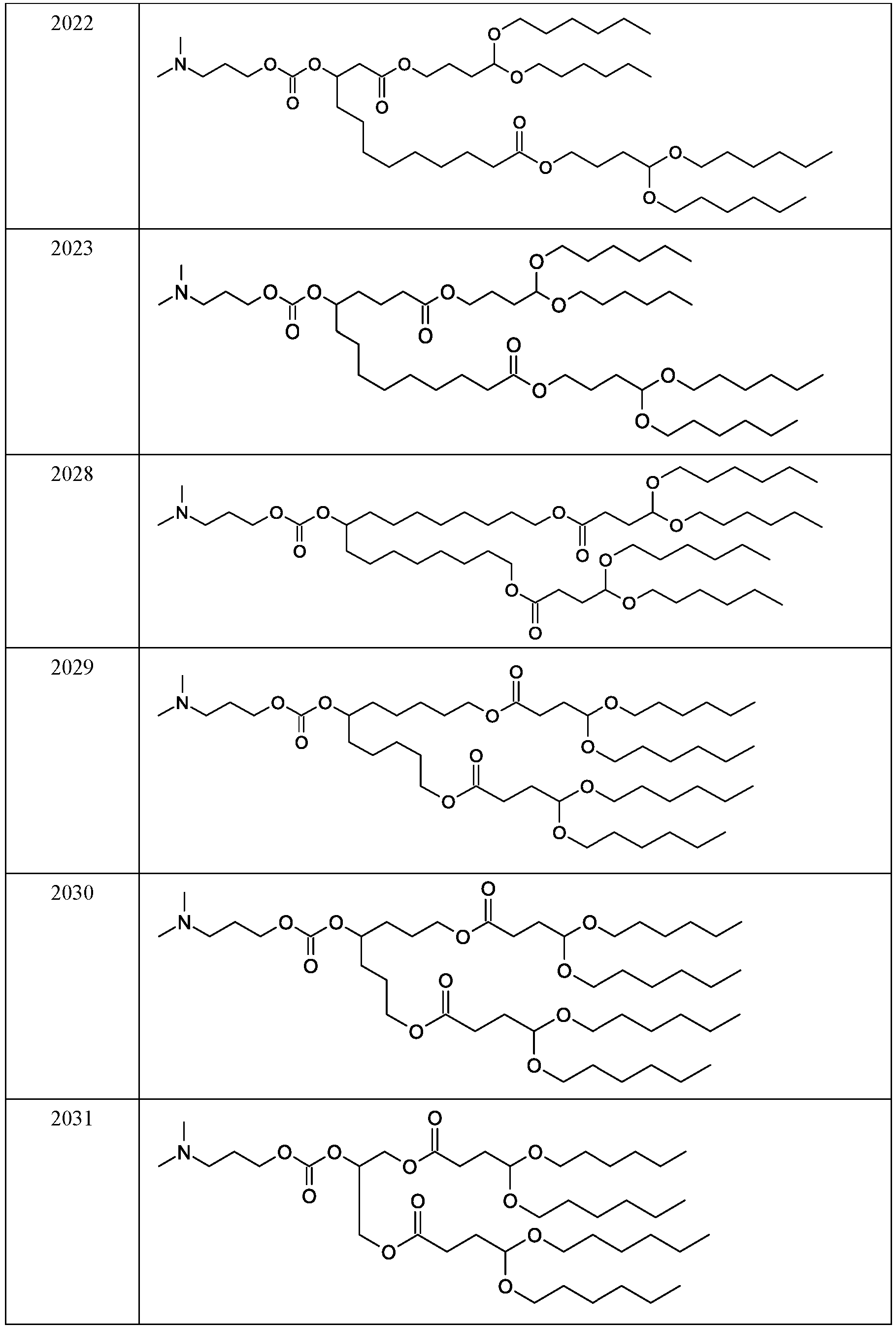

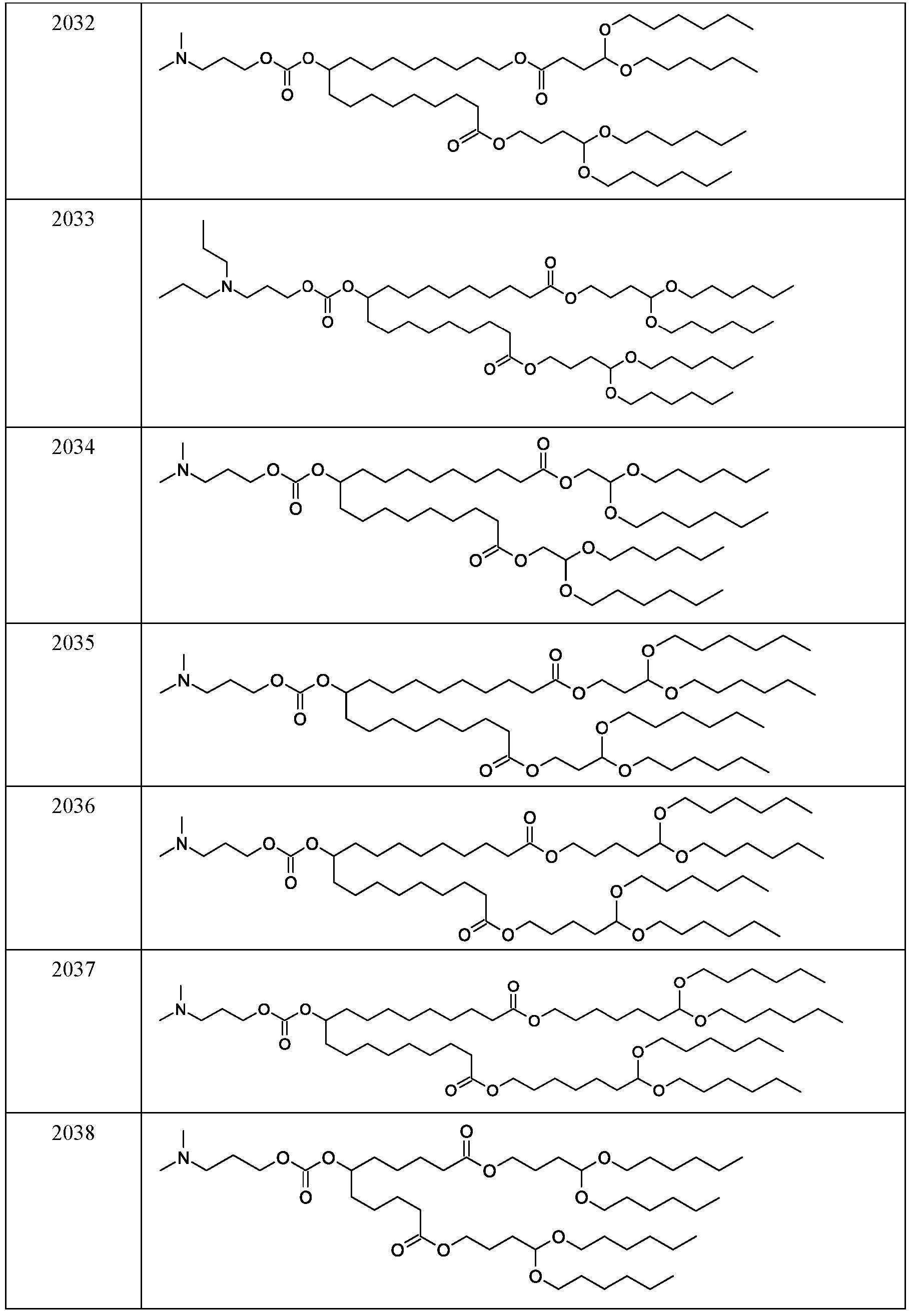

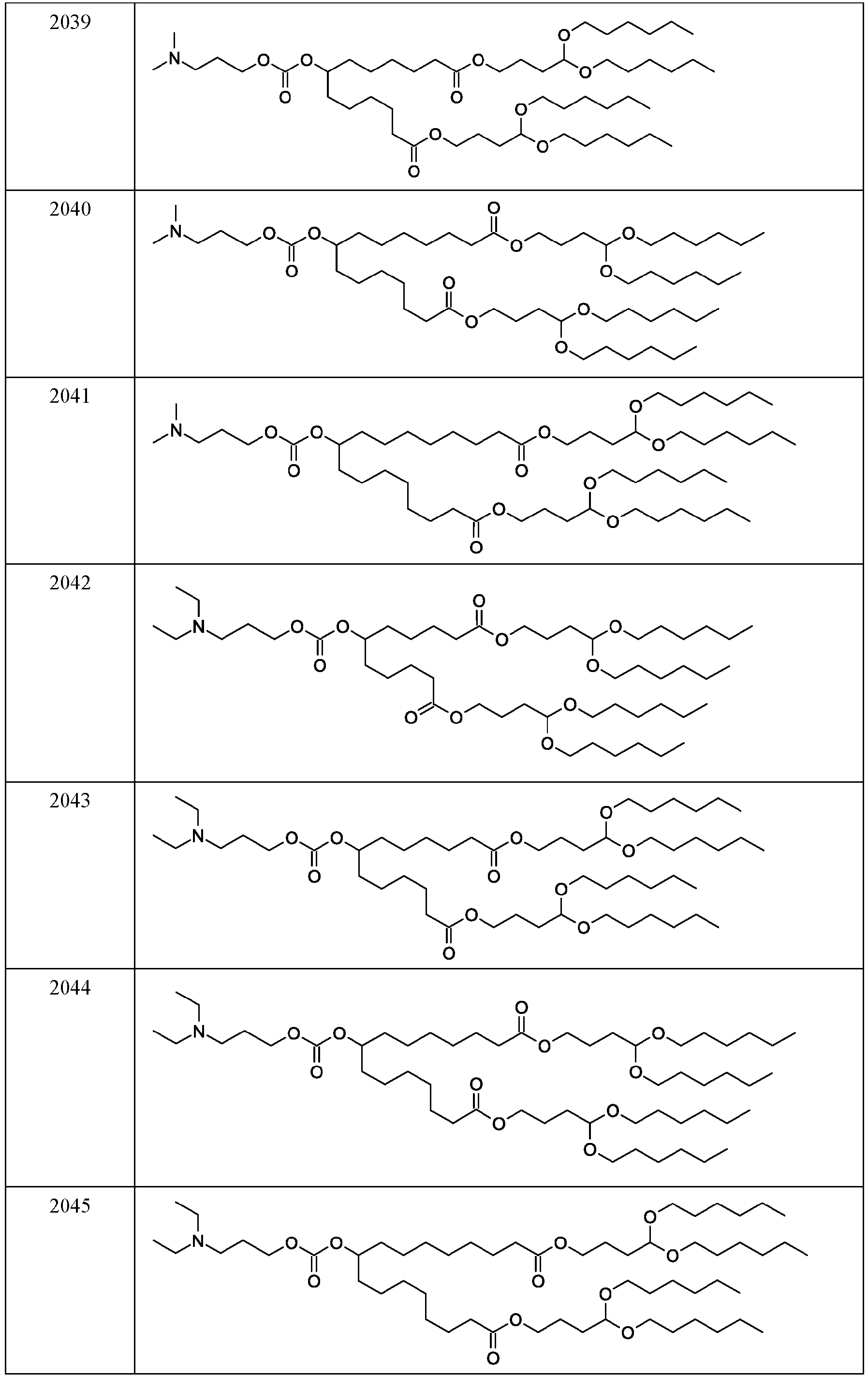

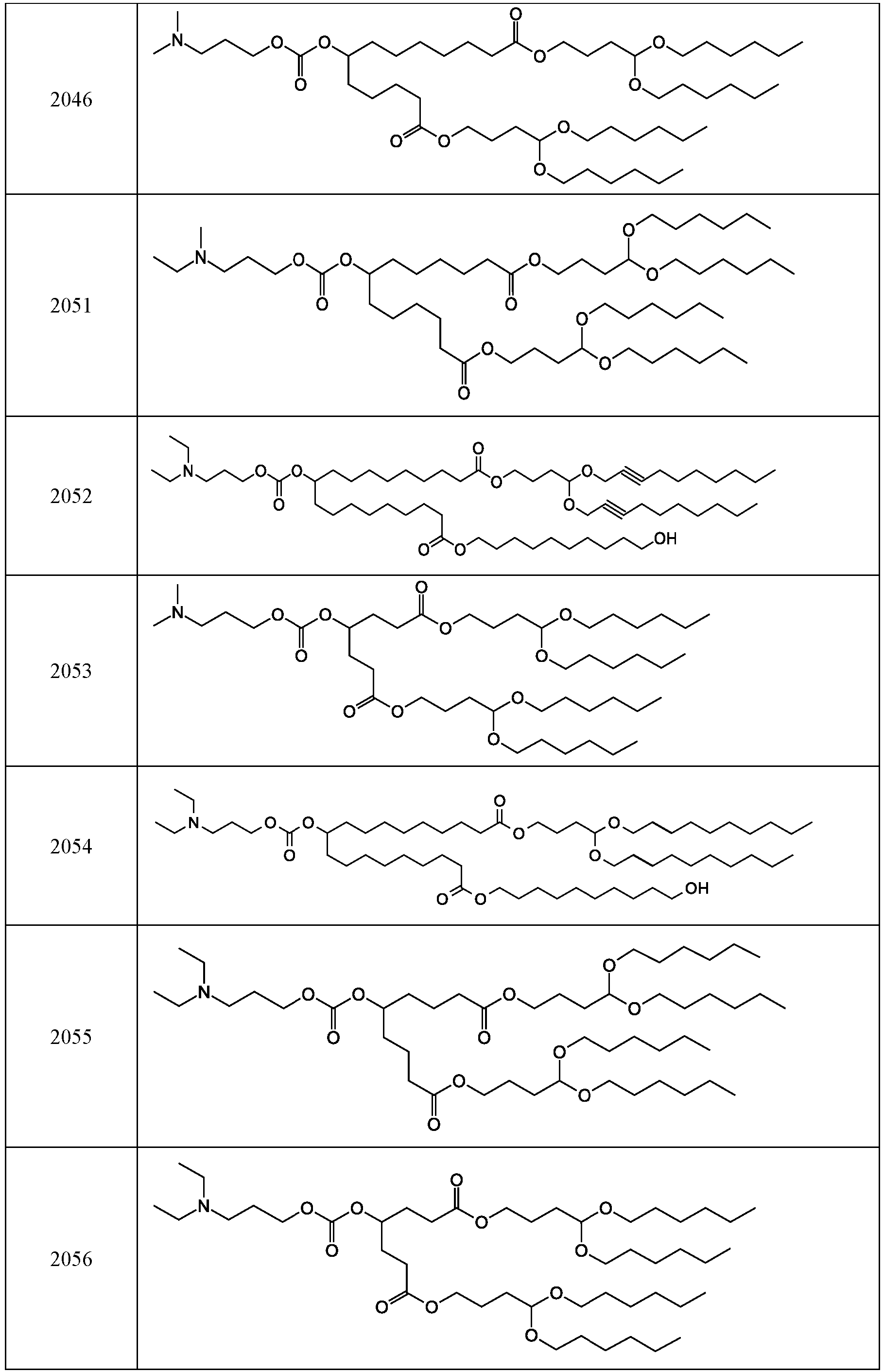

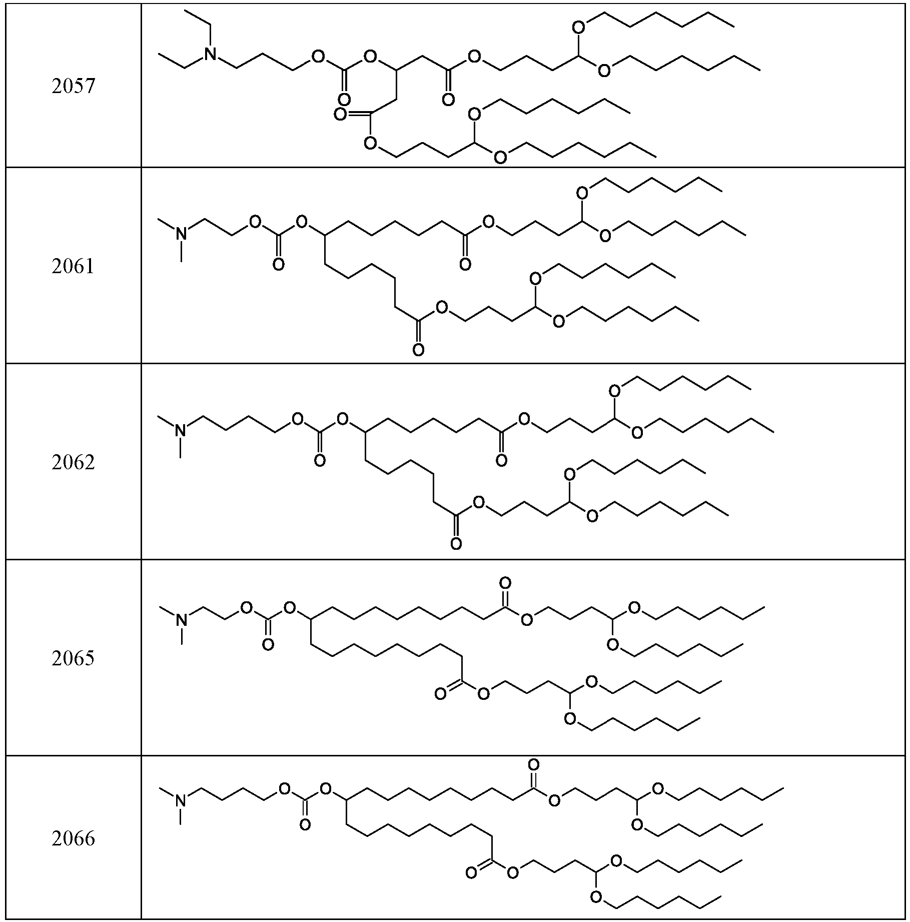

The amino lipid compounds of the present disclosure all have a hydrophobic characteristic due to the presence of long nonpolar residues and simultaneously a hydrophilic characteristic due to the amino group. Due to this amphiphilic characteristic, the amino lipid compounds of the present disclosure can be used to form a lipid nanoparticle, such as a lipid bilayer, a micelle, a liposome, and the like.

In the context of the present disclosure, the term "lipid nanoparticle" means a nanometer-sized material produced by introducing an amino lipid compound into an aqueous solution. The particle is in particular a lipid nanoparticle, a lipid bilayer vesicle (a liposome), a multilayer vesicle or a micelle.

In some embodiments, the lipid nanoparticle is a liposome containing an amino lipid compound of the present disclosure. Within the scope of the present disclosure, a liposome is a microvesicle consisting of a bilayer of lipid amphipathic molecules encapsulating an aqueous compartment.

Liposome formation is not a spontaneous process. When a lipid is introduced into water, a lipid vesicle is firstly formed, thus forming a bilayer or a series of bilayers, each of which is separated by a water molecule. Liposomes can be formed by sonicating lipid vesicles in water.

In the context of the present disclosure, the term "lipid bilayer" means a thin film formed by two layers of lipid molecules. The term "micelle" means an aggregate of surfactant molecules dispersed in a liquid colloid. Typical micelles in an aqueous solution form aggregates with the hydrophilic head region upon contact with water, chelating the hydrophobic single tail region at the center of the micelle.

In one aspect, the present disclosure provides a use of the amino lipid compound of the present disclosure for the manufacture of a vehicle for an active ingredient. In some embodiments, the vehicle is in the form of a lipid nanoparticle, such as a lipid bilayer, micelle, liposome.

### Lipid nanoparticle (LNP)

In another aspect, the present disclosure provides a lipid nanoparticle containing the amino lipid compound of the present disclosure.

In some embodiments, the lipid nanoparticle further contains one or more of a helper lipid, a structural lipid, and a PEG-lipid (polyethylene glycol-lipid).

In some further embodiments, the lipid nanoparticle further contains the helper lipid, the structural lipid, and the PEG-lipid.

In some embodiments, the lipid nanoparticle comprises the amino lipid compound in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0% or 65.0%-75.0%, based on the total amount of the amino lipid compound, the helper lipid, the structural lipid, and the PEG-lipid.

In some embodiments, the lipid nanoparticle comprises the helper lipid in an amount (molar percent) of about 5.0% to 45.0%, such as about 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, 20.0%-25.0%, 25.0%-33.5%, 33.5%-37.0%, 37.0%-40.0%, 40.0%-42.0%, or 42.0%-45.0%, based on the total amount of the amino lipid compound, the helper lipid, the structural lipid, and the PEG-lipid.

In some embodiments, the lipid nanoparticle comprises the structural lipid in an amount (molar percent) of about 0.0% to 50.0%, such as about 0.0%-10.0%, 10.0%-15.5%, 15.5%-18.5%, 18.5%-22.5%, 22.5%-23.5%, 23.5%-28.5%, 28.5%-33.5%, 33.5%-35.0%, 35.0%-36.5%, 36.5%-38.0%, 38.0%-38.5%, 38.5%-39.0%, 39.0%-39.5%, 39.5%-40.5%, 40.5%-41.5%, 41.5%-42.5%, 42.5%-42.7%, 42.7%-43.0%, 43.0%-43.5%, 43.5%-45.0%, 45.0%-46.5%, 46.5%-48.5%, or 46.5%-50.0%, based on the total amount of the amino lipid compound, the helper lipid, the structural lipid, and the PEG-lipid.

In some embodiments, the lipid nanoparticle comprises the PEG-lipid in an amount (molar percent) of about 0.5% to 5.0%, such as about 0.5%-1.0%, 1.0%-1.5%, 1.5%-1.6%, 1.6%-2.0%, 2.0%-2.5%, 2.5%-3.0%, 3.0%-3.5%, 3.5%-4.0%, 4.0%-4.5%, or 4.5%-5.0%, based on the total amount of the amino lipid compound, the helper lipid, the structural lipid, and the PEG-lipid.

In some embodiments as described above, the helper lipid is a phospholipid. The phospholipid is generally semi-synthetic and may also be of natural origin or chemically modified. The phospholipid includes, but is not limited to, DSPC (distearoyl phosphatidylcholine), DOPE (dioleoyl phosphatidylethanolamine), DOPC (dioleoyl lecithin), DOPS (dioleoyl phosphatidylserine), DSPG (1,2-distearoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), lysophospholipid, and the like. Preferably, the helper lipid is one or more selected from the group consisting of DSPC, DOPE, DOPC, and DOPS. In some embodiments, the helper lipid is DSPC and/or DOPE.

In some embodiments, the structural lipid is a sterol, including but not limited to, cholesterol, cholesterol esters, steroid hormones, steroid vitamins, bile acid, cholesterin, ergosterol, β-sitosterol, oxidized cholesterol derivatives, and the like. Preferably, the structural lipid is at least one selected from cholesterol, cholesteryl esters, steroid hormones, steroid vitamins, and bile acid. In some embodiments, the structural lipid is cholesterol, preferably high purity cholesterol, particularly injection grade high purity cholesterol, such as CHO-HP (manufactured by AVT).

As used herein, the term PEG-lipid (polyethylene glycol-lipid) is a conjugate of polyethylene glycol and a lipid structure. Preferably, the PEG-lipid is selected from PEG-DMG and PEG-distearoyl phosphatidylethanolamine (PEG-DSPE), preferably PEG-DMG. Preferably, the PEG-DMG is a polyethylene glycol (PEG) derivative of 1,2-dimyristoyl-sn-glycerol. Preferably, the PEG has an average molecular weight of about 2,000 to 5,000, preferably about 2,000.

In some embodiments as described above, in the lipid nanoparticle, the molar ratio of the amino lipid compound of the present disclosure: helper lipid: structural lipid: PEG-lipid is about 45:10:42.5:2.5, or 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DOPE, and the structural lipid is CHO-HP.

In other embodiments as described above, in the lipid nanoparticle, the molar ratio of the amino lipid compound of the present disclosure: helper lipid: structural lipid: PEG-lipid is about 50.0:10.0:38.5:1.5, or 50.0:9.0:38.0:3.0, or 49.5:10.0:39.0:1.5, or 48.0:10.0:40.5:1.5, or 46.3:9.4:42.7:1.6, or 45.0:9.0:43.0:3.0, or 45.0:11.0:41.5:2.5, or 42.0:10.5:45.0:2.5, or 42.0:16.0:39.5:2.5, or 40.0:16.0:41.5:2.5, or 40.0:18.0:39.5:2.5, or 35.0:40.0:22.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DSPC, and the structural lipid is CHO-HP.

In some embodiments, the lipid nanoparticle has the amino lipid compound of the present disclosure, helper lipid, structural lipid, and PEG-lipid in molar percent (%) as shown in Nos. 1-24 in Table 2 below, based on the total amount of the amino lipid compound, the helper lipid, the structural lipid, and the PEG-lipid:

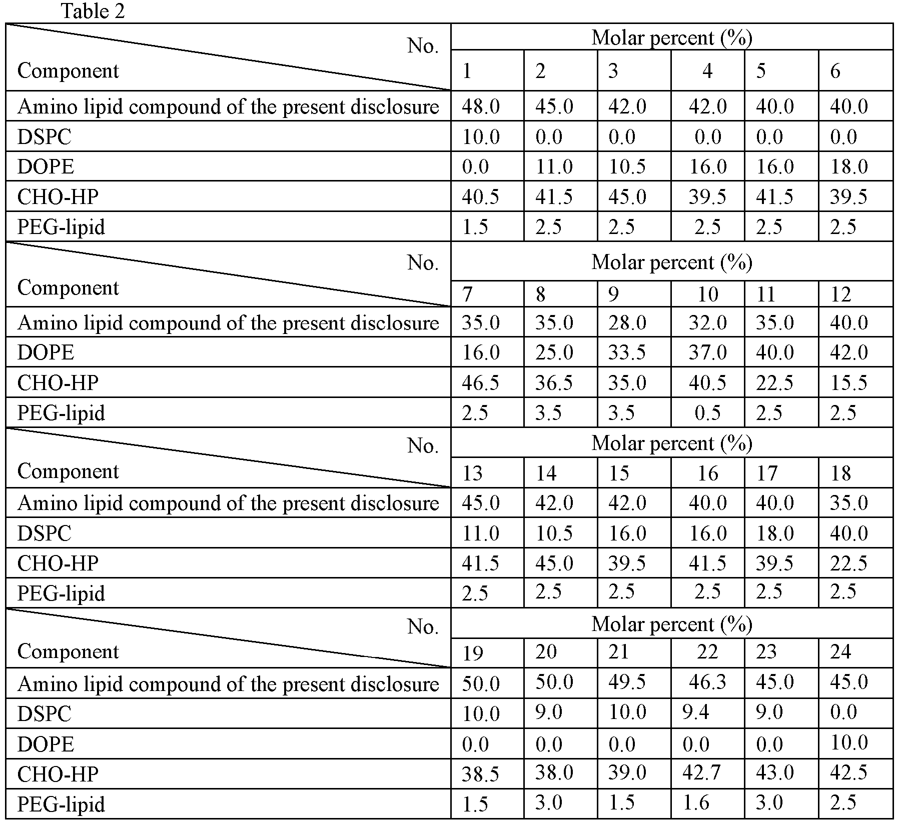

In some embodiments, the lipid nanoparticle has the amino lipid compound of the present disclosure, helper lipid, structural lipid, and PEG-lipid in molar percent (%) as shown in Nos. 25-42 in Table 3 below, based on the total amount of the amino lipid compound, the helper lipid, the structural lipid, and the PEG-lipid:

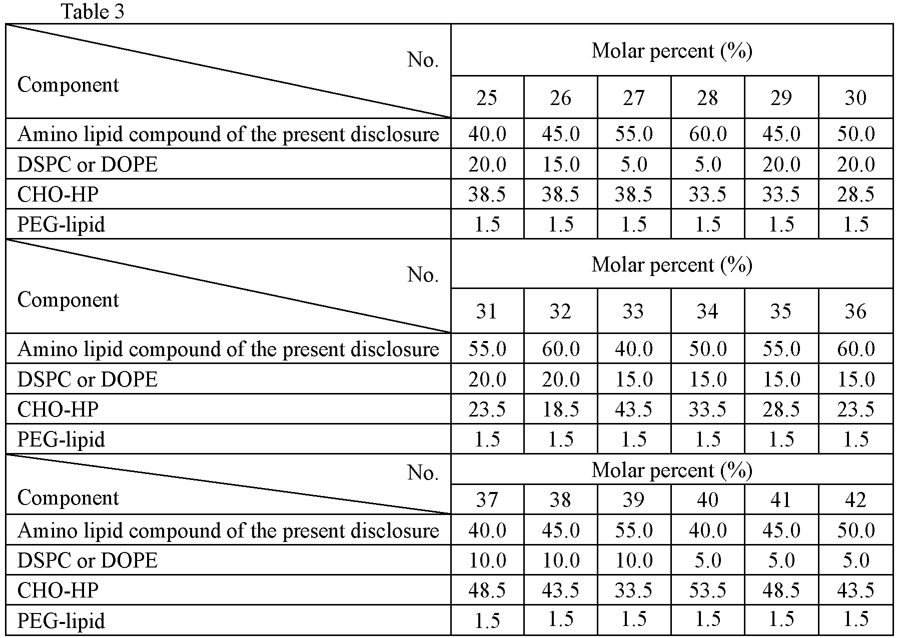

As described above, the lipid nanoparticle of the present disclosure may be used as a delivery vehicle for an active ingredient.

In some embodiments, the active ingredient includes a therapeutic and/or a prophylactic agent.

The term "therapeutic agent" or "prophylactic agent" refers to any agent that, when administered to a subject, has therapeutic, diagnostic, and/or prophylactic effects and/or elicits desired biological and/or pharmacological effects.

An "effective amount" or "therapeutically effective amount" refers to an amount of the compound of the present invention or the lipid nanoparticle comprising the compound of the present invention that is sufficient to effect treatment in a mammal (preferably a human), when administered to the mammal (preferably the human). The amount of the lipid nanoparticle of the present invention that constitutes a "therapeutically effective amount" will depend on the compound, the condition and its severity, the mode of administration, and the age of the mammal to be treated, but may be routinely determined by one of ordinary skill in the art in light of their own knowledge and the present disclosure.

Preferably, the pharmaceutically active ingredient is a biologically active ingredient, which is a substance that has a biological effect when introduced into a cell or a host, for example, by stimulating an immune or inflammatory response, by exerting an enzymatic activity, by complementing a mutation, or the like. A biologically active ingredient includes, but is not limited to, a nucleic acid, a protein, a peptide, an antibody, a small molecule, and a mixture thereof.

Preferably, the biologically active ingredient is a nucleic acid.

In some embodiments, the biologically active ingredient is an antineoplastic agent, an antibiotic, an immunomodulator, an anti-inflammatory agent, an agent acting on the central nervous system, a polypeptide, a polypeptoid, or a mixture thereof.

A lipid nanoparticle may be referred to as "a lipid nanoparticle drug" when it encapsulates the active ingredient in its internal aqueous space.

In the context of the present disclosure, the term "cell" is a generic term and includes the culture of individual cells, tissues, organs, insect cells, avian cells, fish cells, amphibian cells, mammalian cells, primary cells, continuous cell lines, stem cells, and/or genetically engineered cells (e.g., recombinant cells expressing a heterologous polypeptide or protein). Recombinant cells include, for example, cells expressing heterologous polypeptides or proteins (such as growth factors or blood factors).

In some embodiments as described above, the lipid nanoparticle of the present disclosure further comprises a nucleic acid.

In some embodiments, the mass ratio of the amino lipid compound of the present disclosure to the nucleic acid in the lipid nanoparticle is about (5-30): 1, such as about (5-10): 1, (10-15): 1, (15-20): 1, (20-25): 1, or (25-30): 1, preferably about 10:1.

In some embodiments, the nucleic acid is selected from the group consisting of RNA, antisense oligonucleotide, and DNA.

In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA or a mixture thereof.

In some embodiments, the messenger RNA (mRNA) encodes a polypeptide and/or protein of interest. Any naturally or non-naturally occurring or otherwise modified polypeptide is included. In some embodiments, the polypeptide and/or protein encoded by the mRNA may have therapeutic and/or prophylactic effects when expressed in a cell.

In some embodiments, the RNA is an siRNA that is capable of selectively decreasing the expression of a gene of interest or down-regulating the expression of the gene. For example, an siRNA may be selected such that a gene associated with a particular disease, disorder, or condition is silenced upon administration of a lipid nanoparticle comprising the siRNA to a subject in need thereof. An siRNA may comprise a sequence complementary to an mRNA sequence encoding a gene or protein of interest. In some embodiments, the siRNA may be an immunomodulatory siRNA.

In certain embodiments, the RNA is sgRNA and/or cas9 mRNA. The sgRNA and/or cas9 mRNA may be used as a gene editing tool. For example, the sgRNA-Cas9 complex can affect mRNA translation of cellular genes.

In some embodiments, the RNA is an shRNA or a vector or plasmid encoding the same. The shRNA may be produced inside the target cell after delivery of an appropriate construct into the nucleus. Constructs and mechanisms associated with shRNA are well known in the relevant art.

In some embodiments, the DNA is a plasmid.

In some embodiments, the lipid nanoparticle is used to transfer nucleic acids. In some embodiments, the lipid nanoparticle may be used for, for example, gene therapy, gene vaccination, protein replacement therapy, antisense therapy, or therapy by interfering RNA.

### Pharmaceutical composition

In another aspect, the present invention provides a pharmaceutical composition comprising a lipid nanoparticle as described above and a pharmaceutically acceptable carrier, diluent, or excipient.

In some embodiments, the pharmaceutical composition further comprises a buffer solution. In some such embodiments, the buffer solution is selected from a phosphate buffer and a Tris buffer, preferably a phosphate buffer. In some embodiments, the buffer solution has a concentration of about 5 mmol/L to about 30 mmol/L, preferably about 10 mmol/L. In some embodiments, the buffer solution has a pH of about 6 to 8, preferably about 7 to 8, and more preferably about 7 to 7.5.

In some embodiments, the pharmaceutical composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

In some embodiments as described above, the pharmaceutical composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

### Use

The lipid nanoparticle of the present disclosure has excellent properties of encapsulating biologically active ingredients. The lipid nanoparticle comprising biologically active ingredients can be used to deliver any of a variety of therapeutic agents into cells. The present disclosure includes use of the lipid nanoparticle as described above to deliver a biologically active ingredient into a cell. The present invention also provides a method of delivering a biologically active ingredient into a cell, tissue or organ, comprising contacting the lipid nanoparticle of the present disclosure comprising the biologically active ingredient with the cell, tissue or organ. This provides a subject with the possibility of new therapeutic treatment.

In some embodiments, the tissue or organ is selected from the group consisting of spleen, liver, kidney, lung, femur, ocular tissue, vascular endothelium in blood vessels, lymph, and tumor tissue.

Preferably, the cell is a mammalian cell; and further preferably, the mammalian cell is in a mammal. As used herein, a subject may be any mammal, preferably selected from the group consisting of mice, rats, pigs, cats, dogs, horses, goats, cattle, and monkeys, etc. In some preferred embodiments, the subject is a human.

The present disclosure provides a method of producing a polypeptide and/or protein of interest in a mammalian cell, comprising contacting the cell with the lipid nanoparticle comprising mRNA encoding the polypeptide and/or protein of interest, upon contact of the cell with the lipid nanoparticle, the mRNA being able to be taken up into the cell and translated to produce the polypeptide and/or protein of interest.

In yet another aspect, the present disclosure provides a use of the amino lipid compound, lipid nanoparticle, or pharmaceutical composition of the present disclosure in the manufacture of a medicament. Preferably, the medicament is a nucleic acid drug. Preferably, the pharmaceutical composition is used for treatment and/or prevention of a disease.

In some embodiments, the disease is selected from the group consisting of rare diseases, infectious diseases, cancers, genetic diseases, autoimmune diseases, diabetes, neurodegenerative diseases, cardiovascular, renal vascular diseases, and metabolic diseases.

The medicaments are used for, for example, gene therapy, protein replacement therapy, antisense therapy, or therapy by interfering RNA, and gene vaccination.

In some embodiments, the cancers are selected from one or more of lung cancer, stomach cancer, liver cancer, esophageal cancer, colon cancer, pancreatic cancer, brain cancer, lymphoma, blood cancer, or prostate cancer. In some embodiments, the genetic disorders are selected from one or more of hemophilia, thalassemia, and Gaucher's disease.

In some embodiments, the gene vaccination is preferably used to treat and/or prevent cancer, allergy, toxicity, and pathogen infection. In some embodiments, the pathogen is selected from one or more of viruses, bacteria, or fungi.

The present disclosure provides a method of treating a disease or disorder in a mammal in need thereof, comprising administering to the mammal a therapeutically effective amount of the lipid nanoparticle as described above.

Preferably, the disease or disorder is selected from the group consisting of rare diseases, infectious diseases, cancers, genetic diseases, autoimmune diseases, diabetes, neurodegenerative diseases, cardiovascular, renal vascular diseases, and metabolic diseases.

In yet another aspect, the present disclosure provides a use of the amino lipid compound, lipid nanoparticle, or pharmaceutical composition of the disclosure in the manufacture of a medicament for nucleic acid transfer. In some embodiments, the nucleic acid is selected from the group consisting of RNA, antisense oligonucleotide, and DNA. In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof. In some embodiments, the DNA is a plasmid.

### Preparation method

In yet another aspect, the present disclosure also provides a general synthetic process for preparing the amino lipid compound of formula (Id) or (Ie) of the present disclosure, as follows: 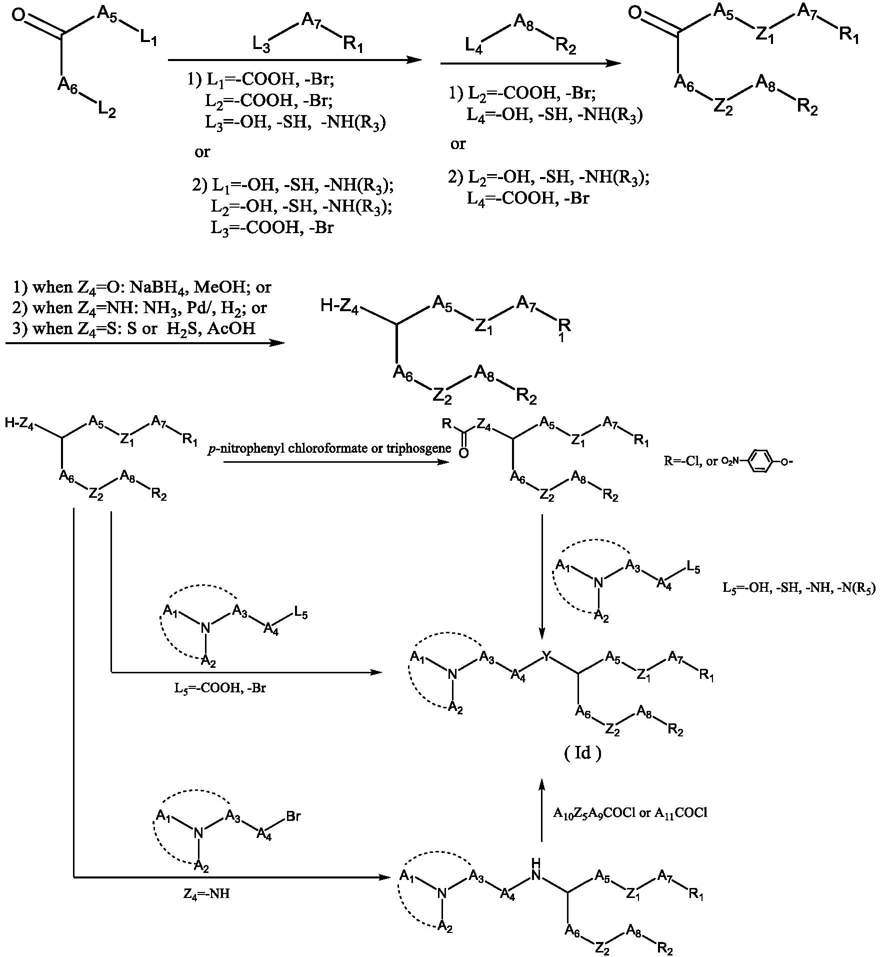 or

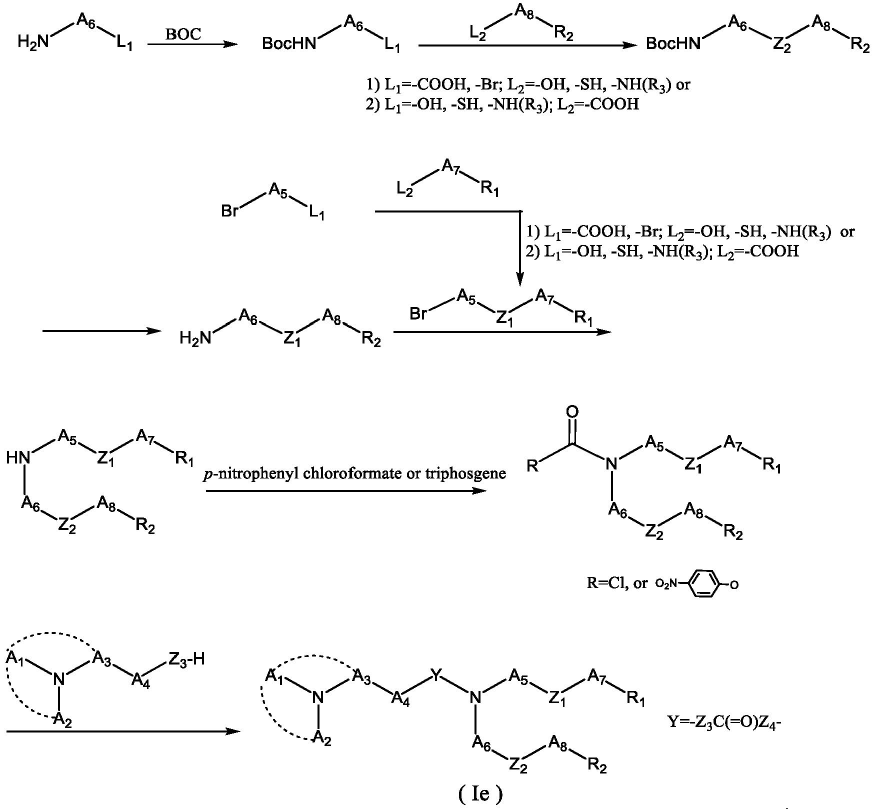

In yet another aspect, the present disclosure also provides a general synthetic process for preparing the amino lipid compound of formula (II-I) of the present disclosure, as follows:
(1) when X is C, Y₁ is -Z₃C(=O)Z₄-, both Z₃ and Z₄ are not a bond, and A₁₂ is a bond, 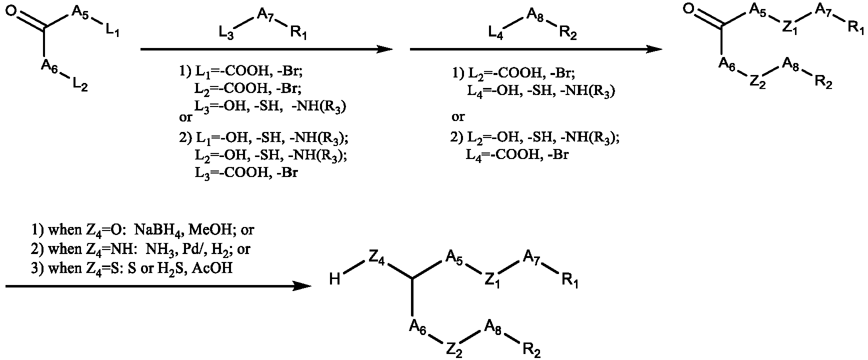 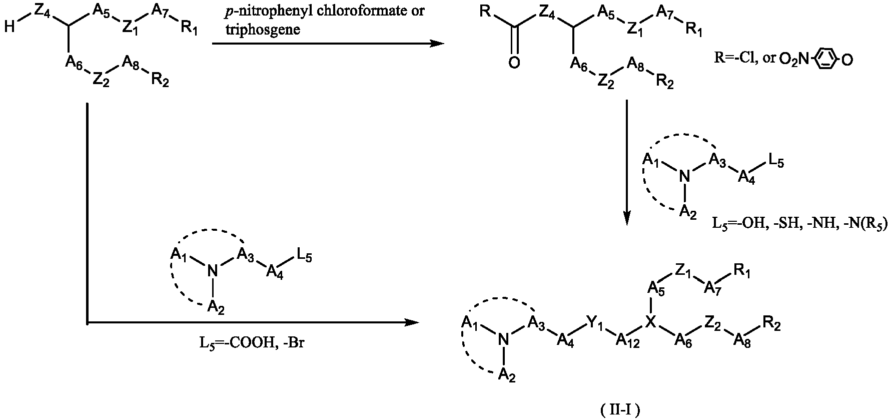 or
(2) when X is C, and Y₁ is -N(R₅), 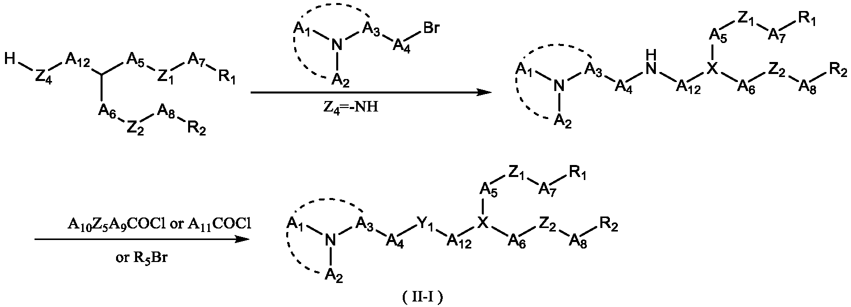 or
(3) when X is C, Y₁ is -Z₃C(=O)Z₄, Z₃ is a bond, and Z₄ is not a bond, 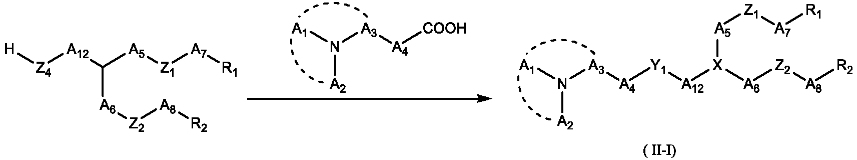 or
(4) when X is C, Y₁ is -Z₃C(=O)Z₄, Z₃ is not a bond, and Z₄ is a bond, 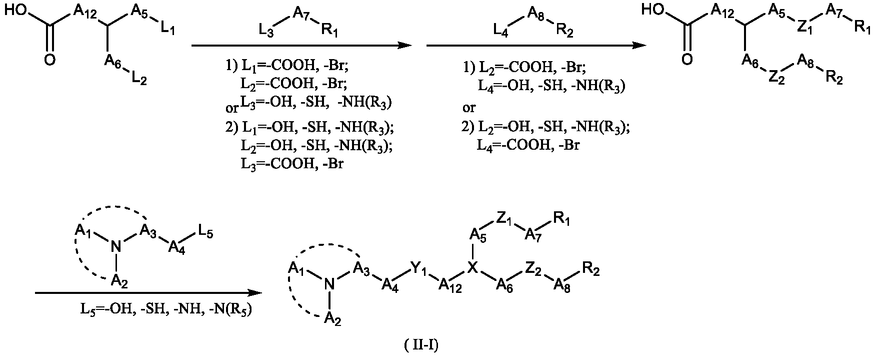 or
(5) when X is N, Y₁ is -Z₃C(=O)Z₄-, Z₄ is a bond, and A₁₂ is a bond, 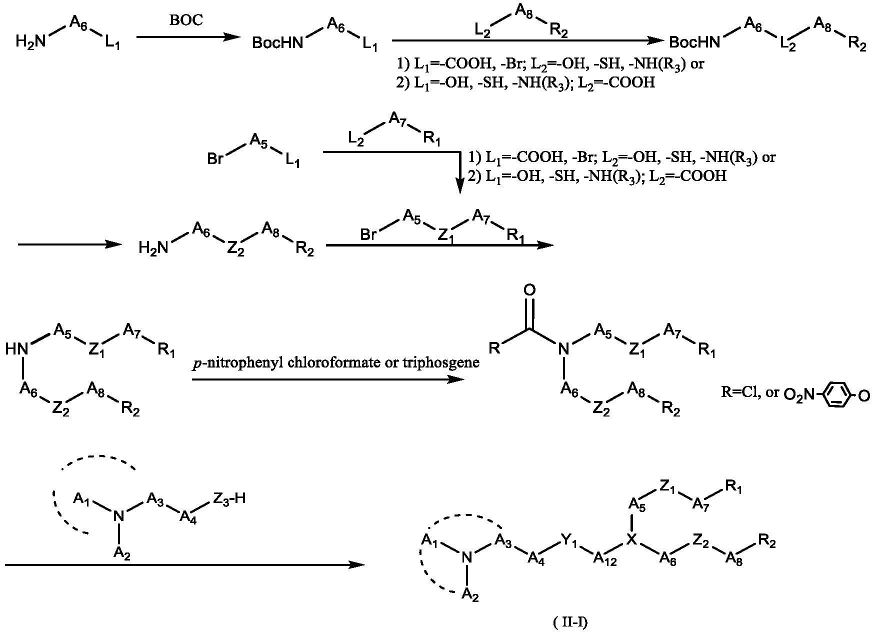 or
(6) when X is N, Y₁ is -Z₃C(=O)Z₄-, Z₄ is a bond, and A₁₂ is not a bond,

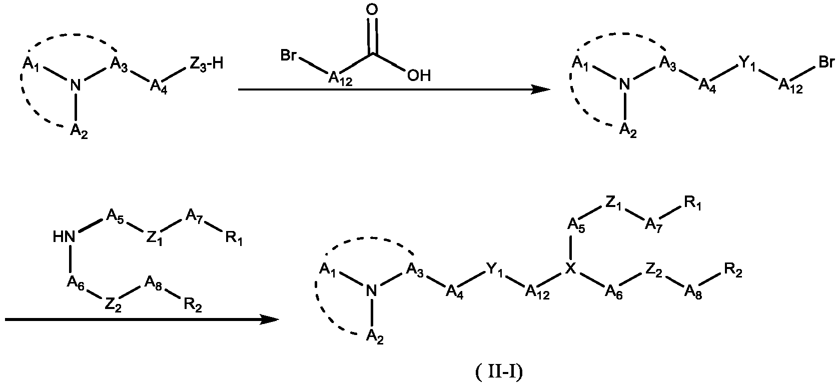

In yet another aspect, the lipid nanoparticle or the pharmaceutical composition of the present disclosure may be prepared according to methods known in the art. For example, the method may comprise the following steps:
(1) formulating: formulating a suitable aqueous phase; and formulating an organic phase comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2); and
(4) sterilization: optionally sterilizing the product of step (3), for example, by means of a sterilizing filter, such as a 0.22 µm microporous membrane.

In some embodiments, the lipid nanoparticle or the pharmaceutical composition of the present disclosure comprising a nucleic acid, particularly mRNA may be prepared by a method comprising the following steps:
(1) formulating: formulating an aqueous phase comprising the nucleic acid; and formulating an organic phase (e.g., an ethanol phase) comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2);
(4) sterilization: optionally sterilizing the product of step (3), for example, by means of a sterilizing filter, such as a 0.22 µm microporous membrane.

### Beneficial effect

The amino lipid compound of the present disclosure can form a vehicle, such as a lipid nanoparticle, with excellent properties of encapsulating biologically active ingredients, can be used for delivering biologically active ingredients, especially water-insoluble drugs or active ingredients that are easily decomposed or degraded (such as nucleic acids), and improving its bioavailability and efficacy, or immunological activity, or or transfection efficiency (for nucleic acids), or safety, or tissue and/or cell targeting or specificity, or preference for a particular organ and/or tissue.

### Brief description of the drawings

FIGs. 1 to 2 show the results of *in vivo* safety (ALT enzymatic activity) test of lipid nanoparticles comprising different amino lipid compounds;
FIGs. 3 to 4 show the results of *in vivo* safety (AST enzymatic activity) test of lipid nanoparticles comprising different amino lipid compounds;
FIG. 5 shows the results of *in vivo* delivery of lipid nanoparticles comprising different amino lipid compounds;
FIG. 6 shows the delivery efficiency of lipid nanoparticles comprising different amino lipid compounds in different immune cell populations in the spleen;
FIG. 7 shows the delivery efficiency of lipid nanoparticles comprising different amino lipid compounds in different immune cell populations in PBMC.

In order to make the purposes, technical solutions, and advantages of the present invention clearer, the present invention is described below with reference to specific examples. The follow examples are merely illustrative of the present invention and are not intended to be limiting.

### Examples

The following examples are provided for purposes of illustration and not limitation.

The experimental methods for which specific conditions are not specified in the examples are usually under conventional conditions or conditions as recommended by the manufacturer of the raw material or commodity; and the reagents of unspecified origin are generally conventional reagents commercially available.

The abbreviations used in the examples have the following meanings:
- Pd/C: Palladium/carbon;
- EA: Ethyl acetate;
- DCM: Dichloromethane;
- TEA: Triethylamine;
- MPa: Megapascal;
- DMF: N,N-dimethylformamide;
- EDCI: 1-(3-Dimethylaminopropyl)-3-ethylcarbodiimide hydrochloride;
- DMAP: 4-Dimethylaminopyridine;
- TBAF: Tetrabutylammonium fluoride
- TEMPO: 2,2,6,6- Tetramethylpiperidinooxy;
- NaDCC: Sodium dichloroisocyanurate;
- TBSCl: Tert-butyldimethylsilyl chloride;
- h: Hour;
- min: Minute.

### Example 1: Synthesis of Amino Lipid Compound 383

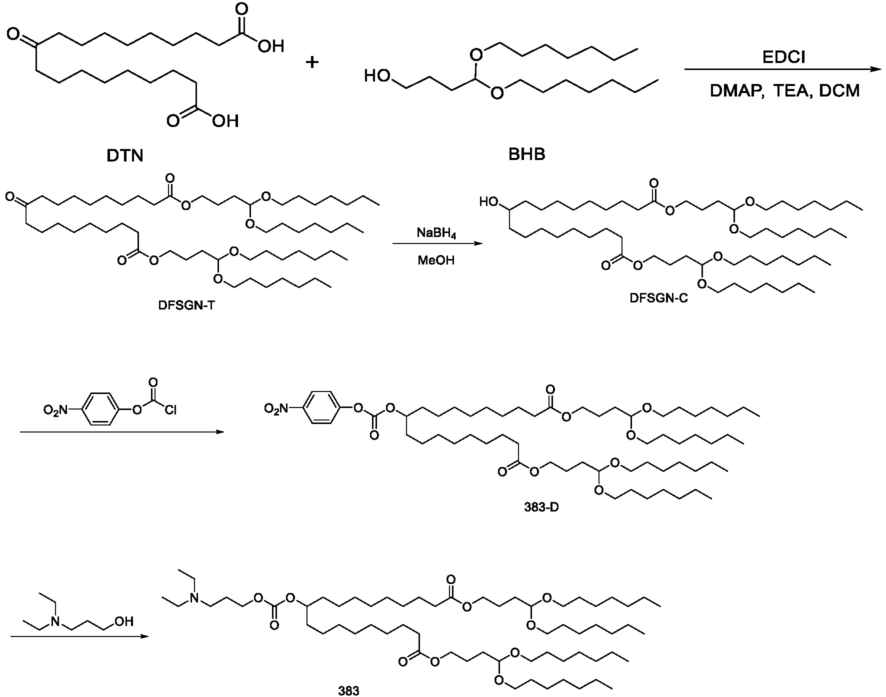

### Step 1: Synthesis of DFSGN-T

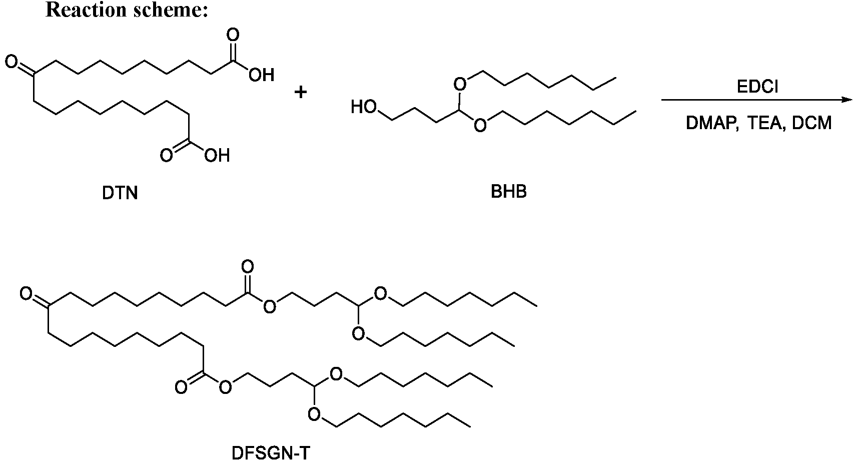

### Experimental procedure:

EDCI (4.43 g, 23.13 mmol), DCM (26 mL), and TEA (2.34 g, 23.13 mmol) were sequentially added to a 100 mL round-bottom flask, shaken and stirred well for 5 min. Then DMAP (0.47 g, 3.86 mmol), BHB (4,4-bis(heptyloxy)butanol) (4.2 g, 13.88 mmol), and DTN (10-oxononadecanedioic acid) (2.64 g, 7.71 mmol) were added and stirred at room temperature overnight. After the reaction was completed, DCM (100 mL) and water (150 mL) were added and stirred for extraction, and partitioned. The organic phase was collected, washed with 50 mL saturated sodium chloride solution, dried over anhydrous sodium sulfate, and concentrated under reduced pressure to obtain 7.7 g crude DFSGN-T, which was purified by silica gel column chromatography and eluted with ethyl acetate: n-heptane = 1.5:1 to obtain 5.13 g DFSGN-T with a yield of 81%.

### Step 2: Synthesis of DFSGN-C

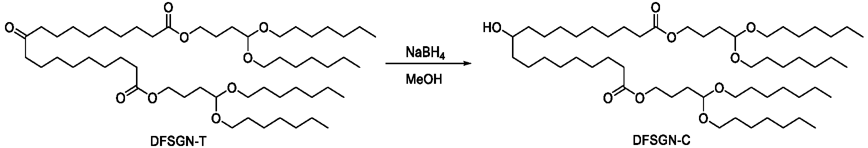

### Experimental procedure:

DFSGN-T (5.13 g, 5.62 mmol) was added to a 250 mL single-necked flask, dissolved in methanol (50 mL), and cooled to 0°C. Sodium borohydride (0.23 g, 6.18 mmol) was added portionwise with stirring, and the reaction was maintained for 1 h after the addition was completed. After the reaction was completed, the solvent was evaporated under reduced pressure, water (100 mL) and DCM (100 mL) were added and stirred for extraction, and partitioned. The organic phase was collected, washed with water (50 mL), and concentrated under reduced pressure at 45°C to obtain 5.2 g crude DFSGN-C, which was purified by silica gel column chromatography and eluted with ethyl acetate: n-heptane = 1.5:1 to obtain 4.5 g DFSGN-C with a yield of 88%.

### Step 3: Synthesis of 383-D

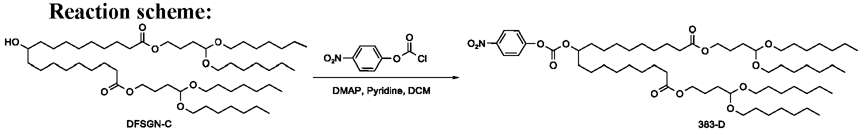

### Experimental procedure:

DFSGN-C (1.8 g, 1.97 mmol), DCM (18 mL), DMAP (72 mg, 0.60 mmol), and pyridine (0.39 g, 4.93 mmol) were added to a 50 mL single-necked round-bottom flask and stirred at room temperature. *P*-nitrophenyl chloroformate (0.79 g, 3.94 mmol) was added portionwise and stirred for another 1 h. After the reaction was completed, the mixture was diluted by adding water (150 mL) and extracted with DCM (100 mL). The organic phase was collected, washed with water (80 mL), and then concentrated under reduced pressure to obtain 3.0 g crude 383-D, which was purified by silica gel column chromatography and eluted with ethyl acetate: n-heptane = 1.5:1 to obtain 1.74 g 383-D with a yield of 82%.

### Step 4: Synthesis of Amino Lipid Compound 383

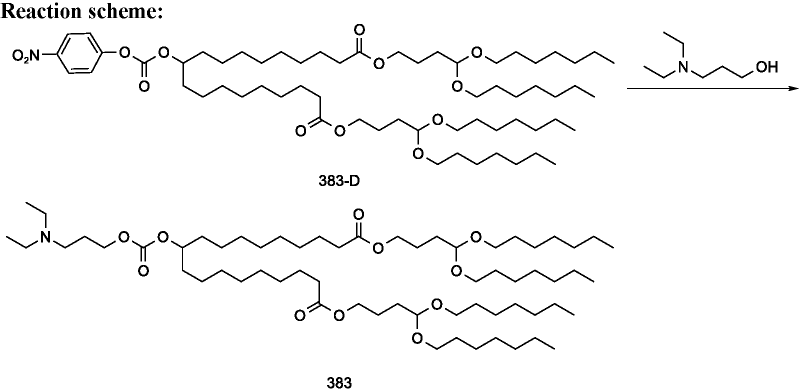

### Experimental procedure:

383-D (1.74 g, 1.61 mmol), DCM (18 mL), DMAP (59 mg, 0.48 mmol), triethylamine (0.49 mg, 4.84 mmol), and diethylaminopropanol (1.27 g, 9.68 mmol) were added to a 50 mL single-necked round-bottom flask and stirred at room temperature for 2 days. After the reaction was completed, the mixture was diluted by adding water (100 mL) and extracted with DCM (100 mL). The organic phase was collected, washed with water (80 mL), and then concentrated under reduced pressure to obtain 2.8 g crude amino lipid compound 383, which was purified by silica gel column chromatography and eluted with ethyl acetate: n-heptane = 1.5:1 to obtain 945 mg amino lipid compound 383 with a yield of 55% and a purity of 96.55%.

### Amino lipid compound 383

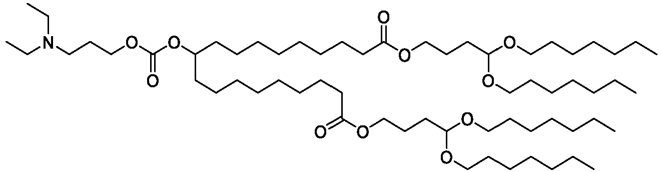

¹H NMR in CDCl₃ δ 4.69-4.65 (m, 1H), 4.48 (t, J= 5.3 Hz, 2H), 4.17 (t, J= 6.6 Hz, 2H), 4.07 (t, J= 6.1 Hz, 4H), 3.58-3.54 (m, 4H), 3.42-3.38 (m, 4H), 2.53-2.49 (m, 6H), 2.28 (t, J= 7.5 Hz, 4H), 1.83-1.79 (m, 2H), 1.71-1.66 (m, 12H), 1.61-1.52 (m, 12H), 1.36-1.28 (m, 52H), 1.01 (t, J= 7.2 Hz, 6H) 0.88 (t, J= 7.1 Hz, 12H).

LC-MS (ESI): Calculated for (M+H) 1070.92, Found 1071.5.

### Example 2: Synthesis of Amino Lipid Compound 382

According to the general synthetic process, amino lipid compound 382 was prepared according to the method of Example 1, with replacing BHB with compound BSB, to obtain 655 mg amino lipid compound 382 with a yield of 58% and a purity of 97.63%.

### BSB

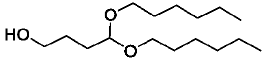

### Amino lipid compound 382

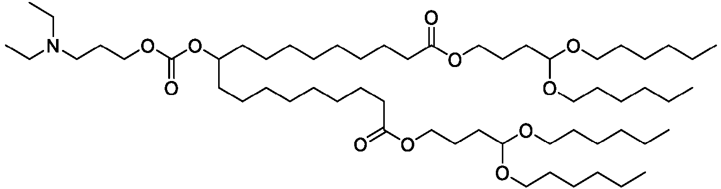

¹H NMR in CDCl₃ δ4.69-4.65 (m, 1H), 4.48 (t, J= 5.1 Hz, 2H), 4.17 (t, J= 6.5 Hz, 2H), 4.07 (t, J= 6.0 Hz, 4H), 3.58-3.55 (m, 4H), 3.42-3.38 (m, 4H), 2.53-2.49 (m, 6H), 2.28 (t, J= 7.6 Hz, 4H), 1.84-1.79 (m, 2H), 1.74-1.65 (m, 12H), 1.61-1.52 (m, 12H), 1.37-1.27 (m, 44H), 1.01 (t, J= 7.2 Hz, 6H) 0.89 (t, J= 6.9 Hz, 12H).

LC-MS (ESI): Calculated for (M+H) 1014.85, Found 1015.4.

### Example 3: Synthesis of Amino Lipid Compound 1245

### Step 1: Synthesis of 3-Bromopropanal

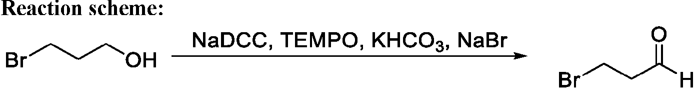

### Experimental procedure:

3-Bromopropanol (10.0 g, 72 mmol) and DCM (150 mL) were added to a 500 mL single-necked flask. TEMPO (225 mg, 1.44 mmol), potassium bicarbonate (5.05 g, 50.4 mmol), and sodium bromide (296 mg, 2.88 mmol) were added with stirring. The reaction solution was cooled to 5°C and added with aqueous NaDCC solution (9.5 g, 43.2 mmol) dropwise. After the dropwise addition was completed, the mixture was reacted at 5°C for 3 h and filtered. The filter cake was washed once with 20 mL water and once with 20 mL DCM. The organic phase was collected and concentrated under reduced pressure to obtain crude 3-bromopropanal, which was purified by silica gel column chromatography and eluted with DCM to obtain 9.0 g 3-bromopropanal with a yield of 91.2%.

### Step 2: Synthesis of 3,3-Dioctyloxybromopropane

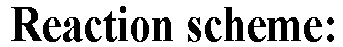

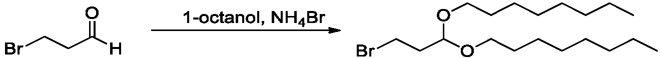

### Experimental procedure:

3-Bromopropanal (9.0 g, 65.7 mmol), cyclopentyl methyl ether (80 mL), 1-octanol (21.4 g, 164.3 mmol), and ammonium bromide (322 mg, 3.3 mmol) were added to a 250 mL single-necked flask. The reaction solution was heated to 130°C with stirring, refluxed for water separation, reacted for 6 h, and filtered. The filtrate was collected and concentrated under reduced pressure to obtain crude 3,3-dioctyloxybromopropane, which was purified by silica gel column chromatography and eluted with n-hexane to obtain 9.63 g 3,3-dioctyloxybromopropane with a yield of 38.6%.

### Step 3: Synthesis of 1245-A

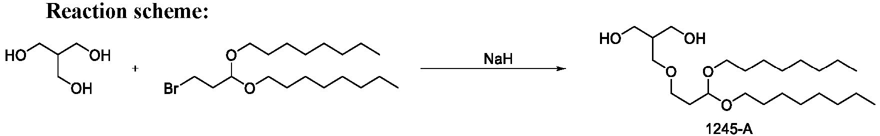

### Experimental procedure:

2-Hydroxymethyl-1,3-propanediol (4.5 g, 42.7 mmol) and DMF (250 mL) were added to a 500 mL two-necked flask. The reaction solution was cooled to 0°C with stirring, added with sodium hydride (1.5 g, 37.0 mmol) portionwise, reacted at 0°C for 1 h, and slowly added with a solution of 3,3-dioctyloxy-1-bromopropane in DMF (11.2 g, 29.5 mmol, dissolved in 20 mL DMF) dropwise. After the dropwise addition was completed, the reaction solution was warmed to 10°C for 6 h, added with 200 mL water for quenching, and then transferred to a 1 L separatory funnel, added with 300 mL water, and extracted twice with 200 mL ethyl acetate. The organic phases were combined, washed with 300 mL water, and then concentrated under reduced pressure to obtain crude 1245-A, which was purified by silica gel column chromatography and eluted with n-hexane: ethyl acetate = 3:1 to obtain 5.87 g 1245-A with a yield of 49.2%.

### Step 4: Synthesis of 1245-B

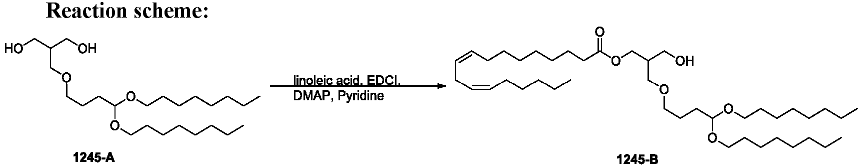

### Experimental procedure:

1245-A (5.87 g, 14.0 mmol) and DCM (280 mL) were added to a 500 mL two-necked flask. The reaction solution was cooled to -10°C with stirring and added with linoleic acid (4.32 g, 15.4 mmol), EDCI (4.03 g, 21.0 mmol), DMAP (171 mg, 1.4 mmol), and pyridine (1.66 g, 21.0 mmol). After the dropwise addition was completed, the mixture was reacted at -10°C for 8 h. The reaction solution was warmed to room temperature, added with 200 mL water for quenching, and then extracted twice with 200 mL DCM. The organic phases were combined and concentrated under reduced pressure to obtain crude 1245-B, which was purified by silica gel column chromatography and eluted with n-hexane: ethyl acetate = 5:1 to obtain 5.6 g 1245-B with a yield of 58.7%.

### Step 5: Synthesis of Amino Lipid Compound 1245

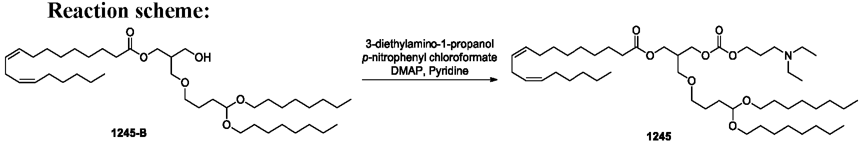

### Experimental procedure:

1245-B (4.85 g, 7.12 mmol), DCM (70 mL), DMAP (261 mg, 2.14 mmol), and pyridine (1.41 g, 17.8 mmol) were added to a 250 mL two-necked flask and stirred at room temperature. Then *p*-nitrophenyl chloroformate (2.87 g, 15.54 mmol) was slowly added portionwise and reacted at room temperature for 1 h. 3-Diethylamino-1-propanol (2.8 g, 21.4 mmol) was added and reacted at room temperature for 12 h. The solvent was evaporated under reduced pressure, and 100 mL water was added. The mixture was extracted twice with 100 mL n-hexane. The organic phases were combined, washed with 150 mL water, and then concentrated under reduced pressure to obtain crude amino lipid compound 1245, which is purified by silica gel column chromatography and eluted with n-hexane: ethyl acetate = 1:1 to obtain 3.28 g amino lipid compound 1245 with a yield of 55.0% and a purity of 96.30%.

LC-MS (ESI): Calculated for (M+H) 838.7, Found 839.1.

### Example 4: Synthesis of Amino Lipid Compound 1246

### Step 1: Synthesis of 4-(tert-butyldimethylsilyl)oxo-1-butanol

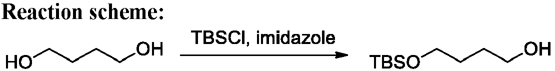

### Experimental procedure:

1,4-butanediol (20.0 g, 222 mmol), DCM (660 mL), and imidazole (22.67 g, 333 mmol) were added to a 1 L single-necked flask. The reaction solution was cooled to 0°C with stirring, added with TBSCl (36.7 g, 244 mmol), reacted at 0°C for 6 h, added with 400 mL water, and extracted twice with 300 mL DCM. The organic phases were combined and concentrated under reduced pressure to obtain crude 4-(tert-butyldimethylsilyl)oxo-1-butanol, which was purified by silica gel column chromatography and eluted with n-hexane: ethyl acetate = 5:1 to obtain 22.5 g 4-(tert-butyldimethylsilyl)oxo-1-butanol with a yield of 49.5%.

### Step 2: Synthesis of 4-(tert-butyldimethylsilyl)oxo-1-butanal

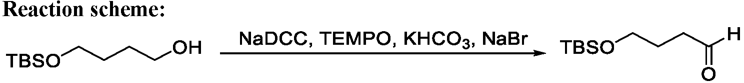

### Experimental procedure:

4-(tert-butyldimethylsilyl)oxo-1-butanol (22.5 g, 110 mmol) and DCM (300 mL) were added to a 1 L single-necked flask, and TEMPO (344 mg, 2.2 mmol), potassium bicarbonate (7.7 g, 77 mmol), and sodium bromide (453 mg, 4.4 mmol) were added with stirring. The reaction solution was cooled to 5°C, added with aqueous NaDCC solution (14.5 g, 66 mmol, dissolved in 150 mL water) dropwise, reacted at 5°C for 3 h, and filtered. The filter cake was washed once with 50 mL water and once with 50 mL DCM. The filtrate was collected and separated into phases. The organic phase was collected, and the aqueous phase was extracted twice with 150 mL DCM. The organic phases were combined and concentrated under reduced pressure to obtain crude 4-(tert-butyldimethylsilyl)oxo-1-butanal, which was purified by silica gel column chromatography and eluted with DCM to obtain 11.4 g 4-(tert-butyldimethylsilyl)oxo-1-butanal with a yield of 51.2%.

### Step 3: Synthesis of 4,4-dioctyloxy-1-TBS butanol

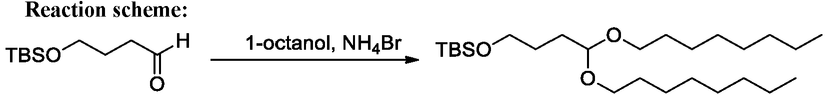

### Experimental procedure:

4-(tert-butyldimethylsilyl)oxo-1-butanal (10.0 g, 49.4 mmol), cyclopentyl methyl ether (80 mL), 1-octanol (16.1 g, 123.5 mmol), and ammonium bromide (242 mg, 2.47 mmol) were added to a 250 mL single-necked flask. The reaction solution was heated to 130°C with stirring, refluxed for water separation, reacted for 6 h, and filtered. The filtrate was collected and concentrated under reduced pressure to obtain crude 4,4-dioctyloxy-1-TBS butanol, which was purified by silica gel column chromatography and eluted with n-hexane to obtain 10.2 g 4,4-dioctyloxy-1-TBS butanol with a yield of 46.4%.

### Step 4: Synthesis of 4,4-dioctyloxy-1-butanol

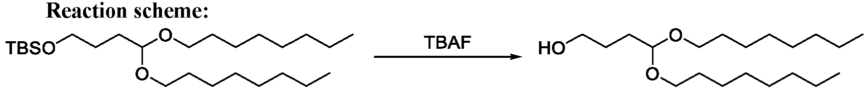

### Experimental procedure:

4,4-dioctyloxy-1-TBS butanol (10.0 g, 22.5 mmol), tetrahydrofuran (110 mL), and TBAF (10.6 g, 33.7 mmol) were added to a 250 mL single-necked flask, reacted at room temperature with stirring for 6 h, added with 80 mL saturated sodium bicarbonate solution, and then extracted twice with 80 mL ethyl acetate. The organic phases were combined, washed with 100 mL saturated sodium chloride solution, dried over anhydrous sodium sulfate, and filtered. The filtrate was collected and concentrated under reduced pressure to obtain crude 4,4-dioctyloxy-1-butanol, which was purified by silica gel column chromatography and eluted with n-hexane: ethyl acetate = 5:1 to obtain 6.5 g 4,4-dioctyloxy-1-butanol with a yield of 87.4%.

### Step 5: Synthesis of 1246-A1

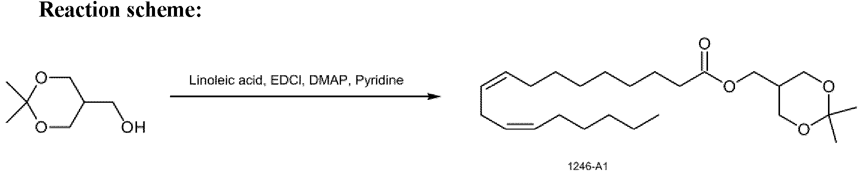

### Experimental procedure:

5-hydroxymethyl-2,2-dimethyl-1,3-dioxane (13.0 g, 88.9 mmol) and DCM (270 mL) were added to a 500 mL two-necked flask. The reaction solution was cooled to -10°C with stirring, added with linoleic acid (25.0 g, 88.9 mmol), EDCI (25.6 g, 133.5 mmol), DMAP (2.18 g, 17.8 mmol), and pyridine (10.6 g, 134.0 mmol), warmed to room temperature for 8 h, added with 200 mL water for quenching, and extracted twice with 200 mL DCM. The organic phases were combined and concentrated under reduced pressure to obtain crude 1246-A1, which was purified by silica gel column chromatography and eluted with n-hexane: ethyl acetate = 20:1 to obtain 23.6 g 1246-A1 with a yield of 64.9%.

### Step 6: Synthesis of 1246-A

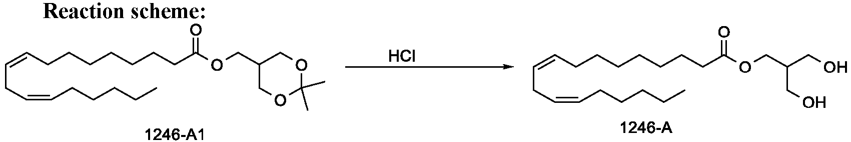

### Experimental procedure:

1246-A1 (23.6 g, 57.8 mmol) and ethanol (360 mL) were added to a 1 L round-bottom flask and stirred at room temperature. Hydrochloric acid (120 mL) was added dropwise and reacted at room temperature for 1 h. The reaction solution was added with 600 mL saturated sodium bicarbonate solution for quenching and extracted twice with 500 mL DCM. The organic phases were combined and concentrated under reduced pressure to obtain crude 1246-A, which was purified by silica gel column chromatography and eluted with n-hexane: ethyl acetate = 3:1 to obtain 17.5 g 1246-A with a yield of 82.2%.

### Step 7: Synthesis of 1246-A-TBS

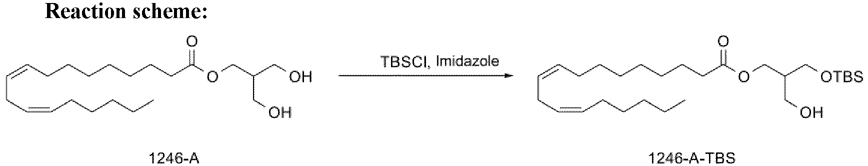

### Experimental procedure:

1246-A (21.3 g, 57.8 mmol) and DCM (600 mL) were added to a 1 L round-bottom flask. The reaction solution was cooled to 0°C with stirring, added with imidazole (5.9 g, 86.7 mmol) and TBSCl (9.6 g, 63.7 mmol), reacted at 0°C for 8 h, added with 500 mL saturated sodium bicarbonate solution for quenching, and extracted twice with DCM (500 mL). The organic phases were combined, washed with 400 mL saturated sodium chloride solution, and then concentrated under reduced pressure to obtain a crude product, which was purified by silica gel column chromatography and eluted with n-hexane: ethyl acetate = 10:1 to obtain 12.4 g 1246-A-TBS with a yield of 44.4%.

### Step 8: Synthesis of 1246-B

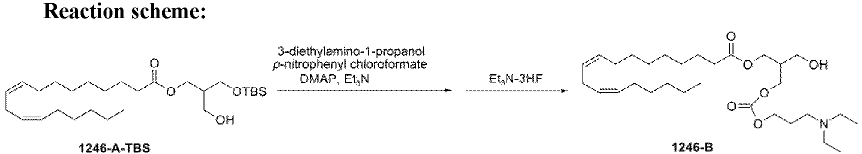

### Experimental procedure:

1246-A-TBS (4.5 g, 9.32 mmol), DCM (45 mL), and DMAP (0.34 g, 2.79 mmol) were added to a 100 mL two-necked flask, and pyridine (1.62 g, 20.5 mmol) was added dropwise with stirring. *P*-nitrophenyl chloroformate (3.75 g, 18.6 mmol) was slowly added portionwise and reacted at room temperature for 1 h. A mixture solution of 3-diethylamino-1-propanol (3.66 g, 27.9 mmol) and triethylamine (0.93 g, 9.32 mmol) was added and reacted at room temperature for 16 h. The solvent was evaporated under reduced pressure, and 200 mL water was added. The mixture was extracted twice with 200 mL n-hexane. The organic phases were combined, washed with 200 mL water and 150 mL saturated sodium bicarbonate solution, and then concentrated under reduced pressure to obtain 5.72 g oil.

The above oil (5.72 g) and tetrahydrofuran (57 mL) were added to a 250 mL round-bottom flask and stirred at room temperature. Triethylamine trihydrofluoride (14.35 g) was added dropwise and reacted at room temperature for 16 h. The reaction solution was concentrated, diluted with 100 mL ethyl acetate, adjusted to pH = 8 with saturated sodium bicarbonate solution, and washed with 200 mL water. The aqueous phase was extracted twice with 200 mL ethyl acetate. The organic phases were combined and concentrated under reduced pressure to obtain crude 1246-B, which was purified by silica gel column chromatography and eluted with DCM: methanol = 10:1 to obtain 960 mg 1246-B with a yield of 19.6%.

### Step 9: Synthesis of 1246-C

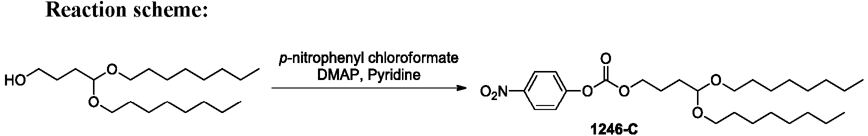

### Experimental procedure:

4,4-dioctyloxy-1-butanol (1.0 g, 3.02 mmol), DCM (10 mL), DMAP (74 mg, 0.6 mmol), and pyridine (0.36 g, 4.5 mmol) were added to a 25 mL single-necked flask and stirred at room temperature. *P*-nitrophenyl chloroformate (0.91 g, 4.5 mmol) was slowly added portionwise and reacted at room temperature for 1 h. The reaction solution was concentrated, added with 100 mL water, and extracted twice with 100 mL n-hexane. The organic phases were combined, washed with 150 mL water and 150 mL saturated sodium bicarbonate solution, and then concentrated under reduced pressure to obtain crude 1246-C, which was purified by silica gel column chromatography and eluted with n-hexane: ethyl acetate = 1:1 to obtain 905 mg 1246-C with a yield of 60.3%.

### Step 10: Synthesis of Amino Lipid Compound 1246

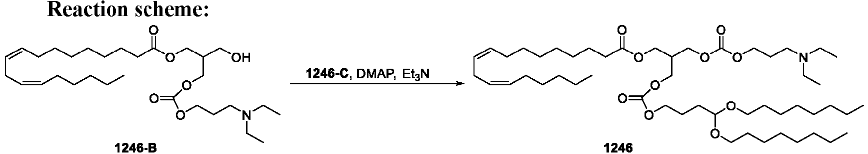

### Experimental procedure:

1246-B (0.96 g, 1.82 mmol), DCM (10 mL), DMAP (67 mg, 0.55 mmol), and triethylamine (0.45 mL, 3.65 mmol) were added to a 25 mL two-necked flask and stirred at room temperature. 1246-C (0.91 g, 1.82 mmol) was added and reacted at room temperature for 12 h. The mixture was added with 100 mL water and extracted with 50 mL DCM. The aqueous phase was extracted twice with 100 mL DCM. The organic phases were combined, washed with 150 mL saturated sodium bicarbonate solution, and then concentrated under reduced pressure to obtain crude amino lipid compound 1246, which was purified by silica gel column chromatography and eluted with n-hexane: ethyl acetate = 1:1 to obtain 1.25 g amino lipid compound 1246 with a yield of 77.6% and a purity of 94.13%.

¹H NMR (600 MHz, CDCl₃) δ 5.41-5.29 (m, 4H), 4.47 (t, *J* = 5.5 Hz, 1H), 4.22-4.12 (m, 10H), 3.55 (dt, *J* = 9.2, 6.7 Hz, 2H), 3.40 (dt, *J* = 9.2, 6.7 Hz, 2H), 2.76 (t, *J* = 6.9 Hz, 2H), 2.50 (q, *J* = 7.2 Hz, 6H), 2.45 (dt, *J* = 11.9, 5.9 Hz, 1H), 2.30 (t, *J* = 7.6 Hz, 2H), 2.04 (q, *J* = 7.0 Hz, 4H), 1.84-1.77 (m, 2H), 1.77-1.64 (m, 4H), 1.63-1.58 (m, 2H), 1.58-1.51 (m, 4H), 1.39-1.20 (m, 34H), 1.00 (t, *J* = 7.1 Hz, 6H), 0.88 (q, *J* = 6.9 Hz, 9H).

¹³C NMR (151 MHz, CDCl₃) δ 173.60, 155.13, 155.11, 130.36, 130.18, 128.18, 128.05, 102.70, 68.30, 67.12, 66.00, 65.03, 65.00, 61.39, 49.21, 47.01, 37.75, 34.23, 31.98, 31.66, 30.02, 29.93, 29.75, 29.58, 29.49, 29.41, 29.32, 29.26, 27.34, 26.60, 26.40, 25.77, 24.98, 24.20, 22.80, 22.71, 14.24, 14.22, 11.87.

LC-MS (ESI): Calculated for (M+H) 882.7, Found 883.1.

### Example 5: Synthesis of Amino Lipid Compound 1247

According to the general synthetic process and the method of Example 3, amino lipid compound 1247 was prepared according to the following reaction scheme to obtain 2.0 g amino lipid compound 1247 with a yield of 54.9% and a purity of 95.75%.

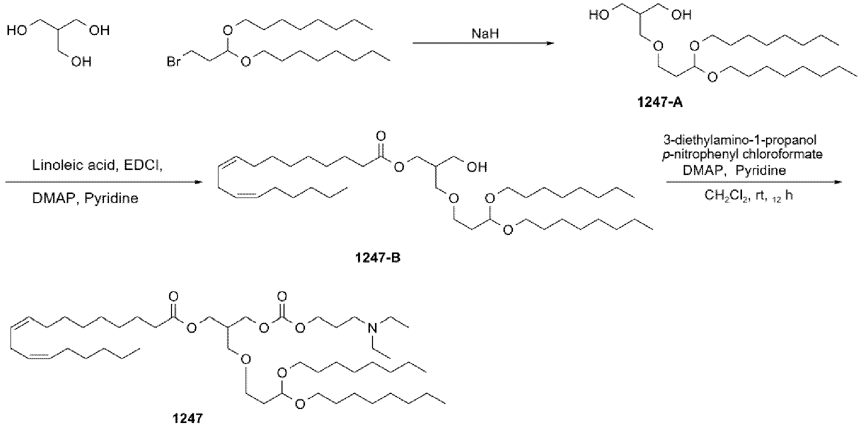

¹H NMR (600 MHz, CDCl₃) δ 5.41-5.29 (m, 4H), 4.58 (t, *J* = 5.8 Hz, 1H), 4.22-4.14 (m, 5H), 4.11 (dd, *J* = 11.1, 6.4 Hz, 1H), 3.56 (dt, *J* = 9.2, 6.7 Hz, 2H), 3.46 (dd, *J* = 13.7, 6.1 Hz, 4H), 3.40 (dt, *J* = 9.3, 6.7 Hz, 2H), 2.77 (t, *J =* 6.9 Hz, 2H), 2.62-2.49 (m, 6H), 2.33-2.28 (m, 3H), 2.04 (q, *J* = 7.0 Hz, 4H), 1.85 (q, *J=* 6.2 Hz, 2H), 1.83-1.78 (m, 2H), 1.63-1.58 (m, 2H), 1.58-1.52 (m, 4H), 1.38-1.22 (m, 34H), 1.00 (t, *J* = 7.1 Hz, 6H), 0.88 (q, *J* = 7.0 Hz, 9H).

¹³C NMR (151 MHz, CDCl₃) δ 173.74, 155.31, 130.36, 130.18, 128.19, 128.05, 100.79, 68.34, 67.59, 66.95, 66.28, 66.27, 65.88, 62.18, 49.23, 47.01, 38.65, 34.33, 34.09, 31.99, 31.67, 30.07, 29.76, 29.60, 29.49, 29.44, 29.34, 29.29, 29.28, 27.34, 26.62, 26.42, 25.77, 25.05, 22.81, 22.72, 14.24, 14.22, 11.88.

LC-MS (ESI): Calculated for (M+H) 824.7, Found 825.1.

### Example 6: Synthesis of Amino Lipid Compound 1248

According to the general synthetic process and the method of Example 3, amino lipid compound 1248 was prepared according to the following reaction scheme to obtain 5.47 g amino lipid compound 1248 with a yield of 82.6% and a purity of 96.52%.

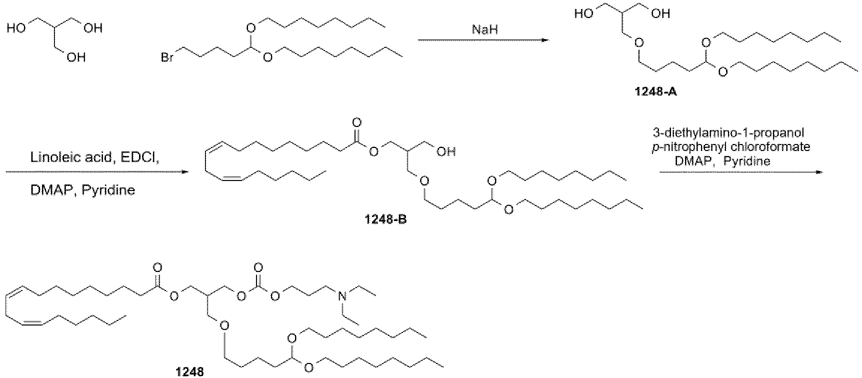

¹H NMR (600 MHz, CDCl₃) δ 5.41-5.19 (m, 4H), 4.44 (t, *J =* 5.7 Hz, 1H), 4.23-4.14 (m, 5H), 4.11 (dd, *J* = 11.2, 6.4 Hz, 1H), 3.55 (dt, *J* = 9.2, 6.7 Hz, 2H), 3.44 (d, *J* = 5.9 Hz, 2H), 3.42-3.36 (m, 4H), 2.77 (t, *J* = 6.8 Hz, 2H), 2.62-2.49 (m, 6H), 2.33-2.28 (m, 3H), 2.04 (q, *J* = 7.0 Hz, 4H), 1.83-1.77 (m, 2H), 1.63-1.58 (m, 4H), 1.58-1.52 (m, 6H), 1.41-1.21 (m, 36H), 1.00 (t, *J* = 7.1 Hz, 6H), 0.88 (q, *J* = 6.9 Hz, 9H).

¹³C NMR (151 MHz, CDCl₃) δ 173.75, 155.31, 130.36, 130.18, 128.18, 128.05, 103.14, 71.44, 68.26, 66.93, 65.93, 65.73, 62.23, 49.23, 47.02, 38.69, 34.34, 33.42, 31.99, 31.67, 30.06, 29.76, 29.59, 29.57, 29.49, 29.43, 29.34, 29.28, 27.34, 26.62, 26.42, 25.77, 25.05, 22.81, 22.72, 21.52, 14.25, 14.22, 11.89.

LC-MS (ESI): Calculated for (M+H) 852.7, Found 853.1.

### Example 7: Synthesis of Amino Lipid Compound 387

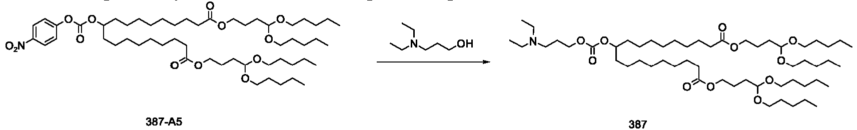

According to the general synthetic process, amino lipid compound 387 was synthesized according to the procedure in Step 4 of Example 1, with replacing 383-D with compound 387-A5, where 0.37 g amino lipid compound 387 was obtained from 387-A5 (1.7 g, 1.76 mmol) and 3-diethylamino-1-propanol (1.39 g, 10.56 mmol) with a yield of 21.93% and a purity of 95.40%.

¹H NMR (600 MHz, CDCl₃) δ 4.67 (m, 1H), 4.48 *(t, J=* 5.3 Hz, 2H), 4.17 *(t, J=* 6.5 Hz, 2H), 4.07 (t, *J=* 6.2 Hz, 4H), 3.56 (dt, *J=* 9.3, 6.7 Hz, 4H), 3.40 (dt, *J=* 9.3, 6.7 Hz, 4H), 2.55-2.47 (m, 6H), 2.28 *(t, J=* 7.6 Hz, 4H), 1.85-1.76 (m, 2H), 1.74-1.63 (m, 8H), 1.63-1.48 (m, 16H), 1.38-1.20 (m, 36H), 1.00 (t, *J=* 7.1 Hz, 6H), 0.90 (m, 12H).

LC-MS (ESI): Calculated for (M+H) 958.79, Found 959.3.

### Example 8: Synthesis of Amino Lipid Compound 388

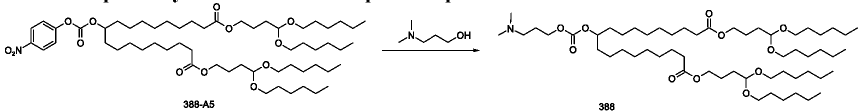

According to the general synthetic process, amino lipid compound 388 was synthesized according to the procedure in Step 4 of Example 1, with replacing 383-D with compound 388-A5, where 0.37 g amino lipid compound 388 was obtained from 388-A5 (1.2 g, 1.17 mmol) and 3-dimethylamino-1-propanol (0.72 g, 7.02 mmol) with a yield of 31.95% and a purity of 94.45%.

¹H NMR (600 MHz, CDCl₃) δ 4.71-4.63 (m, 1H), 4.48 (t, *J* = 5.3 Hz, 2H), 4.17 (t, *J =* 6.6 Hz, 2H), 4.08 (t, *J* = 6.2 Hz, 4H), 3.57 (dt, *J* = 9.3, 6.7 Hz, 4H), 3.41 (dt, *J* = 9.3, 6.7 Hz, 4H), 2.39-2.34 (m, 2H), 2.28 (t, *J =* 7.6 Hz, 4H), 2.22 (s, 6H), 1.88-1.80 (m, 2H), 1.74-1.63 (m, 10H), 1.63-1.47 (m, 16H), 1.39-1.22 (m, 42H), 0.95-0.81 (m, 12H).

LC-MS (ESI): Calculated for (M+H) 986.82, Found 987.4.

### Example 9: Synthesis of Amino Lipid Compound 389

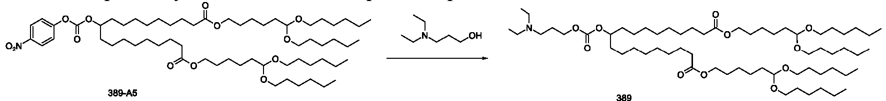

According to the general synthetic process, amino lipid compound 389 was synthesized according to the procedure in Step 4 of Example 1, with replacing 383-D with compound 389-A5, where 1.05 g amino lipid compound 389 was obtained from 389-A5 (2.4 g, 2.23 mmol) and 3-diethylamino-1-propanol (1.75 g, 13.35 mmol) with a yield of 43.97% and a purity of 92.22%.

¹H NMR (600 MHz, CDCl₃) δ 4.72-4.62 (m, 1H), 4.45 (t, *J* = 5.7 Hz, 2H), 4.17 (t, *J =* 6.5 Hz, 2H), 4.05 (t, *J* = 6.7 Hz, 4H), 3.56 (dt, *J* = 9.3, 6.7 Hz, 4H), 3.40 (dt, *J* = 9.3, 6.7 Hz, 4H), 2.61-2.43 (m, 6H), 2.28 (t, *J=* 7.6 Hz, 4H), 1.87-1.76 (m, 2H), 1.57 (m, 26H), 1.43-1.16 (m, 50H), 1.01 (t, *J* = 7.1 Hz, 6H), 0.89 (t, *J* = 6.9 Hz, 12H).

LC-MS (ESI): Calculated for (M+H) 1070.67, Found.

### Example 10: Synthesis of Amino Lipid Compound 390

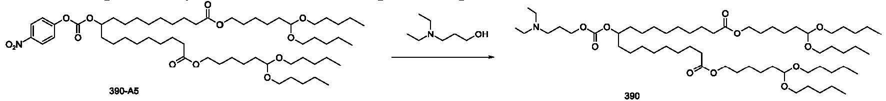

According to the general synthetic process, amino lipid compound 390 was synthesized according to the procedure in Step 4 of Example 1, with replacing 383-D with compound 390-A5, where 0.44 g amino lipid compound 390 was obtained from 390-A5 (1.2 g, 1.17 mmol) and 3-diethylamino-1-propanol (0.92 g, 7.02 mmol) with a yield of 37.06% and a purity of 91.06%.

¹H NMR (600 MHz, CDCl₃) δ 4.71-4.61 (m, 1H), 4.45 (t, *J* = 5.7 Hz, 2H), 4.17 (t, *J =* 6.5 Hz, 2H), 4.05 (t, *J* = 6.7 Hz, 4H), 3.56 (dt, *J=* 9.3, 6.7 Hz, 4H), 3.40 (dt, *J* = 9.3, 6.7 Hz, 4H), 2.51 (td, *J* = 7.1, 3.4 Hz, 6H), 2.28 (t, *J* = 7.6 Hz, 4H), 1.85-1.76 (m, 2H), 1.76-1.47 (m, 26H), 1.43-1.17 (m, 42H), 1.01 (t, *J* = 7.1 Hz, 6H), 0.90 (m, 12H).

LC-MS (ESI): Calculated for (M+H) 1014.85, Found 1015.3.

### Example 11: Synthesis of Amino Lipid Compound 391

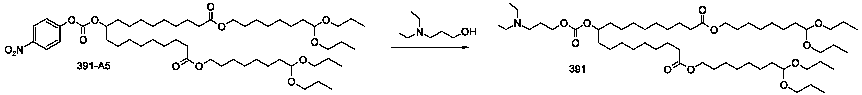

According to the general synthetic process, amino lipid compound 391 was synthesized according to the procedure in Step 4 of Example 1, with replacing 383-D with compound 391-A5, where 0.847 g amino lipid compound 391 was obtained from 391-A5 (2 g, 2.03 mmol) and 3-diethylamino-1-propanol (1.6 g, 12.18 mmol) with a yield of 43.53% and a purity of 92.68%.

¹H NMR (600 MHz, CDCl₃) δ 4.71-4.61 (m, 1H), 4.45 (t, J = 5.7 Hz, 2H), 4.17 (t, J = 6.5 Hz, 2H), 4.05 (t, J = 6.7 Hz, 4H), 3.56 (dt, J = 9.3, 6.7 Hz, 4H), 3.40 (dt, J = 9.3, 6.7 Hz, 4H), 2.51 (td, J = 7.1, 3.4 Hz, 6H), 2.28 (t, J = 7.6 Hz, 4H), 1.85-1.76 (m, 2H), 1.76-1.47 (m, 26H), 1.43-1.17 (m, 34H), 1.01 (t, J = 7.1 Hz, 6H), 0.90 (m, 12H).

LC-MS (ESI): Calculated for (M+H) 958.46, Found 959.1.

### Example 12: Synthesis of Amino Lipid Compound 392

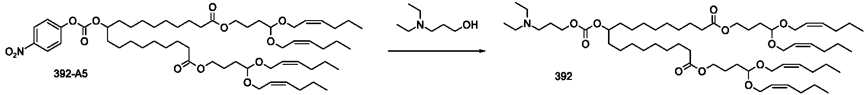

According to the general synthetic process, amino lipid compound 392 was synthesized according to the procedure in Step 4 of Example 1, with replacing 383-D with compound 392-A5, where 0.29 g amino lipid compound 392 was obtained from 392-A5 (0.86 g, 0.85 mmol) and 3-diethylamino-1-propanol (0.67g, 5.1mmol) with a yield of 33.9% and a purity of 97.93%.

¹H NMR (600 MHz, CDCl₃) δ 5.62-5.48 (m, 8H), 4.69-4.65 (m, 1H), 4.62-4.53 (m, 2H), 4.20-4.11 (m, 6H), 4.09-4.06 (m, 8H), 2.54-2.45 (m, 6H), 2.28 (t, *J =* 7.6 Hz, 4H), 2.06-2.03 (m, 8H), 1.84-1.76 (m, 2H), 1.75-1.66 (m, 8H), 1.64-1.46 (m, 8H), 1.43-1.35 (m, 8H), 1.35-1.22 (m, 20H), 1.00 (t, *J =* 7.1 Hz, 6H), 0.90 (t, *J* = 7.4 Hz, 12H).

LC-MS (ESI): Calculated for (M+H) 1006.50, Found 1007.1.

### Example 13: Synthesis of Amino Lipid Compound 396

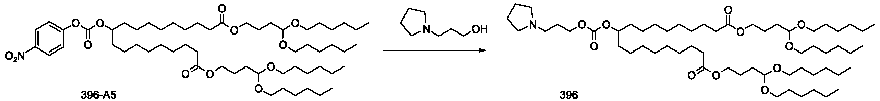

According to the general synthetic process, amino lipid compound 396 was synthesized according to the procedure in Step 4 of Example 1, with replacing 383-D with compound 396-A5, where 0.29 g amino lipid compound 396 was obtained from 396-A5 (1.3 g, 1.27 mmol) and 1-(3-hydroxypropyl)pyrrolidine (0.67 g, 5.18 mmol) with a yield of 22.55% and a purity of 95.18%.

¹H NMR (600 MHz, CDCl₃) δ 4.71-4.62 (m, 1H), 4.48 (t, J = 5.2 Hz, 2H), 4.18 (t, J = 6.6 Hz, 2H), 4.07 (t, J = 6.1 Hz, 4H), 3.56 (dt, J = 9.3, 6.7 Hz, 4H), 3.40 (dt, J = 9.3, 6.7 Hz, 4H), 2.55-2.49 (m, 6H), 2.28 (t, J = 7.6 Hz, 4H), 1.92-1.97 (m, 2H), 1.80-1.63 (m, 14H), 1.63-1.45 (m, 16H), 1.43-1.16 (m, 42H), 0.89 (t, J = 6.9 Hz, 12H).

LC-MS (ESI): Calculated for (M+H) 1012.55, Found 1013.2.

### Example 14: Synthesis of Amino Lipid Compound 398

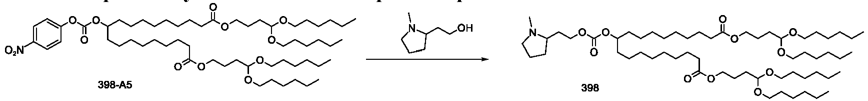

According to the general synthetic process, amino lipid compound 398 was synthesized according to the procedure in Step 4 of Example 1, with replacing 383-D with compound 398-A5, where 0.29 g amino lipid compound 398 was obtained from 398-A5 (1 g, 0.98 mmol) and N-methyl-2-(2-hydroxyethyl)pyrrolidine (0.76 g, 5.88 mmol) with a yield of 29.23% and a purity of 96.77%.

¹H NMR (600 MHz, CDCl₃) δ 4.72-4.62 (m, 1H), 4.48 (d, J = 5.3 Hz, 2H), 4.25-4.18 (m, 1H), 4.18-4.12 (m, 1H), 4.07 (t, J = 6.0 Hz, 4H), 3.56 (dt, J = 8.5, 6.7 Hz, 4H), 3.40 (dt, J = 8.9, 6.8 Hz, 4H), 3.05 (t, J = 8.6 Hz, 1H), 2.28 (dd, J = 16.8, 9.3 Hz, 7H), 2.20-2.02 (m, 3H), 2.00-1.94 (m, 1H), 1.83-1.63 (m, 12H), 1.63-1.43 (m, 18H), 1.40-1.19 (m, 42H), 0.88 (t, J = 6.9 Hz, 12H).

LC-MS (ESI): Calculated for (M+H) 1012.55, Found 1013.2.

### Example 15: Synthesis of Amino Lipid Compound 399

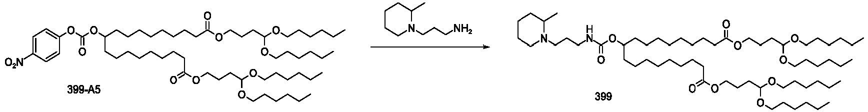

According to the general synthetic process, amino lipid compound 399 was synthesized according to the procedure in Step 4 of Example 1, with replacing 383-D with compound 399-A5, where 0.7 g amino lipid compound 399 was obtained from 399-A5 (1 g, 0.98 mmol) and 3-(2-methylpiperidin-1-yl)propan-1-amine (0.92 g, 5.88 mmol) with a yield of 68.71% and a purity of 96.77%.

¹H NMR (600 MHz, CDCl₃) δ 4.69 (s, 1H), 4.48-4.47 (m, 2H), 4.08-4.06 (t, *J* = 5.9 Hz, 4H), 3.56 (dt, *J =* 13.6, 6.7 Hz, 4H), 3.40 (dt, *J* = 13.8, 6.8 Hz, 4H), 3.32-3.21 (m, 1H), 3.17-3.16 (m, 1H), 2.85-2.83 (m, 1H), 2.79-2.74 (m, 1H), 2.28 (t, *J* = 7.5 Hz, 6H), 2.06 (t, *J* = 10.7 Hz, 1H), 1.81-1.41 (m, 30H), 1.37-1.27 (m, 46H), 1.04 (d, *J* = 6.2 Hz, 3H), 0.89 (t, *J* = 6.8 Hz, 12H).

LC-MS (ESI): Calculated for (M+H) 1039.62, Found 1040.3.

### Example 16: Synthesis of Amino Lipid Compound 416

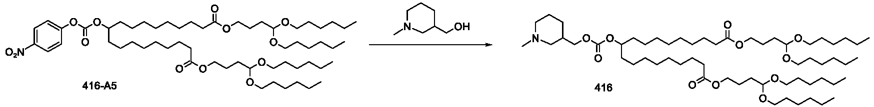

According to the general synthetic process, amino lipid compound 416 was synthesized according to the procedure in Step 4 of Example 1, with replacing 383-D with compound 416-A5, where 0.6 g amino lipid compound 416 was obtained from 416-A5 (1 g, 0.98 mmol) and 1-methylpiperidine-3-methanol (0.76 g, 5.88 mmol) with a yield of 60.47% and a purity of 96.77%.

¹H NMR (600 MHz, CDCl₃) δ 4.70-4.62 (m, 1H), 4.48 (t, *J =* 5.2 Hz, 2H), 4.08-4.03 (m, 5H), 3.94 (dd, *J* = 10.6, 7.4 Hz, 1H), 3.56 (dt, *J* = 9.2, 6.7 Hz, 4H), 3.40 (dt, *J* = 9.2, 6.7 Hz, 4H), 2.85 (d, *J* = 10.2 Hz, 1H), 2.74 (d, *J* = 10.5 Hz, 1H), 2.29-2.25 (m, 7H), 2.06-1.94 (m, 1H), 1.90 (t, *J* = 10.8 Hz, 1H), 1.80-1.44 (m, 29H), 1.42-1.08 (m, 44H), 0.89 (t, *J =* 6.9 Hz, 12H).

LC-MS (ESI): Calculated for (M+H) 1012.55, Found 1013.2.

### Example 17: Synthesis of Amino Lipid Compound 423

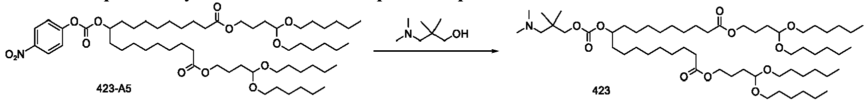

According to the general synthetic process, amino lipid compound 423 was synthesized according to the procedure in Step 4 of Example 1, with replacing 383-D with compound 423-A5, where 0.7 g amino lipid compound 423 was obtained from 423-A5 (1 g, 0.98 mmol) and 3-dimethylamino-2,2-dimethyl-1-propanol (0.77 g, 5.88 mmol) with a yield of 70.4% and a purity of 91.78%.

¹H NMR (600 MHz, CDCl₃) δ 4.72-4.63 (m, 1H), 4.48 (t, *J* = 5.2 Hz, 2H), 4.08 (t, *J =* 6.1 Hz, 4H), 3.94 (s, 2H), 3.57 (dt, *J* = 9.1, 6.7 Hz, 4H), 3.41 (dt, *J* = 9.2, 6.7 Hz, 4H), 2.30-2.23 (m, 10H), 2.17 (s, 2H), 1.75-1.47 (m, 26H), 1.42-1.17 (m, 42H), 0.92 (s, 6H), 0.89 (t, *J* = 6.9 Hz, 12H)

LC-MS (ESI): Calculated for (M+H) 1014.57, Found 1015.2.

### Example 18: Synthesis of Amino Lipid Compound 429

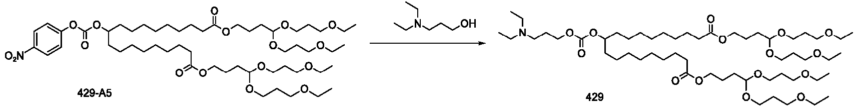

According to the general synthetic process, amino lipid compound 429 was synthesized according to the procedure in Step 4 of Example 1, with replacing 383-D with compound 429-A5, where 0.84 g amino lipid compound 429 was obtained from 429-A5 (2 g, 1.94 mmol) and 3-diethylamino-1-propanol (1.53 g, 11.64 mmol) with a yield of 42.35% and a purity of 97.22%.

¹H NMR (600 MHz, CDCl₃) δ 4.71-4.62 (m, 1H), 4.48 (t, *J* = 5.1 Hz, 2H), 4.17 (t, *J =* 6.5 Hz, 2H), 4.07 (t, *J =* 6.0 Hz, 4H), 3.67 (dt, *J* = 9.3, 6.3 Hz, 4H), 3.55-3.36 (m, 20H), 2.53 (td, *J* = 7.1, 3.4 Hz, 6H), 2.28 (t, *J* = 7.6 Hz, 4H), 1.86-1.81 (m, 10H), 1.73-1.63 (m, 8H), 1.63-1.45 (m, 8H), 1.27 (m, 20H), 1.19 (t, *J* = 7.0 Hz, 12H), 1.03-1.00 (m, 6H).

LC-MS (ESI): Calculated for (M+H) 1022.45, Found 1023.1.

### Example 19: Synthesis of Amino Lipid Compound 430

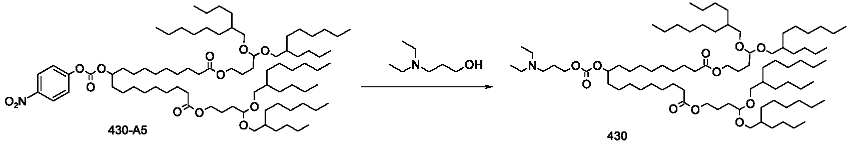

According to the general synthetic process, amino lipid compound 430 was synthesized according to the procedure in Step 4 of Example 1, with replacing 383-D with compound 430-A5, where 0.84 g amino lipid compound 430 was obtained from 430-A5 (2.3 g, 1.7 mmol) and 3-diethylamino-1-propanol (1.34 g, 10.2 mmol) with a yield of 36.7% and a purity of 98.29%.

¹H NMR (600 MHz, CDCl₃) δ 4.70-4.63 (m, 1H), 4.43 (t, *J* = 5.2 Hz, 2H), 4.17 (t, *J* = 6.5 Hz, 2H), 4.07 (t, *J* = 6.2 Hz, 4H), 3.46 (dd, *J* = 9.2, 5.8 Hz, 4H), 3.27 (dd, *J* = 9.2, 5.8 Hz, 4H), 2.57-2.45 (m, 6H), 2.28 (t, *J* = 7.6 Hz, 4H), 1.86-1.76 (m, 2H), 1.74-1.45 (m, 24H), 1.42-1.18 (m, 80H), 1.01 (t, *J* = 7.1 Hz, 6H), 0.89 (q, *J* = 6.9 Hz, 24H).

LC-MS (ESI): Calculated for (M+H) 1351.21, Found 1351.8.

### Example 20: Synthesis of Amino Lipid Compound 490

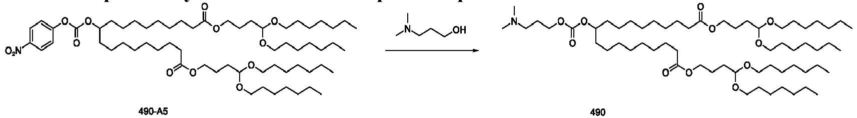

According to the general synthetic process, amino lipid compound 490 was synthesized according to the procedure in Step 4 of Example 1, with replacing 383-D with compound 490-A5, where 1.3 g amino lipid compound 490 was obtained from 490-A5 (2.9 g, 2.69 mmol) and 3-dimethylamino-1-propanol (1.67 g, 16.14 mmol) with a yield of 46.35% and a purity of 93.50%.

¹H NMR (600 MHz, CDCl₃) δ 4.67 (s, 1H), 4.47 (t, *J* = 5.3 Hz, 2H), 4.17 (t, *J* = 6.6 Hz, 2H), 4.07 (t, *J* = 6.2 Hz, 4H), 3.56 (dt, *J* = 9.3, 6.7 Hz, 4H), 3.40 (dt, *J* = 9.3, 6.7 Hz, 4H), 2.37-2.34 (m, 2H), 2.28 (t, *J* = 7.6 Hz, 4H), 2.22 (s, 6H), 1.87-1.81 (m, 2H), 1.68 (t, *J* = 6.6 Hz, 12H), 1.56 (dt, *J* = 14.5, 4.6 Hz, 12H), 1.34-1.24 (m, 52H), 0.88 (t, *J=* 7.0 Hz, 12H).

LC-MS (ESI): Calculated for 1042.62, Found (M+H): 1043.4.

### Example 21: Synthesis of Amino Lipid Compound 849

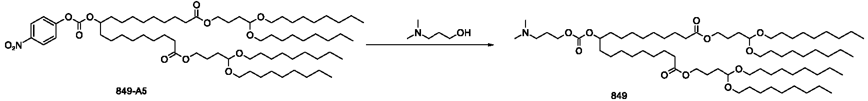

According to the general synthetic process, amino lipid compound 849 was synthesized according to the procedure in Step 4 of Example 1, with replacing 383-D with compound 849-A5, where 0.8 g amino lipid compound 849 was obtained from 849-A5 (2.0 g, 1.68 mmol) and 3-dimethylamino-1-propanol (1.04 g, 10.08 mmol) with a yield of 41.2% and a purity of 93.97%.

¹H NMR (600 MHz, CDCl₃) δ 4.66 (m, 1H), 4.47 (t, *J* = 5.2 Hz, 2H), 4.17 (t, *J* = 6.6 Hz, 2H), 4.07 (t, *J* = 6.1 Hz, 4H), 3.55 (dt, *J* = 9.2, 6.7 Hz, 4H), 3.40 (dt, *J* = 9.2, 6.7 Hz, 4H), 2.38-2.33 (m, 2H), 2.27 (t, *J* = 7.6 Hz, 4H), 2.22 (s, 6H), 1.87-1.80 (m, 2H), 1.71-1.63 (m, 8H), 1.63-1.49 (m, 16H), 1.37-1.20 (m, 66H), 0.87 (t, *J* = 7.0 Hz, 12H).

LC-MS (ESI): Calculated for (M+H) 1154.84, Found 1155.5.

### Example 22: Synthesis of Amino Lipid Compound 859

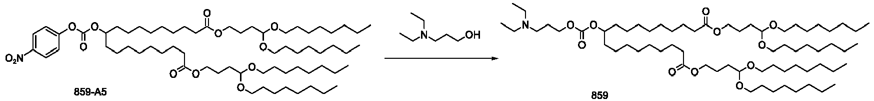

According to the general synthetic process, amino lipid compound 859 was synthesized according to the procedure in Step 4 of Example 1, with replacing 383-D with compound 859-A5, where 270 mg amino lipid compound 859 was obtained from 859-A5 (1.1 g, 0.97 mmol) and 3-diethylamino-1-propanol (0.76 g, 5.82 mmol) with a yield of 24.72% and a purity of 95.67%.

¹H NMR (600 MHz, CDCl₃) δ 4.72-4.59 (m, 1H), 4.48 (t, *J* = 5.1 Hz, 2H), 4.17 (t, *J =* 6.5 Hz, 2H), 4.08 (t, *J* = 6.1 Hz, 4H), 3.56 (dt, *J* = 9.1, 6.7 Hz, 4H), 3.40 (dt, *J* = 9.1, 6.7 Hz, 4H), 2.51 (td, *J* = 7.1, 3.4 Hz, 6H), 2.28 (t, *J* = 7.6 Hz, 4H), 1.84-1.77 (m, 2H), 1.73-1.50 (m, 28H), 1.31 (dd, *J =* 30.6, 6.5 Hz, 56H), 1.01 (t, *J* = 7.1 Hz, 6H), 0.88 (t, *J* = 7.0 Hz, 12H).

LC-MS (ESI): Calculated for 1126.78, Found (M+H): 1127.4.

### Example 23: Synthesis of Amino Lipid Compound 2021

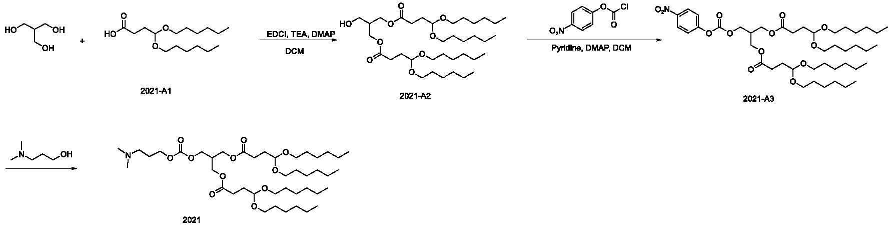

### Step 1: Synthesis of 2021-A2

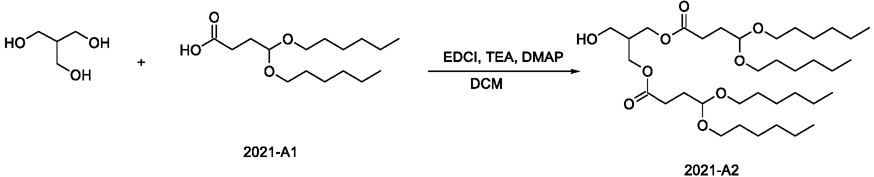

DCM (120 ml) and TEA (4.18 g, 41.75 mmol) were added to EDCI (8.0 g, 41.75 mmol), and 2021-A1 (9.6 g, 33.4 mmol) was added with stirring at room temperature. After the reaction solution was clear, 2-hydroxymethyl-1,3-propanediol (1.8 g, 16.7 mmol) and DMAP (377 mg, 3.09 mmol) were added and reacted at room temperature for 16 h. The reaction solution was washed with saturated potassium bicarbonate (150 ml), and partitioned. The organic phase was collected, and the aqueous phase was extracted once with DCM (80 ml). The organic phases were combined, and the solvent was evaporated under reduced pressure to obtain crude 2021-A2, which was purified by silica gel column chromatography and eluted with n-hexane: ethyl acetate = 20:1 to obtain 4.5 g 2021-A2 with a yield of 41.65%.

### Step 2: Synthesis of 2021-A3

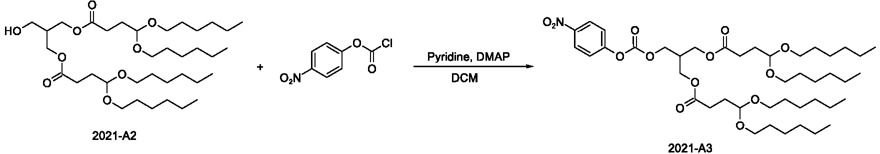

2012-A2 (2.0 g, 3.09 mmol) was added to a 50 mL single-necked round-bottom flask and dissolved in DCM (20 mL). DMAP (113 mg, 0.93 mmol) and pyridine (0.61 g, 7.73 mmol) were added and stirred at room temperature. *P*-nitrophenyl chloroformate (1.25 g, 6.18 mmol) was added portionwise and stirred for another 1 h. The mixture was diluted with water (150 mL) and extracted with DCM (100 mL). The organic phase was collected, washed with water (80 mL), and concentrated to obtain 3.5 g crude 2021-A3, which was purified by silica gel column chromatography and eluted with ethyl acetate: n-heptane = 1.5:1 to obtain 2.13 g 2021-A3 with a yield of 85%.

### Step 3: Synthesis of 2021

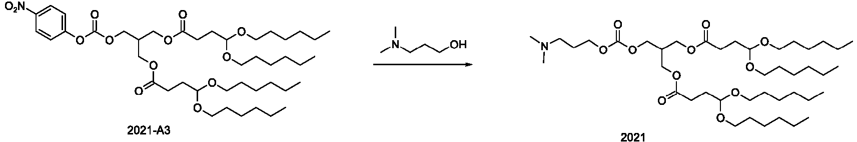

2021-A3 (2.0 g, 2.46 mmol) was added to a 50 mL single-necked round-bottom flask and dissolved in DCM (20 mL). DMAP (92 mg, 0.75 mmol), triethylamine (0.75 mg, 7.38 mmol), and dimethylaminopropanol (1.52 g, 14.76 mmol) were added and stirred at room temperature for 2 days. The mixture was diluted with water (100 mL) and extracted with dichloromethane (100 mL). The organic phase was collected, washed with water (80 mL), and concentrated under reduced pressure to obtain 2.6 g crude 2021, which was purified by silica gel column chromatography and eluted with ethyl acetate: n-heptane = 1.5:1 to obtain 1.16 g amino lipid compound 2021 with a yield of 61% and a purity of 95.32%.

¹H NMR (600 MHz, CDCl₃) δ 4.47 (t, *J =* 5.6 Hz, 2H), 4.18 (t, *J* = 6.1 Hz, 4H), 4.13 (dd, *J*= 6.0, 1.8 Hz, 4H), 3.55 (dt, *J* = 9.3, 6.7 Hz, 4H), 3.39 (dt, *J* = 9.3, 6.7 Hz, 4H), 2.43-2.37 (m, 5H), 2.36-2.32 (m, 2H), 2.21 (s, 6H), 1.91 (td, *J* = 7.6, 5.7 Hz, 4H), 1.85-1.80 (m, 2H), 1.57-1.51 (m, 8H), 1.35-1.24 (m, 24H), 0.88 (t, *J =* 7.0 Hz, 12H).

LC-MS (ESI): Calculated for 776.11, Found (M+H): 776.7.

### Example 24: Synthesis of Amino Lipid Compound 2035

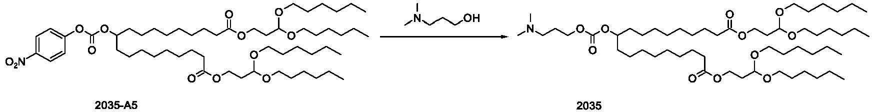

According to the general synthetic process, amino lipid compound 2035 was synthesized according to the procedure in Step 4 of Example 1, with replacing 383-D with compound 2035-A5, where 3.0 g amino lipid compound 2035 was obtained from 2035-A5 (4.1 g, 4.12 mmol) and 3-dimethylamino-1-propanol (2.55 g, 24.6 mmol) with a yield of 75.97% and a purity of 92.02%.

¹H NMR (600 MHz, CDCl₃) δ 4.71-4.63 (m, 1H), 4.60 (t, *J* = 5.8 Hz, 2H), 4.23-4.10 (m, 6H), 3.58 (dt, *J* = 9.2, 6.7 Hz, 4H), 3.42 (dt, *J* = 9.2, 6.7 Hz, 4H), 2.47-2.38 (m, 2H), 2.32-2.24 (m, 10H), 1.94 (q, *J =* 6.4 Hz, 4H), 1.90-1.81 (m, 2H), 1.63-1.49 (m, 16H), 1.38-1.22 (m, 44H), 0.89 (t, *J* = 7.0 Hz, 12H).

LC-MS (ESI): Calculated for (M+H) 958.46, Found 959.2.

### Example 25: Synthesis of Amino Lipid Compound 2036

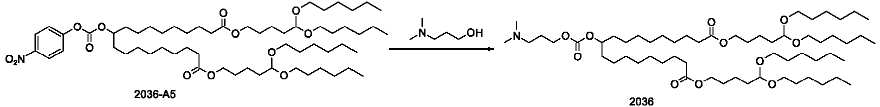

According to the general synthetic process, amino lipid compound 2036 was synthesized according to the procedure in Step 4 of Example 1, with replacing 383-D with compound 2036-A5, where 0.9 g amino lipid compound 2036 was obtained from 2036-A5 (4.85 g, 4.62 mmol) and 3-dimethylamino-1-propanol (2.86 g, 27.7 mmol) with a yield of 19.2% and a purity of 92.23%.

¹H NMR (600 MHz, CDCl₃) δ 4.67 (m, 1H), 4.46 (t, J = 5.7 Hz, 2H), 4.17 (t, J = 6.6 Hz, 2H), 4.06 (t, J = 6.7 Hz, 4H), 3.56 (dt, J = 9.3, 6.7 Hz, 4H), 3.40 (dt, J = 9.3, 6.7 Hz, 4H), 2.39-2.34 (m, 2H), 2.28 (t, J = 7.6 Hz, 4H), 2.22 (s, 6H), 1.84 (dd, J = 14.2, 7.1 Hz, 2H), 1.66-1.54 (m, 30H), 1.45-1.27 (m, 42H), 0.89 (t, J = 7.0 Hz, 12H).

LC-MS (ESI): Calculated for (M+H) 1014.57, Found 1015.3.

### Example 26: Synthesis of Amino Lipid Compound 2038

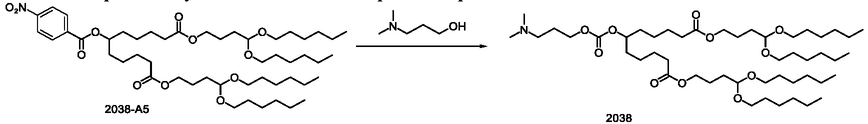

According to the general synthetic process, amino lipid compound 2038 was synthesized according to the procedure in Step 4 of Example 1, with replacing 383-D with compound 2038-A5, where 425 mg amino lipid compound 2038 was obtained from 2038-A5 (0.91 g, 1.02 mmol) and 3-dimethylamino-1-propanol (0.63 g, 6.12 mmol) with a yield of 47.77% and a purity of 94.67%.

¹H-NMR (600 MHz, CDCl₃) δ 4.70-4.63 (m, 1H), 4.48 (t, J = 5.2 Hz, 2H), 4.22 (t, J = 5.8 Hz, 2H), 4.07 (t, J = 6.2 Hz, 4H), 3.57 (dt, J = 9.2, 6.7 Hz, 4H), 3.41 (dt, J = 9.2, 6.7 Hz, 4H), 2.65-2.57 (m, 4H), 2.30 (ddd, J = 15.3, 11.8, 6.9 Hz, 14H), 1.70-1.54 (m, 20H), 1.38-1.27 (m, 28H), 0.89 (t, J = 6.9 Hz, 12H).

LC-MS (ESI): Calculated for 874.29, Found (M+H): 875.1.

### Example 27: Synthesis of Amino Lipid Compound 2039

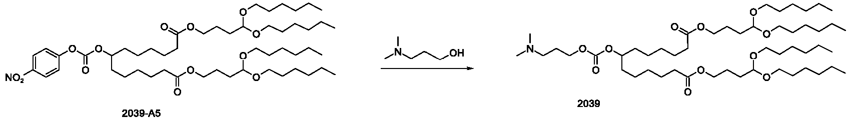

According to the general synthetic process, amino lipid compound 2039 was synthesized according to the procedure in Step 4 of Example 1, with replacing 383-D with compound 2039-A5, where 1.05 g amino lipid compound 2039 was obtained from 2039-A5 (5.0 g, 5.33 mmol) and 3-dimethylamino-1-propanol (3.3 g, 31.97 mmol) with a yield of 21.83% and a purity of 94.51%.

¹H NMR (600 MHz, CDCl₃) δ 4.67 (m, 1H), 4.48 *(t, J=* 5.3 Hz, 2H), 4.17 *(t, J=* 6.6 Hz, 2H), 4.08 (t, *J* = 6.2 Hz, 4H), 3.57 (dt, *J* = 9.3, 6.7 Hz, 4H), 3.41 (dt, *J* = 9.3, 6.7 Hz, 4H), 2.38-2.35 (m, 2H), 2.31-2.26 (m, 4H), 2.23 (s, 6H), 1.85 (dt, *J* = 13.8, 6.7 Hz, 4H), 1.73-1.65 (m, 8H), 1.58 (m, 16H), 1.57 -1.39-1.25 (m, 30H), 0.89 (t, *J* = 7.0 Hz, 12H).

LC-MS (ESI): Calculated for 902.35, Found (M+H): 903.30.

### Example 28: Synthesis of Amino Lipid Compound 2040

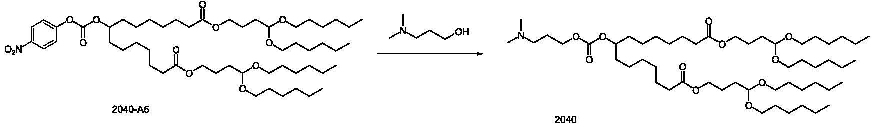

According to the general synthetic process, amino lipid compound 2040 was synthesized according to the procedure in Step 4 of Example 1, with replacing 383-D with compound 2040-A5, where 0.77 g amino lipid compound 2040 was obtained from 2040-A5 (2.0 g, 2.07 mmol) and 3-dimethylamino-1-propanol (1.28 g, 12.42 mmol) with a yield of 40.0% and a purity of 92.49%.

¹H NMR (600 MHz, CDCl₃) δ 4.68 (m, 1H), 4.48 (t, J = 5.3 Hz, 2H), 4.17 (t, J = 6.6 Hz, 2H), 4.08 (t, J = 6.2 Hz, 4H), 3.57 (dt, J = 9.3, 6.7 Hz, 4H), 3.41 (dt, J = 9.3, 6.7 Hz, 4H), 2.38-2.35 (m, 2H), 2.31-2.26 (m, 4H), 2.23 (s, 6H), 1.85 (dt, J = 13.8, 6.7 Hz, 4H), 1.73-1.65 (m, 8H), 1.58 (m, 16H), 1.39-1.25 (m, 34H), 0.89 (t, J = 7.0 Hz, 12H).

LC-MS (ESI): Calculated for 930.40, Found (M+H): 931.2.

### Example 29: Synthesis of Amino Lipid Compound 2041

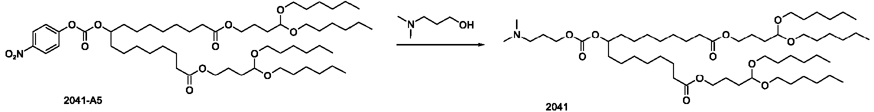

According to the general synthetic process, amino lipid compound 2041 was synthesized according to the procedure in Step 4 of Example 1, with replacing 383-D with compound 2041-A5, where 0.66 g amino lipid compound 2041 was obtained from 2041-A5 (2.5 g, 2.51 mmol) and 3-dimethylamino-1-propanol (1.56 g, 15.08 mmol) with a yield of 27.43% and a purity of 91.97%.

¹H NMR (600 MHz, CDCl₃) δ 4.68 (m, 1H), 4.48 (t, *J* = 5.3 Hz, 2H), 4.17 (t, *J* = 6.6 Hz, 2H), 4.07 (t, *J* = 6.2 Hz, 4H), 3.41 (dt, *J* = 9.3, 6.7 Hz, 4H), 2.56-2.46 (m, 6H), 2.32-2.24 (m, 4H), 1.71-1.66 (m, 12H), 1.62 (m, 4H), 1.59-1.54 (m, 10H), 1.40-1.25 (m, 40H), 1.01 (t, *J* = 7.1 Hz, 6H), 0.89 (t, *J=* 7.0 Hz, 12H).

LC-MS (ESI): Calculated for (M+H) 958.46, Found 959.0.

### Example 30: Synthesis of Amino Lipid Compound 2042

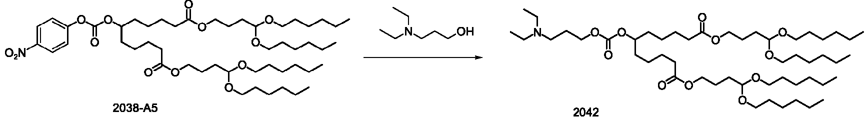

According to the general synthetic process, amino lipid compound 2042 was synthesized according to the procedure in Step 4 of Example 1, with replacing 383-D with compound 2038-A5, where 0.35 g amino lipid compound 2042 was obtained from 2038-A5 (1.0 g, 1.10 mmol) and 3-diethylamino-1-propanol (0.86 g, 6.60 mmol) with a yield of 35.26% and a purity of 96.36%.

¹H NMR (600 MHz, CDCl₃) δ 4.68 (m, 1H), 4.48 (t, *J* = 5.3 Hz, 2H), 4.17 (t, *J* = 6.6 Hz, 2H), 4.07 (t, *J* = 6.2 Hz, 4H), 3.57 (dt, *J* = 9.3, 6.7 Hz, 4H), 3.41 (dt, *J* = 9.3, 6.7 Hz, 4H), 2.56-2.46 (m, 6H), 2.32-2.24 (m, 4H), 1.71-1.66 (m, 12H), 1.62 (m, 4H), 1.59-1.54 (m, 10H), 1.40-1.25 (m, 28H), 1.01 (t, *J* = 7.1 Hz, 6H), 0.89 (t, *J* = 7.0 Hz, 12H).

LC-MS (ESI): Calculated for 902.35, Found (M+H): 903.2.

### Example 31: Synthesis of Amino Lipid Compound 2043

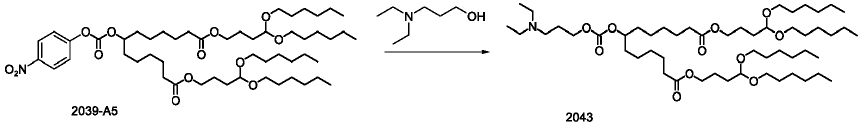

According to the general synthetic process, amino lipid compound 2043 was synthesized according to the procedure in Step 4 of Example 1, with replacing 383-D with compound 2039-A5, where 0.56 g amino lipid compound 2043 was obtained from 2039-A5 (1.2 g, 1.28 mmol) and 3-diethylamino-1-propanol (1.01 g, 7.67 mmol) with a yield of 47.02% and a purity of 90.21%.

¹H NMR (600 MHz, CDCl₃) δ 4.67 (m, 1H), 4.48 (t, *J* = 5.3 Hz, 2H), 4.17 (t, *J* = 6.4 Hz, 2H), 4.07 (t, *J* = 6.2 Hz, 4H), 3.57 (dt, *J* = 9.3, 6.7 Hz, 4H), 3.41 (dt, *J* = 9.3, 6.7 Hz, 4H), 2.56-2.47 (m, 6H), 2.28 (t, *J* = 7.6 Hz, 4H), 1.86-1.78 (m, 4H), 1.72-1.65 (m, 8H), 1.62-1.52 (m, 16H), 1.37-1.24 (m, 30H), 1.01 (t, *J* = 7.1 Hz, 6H), 0.89 (t, *J* = 7.0 Hz, 12H).

LC-MS (ESI): Calculated for 930.40, Found (M+H): 931.3.

### Example 32: Synthesis of Amino Lipid Compound 2044

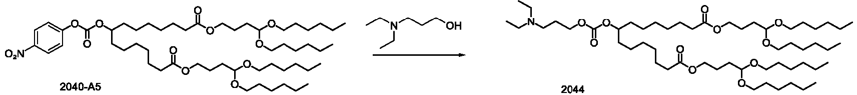

According to the general synthetic process, amino lipid compound 2044 was synthesized according to the procedure in Step 3 of Example 1, with replacing 383-D with compound 2040-A5, where 0.4 g amino lipid compound 2044 was obtained from 2044-A5 (0.93 g, 0.96 mmol) and 3-diethylamino-1-propanol (0.76 g, 5.76 mmol) with a yield of 43.50% and a purity of 90.45%.

¹H NMR (600 MHz, CDCl₃) δ 4.71-4.60 (m, 1H), 4.48 (t, *J* = 5.1 Hz, 2H), 4.20-4.12 (m, 2H), 4.08 (t, *J* = 6.0 Hz, 4H), 3.57 (dt, *J* = 9.2, 6.7 Hz, 4H), 3.41 (dt, *J* = 9.3, 6.7 Hz, 4H), 2.57-2.45 (m, 6H), 2.28 (t, *J* = 7.6 Hz, 4H), 1.93-1.84 (m, 4H), 1.73-1.65 (m, 8H), 1.65-1.51 (m, 16H), 1.35-1.25 (m, 34H), 1.02 (t, *J* = 7.1 Hz, 6H), 0.89 (t, *J* = 6.8 Hz, 12H).

LC-MS (ESI): Calculated for 958.46, Found (M+H): 959.3.

### Example 33: Synthesis of Amino Lipid Compound 2045

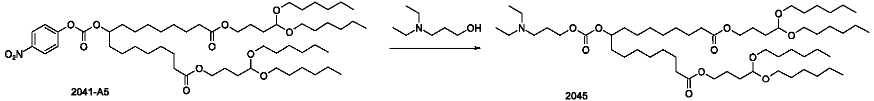

According to the general synthetic process, amino lipid compound 2045 was synthesized according to the procedure in Step 4 of Example 1, with replacing 383-D with compound 2041-A5, where 1.2 g amino lipid compound 2045 was obtained from 2045-A5 (2.5 g, 2.51 mmol) and 3-diethylamino-1-propanol (1.98 g, 15.08 mmol) with a yield of 48.46% and a purity of 90.78%.

¹H NMR (600 MHz, CDCl₃) δ 4.71-4.61 (m, 1H), 4.47 (t, *J* = 5.1 Hz, 2H), 4.16 (t, *J =* 6.5 Hz, 2H), 4.06 (t, *J* = 6.1 Hz, 4H), 3.56 (dt, *J* = 9.3, 6.7 Hz, 4H), 3.39 (dt, *J* = 9.3, 6.7 Hz, 4H), 2.58-2.43 (m, 6H), 2.27 (t, *J =* 7.6 Hz, 4H), 1.90-1.74 (m, 6H), 1.74-1.45 (m, 20H), 1.42-1.16 (m, 40H), 1.00 (t, *J* = 7.1 Hz, 6H), 0.88 (t, *J* = 6.8 Hz, 12H).

LC-MS (ESI): Calculated for (M+H) 986.51, Found 987.1.

### Example 34: Synthesis of Amino Lipid Compound 2061

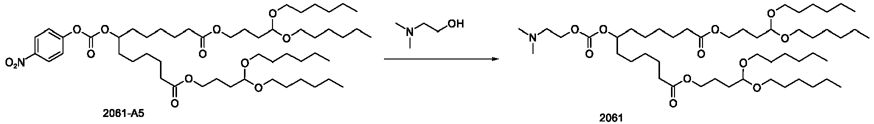

According to the general synthetic process, amino lipid compound 2061 was synthesized according to the procedure in Step 4 of Example 1, with replacing 383-D with compound 2061-A5, where 0.942 g amino lipid compound 2061 was obtained from 2061-A5 (1.87 g, 1.99 mmol) and 3-diethylamino-1-propanol (1.23 g, 11.94 mmol) with a yield of 53.29% and a purity of 94.25%.

¹H-NMR (600 MHz, CDCl₃) δ 4.70-4.63 (m, 1H), 4.48 (t, J = 5.2 Hz, 2H), 4.22 (t, J = 5.8 Hz, 2H), 4.07 (t, J = 6.2 Hz, 4H), 3.57 (dt, J = 9.2, 6.7 Hz, 4H), 3.41 (dt, J = 9.2, 6.7 Hz, 4H), 2.65-2.57 (m, 4H), 2.30 (ddd, J = 15.3, 11.8, 6.9 Hz, 14H), 1.70-1.54 (m, 22H), 1.38-1.27 (m, 28H), 0.89 (t, J = 6.9 Hz, 12H).

LC-MS (ESI): Calculated for 888.32, Found (M+H): 888.7.

### Example 35: Synthesis of Amino Lipid Compound 851

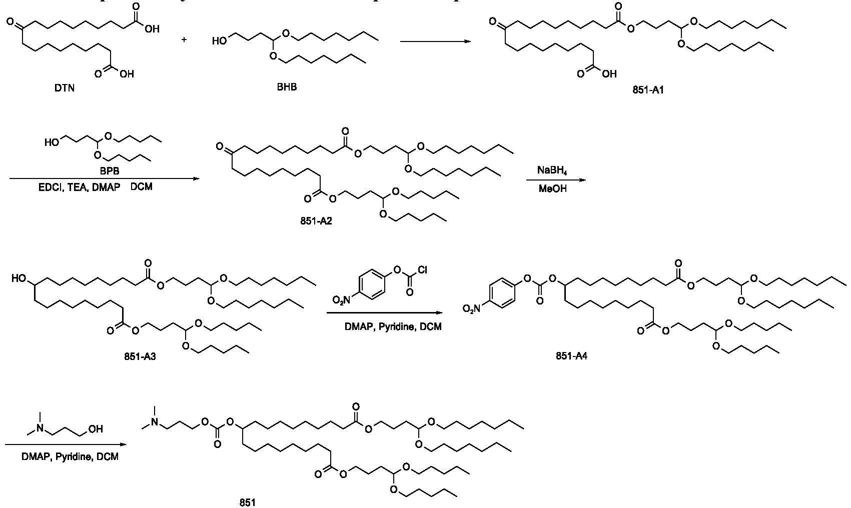

### Step 1: Synthesis of 851-A1

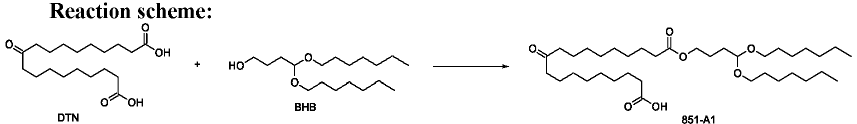

### Experimental procedure:

EDCI (2.8 g, 14.6 mmol), DCM (50 mL), and TEA (1.47 g, 14.6 mmol) were sequentially added to a 100 mL round-bottom flask, shaken and stirred well for 5 min. Then DMAP (0.89 g, 7.3 mmol), BHB (4,4-bis(heptyloxy)butanol) (3.09 g, 10.22 mmol), DTN (10-oxononadecanedioic acid) (5.00 g, 14.6 mmol) were added and stirred at room temperature overnight. DCM (100 mL) and water (150 mL) were added and stirred for extraction, and partitioned. The organic phase was collected, washed with 50 mL saturated sodium chloride solution, and then concentrated under reduced pressure to obtain 8.2 g crude 851-A1, which was purified by silica gel column chromatography and eluted with ethyl acetate: n-heptane = 2:1 to obtain 3.36 g 851-A1 with a yield of 52.3%.

### Step 2: Synthesis of 851-A2

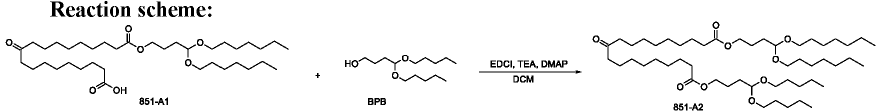

### Experimental procedure:

EDCI (1.1 g, 5.74 mmol), DCM (30 mL), and TEA (0.58 g, 5.74 mmol) were sequentially added to a 100 mL round-bottom flask, shaken and stirred well for 5 min. Then DMAP (0.29 g, 2.39 mmol), BPB (4,4-bis(pentyloxy)butanol) (1.41 g, 5.74 mmol), and 851-A1 (3.00 g, 4.78 mmol) were added and stirred at room temperature overnight. DCM (100 mL) and water (150 mL) were added and stirred for extraction, and partitioned. The organic phase was collected, washed with 50 mL saturated sodium chloride solution, and then concentrated under reduced pressure to obtain 4.8 g crude 851-A2, which was purified by silica gel column chromatography and eluted with ethyl acetate: n-heptane = 1.5:1 to obtain 3.42 g 851-A2 with a yield of 83.6%.

### Step 3: Synthesis of 851-A3

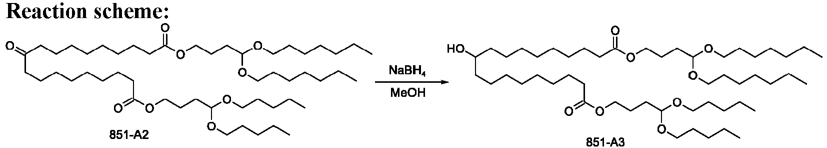

### Experimental procedure:

851-A2 (3.00 g, 3.51 mmol) was added to a 250 mL single-necked flask, dissolved in methanol (30 mL), and cooled to 0°C. Sodium borohydride (0.135 g, 3.51 mmol) was added portionwise with stirring, and the reaction was maintained for 1 h after the addition was completed. The solvent was evaporated under reduced pressure, and water (100 mL) and DCM (100 mL) were added and stirred for extraction. The organic phase was collected, washed with water (50 mL), and then concentrated under reduced pressure to obtain 3.1 g crude 851-A3, which was purified by silica gel column chromatography and eluted with ethyl acetate: n-heptane = 1.5:1 to obtain 2.86 g 851-A3 with a yield of 95.1%.

### Step 4: Synthesis of 851-A4

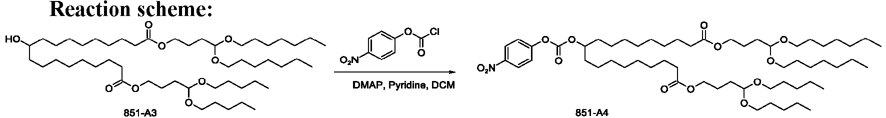

### Experimental procedure:

851-A3 (2.86 g, 3.34 mmol) was added to a 50 mL single-necked round-bottom flask and dissolved in DCM (30 mL). DMAP (122 mg, 1.00 mmol) and pyridine (0.66 g, 8.35 mmol) were added and stirred at room temperature. P-nitrophenyl chloroformate (1.35 g, 6.68 mmol) was added portionwise and stirred for another 1 h. Water (150 mL) and DCM (100 mL) were added and stirred for extraction. The organic phase was collected, washed with water (80 mL), and then concentrated under reduced pressure to obtain 5.0 g crude 851-A4, which was purified by silica gel column chromatography and eluted with ethyl acetate: n-heptane = 1.5:1 to obtain 3.06 g 851-A4 with a yield of 89.6%.

### Step 5: Synthesis of Amino Lipid Compound 851

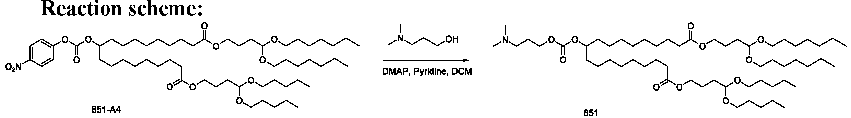

### Experimental procedure:

851-A4 (3.00 g, 2.93 mmol) was added to a 50 mL single-necked round-bottom flask and dissolved in DCM (30 mL). DMAP (108 mg, 0.88 mmol), triethylamine (0.88 mg, 8.8 mmol), and dimethylaminopropanol (1.81 g, 17.58 mmol) were added and stirred at room temperature for 2 days. Water (100 mL) and DCM (100 mL) were added and stirred for extraction. The organic phase was collected, washed with water (80 mL), and then concentrated under reduced pressure to obtain 3.6 g crude amino lipid compound 851, which was purified by silica gel column chromatography and eluted with ethyl acetate: n-heptane = 1.5:1 to obtain 1.69 g amino lipid compound 851 with a yield of 58.3% and a purity of 97.21%.

¹H NMR (600 MHz, CDCl₃) δ 4.66 (m, 1H), 4.47 (dd, *J =* 5.3, 4.5 Hz, 2H), 4.16 (t, *J =* 6.6 Hz, 2H), 4.07 (t, *J* = 5.9 Hz, 4H), 3.56 (dtd, *J* = 8.8, 6.7, 2.0 Hz, 4H), 3.40 (dt, *J* = 9.2, 6.7 Hz, 4H), 2.37-2.33 (m, 2H), 2.27 (t, *J* = 7.6 Hz, 4H), 2.22 (s, 6H), 1.87-1.80 (m, 2H), 1.68 (dt, *J* = 13.0, 7.4 Hz, 8H), 1.62-1.50 (m, 16H), 1.35-1.24 (m, 44H), 0.92-0.85 (m, 12H).

LC-MS (ESI): Calculated for 986.51, Found (M+H): 987.2.

### Example 36: Synthesis of Amino Lipid Compound 854

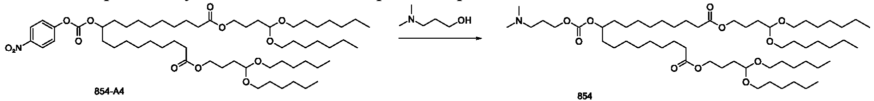

According to the general synthetic process, amino lipid compound 854 was synthesized according to the procedure in Step 5 of Example 35, with replacing 851-A4 with compound 854-A4, where 1.2 g amino lipid compound 854 was obtained from 854-A4 (2.37 g, 2.26 mmol) and 3-dimethylamino-1-propanol (1.40 g, 13.53 mmol) with a yield of 52.33% and a purity of 91.41%.

¹H NMR (600 MHz, CDCl₃) δ 4.66 (s, 1H), 4.47 (t, *J =* 5.2 Hz, 2H), 4.16 (t, *J =* 6.6 Hz, 2H), 4.06 (t, *J =* 6.2 Hz, 4H), 3.55 (dtd, *J =* 8.3, 6.7, 1.5 Hz, 4H), 3.39 (dtd, *J* = 7.4, 6.7, 0.6 Hz, 4H), 2.39-2.34 (m, 2H), 2.27 (t, *J =* 7.6 Hz, 4H), 2.22 (s, 6H), 1.87-1.80 (m, 2H), 1.71-1.63 (m, 8H), 1.62-1.48 (m, 16H), 1.34-1.22 (m, 48H), 0.87 (m, 12H).

LC-MS (ESI): Calculated for 1014.57, Found (M+H): 1015.2.

### Example 37: Synthesis of Amino Lipid Compound 856

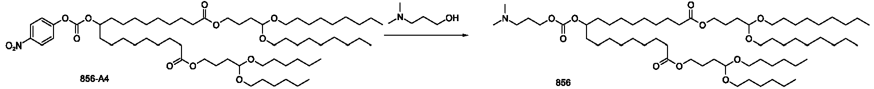

According to the general synthetic process, amino lipid compound 856 was synthesized according to the procedure in Step 5 of Example 35, with replacing 851-A4 with compound 856-A4, where 500 mg amino lipid compound 856 was obtained from 856-A4 (1.5 g, 1.36 mmol) and 3-dimethylamino-1-propanol (0.84 g, 8.13 mmol) with a yield of 34.45% and a purity of 94.85%.

¹H NMR (600 MHz, CDCl₃) δ 4.67 (m, 1H), 4.47 *(t, J=* 5.0 Hz, 2H), 4.17 (t, *J=* 6.6 Hz, 2H), 4.07 (t, *J =* 6.0 Hz, 4H), 3.59-3.53 (m, 4H), 3.43-3.36 (m, 4H), 2.38-2.32 (m, 2H), 2.28 (t, *J=* 7.6 Hz, 4H), 2.22 (s, 6H), 1.86-1.80 (m, 2H), 1.68 (dd, *J* = 18.0, 8.4 Hz, 8H), 1.61-1.49 (m, 16H), 1.36-1.24 (m, 56H), 0.88 (m, 12H).

LC-MS (ESI): Calculated for 1070.67, Found (M+H): 1071.4.

### Example 38: Synthesis of Amino Lipid Compound 861

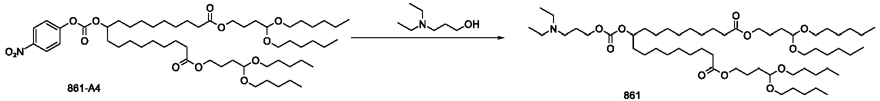

According to the general synthetic process, amino lipid compound 861 was synthesized according to the procedure in Step 5 of Example 35, with replacing 851-A4 with compound 861-A4, where 900 mg amino lipid compound 861 was obtained from 861-A4 (1.5 g, 1.51 mmol) and 3-diethylamino-1-propanol (1.19 g, 9.06 mmol) with a yield of 60.48% and a purity of 94.43%.

¹H NMR (600 MHz, CDCl₃) δ 4.71-4.63 (m, 1H), 4.48 (t, *J* = 5.2 Hz, 2H), 4.17 (t, *J* = 6.5 Hz, 2H), 4.08 (t, *J* = 6.1 Hz, 4H), 3.57 (dt, *J* = 9.2, 6.7 Hz, 4H), 3.41 (dt, *J* = 9.3, 6.7 Hz, 4H), 2.54-2.46 (m, 6H), 2.28 (t, *J =* 7.6 Hz, 4H), 1.85-1.77 (m, 2H), 1.74-1.49 (m, 24H), 1.40-1.23 (m, 40H), 1.01 (t, *J* = 7.1 Hz, 6H), 0.95-0.83 (m, 12H).

LC-MS (ESI): Calculated for 986.51, Found (M+H): 987.2.

### Example 39: Synthesis of Amino Lipid Compound 862

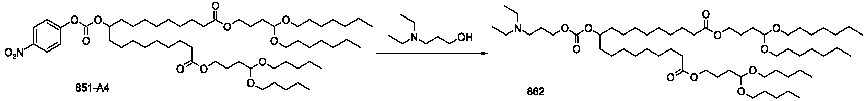

According to the general synthetic process, amino lipid compound 862 was synthesized according to the procedure in Step 5 of Example 35, where 910 mg amino lipid compound 862 was obtained from 851-A4 (2.2 g, 2.15 mmol) and 3-diethylamino-1-propanol (1.69 g, 12.91 mmol) with a yield of 41.69% and a purity of 93.61%.

¹H NMR (600 MHz, CDCl₃) δ 4.71-4.62 (m, 1H), 4.48(t, *J* = 4.7 Hz, 2H), 4.17 (t, *J* = 6.5 Hz, 2H), 4.08 (t, *J* = 5.9 Hz, 4H), 3.57 (m, 4H), 3.41 (dt, *J* = 9.0, 6.8 Hz, 4H), 2.56-2.45 (m, 6H), 2.33-2.25 (m, 4H), 1.85-1.76 (m, 2H), 1.74-1.49 (m, 26H), 1.39-1.22 (m, 42H), 1.01 (t, *J* = 7.1 Hz, 6H), 0.93-0.83 (m, 12H).

LC-MS (ESI): Calculated for 1014.57, Found (M+H): 1015.3.

### Example 40: Synthesis of Amino Lipid Compound 863

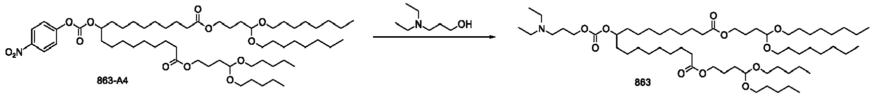

According to the general synthetic process, amino lipid compound 863 was synthesized according to the procedure in Step 5 of Example 35, with replacing 851-A4 with compound 863-A4, where 950 mg amino lipid compound 863 was obtained from 863-A4 (1.81 g, 1.72 mmol) and 3-diethylamino-1-propanol (1.36 g, 10.34 mmol) with a yield of 52.88% and a purity of 93.08%.

¹H NMR (600 MHz, CDCl₃) δ 4.71-4.62 (m, 1H), 4.48 (t, *J* = 4.7 Hz, 2H), 4.17 (t, *J* = 6.5 Hz, 2H), 4.08 (t, *J* = 5.7 Hz, 4H), 3.65-3.51 (m, 4H), 3.41 (dt, *J* = 9.0, 6.8 Hz, 4H), 2.60-2.44 (m, 6H), 2.28 (t, *J* = 7.5 Hz, 4H), 1.83-1.76 (m, 2H), 1.74-1.50 (m, 26H), 1.42-1.19 (m, 46H), 1.01 (t, *J* = 7.1 Hz, 6H), 0.89 (dt, *J* = 13.7, 7.0 Hz, 12H).

LC-MS (ESI): Calculated for 1042.62, Found (M+H): 1043.2.

### Example 41: Synthesis of Amino Lipid Compound 864

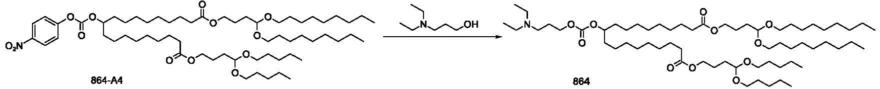

According to the general synthetic process, amino lipid compound 864 was synthesized according to the procedure in Step 5 of Example 35, with replacing 851-A4 with compound 864-A4, where 1.2 g amino lipid compound 864 was obtained from 864-A4 (2.81 g, 2.61 mmol) and 3-diethylamino-1-propanol (2.08 g, 15.63 mmol) with a yield of 42.94% and a purity of 92.67%.

¹H NMR (400 MHz, CDCl₃) δ 4.73-4.60 (m, 1H), 4.48 (dd, *J* = 5.1, 4.5 Hz, 2H), 4.12 (dt, *J* = 11.4, 6.3 Hz, 6H), 3.57 (dtd, *J* = 8.8, 6.7, 2.0 Hz, 4H), 3.41 (dt, *J* = 9.2, 6.7 Hz, 4H), 2.60-2.43 (m, 6H), 2.28 (t, *J* = 7.6 Hz, 4H), 1.86-1.77 (m, 2H), 1.74-1.50 (m, 24H), 1.41-1.20 (m, 52H), 1.01 (t, *J* = 7.1 Hz, 6H), 0.90 (dt, *J* = 13.7, 4.4 Hz, 12H).

LC-MS (ESI): Calculated for 1070.67, Found (M+H): 1071.3.

### Example 42: Synthesis of Amino Lipid Compound 865

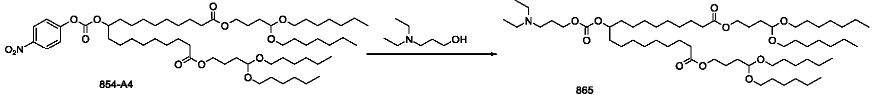

According to the general synthetic process, amino lipid compound 865 was synthesized according to the procedure in Step 5 of Example 35, with replacing 851-A4 with compound 854-A4, where 0.65 g amino lipid compound 865 was obtained from 854-A4 (1.5 g, 1.43 mmol) and 3-diethylamino-1-propanol (1.12 g, 8.57 mmol) with a yield of 43.60% and a purity of 96.53%.

¹H NMR (600 MHz, CDCl₃) δ 4.71-4.63 (m, 1H), 4.48 (t, *J* = 5.2 Hz, 2H), 4.17 (t, *J* = 6.5 Hz, 2H), 4.08 (t, *J* = 6.2 Hz, 4H), 3.57 (m, 4H), 3.44-3.36 (m, 4H), 2.51 (td, *J* = 7.1, 3.4 Hz, 6H), 2.28 (t, *J* = 7.6 Hz, 4H), 1.85-1.77 (m, 2H), 1.74-1.50 (m, 24H), 1.41-1.20 (m, 48H), 1.32 (m, 6H), 0.89 (td, *J* = 7.0, 3.5 Hz, 12H).

LC-MS (ESI): Calculated for 1042.62, Found (M+H): 1043.3.

### Example 43: Synthesis of Amino Lipid Compound 867

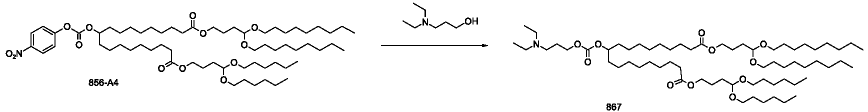

According to the general synthetic process, amino lipid compound 867 was synthesized according to the procedure in Step 5 of Example 35, with replacing 851-A4 with compound 856-A4, where 0.60 g amino lipid compound 867 was obtained from 856-A4 (2.0 g, 1.81 mmol) and 3-diethylamino-1-propanol (1.42 g, 10.84 mmol) with a yield of 30.17% and a purity of 91.94%.

¹H NMR (600 MHz, CDCl₃) δ 4.67 (m, 1H), 4.48 (dd, *J* = 5.3, 4.7 Hz, 2H), 4.17 (t, *J* = 6.5 Hz, 2H), 4.08 (t, *J* = 6.0 Hz, 4H), 3.57 (m, 4H), 3.41 (m, 4H), 2.55-2.48 (m, 6H), 2.28 (t, *J* = 7.6 Hz, 4H), 1.86-1.50 (m, 28H), 1.38-1.22 (m, 54H), 1.01 (t, *J* = 7.1 Hz, 6H), 0.91-0.85 (m, 12H).

LC-MS (ESI): Calculated for 1098.73, Found (M+H): 1099.3.

### Example 44: Synthesis of Amino Lipid Compound 868

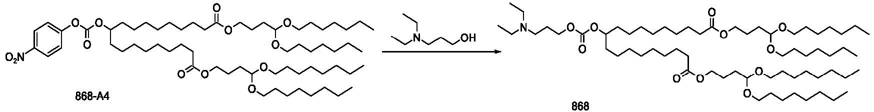

According to the general synthetic process, amino lipid compound 868 was synthesized according to the procedure in Step 5 of Example 35, with replacing 851-A4 with compound 868-A4, where 84 mg amino lipid compound 868 was obtained from 868-A4 (1.0 g, 0.90 mmol) and 3-diethylamino-1-propanol (0.71 g, 5.4 mmol) with a yield of 8.49% and a purity of 92.68%.

¹H NMR (600 MHz, CDCl₃) δ 4.67 (m, 1H), 4.48 (t, *J* = 5.3 Hz, 2H), 4.17 (t, *J* = 6.5 Hz, 2H), 4.08 (t, *J* = 6.2 Hz, 4H), 3.56 (dt, *J* = 9.1, 6.7 Hz, 4H), 3.40 (dt, *J* = 9.2, 6.7 Hz, 4H), 2.54-2.46 (m, 6H), 2.28 (t, *J* = 7.6 Hz, 4H), 1.84-1.77 (m, 2H), 1.73-1.50 (m, 28H), 1.37-1.23 (m, 52H), 1.01 (t, *J* = 7.1 Hz, 6H), 0.88 (td, *J* = 6.9, 1.4 Hz, 12H).

LC-MS (ESI): Calculated for 1098.73, Found (M+H): 1099.4.

### Example 45: Synthesis of Amino Lipid Compound 869

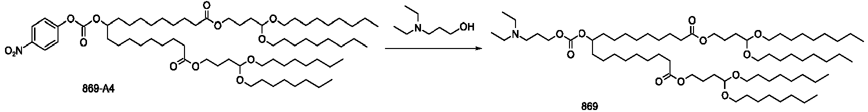

According to the general synthetic process, amino lipid compound 869 was synthesized according to the procedure in Step 5 of Example 35, with replacing 851-A4 with compound 869-A4, where 50 mg amino lipid compound 869 was obtained from 869-A4 (1.25 g, 1.08 mmol) and 3-diethylamino-1-propanol (0.85 g, 6.48 mmol) with a yield of 4.00% and a purity of 93.28%.

¹H NMR (600 MHz, CDCl₃) δ 4.67 (m, 1H), 4.48 (t, *J* = 5.3 Hz, 2H), 4.17 (t, *J* = 6.5 Hz, 2H), 4.08 (t, *J* = 6.2 Hz, 4H), 3.56 (dt, *J* = 9.1, 6.7 Hz, 4H), 3.40 (dt, *J* = 9.2, 6.7 Hz, 4H), 2.54-2.46 (m, 6H), 2.28 (t, *J* = 7.6 Hz, 4H), 1.84-1.77 (m, 2H), 1.73-1.50 (m, 28H), 1.37-1.23 (m, 60H), 1.01 (t, *J* = 7.1 Hz, 6H), 0.88 (td, *J* = 6.9, 1.4 Hz, 12H).

LC-MS (ESI): Calculated for 1154.84, Found (M+H): 1155.3.

### Example 46: Synthesis of Amino Lipid Compound 434

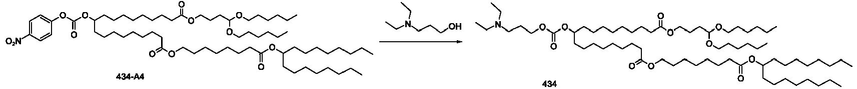

According to the general synthetic process, amino lipid compound 434 was synthesized according to the procedure in Step 4 of Example 1, with replacing 383-D with compound 434-A4, where 1.1 g amino lipid compound 434 was obtained from 434-A4 (2.0 g, 1.74 mmol) and 3-diethylamino-1-propanol (1.37 g, 10.46 mmol) with a yield of 55.51% and a purity of 95.69%.

¹H NMR (600 MHz, CDCl₃) δ 4.70-4.62 (m, 1H), 4.48 (t, *J=* 5.2 Hz, 2H), 4.17 (t, *J =* 6.5 Hz, 2H), 4.08 (t, *J* = 6.2 Hz, 4H), 3.57 (m, 4H), 3.44-3.36 (m, 6H), 2.49-2.44 (m, 6H), 2.28 (t, *J* = 7.6 Hz, 4H), 1.73-1.48 (m, 22H), 1.32 (m, 62H), 0.95(m, 6H), 0.89 (td, *J* = 7.0, 3.5 Hz, 12H).

LC-MS (ESI): Calculated for 1038.98, Found (M+H): 1039.4.

### Example 47: Synthesis of Amino Lipid Compound 436

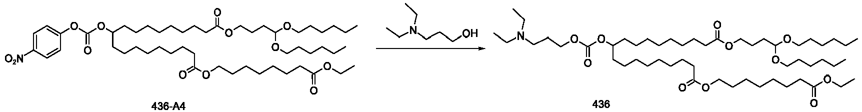

According to the general synthetic process, amino lipid compound 436 was synthesized according to the procedure in Step 4 of Example 1, with replacing 383-D with compound 436-A4, where 0.79 g amino lipid compound 436 was obtained from 436-A4 (2.0 g, 2.14 mmol) and 3-diethylamino-1-propanol (1.68 g, 12.84 mmol) with a yield of 39.6% and a purity of 90.91%.

¹H NMR (600 MHz, CDCl₃) δ 4.70-4.62 (m, 1H), 4.58-4.53 (t, *J* = 7.0 Hz, 1H), 4.48 (t, *J* = 5.2 Hz, 2H), 4.08 (t, *J* = 6.2 Hz, 4H), 3.95 (q, *J* = 7.1 Hz, 2H), 3.57 (m, 4H), 3.44-3.36 (m, 6H), 2.49-2.44 (m, 6H), 2.28 (t, *J* = 7.6 Hz, 4H), 1.73-1.48 (m, 18H), 1.32 (m, 41H), 0.95(m, 6H), 0.89 (t, *J* = 7.0, 6H).

LC-MS (ESI): Calculated for 928.39, Found (M+H): 929.1.

### Example 48: Synthesis of Amino Lipid Compound 437

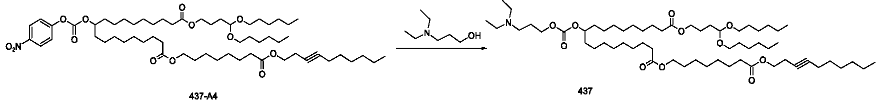

According to the general synthetic process, amino lipid compound 437 was synthesized according to the procedure in Step 4 of Example 1, with replacing 383-D with compound 437-A4, where 0.6 g amino lipid compound 437 was obtained from 437-A4 (1.3 g, 1.24 mmol) and 3-diethylamino-1-propanol (0.98 g, 7.44 mmol) with a yield of 46.7% and a purity of 94.75%.

¹H NMR (600 MHz, CDCl₃) δ 4.70-4.62 (m, 1H), 4.58-4.53 (t, *J =* 7.0 Hz, 1H), 4.48 (t, *J* = 5.2 Hz, 2H), 4.08 (t, *J =* 6.2 Hz, 4H), 3.95 (q, *J =* 7.1 Hz, 2H), 3.57 (m, 4H), 3.44-3.36 (m, 6H), 3.25-3.16 (t, *J =* 6.7, 2H), 2.98-2.89 (t, *J =* 7.5, 2H), 2.49-2.44 (m, 6H), 2.28 (t, *J =* 7.6 Hz, 4H), 1.73-1.48 (m, 18H), 1.32 (m, 46H), 1.05(m, 3H), 0.89 *(t, J=* 7.0, 12H).

LC-MS (ESI): Calculated for 1036.57, Found (M+H): 1037.3.

### Example 49: Synthesis of Amino Lipid Compound 438

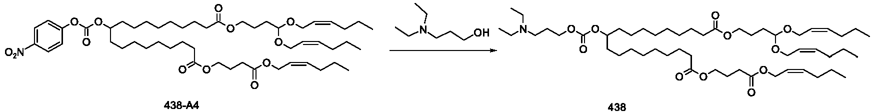

According to the general synthetic process, amino lipid compound 438 was synthesized according to the procedure in Step 4 of Example 1, with replacing 383-D with compound 438-A4, where 0.82 g amino lipid compound 438 was obtained from 438-A4 (1.6 g, 1.72 mmol) and 3-diethylamino-1-propanol (1.35 g, 10.32 mmol) with a yield of 51.6% and a purity of 92.59%.

¹H NMR (600 MHz, CDCl₃) δ 5.53-5.51(dt, *J =* 11.0, 7.8 Hz, 3H), 5.48-5.46(dt, *J* = 10.7, 7.4 Hz, 3H), 4.71-4.63 (m, 1H), 4.58-4.53 (t, *J* = 7.0 Hz, 1H), 4.48 (t, *J* = 5.2 Hz, 2H), 4.15 (d, *J* = 9.66 Hz, 2H), 4.08 (t, *J* = 6.2 Hz, 4H), 3.57 (d, *J* = 7.8 Hz, 4H), 3.44-3.36 (m, 6H), 3.25-3.16 (m, 6H), 2.49-2.44 (q, *J* = 7.6 Hz, 6H), 2.28 (m, 6H), 1.73-1.48 (m, 20H), 1.32 (m, 16H), 0.96(m, 3H), 0.89 (t, *J* = 7.0, 12H).

LC-MS (ESI): Calculated for 922.34, Found (M+H): 923.1.

### Example 50: Synthesis of Amino Lipid Compound 439

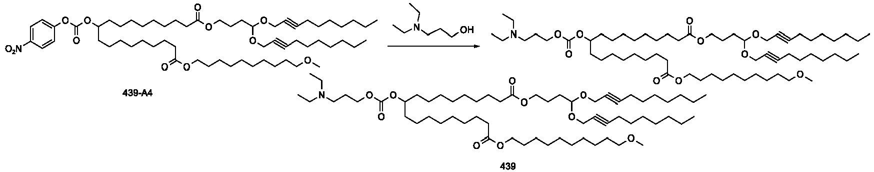

According to the general synthetic process, amino lipid compound 439 was synthesized according to the procedure in Step 4 of Example 1, with replacing 383-D with compound 439-A4, where 0.37 g amino lipid compound 439 was obtained from 439-A4 (1.2 g, 1.15 mmol) and 3-diethylamino-1-propanol (0.91 g, 6.90 mmol) with a yield of 31.6% and a purity of 95.92%.

¹H NMR (600 MHz, CDCl₃) δ 4.86 (t, *J* = 4.9 Hz, 1H), 4.71 (m, 1H), 4.26 (s, 4H), 4.24-4.16 (m, 2H), 4.16-4.04 (m, 4H), 3.40 (t, *J =* 6.6 Hz, 2H), 3.37 (s, 3H), 2.58-2.50 (m, 6H), 2.32 (td, *J* = 7.6, 1.9 Hz, 4H), 2.25 (t, *J =* 7.2 Hz, 4H), 1.76 (t, *J =* 7.8 Hz, 4H), 1.70-1.51 (m, 20H), 1.44-1.26 (m, 46H), 1.05 (t, *J* = 7.1 Hz, 6H), 0.92 (t, *J* = 6.8 Hz, 6H).

LC-MS (ESI): Calculated for 1032.58, Found (M+H): 1033.3.

### Example 51: Synthesis of Amino Lipid Compound 440

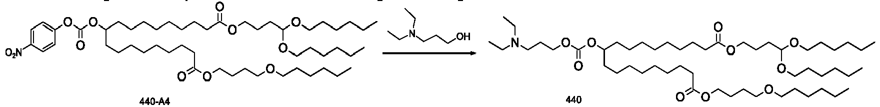

According to the general synthetic process, amino lipid compound 440 was synthesized according to the procedure in Step 4 of Example 1, with replacing 383-D with compound 440-A4, where 0.52 g amino lipid compound 440 was obtained from 440-A4 (1.0 g, 1.08 mmol) and 3-diethylamino-1-propanol (0.85 g, 6.48 mmol) with a yield of 52.5% and a purity of 92.61%.

¹H NMR (600 MHz, CDCl₃) δ 4.70 (m, 1H), 4.51 (t, *J* = 5.3 Hz, 1H), 4.21 (t, *J* = 6.6 Hz, 2H), 4.10 (dt, *J =* 13.5, 6.5 Hz, 4H), 3.60 (dt, *J=* 9.3, 6.7 Hz, 2H), 3.48-3.40 (m, 2H), 3.35 (t, *J =* 6.6 Hz, 4H), 2.43 (t, *J* = 7.8 Hz, 2H), 2.31 (t, *J* = 7.8 Hz, 4H), 2.28 (m, 4H), 1.75-1.54 (m, 24H), 1.37-1.25 (m, 38H), 1.05 (t, *J* = 7.1 Hz, 6H), 0.92 (t, *J* = 6.8 Hz, 9H).

LC-MS (ESI): Calculated for 914.40, Found (M+H): 914.6.

### Example 52: Synthesis of Amino Lipid Compound 441

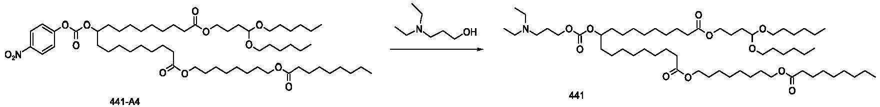

According to the general synthetic process, amino lipid compound 441 was synthesized according to the procedure in Step 4 of Example 1, with replacing 383-D with compound 441-A4, where 0.6 g amino lipid compound 441 was obtained from 441-A4 (1.8 g, 1.74 mmol) and 3-diethylamino-1-propanol (1.37 g, 10.44 mmol) with a yield of 33.2% and a purity of 94.67%.

¹H NMR (600 MHz, CDCl₃) δ 4.70-4.62 (m, 1H), 4.58-4.53 (t, *J =* 7.0 Hz, 1H), 4.48 (t, *J* = 5.2 Hz, 2H), 4.08 (t, *J =* 6.2 Hz, 4H), 3.95 (q, *J =* 7.1 Hz, 2H), 3.57 (m, 4H), 3.44-3.36 (m, 6H), 2.49-2.44 (m, 6H), 2.28 (t, *J =* 7.6 Hz, 4H), 1.73-1.48 (m, 20H), 1.32 (m, 50H), 0.95(m, 6H), 0.89 (t, *J=* 7.0, 9H).

LC-MS (ESI): Calculated for 1026.58, Found (M+H): 1027.3.

### Example 53: Synthesis of Amino Lipid Compound 442

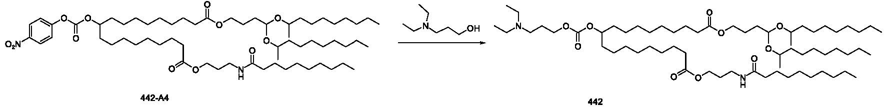

According to the general synthetic process, amino lipid compound 442 was synthesized according to the procedure in Step 4 of Example 1, with replacing 383-D with compound 442-A4, where 0.29 g amino lipid compound 442 was obtained from 442-A4 (1.3 g, 1.19 mmol) and 3-diethylamino-1-propanol (0.94 g, 7.14 mmol) with a yield of 22.8% and a purity of 94.29%.

¹H NMR (600 MHz, CDCl₃) δ 4.70-4.62 (m, 1H), 4.58-4.53 (t, *J =* 7.0 Hz, 1H), 4.48 (t, *J* = 5.2 Hz, 2H), 4.08 (t, *J =* 6.2 Hz, 4H), 3.97-3.94 (m, 2H), 3.66-3.62 (t, *J =* 6.75 Hz, 2H), 3.44-3.36 (m, 6H), 2.49-2.44 (m, 6H), 2.28 (t, *J* = 7.6 Hz, 6H), 1.73-1.48 (m, 16H), 1.32 (m, 56H), 1.25(d, *J* = 6.17 Hz, 6H), 0.95(m, 6H), 0.89 (t, *J* = 7.0, 9H).

LC-MS (ESI): Calculated for 1081.70, Found (M+H): 1082.3.

### Example 54: Synthesis of Amino Lipid Compound 393

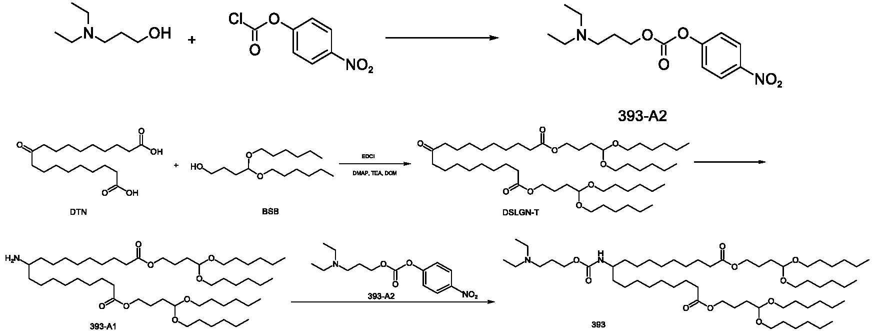

### Step 1: Synthesis of DLSGN-T

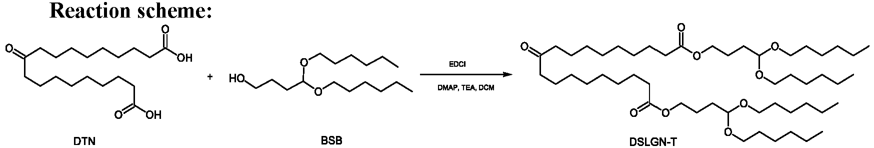

### Experimental procedure:

EDCI (8.40 g, 43.80 mmol), DMAP (0.64 g, 5.26 mmol), and triethylamine (4.44 g, 43.80 mmol) were sequentially added to a 250 mL single-necked flask, dissolved in DCM (60 mL), and stirred at room temperature. Then DTN (6.00 g, 17.5 mmol) and BSB (8.66 g, 31.54 mmol) were added and reacted at room temperature overnight. The reaction was quenched by addition of saturated aqueous ammonium chloride solution (100 mL), and the phases were separated. The organic phase was collected, and the aqueous phase was extracted twice with DCM (100 mL). The organic phases were combined and washed with 100 mL saturated sodium chloride solution. Then, the solvent was evaporated to obtain 15.9 g crude DFSGN-T, which was purified by silica gel column chromatography and eluted with ethyl acetate: n-heptane = 1:10 to obtain 13.6 g DSLGN-T.

### Step 2: Synthesis of 393-A1

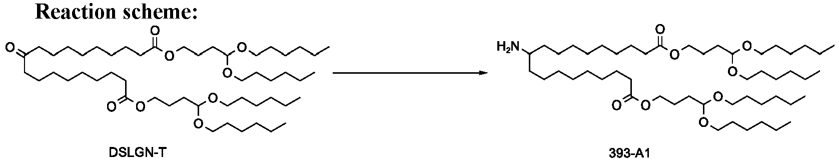

### Experimental procedure:

DSLGN-T (4 g, 4.71 mmol), DCM (20 mL), methanol (20 mL), and ammonium acetate (3.63 g, 47.1 mmol) were sequentially added to a 100 mL single-necked flask and stirred at room temperature for 1 h. Sodium cyanoborohydride (0.89 g, 14.13 mmol) was added and reacted at room temperature overnight. The solvent was evaporated under reduced pressure, and water (50 mL) was added. The mixture was extracted with DCM (50 mL×2). The organic phase was collected, and the solvent was evaporated under reduced pressure to obtain 4.1 g crude 393-A1, which was purified by silica gel column chromatography and eluted with n-hexane: ethyl acetate = 5:1 to obtain 2.46 g 393-A1 with a yield of 61%.

### Step 3: Synthesis of 393-A2

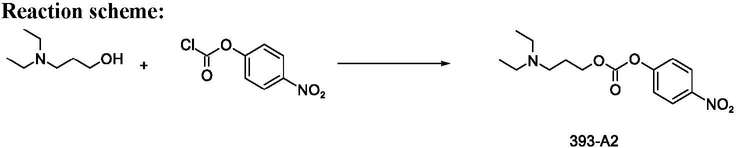

### Experimental procedure:

*P*-nitrophenyl chloroformate (5.53 g, 27.43 mmol) and DCM (15 mL) were added to a 250 mL single-necked flask. Then DMAP (0.28 g, 2.29 mmol), 3-diethylamino-1-propanol (3 g, 22.86 mmol), and pyridine (2.17 g, 27.43 mmol) were dissolved in DCM (15 mL) and slowly added dropwise to the reaction system, followed by reacting for 2 h. The reaction was quenched by addition of 50 mL water. The organic phase was collected, and the aqueous phase was extracted once with 50 mL DCM. The organic phases were combined and concentrated under reduced pressure to obtain a crude product, which was purified by silica gel column chromatography using n-hexane: ethyl acetate = 20:1 to obtain 6.48 g 393-A1 with a yield of 95.6%.

### Step 4: Synthesis of Amino Lipid Compound 393

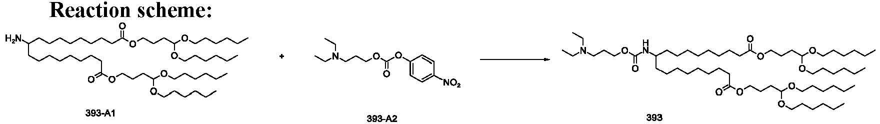

### Experimental procedure:

383-A1 (1 g, 1.17 mmol), DCM (10 mL), DMAP (72 mg, 0.59 mmol), pyridine (0.28 g, 3.51 mmol), and 393-A2 (1.04 g, 3.51 mmol) were added to a 100 mL single-necked flask and reacted at room temperature overnight. Water (20 mL) was added, and the mixture was extracted with DCM (20 mL× 2). The organic phase was collected and concentrated under reduced pressure to obtain crude amino lipid compound 393, which was purified by silica gel column chromatography and eluted with n-hexane: ethyl acetate = 3:1 to obtain 700 mg amino lipid compound 393 with a purity of 94.11% and a yield of 59%.

¹H NMR (600 MHz, CDCl₃) δ 4.48 (t, J = 5.2 Hz, 2H), 4.07 (t, J = 6.1 Hz, 6H), 3.56 (dt, J = 9.2, 6.7 Hz, 5H), 3.40 (dt, J = 9.2, 6.7 Hz, 4H), 2.56-2.47 (m, 6H), 2.28 (t, J = 7.6 Hz, 4H), 1.76 (m, 2H), 1.72-1.63 (m, 8H), 1.57 (m, 12H), 1.45 (m, 2H), 1.38-1.21 (m, 46H), 1.02 (t, J = 7.1 Hz, 6H), 0.89 (t, J = 6.9 Hz, 12H).

LC-MS (ESI): Calculated for (M+H) 1013.87, Found 1014.3.

### Example 55: Synthesis of Amino Lipid Compound 394

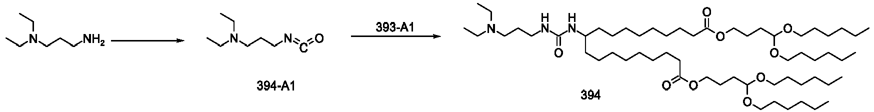

### Experimental procedure:

Triphosgene (214 mg, 0.73 mmol) and DCM (10 mL) were added to a 100 mL single-necked flask. A solution of 3-diethylaminopropylamine (190 mg, 1.46 mmol) in DCM (5 mL) was added dropwise at -10°C and stirred at room temperature for 1 h. Triethylamine (295 mg, 2.92 mmol) was added dropwise and stirred for 10 min. The solvent was evaporated under reduced pressure to obtain a white solid. DCM (10 mL) and 393-A1 (1.2 g, 1.4 mmol) were added and stirred overnight. Water (20 mL) was added and stirred, and partitioned. The organic phase was collected, and the aqueous phase was extracted once with DCM (20 mL). The organic phases were combined and concentrated under reduced pressure to obtain crude amino lipid compound 394, which was purified by silica gel column chromatography and eluted with ethyl acetate: methanol = 30:1 to obtain 1.18 g amino lipid compound 394 with a yield of 83.16%.

¹H NMR (600 MHz, CDCl₃) δ 4.48 (t, J = 5.3 Hz, 2H), 4.07 (t, J = 6.2 Hz, 4H), 3.66-3.59 (m, 1H), 3.57 (dt, J = 9.3, 6.7 Hz, 4H), 3.41 (dt, J = 9.3, 6.7 Hz, 4H), 3.38-3.32 (m, 2H), 3.11-3.07 (m, 4H), 3.03 (t, J = 6.4 Hz, 2H), 2.28 (t, J = 7.6 Hz, 4H), 2.02-1.94 (m, 2H), 1.73-1.63 (m, 8H), 1.63-1.51 (m, 12H), 1.43 (m, 2H), 1.38-1.23 (m, 52H), 0.89 (t, J = 6.8Hz, 12H).

LC-MS (ESI): Calculated for (M+H) 1012.89, Found 1013.6.

### Experimental Example 1: Preparation of Lipid Nanoparticle Encapsulating Luciferase mRNA (Fluc mRNA)

(1) Formulating:
   A specified amount of Fluc mRNA stock solution, 0.2 M sodium acetate buffer, and DEPC water were added to a container, and mixed well to obtain a water phase;
   The amino lipid compound of the present disclosure, a helper lipid (DSPC), a structural lipid (cholesterol), and a PEG-lipid were separately dissolved in absolute ethanol to prepare respective solutions at concentrations of 20 mg/mL, 10 mg/mL, 20 mg/mL, and 25 mg/mL, respectively. The above four solutions were pipetted at a molar ratio of Lipid: DSPC: CHO-HP: M-DMG-2000 of 48:10:40.5:1.5, and mixed well to prepare an alcohol phase.
(2) Encapsulation: The water phase and the alcohol phase were aspirate into water phase and alcoholic phase syringes at a flow rate of water phase: alcohol phase = 9 mL/min: 3 mL/min by using a microfluidic preparation instrument (MPE-L2), and encapsulation was carried out at a flow rate of water phase: alcohol phase = 12 mL/min: 4 mL/min to obtain mRNA-encapsulating lipid nanoparticle (mRNA-LNP).
(3) Dialysis: The product of step (2) was loaded in a dialysis bag and placed in a Tris Buffer-8% (m/V) sucrose solution for replacement to remove ingredients such as residual ethanol, unassembled lipids. Dialysis was conducted for 2 h with magnetic stirring at room temperature and under protection from light (dialysate was replaced every 1 hour).
(4) The product of step (3) was sterilized by passing through a 0.22 µm microporous membrane, and then packaged.

A variety of lipid nanoparticle formulations (LNP formulations) encapsulating Fluc mRNA were prepared, wherein the amino lipid compounds contained in the variety of LNP formulations were different from each other, and each LNP formulation had a Fluc mRNA concentration of 0.2 µg/µL, a mass ratio of Fluc mRNA to Lipid of 1:10, a particle size of 80 nm-130 nm, and an encapsulation efficiency of 85% or higher.

### Experimental Example 2: Performance Evaluation of in vivo Delivery of Lipid Nanoparticle

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: prior to injection of the test LNP formulation, the LNP formulation was gently and repeatedly inverted to thoroughly mix the sample of the formulation. A corresponding amount of the LNP formulation was aspirated with a 1 ml insulin syringe, and the LNP formulation was injected by tail vein injection (IV), with 3 mice in duplicate per formulation. Each mouse was injected with 70 µL of the corresponding LNP formulation encapsulating the luciferase mRNA (Fluc mRNA) prepared in Experimental Example 1.

6 hours after injection of LNP formulations, mice were injected with 200 µL D-Luciferin luciferase developing substrate (Catalog No.122799; Manufacturer: Perkin Elmer). After the substrate was injected, the mice were anesthetized with isoflurane inhalation, and the injection time of luciferase developing substrate was recorded. 10 minutes after the substrate injection, the animals were placed in supine position, and the signal distribution and expression intensity of luciferase in the body and various organs of the animals were observed with In Vivo Imaging System (IVIS).

The encapsulation efficiency of the lipid nanoparticle encapsulating luciferase mRNA (Fluc mRNA) with a representative amino lipid compound and the fluorescence expression intensity induced by the same are shown in Table 4, with the LNP formulation containing SM-102, 1001 or ALC-0315 as a control LNP formulation. The preparation method of the control LNP formulation was basically the same as that of the LNP formulations in Experimental Example 1, with the difference lying in that the amino lipid compounds of the present disclosure were replaced with SM-102, 1001 or ALC-0315 in the preparation of the control LNP formulation.

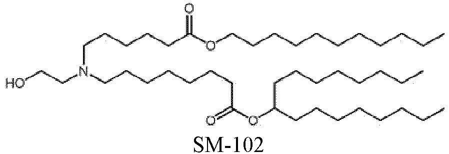

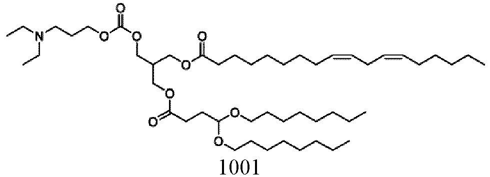

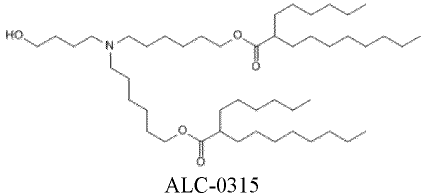

**Table 4**

| Amino lipid compounds | Average number of photons (Total *in-vivo* flux) | Average number of photons (Liver) | Average number of photons (Spleen) |
|---|---|---|---|
| 383 | 6.10E+07 | 8.57E+06 | 1.53E+06 |
| 381 | 2.46E+08 | 4.40E+07 | 1.96E+07 |
| 380 | 4.05E+08 | 7.25E+07 | 6.79E+07 |
| 1246 | 4.92E+07 | 6.85E+06 | 3.83E+06 |
| 1248 | 8.20E+07 | 1.79E+07 | 3.48E+06 |
| 434 | 2.50E+07 | 3.52E+06 | 1.40E+05 |
| 436 | 2.50E+07 | 6.68E+06 | 5.08E+05 |
| 389 | 2.99E+07 | 4.14E+06 | 8.58E+04 |
| 390 | 5.23E+07 | 7.04E+06 | 3.84E+05 |
| 382 | 5.28E+07 | 6.78E+06 | 1.88E+05 |
| 388 | 1.94E+08 | 3.66E+07 | 1.34E+06 |
| 457 | 1.43E+07 | 1.55E+06 | 2.70E+06 |
| 437 | 1.66E+07 | 2.66E+06 | 2.62E+06 |
| 433 | 2.56E+07 | 3.83E+06 | 2.44E+05 |
| 451 | 2.86E+07 | 4.63E+06 | 1.74E+07 |
| 441 | 3.09E+07 | 5.62E+06 | 5.52E+05 |
| 438 | 5.78E+07 | 8.47E+06 | 1.07E+06 |
| 387 | 6.66E+07 | 7.15E+06 | 6.77E+05 |
| 392 | 8.20E+07 | 1.50E+07 | 6.65E+05 |
| 393 | 2.80E+08 | 5.44E+07 | 1.00E+07 |
| 452 | 3.27E+08 | 4.94E+07 | 2.22E+07 |
| 450 | 4.38E+07 | 7.96E+05 | 3.18E+06 |
| 399 | 9.00E+07 | 1.96E+07 | 2.05E+06 |
| 458 | 1.29E+08 | 2.69E+07 | 2.60E+06 |
| 449 | 1.65E+08 | 2.94E+07 | 7.31E+06 |
| 398 | 1.79E+08 | 2.78E+07 | 6.36E+05 |
| 396 | 1.85E+08 | 3.24E+07 | 9.78E+05 |
| 416 | 2.16E+08 | 5.13E+07 | 3.99E+06 |
| 459 | 3.42E+07 | 5.09E+06 | 2.52E+06 |
| 439 | 5.87E+07 | 1.25E+07 | 1.96E+06 |
| 440 | 2.30E+08 | 3.22E+07 | 2.14E+06 |
| 2044 | 4.94E+07 | 6.90E+06 | 1.18E+06 |
| 2045 | 5.83E+07 | 8.90E+06 | 1.89E+06 |
| 2040 | 1.53E+08 | 2.97E+07 | 1.11E+07 |
| 2043 | 1.64E+08 | 2.59E+07 | 1.07E+07 |
| 2039 | 2.05E+08 | 4.26E+07 | 3.75E+07 |
| 2041 | 2.08E+08 | 4.16E+07 | 1.07E+07 |
| 2021 | 4.49E+06 | 1.19E+06 | 1.04E+06 |
| 869 | 1.22E+07 | 2.15E+06 | 5.40E+04 |
| 867 | 2.15E+07 | 4.12E+06 | 1.36E+05 |
| 859 | 2.31E+07 | 3.71E+06 | 7.21E+04 |
| 490 | 2.65E+07 | 3.68E+06 | 5.06E+05 |
| 849 | 2.67E+07 | 4.12E+06 | 1.82E+05 |
| 868 | 3.04E+07 | 5.35E+06 | 1.74E+05 |
| 856 | 3.40E+07 | 9.34E+06 | 3.58E+05 |
| 864 | 3.66E+07 | 6.58E+06 | 2.33E+05 |
| 865 | 4.34E+07 | 8.60E+06 | 3.25E+05 |
| 863 | 4.43E+07 | 7.13E+06 | 2.88E+05 |
| 2036 | 5.60E+07 | 1.04E+07 | 5.75E+05 |
| 861 | 6.67E+07 | 1.17E+07 | 3.52E+05 |
| 862 | 8.57E+07 | 1.53E+07 | 6.67E+05 |
| 851 | 1.28E+08 | 2.21E+07 | 1.93E+06 |
| 2035 | 1.49E+08 | 3.12E+07 | 7.61E+05 |
| ALC-0315 | 6.39E+08 | 1.23E+08 | 3.45E+06 |
| 1001 | 9.68E+07 | 9.38E+06 | 1.65E+07 |
| SM-102 | 3.00E+08 | 6.19E+07 | 6.42E+06 |

### Experimental Example 3: Evaluation of in vivo Safety of Lipid Nanoparticle

A variety of lipid nanoparticle formulations (LNP formulations) encapsulating human erythropoietin mRNA (hEPO mRNA) were prepared according to the method as described in Experimental Example 1, with replacing the luciferase mRNA (Fluc mRNA) with human erythropoietin mRNA (hEPO mRNA), with a concentration of human erythropoietin mRNA (hEPO mRNA) of 0.5 µg/µL, a mass ratio of hEPO mRNA to Lipid of 1:10, a particle size of 90 nm-130 nm, and an encapsulation efficiency of above 90% or higher for each LNP formulation. MC3 in the MC3-containing LNP formulation was

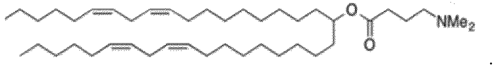

Animal preparation: Female SD rats of 6-8 weeks old were selected and raised in an SPF grade breeding room. Animal testing was conducted in strict accordance with the guidelines of national health institutions and animal ethics requirements.

*In vivo* Delivery: Prior to injection of the test LNP formulations, the LNP formulations were gently and repeatedly inverted to thoroughly mix the formulation samples. A corresponding amount of the formulation samples were aspirated with a 2 ml syringe, and the LNP formulations were injected by tail vein injection (IV), with 4 rats per formulation. Each rat was injected with (1200 µL-hEPO 3mpk) of the human erythropoietin mRNA (hEPO mRNA)-encapsulating LNP formulation. Tris buffer was used as a blank control.

Serum acquisition: Blood samples of rats were collected 12 h after injection, placed in tubes without anticoagulants, and naturally coagulated at room temperature for 30 min-60 min, and then centrifuged at a speed of 3500 rpm for 10 min to obtain the supernatant, which was the serum.

The detection of alanine transaminase was carried out according to instructions of the kit (Nanjing Jiancheng Bioengineering Institute, Catalog. No. C009-2-1), and a standard curve was made using the standard provided in the kit. D-PBS was used in the experiment, which was purchased from Sangon Biotech (Shanghai) Co., Ltd., Catalog No. E607009-0600.

### The method of detection of enzyme activity of alanine aminotransferase (ALT) in serum of the mice is as follows:

Preparation of ALT standard curve:
(1) Enzymatic reaction: 0, 2, 4, 6, 8 and 10 µL of 2 µmol/mL sodium pyruvate standard solution were sequentially added to 5 µL of 0.1 mol/L phosphate buffer, and the volume was supplemented to 25 µL with a ALT matrix solution (alanine aminotransferase matrix solution), and repeatedly aspirating and spitting with a pipette for mixing well;
(2) Addition reaction: 20 µL 2,4-dinitrophenylhydrazine solution was added to all reaction wells in (1), mixed by aspirating and spitting, and then placed in an incubator at 37°C to react for 20 min;
(3) Developing: 200 µL of 0.4 mol/L NaOH solution was added to all reaction wells in (2) to stop the reaction, mixed by aspirating and spitting, and incubated at room temperature for 15 min. The OD value of each well was measured at 510 nm in a microplate reader; and
(4) Data processing of standard curve: The corresponding absolute OD value for each well was obtained by subtracting the OD value for the well with 0 µL sodium pyruvate from the measured OD value for each well, the corresponding ALT Karmen units being 0, 28, 57, 97, 150 and 200, respectively. The standard curve was obtain by taking the absolute OD value as the abscissa and the corresponding Karmen unit as the ordinate.

Detection of ALT enzyme activity in serum samples:
(1) Reagent preparation: an ALT matrix solution was placed in an incubator at 37°C for preheating;
(2) Enzymatic reaction: 5 µL diluted serum was aspirated and added to a 96-well plate, then 20 µL matrix solution was added to the corresponding sample well and mixed by repeatedly aspirating and spitting to avoid bubbling, and then placed in an incubator at 37°C for 30 min;
(3) Addition reaction: 20 µL 2,4-dinitrophenylhydrazine was added to all reaction wells in (2), mixed by aspirating and spitting, and then reacted in an incubator at 37°C for 20 min;
(4) Developing: 200 µL of 0.4 mol/L NaOH solution was added to all reaction wells in (3) to stop the reaction, mixed by aspirating and spitting, and then incubated at room temperature for 15 min. The OD value for each well was measured at a wavelength of 510 nm in a microplate reader; and
(5) Calculation of ALT enzyme activity in serum: The absolute OD value of the corresponding sample well was obtained by subtracting the OD value for the control well sample well without biochemical reaction) from the obtained OD value of the sample well, and was substituted into the standard curve formula to calculate the ALT enzyme activity (Karmen unit) for the corresponding serum sample. The Karman unit was converted into an activity unit (1 Karmen unit = 0.482 U/L).

### The method of detection of enzyme activity of aspartate aminotransferase (AST) in serum of the mice is as follows:

Preparation of AST standard curve:
(1) Enzymatic reaction: 0, 2, 4, 6, and 8 µL of 2 µmol/mL sodium pyruvate standard solution were sequentially added to 5 µL of 0.1 mol/L phosphate buffer, and the volume was supplemented to 25 µL with a AST matrix solution (aspartate aminotransferase matrix solution), and repeatedly aspirating and spitting with a pipette for mixing well;
(2) Addition reaction: 20 µL 2,4-dinitrophenylhydrazine solution was added to all reaction wells in (1), mixed by aspirating and spitting, and then placed in an incubator at 37°C to react for 20 min;
(3) Developing: 200 µL of 0.4 mol/L NaOH solution was added to all reaction wells in (2) to stop the reaction, mixed by aspirating and spitting, and incubated at room temperature for 15 min. The OD value of each well was measured at 510 nm in a microplate reader; and
(4) Data processing of standard curve: The corresponding absolute OD value for each well was obtained by subtracting the OD value for the well with 0 µL sodium pyruvate from the measured OD value for each well, the corresponding AST Karmen units being 0, 24, 61, 114 and 190, respectively. The standard curve was obtain by taking the absolute OD value as the abscissa and the corresponding Karmen unit as the ordinate.

Detection of AST enzyme activity in serum samples:
(1) Reagent preparation: an AST matrix solution was placed in an incubator at 37°C for preheating;
(2) Enzymatic reaction: 5 µL diluted serum was aspirated and added to a 96-well plate, then 20 µL matrix solution was added to the corresponding sample well and mixed by repeatedly aspirating and spitting to avoid bubbling, and then placed in an incubator at 37°C for 30 min;
(3) Addition reaction: 20 µL 2,4-dinitrophenylhydrazine was added to all reaction wells in (2), mixed by aspirating and spitting, and then reacted in an incubator at 37°C for 20 min;
(4) Developing: 200 µL of 0.4 mol/L NaOH solution was added to all reaction wells in (3) to stop the reaction, mixed by aspirating and spitting, and then incubated at room temperature for 15 min. The OD value for each well was measured at a wavelength of 510 nm in a microplate reader; and
(5) Calculation of AST enzyme activity in serum: The absolute OD value of the corresponding sample well was obtained by subtracting the OD value for the control well sample well without biochemical reaction) from the obtained OD value of the sample well, and was substituted into the standard curve formula to calculate the AST enzyme activity (Karmen unit) for the corresponding serum sample. The Karman unit was converted into an activity unit (1 Karmen unit = 0.482 U/L).

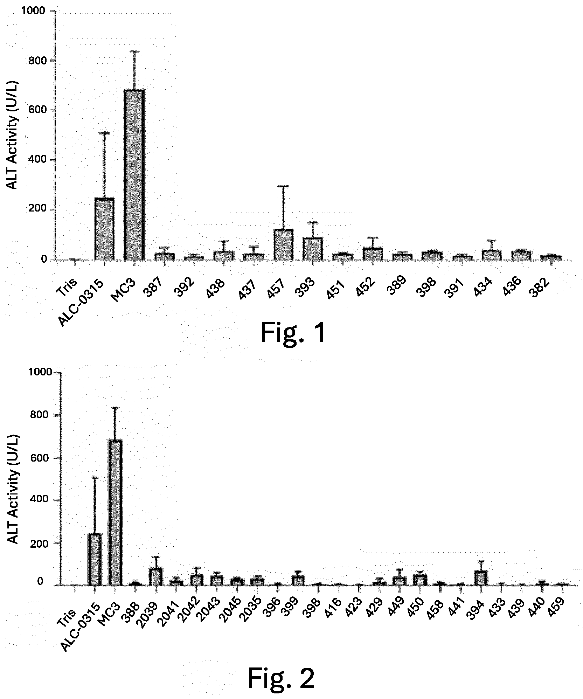

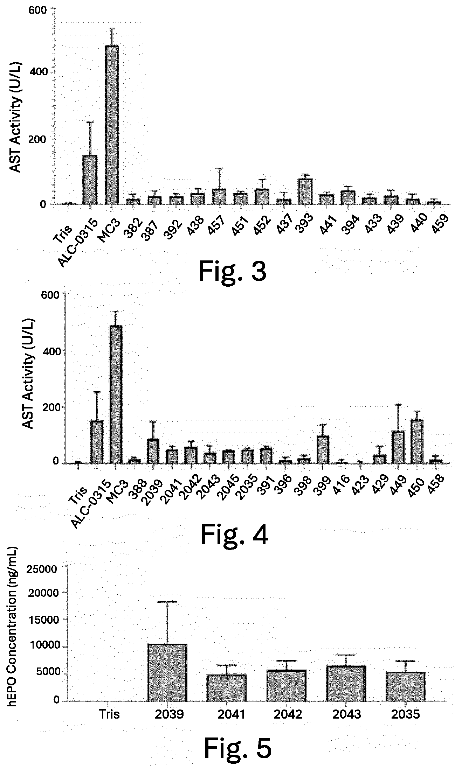

The *in vivo* delivery and safety evaluation results of the lipid nanoparticles encapsulating human erythropoietin (hEPO) mRNA with the representative amino lipid compounds are shown in FIG. 1 to FIG. 5, with Tris, ALC-0315 and MC3 as controls. "Tris" refers to the control group injected with Tris buffer solution only.

As can be seen from FIG. 1 to FIG. 4, as compared to the commercially available ALC-0315 and MC3, the representative amino lipid compounds exhibit lower ALT enzyme activity and lower AST enzyme activity.

### Experimental Example 4: Evaluation of the delivery efficiency of lipid nanoparticles to immune cell populations in different tissues and organs

C57BL/6 mice (3 mice per group) were intravenously injected with eGFP mRNA encapsulating LNP. After 12 hours, the mice were dissected, and the spleen and peripheral blood, were collected to prepare single-cell suspensions of various tissues and organs. After blocking with Fc blocking antibodies and surface staining with fluorescent-labeled antibodies, flow cytometry was conducted to analyze the expression levels of eGFP in various immune cell populations to confirm the delivery efficiency of lipid nanoparticles comprising different amino lipid compounds to immune cell populations in different tissues and organs.

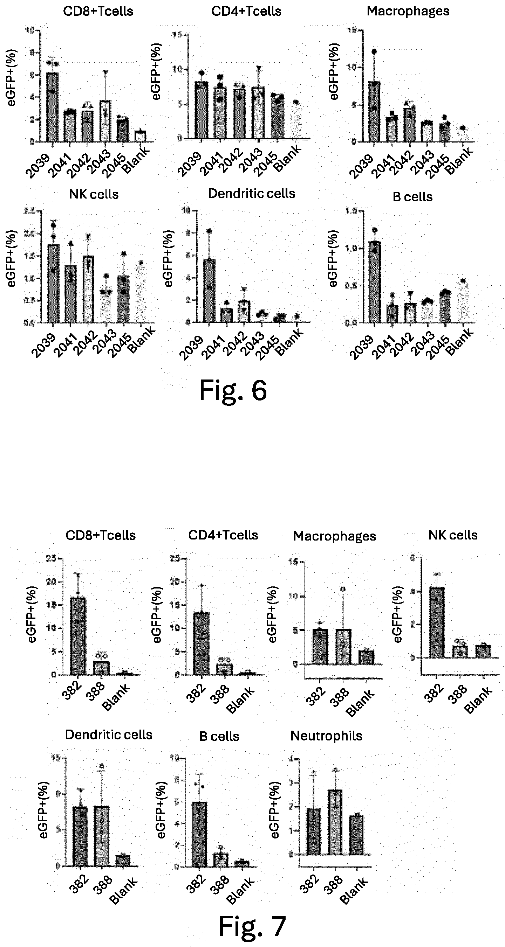

The test results are shown in FIG. 6 to FIG. 7. "Blank" in FIG. 6 and FIG. 7 refers to the control group injected with 100 µL Tris buffer. As can be seen from FIG. 6 to FIG. 7, the lipid nanoparticles comprising the amino lipid compounds 382, 388, 2039, 2041, 2042, 2043, and 2045 have different delivery preferences to different immune cells.

In addition to those described in this disclosure, various modifications to the present invention will be apparent to those skilled in the art from the foregoing description. Such modifications are also intended to fall within the scope of the appended claims.

## Claims

1. An amino lipid compound having a structure of formula (II-I): 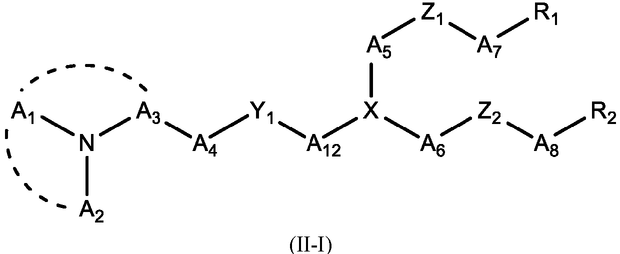
or a pharmaceutically acceptable salt thereof, or a stereoisomer thereof,
wherein:
A₁, A₂, and A₃ are one of the following:
(1) when the dashed line connecting A₁ and A₂ and the dashed line connecting A₁ and A₃ are absent, A₁ is H, C₁-C₅ hydrocarbyl, or C₁-C₅ heterohydrocarbyl, A₂ is H, C₁-C₅ hydrocarbyl, or C₁-C₅ heterohydrocarbyl, and A₃ is C₁-C₅ hydrocarbylene or a bond;
(2) when the dashed line connecting A₁ and A₂ is a bond and the dashed line connecting A₁ and A₃ is absent, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene or a bond, and A₁ and A₂, together with the nitrogen atom to which they are both attached, form an N-containing heterocyclic ring;
(3) when the dashed line connecting A₁ and A₃ is a bond and the dashed line connecting A₁ and A₂ is absent, A₁ is C₁-C₅ hydrocarbylene or C₁-C₅ heterohydrocarbylene, A₂ is H, C₁-C₅ hydrocarbyl, or C₁-C₅ heterohydrocarbyl, A₃ is C₁-C₅ hydrocarbylene, and A₁ and A₃, together with the nitrogen atom to which they are both attached, form an N-containing heterocyclic ring;
(4) when the dashed line connecting A₁ and A₂ and the dashed line connecting A₁ and A₃ are a bond, A₁ is C₁-C₅ hydrocarbylene or C₁-C₅ heterohydrocarbylene, A₂ is C₁-C₅ hydrocarbylene or C₁-C₅ heterohydrocarbylene, A₃ is C₁-C₅ hydrocarbylene, and A₁, A₂ and A₃, together with the nitrogen atom to which they are all attached, form an N-containing spiro-, fused, or bridged heterocyclic ring;
A₄ is C₁-C₅ hydrocarbylene or a bond;
A₅, A₆, A₇, A₈, and A₁₂ are each independently C₁-C₁₈ hydrocarbylene, C₁-C₁₈ heterohydrocarbylene, or a bond;
Z₁ and Z₂ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O, -OP(=O)O-, -OP(=O)(OH)O-, -OP(=O)(H)O-, -OS(=O)₂NH-, or -NHS(=O)₂O-;
Y₁ is -Z₃C(=O)Z₄-, -N(R₅)-, -Z₃C(=S)Z₄-, -OS(=O)₂O-, -OS(=O)O-, -OS(=O)₂NH-, -NHS(=O)₂NH-, -NHS(=O)₂-, -S(=O)₂NH-, -S(=O)₂O-, -OS(=O)₂-, -OP(=O)O-, -OP(=O)(OH)O-, -OP(=O)(H)O-, -O-, -S-, -NHS(=O)₂O-, -NHP(=O)O-, -OP(=O)NH-, -NHP(=O)(OH)O-, -OP(=O)(OH)NH-, -OC(=O)S-, or -SC(=O)O-;
X is C or N;
R₁ is H, C₁-C₂₄ hydrocarbyl, C₁-C₂₄ heterohydrocarbyl, Z₆R₈, -C(R₆)(OA₁₃Z₈R₉)₂, -C(R₆)(SA₁₃Z₈R₉)₂, or -C(R₆)(SA₁₃Z₈R₉)(OA₁₃Z₈R₉);
R₂ is H, C₁-C₂₄ hydrocarbyl, C₁-C₂₄ heterohydrocarbyl, Z₇R₁₀, -C(R₆)(OA₁₄Z₉R₁₁)₂, -C(R₆)(SA₁₄Z₉R₁₁)₂, or -C(R₆)(SA₁₄Z₉R₁₁)(OA₁₄Z₉R₁₁);
Z₃ and Z₄ are each independently -O-, -N(R₆)-, -S-, or a bond;
Z₆ and Z₇ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O, -OP(=O)O-, -OP(=O)(OH)O-, -OP(=O)(H)O-, -OS(=O)₂NH-, or -NHS(=O)₂O-;
R₅ is -C(=O)A₉Z₅A₁₀, -C(=O)A₁₁, H, or C₁-C₈ hydrocarbyl;
Z₅ is -O- or -S-;
Z₈ and Z₉ are each independently -C(=O)O-, -OC(=O)-, -O-, -C(=O)-, -S-, -C(=O)S-, -SC(=O)-, -C(=O)N(R₆)-, -N(R₆)C(=O)-, -OC(=O)O-, -OC(=O)S-, -SC(=O)O-, -SC(=O)S-, -N(R₆)C(=O)O-, -OC(=O)N(R₆)-, -N(R₆)C(=O)N(R₆)-, -SC(=O)N(R₆)-, -N(R₆)C(=O)S-, -N(C(=O)A₉OA₁₀)-, -N(C(=O)A₉SA₁₀)-, -N(C(=O)A₁₁), -N(A₉OA₁₀)-, -N(A₉SA₁₀)-, -N(A₁₁)-, -OS(=O)O-, -OS(=O)₂O, -OP(=O)O-, -OP(=O)(OH)O-, -OP(=O)(H)O-, -OS(=O)₂NH-, -NHS(=O)₂O-, or a bond;
each R₆ is independently H or C₁-C₈ hydrocarbyl;
R₈ and R₁₀ are each independently H, C₁-C₂₄ hydrocarbyl, or C₁-C₂₄ heterohydrocarbyl containing O or S;
R₉ and R₁₁ are each independently C₁-C₂₄ hydrocarbyl or C₁-C₂₄ heterohydrocarbyl containing O or S;
A₉ is C₁-C₈ hydrocarbylene or a bond;
A₁₀ and A₁₁ are each independently C₁-C₈ hydrocarbyl;
A₁₃ and A₁₄ are each independently C₁-C₈ hydrocarbylene, C₁-C₈ heterohydrocarbylene, or a bond;
preferably, the hydrocarbyl is an alkyl, alkenyl, or alkynyl;
preferably, the hydrocarbylene is an alkylene, alkenylene, or alkynylene;
preferably, the heterohydrocarbyl is a heteroalkyl, heteroalkenyl, or heteroalkynyl;
preferably, the heterohydrocarbylene is heteroalkylene, heteroalkenylene, or heteroalkynylene.

2. The amino lipid compound according to claim 1, having a structure represented by formula (II-VIa): 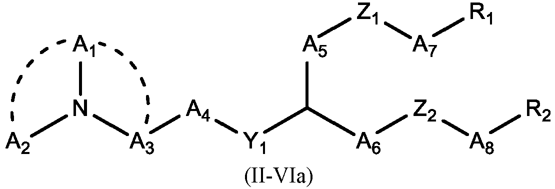 or a pharmaceutically acceptable salt thereof, or a stereoisomer thereof.

3. The amino lipid compound according to claim 1, having a structure represented by formula (II-IVa): 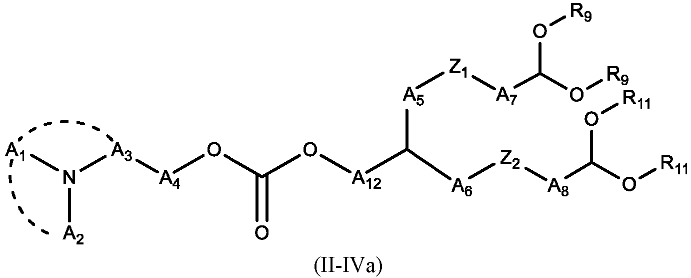 or a pharmaceutically acceptable salt thereof, or a stereoisomer thereof.

4. The amino lipid compound according to claim 1, having a structure represented by formula (II-VIa-c): 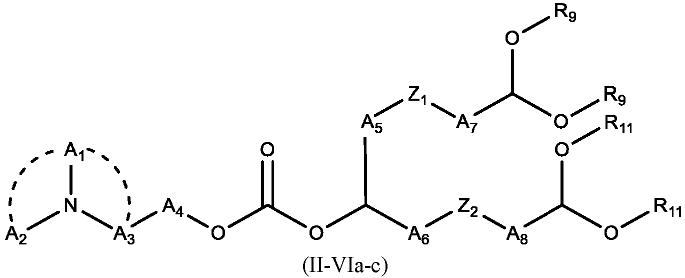 or a pharmaceutically acceptable salt thereof, or a stereoisomer thereof.

5. The amino lipid compound according to any one of claims 1 and 2, or a pharmaceutically acceptable salt thereof, or a stereoisomer thereof, wherein Y₁ is -Z₃C(=O)Z₄- or -N(R₅)-.

6. The amino lipid compound according to any one of claims 1, 2, and 5, or a pharmaceutically acceptable salt thereof, or a stereoisomer thereof, wherein Y₁ is -N(R₆)C(=O)O-, -OC(=O)N(R₆)-, or -OC(=O)O-.

7. The amino lipid compound according to any one of claims 1, 2, 5, and 6, or a pharmaceutically acceptable salt thereof, or a stereoisomer thereof, wherein Y₁ is -OC(=O)O-.

8. The amino lipid compound according to any one of claims 1, 2, 5, and 6, or a pharmaceutically acceptable salt thereof, or a stereoisomer thereof, wherein Y₁ is -N(H)C(=O)O-.

9. The amino lipid compound according to any one of claims 1, 2, 5, and 6, or a pharmaceutically acceptable salt thereof, or a stereoisomer thereof, wherein Y₁ is -OC(=O)N(H)-.

10. The amino lipid compound according to any one of claims 1, 2, and 5, or a pharmaceutically acceptable salt thereof, or a stereoisomer thereof, wherein Y₁ is -N(R₅)-.

11. The amino lipid compound according to any one of claims 1, 2, 5, and 10, or a pharmaceutically acceptable salt thereof, or a stereoisomer thereof, wherein Y₁ is -N(C(=O)A₉OA₁₀)-.

12. The amino lipid compound according to any one of claims 1, 2, 5, and 10, or a pharmaceutically acceptable salt thereof, or a stereoisomer thereof, wherein Y₁ is -N(C(=O)A₁₀)-.

13. The amino lipid compound according to any one of claims 1, 2, and 5 to 12, or a pharmaceutically acceptable salt thereof, or a stereoisomer thereof, wherein Z₁ and Z₂ are each independently -C(=O)O- or -OC(=O)-.

14. The amino lipid compound according to any one of claims 1, 2, and 5 to 13, or a pharmaceutically acceptable salt thereof, or a stereoisomer thereof, wherein Z₁ is -C(=O)O-.

15. The amino lipid compound according to any one of claims 1, 2, and 5 to 13, or a pharmaceutically acceptable salt thereof, or a stereoisomer thereof, wherein Z₁ is -OC(=O)-.

16. The amino lipid compound according to any one of claims 1, 2, and 5 to 13, or a pharmaceutically acceptable salt thereof, or a stereoisomer thereof, wherein Z₂ is -C(=O)O-.

17. The amino lipid compound according to any one of claims 1, 2, and 5 to 13, or a pharmaceutically acceptable salt thereof, or a stereoisomer thereof, wherein Z₂ is -OC(=O)-.

18. The amino lipid compound according to any one of claims 1, 2, 5 to 14, and 16, or a pharmaceutically acceptable salt thereof, or a stereoisomer thereof, wherein Z₁ and Z₂ are -C(=O)O-.

19. The amino lipid compound according to any one of claims 1, 2, 5 to 13, 15, and 17, or a pharmaceutically acceptable salt thereof, or a stereoisomer thereof, wherein Z₁ and Z₂ are -OC(=O)-.

20. The amino lipid compound according to any one of claims 1, 2, 5 to 14, and 17, or a pharmaceutically acceptable salt thereof, or a stereoisomer thereof, wherein Z₁ is -C(=O)O-, and Z₂ is -OC(=O)-.

21. The amino lipid compound according to any one of claims 1, 2, and 5 to 20, or a pharmaceutically acceptable salt thereof, or a stereoisomer thereof, wherein R₁ is -C(R₆)(OR₉)₂.

22. The amino lipid compound according to any one of claims 1, 2, and 5 to 21, or a pharmaceutically acceptable salt thereof, or a stereoisomer thereof, wherein R₁ is -C(H)(OR₉)₂.

23. The amino lipid compound according to any one of claims 1, 2, and 5 to 22, or a pharmaceutically acceptable salt thereof, or a stereoisomer thereof, wherein R₂ is -C(R₆)(OR₁₁)₂.

24. The amino lipid compound according to any one of claims 1, 2, and 5 to 23, or a pharmaceutically acceptable salt thereof, or a stereoisomer thereof, wherein R₂ is -C(H)(OR₁₁)₂.

25. The amino lipid compound according to any one of claims 1, 2, and 5 to 22, or a pharmaceutically acceptable salt thereof, or a stereoisomer thereof, wherein R₂ is Z₇R₁₀.

26. The amino lipid compound according to any one of claims 1, 2, and 5 to 22, or a pharmaceutically acceptable salt thereof, or a stereoisomer thereof, wherein R₂ is -C(=O)OR₁₀.

27. The amino lipid compound according to any one of claims 1, 2, and 5 to 22, or a pharmaceutically acceptable salt thereof, or a stereoisomer thereof, wherein R₂ is -OC(=O)R₁₀.

28. The amino lipid compound according to any one of claims 1, 2, and 5 to 22, or a pharmaceutically acceptable salt thereof, or a stereoisomer thereof, wherein R₂ is -OR₁₀.

29. The amino lipid compound according to any one of claims 1, 2, and 5 to 22, or a pharmaceutically acceptable salt thereof, or a stereoisomer thereof, wherein R₂ is -N(H)C(=O)R₁₀.

30. The amino lipid compound according to any one of claims 1, 2, and 5 to 22, or a pharmaceutically acceptable salt thereof, or a stereoisomer thereof, wherein R₂ is -C(=O)N(R₆)R₁₀.

31. The amino lipid compound according to any one of claims 1, 2, and 5 to 22, or a pharmaceutically acceptable salt thereof, or a stereoisomer thereof, wherein R₂ is substituted C₁-C₂₄ alkyl.

32. The amino lipid compound according to any one of claims 1, 2, and 5 to 22, or a pharmaceutically acceptable salt thereof, or a stereoisomer thereof, wherein R₁ is -C(R₆)(OR₉)₂, and R₂ is -C(R₆)(OR₁₁)₂.

33. The amino lipid compound according to any one of claims 1, 2, and 5 to 22, or a pharmaceutically acceptable salt thereof, or a stereoisomer thereof, wherein R₁ is -C(H)(OR₉)₂, and R₂ is -C(H)(OR₁₁)₂.

34. The amino lipid compound according to any one of claims 1, 2, and 5 to 22, or a pharmaceutically acceptable salt thereof, or a stereoisomer thereof, wherein R₁ is -C(H)(OR₉)₂, and R₂ is -C(=O)OR₁₀.

35. The amino lipid compound according to any one of claims 1, 2, and 5 to 22, or a pharmaceutically acceptable salt thereof, or a stereoisomer thereof, wherein R₁ is -C(H)(OR₉)₂, and R₂ is -OC(=O)R₁₀.

36. The amino lipid compound according to any one of claims 1, 2, and 5 to 22, or a pharmaceutically acceptable salt thereof, or a stereoisomer thereof, wherein R₁ is -C(H)(OR₉)₂, and R₂ is -OR₁₀.

37. The amino lipid compound according to any one of claims 1, 2, and 5 to 22, or a pharmaceutically acceptable salt thereof, or a stereoisomer thereof, wherein R₁ is -C(H)(OR₉)₂, and R₂ is C₁-C₂₄ alkyl substituted with hydroxyl.

38. The amino lipid compound according to any one of claims 1, 2, 5 to 7, and 13 to 37, or a pharmaceutically acceptable salt thereof, or a stereoisomer thereof, wherein Y₁ is -OC(=O)O-, R₁ is -C(H)(OR₉)₂, and R₂ is -C(=O)OR₁₀.

39. The amino lipid compound according to any one of claims 1, 2, 5 to 7, and 13 to 37, or a pharmaceutically acceptable salt thereof, or a stereoisomer thereof, wherein Y₁ is -OC(=O)O-, R₁ is -C(H)(OR₉)₂, and R₂ is -OC(=O)R₁₀.

40. The amino lipid compound according to any one of claims 1, 2, 5 to 7, and 13 to 37, or a pharmaceutically acceptable salt thereof, or a stereoisomer thereof, wherein Y₁ is -OC(=O)O-, R₁ is -C(H)(OR₉)₂, and R₂ is -OR₁₀.

41. The amino lipid compound according to any one of claims 1, 2, 5 to 7, and 13 to 37, or a pharmaceutically acceptable salt thereof, or a stereoisomer thereof, wherein Y₁ is -OC(=O)O-, R₁ is -C(H)(OR₉)₂, and R₂ is C₁-C₂₄ alkyl substituted with hydroxyl.

42. The amino lipid compound according to any one of claims 1, 2, 5 to 7, and 13 to 37, or a pharmaceutically acceptable salt thereof, or a stereoisomer thereof, wherein Y₁ is -OC(=O)O-, A₇ and As are unsubstituted C₁-C₅ alkylene, R₁ is -C(H)(OR₉)₂, and R₂ is -C(=O)OR₁₀.

43. The amino lipid compound according to any one of claims 1, 2, 5 to 7, and 13 to 37, or a pharmaceutically acceptable salt thereof, or a stereoisomer thereof, wherein Y₁ is -OC(=O)O-, A₇ and As are unsubstituted C₁-C₅ alkylene, R₁ is -C(H)(OR₉)₂, and R₂ is -OC(=O)R₁₀.

44. The amino lipid compound according to any one of claims 1, 2, 5, 6, 8, and 13 to 37, or a pharmaceutically acceptable salt thereof, or a stereoisomer thereof, wherein Y₁ is -N(H)C(=O)O-, R₁ is -C(H)(OR₉)₂, and R₂ is -C(H)(OR₁₁)₂.

45. The amino lipid compound according to any one of claims 1, 2, 5, 6, 9, and 13 to 37, or a pharmaceutically acceptable salt thereof, or a stereoisomer thereof, wherein Y₁ is -OC(=O)N(H)-, R₁ is -C(H)(OR₉)₂, and R₂ is -C(H)(OR₁₁)₂.

46. The amino lipid compound according to any one of claims 1, 2, 5, 6, 11, and 13 to 37, or a pharmaceutically acceptable salt thereof, or a stereoisomer thereof, wherein Y₁ is -N(C(=O)A₉OA₁₀)-, R₁ is -C(H)(OR₉)₂, and R₂ is -C(H)(OR₁₁)₂.

47. The amino lipid compound according to any one of claims 1, 2, 5, 6, 12, and 13 to 37, or a pharmaceutically acceptable salt thereof, or a stereoisomer thereof, wherein Y₁ is -N(C(=O)A₁₀)-, R₁ is -C(H)(OR₉)₂, and R₂ is -C(H)(OR₁₁)₂.

48. The amino lipid compound according to any one of claims 1, 2, 5, 6, 12, and 13 to 37, or a pharmaceutically acceptable salt thereof, or a stereoisomer thereof, wherein Y₁ is -N(C(=O)A₁₀)-, R₁ is -C(H)(OR₉)₂, and R₂ is C₁-C₂₄ alkyl substituted with hydroxyl.

49. The amino lipid compound according to any one of claims 1, 2, and 5 to 48, or a pharmaceutically acceptable salt thereof, or a stereoisomer thereof, wherein A₉ is C₁-C₅ alkylene.

50. The amino lipid compound according to any one of claims 1, 2, and 5 to 49, or a pharmaceutically acceptable salt thereof, or a stereoisomer thereof, wherein A₁₀ is C₁-C₈ alkyl.

51. The amino lipid compound according to any one of claims 1, 2, and 5 to 50, or a pharmaceutically acceptable salt thereof, or a stereoisomer thereof, wherein R₁₀ is C₁-C₂₄ alkyl, C₂-C₂₄ alkenyl, or C₂-C₂₄ alkynyl.

52. The amino lipid compound according to any one of claims 1, 2, and 5 to 51, or a pharmaceutically acceptable salt thereof, or a stereoisomer thereof, wherein R₁₀ is a straight C₂-C₈ alkyl.

53. The amino lipid compound according to any one of claims 1, 2, and 5 to 51, or a pharmaceutically acceptable salt thereof, or a stereoisomer thereof, wherein R₁₀ is a branched C₇-C₁₈ alkyl.

54. The amino lipid compound according to any one of claims 1, 2, and 5 to 51, or a pharmaceutically acceptable salt thereof, or a stereoisomer thereof, wherein R₁₀ is C₅-C₁₂ alkenyl.

55. The amino lipid compound according to any one of claims 1, 2, and 5 to 51, or a pharmaceutically acceptable salt thereof, or a stereoisomer thereof, wherein R₁₀ is C₅-C₁₂ alkynyl.

56. The amino lipid compound according to any one of claims 1, 2, and 5 to 55, or a pharmaceutically acceptable salt thereof, or a stereoisomer thereof, wherein R₉ is C₉-C₁₁ alkynyl, such as C₁₀ alkynyl.

57. The amino lipid compound according to any one of claims 1, 2, and 5 to 56, or a pharmaceutically acceptable salt thereof, or a stereoisomer thereof, wherein R₁₄ is C₁-C₅ alkyl.

58. The amino lipid compound according to any one of claims 1, 2, and 5 to 56, or a pharmaceutically acceptable salt thereof, or a stereoisomer thereof, wherein R₁₄ is C₄-C₈ alkenyl with one double bond.

59. The amino lipid compound according to any one of claims 1 and 3, or a pharmaceutically acceptable salt thereof, or a stereoisomer thereof, wherein A₁₂ is C₂-C₃ alkylene.

60. The amino lipid compound according to any one of claims 1 and 3, or a pharmaceutically acceptable salt thereof, or a stereoisomer thereof, wherein A₁₂ is C₁-C₃ alkylene (e.g., C₁ alkylene), and As and A₆ are each independently C₃-C₉ alkylene (e.g., C₄-C₉ alkylene, C₅-C₉ alkylene, C₆ alkylene, C₇ alkylene, C₈ alkylene).

61. The amino lipid compound according to any one of claims 1, 3, and 4, or a pharmaceutically acceptable salt thereof, or a stereoisomer thereof, wherein Z₁ and Z₂ are each independently -C(=O)O-, -OC(=O)-, -O-, -C(=O)-, -C(=O)N(R₆)-, -N(R₆)C(=O)-, -N(R₆)C(=O)O-, -OC(=O)N(R₆)-, -N(R₆)C(=O)N(R₆)-, -C(=O)S-, -SC(=O)-, -OC(=O)O-, -OC(=O)S-, -SC(=O)O-, -SC(=O)S-, -OS(=O)O-, -OS(=O)₂O-, -OP(=O)O-, -OP(=O)(OH)O-, or -OP(=O)(H)O-.

62. The amino lipid compound according to any one of claims 1, 3, 4, and 59 to 61, or a pharmaceutically acceptable salt thereof, or a stereoisomer thereof, wherein Z₁ is -C(=O)O-.

63. The amino lipid compound according to any one of claims 1, 3, 4, and 59 to 61, or a pharmaceutically acceptable salt thereof, or a stereoisomer thereof, wherein Z₁ is -OC(=O)-.

64. The amino lipid compound according to any one of claims 1, 3, 4, and 59 to 61, or a pharmaceutically acceptable salt thereof, or a stereoisomer thereof, wherein Z₁ is -OC(=O)O-.

65. The amino lipid compound according to any one of claims 1, 3, 4, and 59 to 61, or a pharmaceutically acceptable salt thereof, or a stereoisomer thereof, wherein Z₁ is -N(H)C(=O)O-.

66. The amino lipid compound according to any one of claims 1, 3, 4, and 59 to 61, or a pharmaceutically acceptable salt thereof, or a stereoisomer thereof, wherein Z₁ is -OC(=O)N(H)-.

67. The amino lipid compound according to any one of claims 1, 3, 4, and 59 to 61, or a pharmaceutically acceptable salt thereof, or a stereoisomer thereof, wherein Z₂ is -C(=O)O-.

68. The amino lipid compound according to any one of claims 1, 3, 4, and 59 to 61, or a pharmaceutically acceptable salt thereof, or a stereoisomer thereof, wherein Z₂ is -OC(=O)-.

69. The amino lipid compound according to any one of claims 1, 3, 4, and 59 to 61, or a pharmaceutically acceptable salt thereof, or a stereoisomer thereof, wherein Z₂ is -OC(=O)O-.

70. The amino lipid compound according to any one of claims 1, 3, 4, and 59 to 61, or a pharmaceutically acceptable salt thereof, or a stereoisomer thereof, wherein Z₂ is -N(H)C(=O)O-.

71. The amino lipid compound according to any one of claims 1, 3, 4, and 59 to 61, or a pharmaceutically acceptable salt thereof, or a stereoisomer thereof, wherein Z₂ is -OC(=O)N(H)-.

72. The amino lipid compound according to any one of claims 1, 3, 4, and 59 to 61, or a pharmaceutically acceptable salt thereof, or a stereoisomer thereof, wherein Z₁ and Z₂ are -C(=O)O-.

73. The amino lipid compound according to any one of claims 1, 3, 4, and 59 to 61, or a pharmaceutically acceptable salt thereof, or a stereoisomer thereof, wherein Z₁ and Z₂ are -OC(=O)-.

74. The amino lipid compound according to any one of claims 1, 3, 4, and 59 to 61, or a pharmaceutically acceptable salt thereof, or a stereoisomer thereof, wherein Z₁ and Z₂ are -OC(=O)O-.

75. The amino lipid compound according to any one of claims 1, 3, 4, and 59 to 61, or a pharmaceutically acceptable salt thereof, or a stereoisomer thereof, wherein Z₁ and Z₂ are -N(H)C(=O)O-.

76. The amino lipid compound according to any one of claims 1, 3, 4, and 59 to 61, or a pharmaceutically acceptable salt thereof, or a stereoisomer thereof, wherein Z₁ and Z₂ are -OC(=O)N(H)-.

77. The amino lipid compound according to any one of claims 1, 3, 4, and 59 to 61, or a pharmaceutically acceptable salt thereof, or a stereoisomer thereof, wherein Z₁ is -C(=O)O-, and Z₂ is -OC(=O)-.

78. The amino lipid compound according to any one of claims 1 to 77, or a pharmaceutically acceptable salt thereof, or a stereoisomer thereof, wherein A₄ is a bond.

79. The amino lipid compound according to any one of claims 1 to 78, or a pharmaceutically acceptable salt thereof, or a stereoisomer thereof, wherein A₅ and A₆ are each independently C₃-C₁₀ alkylene.

80. The amino lipid compound according to any one of claims 1 to 79, or a pharmaceutically acceptable salt thereof, or a stereoisomer thereof, wherein A₇ and A₅ are each independently C₂-C₄ alkylene or C₅-C₁₀ alkylene.

81. The amino lipid compound according to any one of claims 1 to 80, or a pharmaceutically acceptable salt thereof, or a stereoisomer thereof, wherein each R₉ is independently C₃-C₉ alkyl.

82. The amino lipid compound according to any one of claims 1 to 80, or a pharmaceutically acceptable salt thereof, or a stereoisomer thereof, wherein each R₉ is independently C₅-C₈ alkenyl.

83. The amino lipid compound according to any one of claims 1 to 82, or a pharmaceutically acceptable salt thereof, or a stereoisomer thereof, wherein each R₁₁ is independently C₃-C₉ alkyl.

84. The amino lipid compound according to any one of claims 1 to 82, or a pharmaceutically acceptable salt thereof, or a stereoisomer thereof, wherein each R₁₁ is independently C₅-C₈ alkenyl.

85. The amino lipid compound according to any one of claims 1 to 84, or a pharmaceutically acceptable salt thereof, or a stereoisomer thereof, wherein the dashed line connecting A₁ and A₂ and the dashed line connecting A₁ and A₃ are absent.

86. The amino lipid compound according to any one of claims 1 to 84, or a pharmaceutically acceptable salt thereof, or a stereoisomer thereof, wherein the dashed line connecting A₁ and A₂ is a bond, and the dashed line connecting A₁ and A₃ is absent.

87. The amino lipid compound according to any one of claims 1 to 84, or a pharmaceutically acceptable salt thereof, or a stereoisomer thereof, wherein the dashed line connecting A₁ and A₃ is a bond, and the dashed line connecting A₁ and A₂ is absent.

88. The amino lipid compound according to any one of claims 1 to 87, or a pharmaceutically acceptable salt thereof, or a stereoisomer thereof, wherein A₁ and A₂ are each independently C₁-C₅ alkyl or C₁-C₅ alkylene.

89. The amino lipid compound according to any one of claims 1 to 85 and 88, or a pharmaceutically acceptable salt thereof, or a stereoisomer thereof, wherein A₁ and A₂ are each independently C₁-C₃ alkyl.

90. The amino lipid compound according to claim 89, or a pharmaceutically acceptable salt thereof, or a stereoisomer thereof, wherein A₁ and A₂ are each independently C₁-C₂ alkyl.

91. The amino lipid compound according to claim 88, or a pharmaceutically acceptable salt thereof, or a stereoisomer thereof, wherein A₂ is C₁-C₃ alkyl.

92. The amino lipid compound according to claim 88, or a pharmaceutically acceptable salt thereof, or a stereoisomer thereof, wherein A₂ is C₁-C₂ alkyl.

93. The amino lipid compound according to any one of claims 1 to 92, or a pharmaceutically acceptable salt thereof, or a stereoisomer thereof, wherein A₃ is C₁-C₅ alkylene.

94. The amino lipid compound according to any one of claims 1 to 93, or a pharmaceutically acceptable salt thereof, or a stereoisomer thereof, wherein A₃ is C₂-C₄ alkylene.

95. The amino lipid compound according to any one of claims 1 to 84, 86, 88, 93, and 94, or a pharmaceutically acceptable salt thereof, or a stereoisomer thereof, wherein when the dashed line connecting A₁ and A₂ is a bond and the dashed line connecting A₁ and A₃ is absent, A₁ and A₂, together with the nitrogen atom to which they are both attached, form an N-containing four-, five-, or six-membered heterocyclic ring.

96. The amino lipid compound according to any one of claims 1 to 84, 87, 88, and 91 to 94, or a pharmaceutically acceptable salt thereof, or a stereoisomer thereof, wherein when the dashed line connecting A₁ and A₃ is a bond, and the dashed line connecting A₁ and A₂ is absent, A₁ and A₃, together with the nitrogen atom to which they are both attached, form an N-containing four-, five-, or six-membered heterocyclic ring.

97. The amino lipid compound according to any one of claims 1 to 96, wherein the amino lipid compound has one of the structures shown below:
| Amino lipid compounds | Structural formulae |
|---|---|
| 382 | 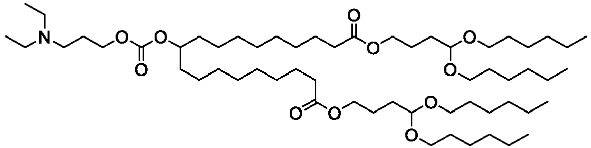 |
| 383 | 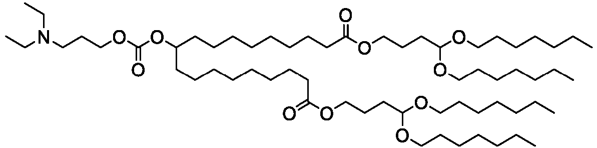 |
| 387 | 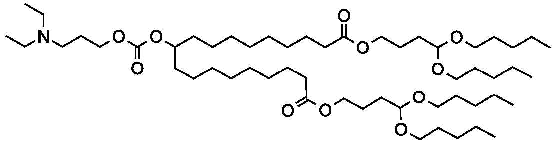 |
| 388 | 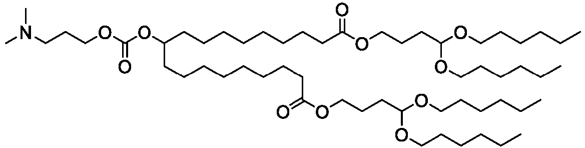 |
| 389 | 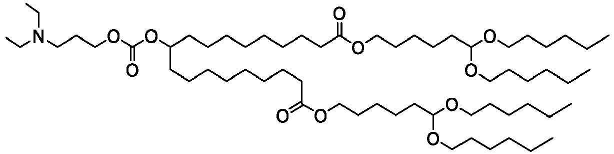 |
| 390 | 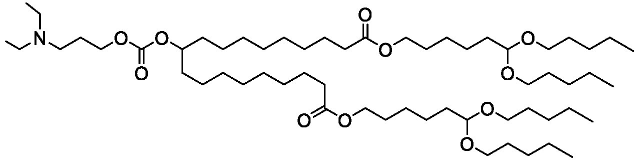 |
| 391 | 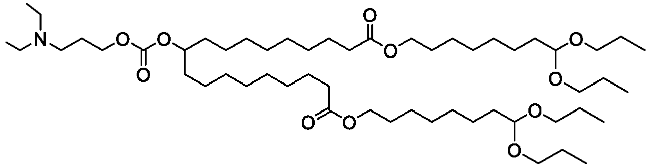 |
| 490 | 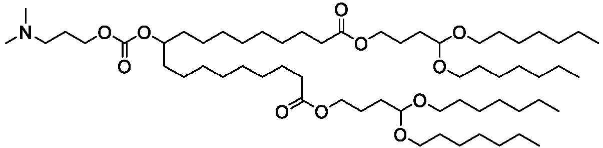 |
| 849 | 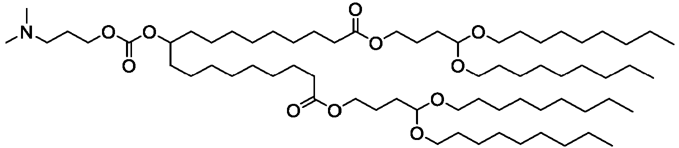 |
| 851 | 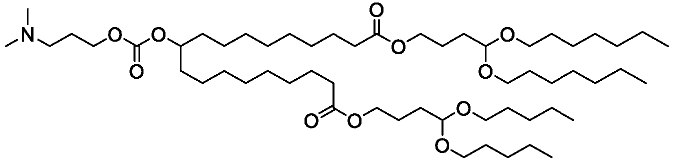 |
| 856 | 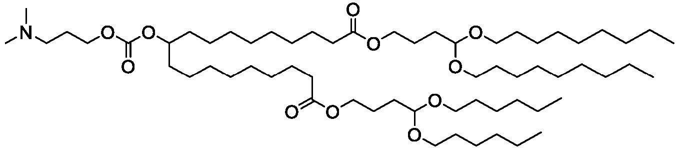 |
| 859 | 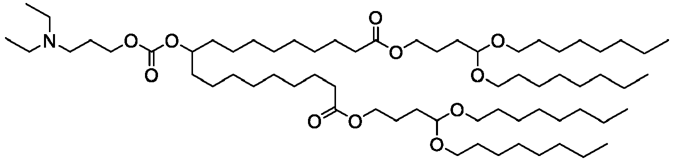 |
| 861 | 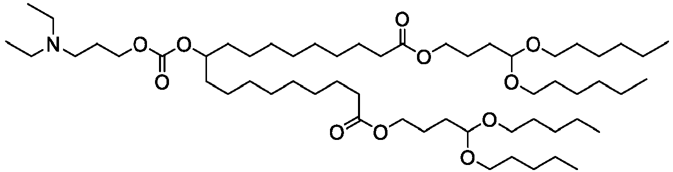 |
| 862 | 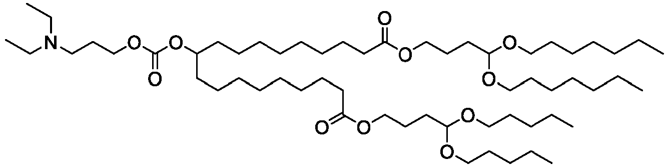 |
| 863 | 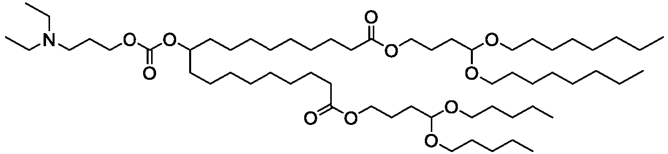 |
| 864 | 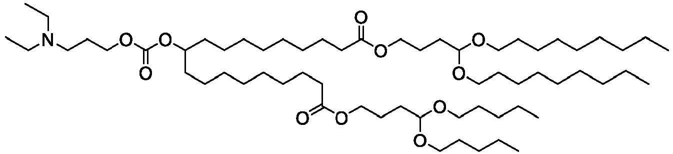 |
| 865 | 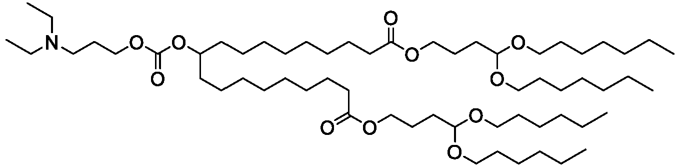 |
| 867 | 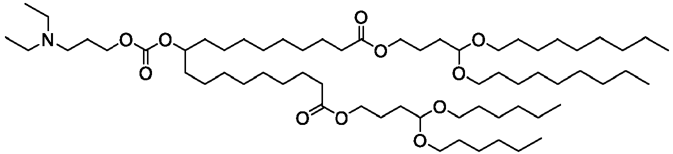 |
| 868 | 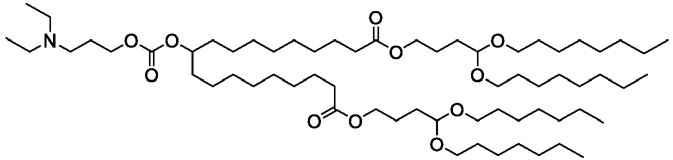 |
| 869 | 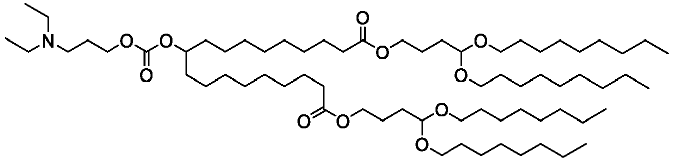 |
| 2035 | 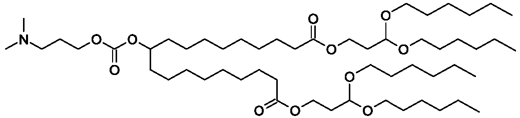 |
| 2036 | 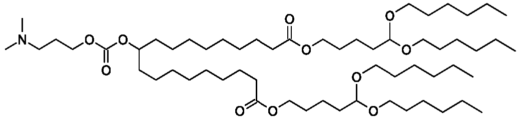 |
| 2039 | 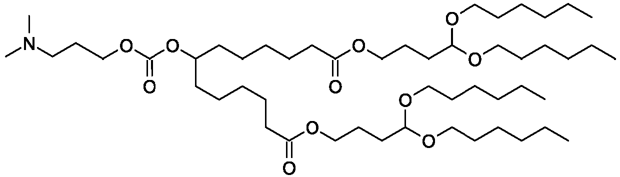 |
| 2040 | 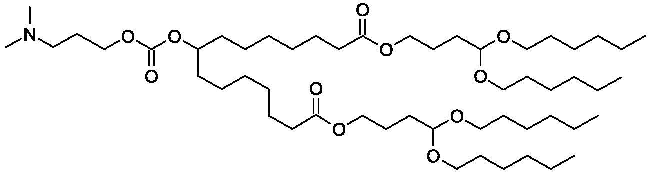 |
| 2041 | 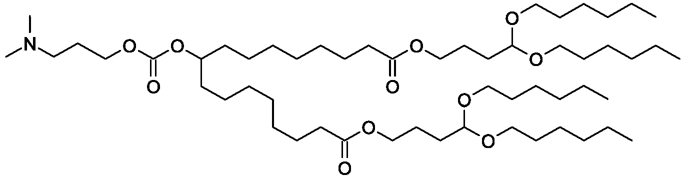 |
| 2042 | 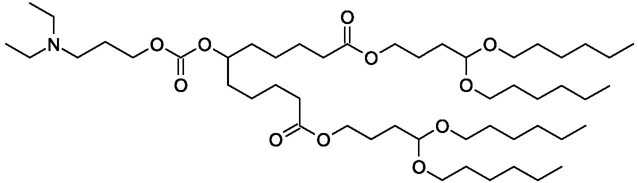 |
| 2043 | 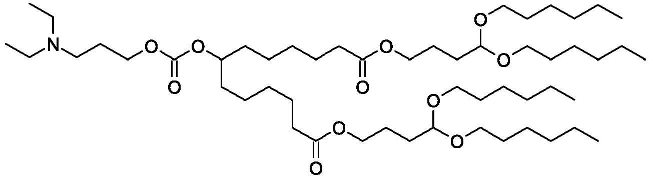 |
| 2044 | 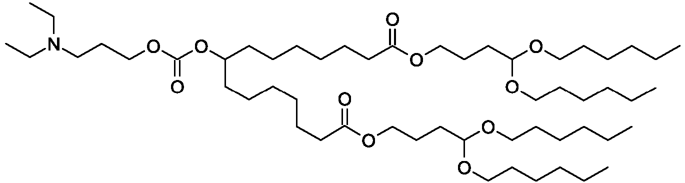 |
| 2045 | 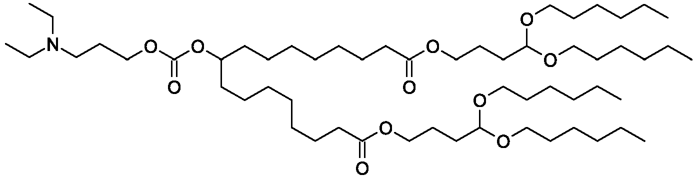 |

98. A lipid nanoparticle comprising the amino lipid compound of any one of claims 1 to 97.

99. The lipid nanoparticle according to claim 98, further comprising a biologically active ingredient;
preferably, the biologically active ingredient is a nucleic acid.

100. A pharmaceutical composition comprising the amino lipid compound of any one of claims 1 to 97 or the lipid nanoparticle of any one of claims 98 and 99, and a pharmaceutically acceptable carrier, diluent, or excipient.

101. Use of the amino lipid compound of any one of claims 1 to 97 in the manufacture of a vehicle for an active ingredient;
preferably, the active ingredient is a pharmaceutically active ingredient, preferably a nucleic acid.

102. Use of the amino lipid compound of any one of claims 1 to 97, the lipid nanoparticle of any one of claims 98 and 99, or the pharmaceutical composition of claim 100 in the manufacture of a medicament.

103. The use according to claim 101, wherein the medicament is for use in the treatment and/or prevention of a disease;
preferably, the medicament is for use in gene therapy, protein replacement therapy, antisense therapy, therapy by interfering RNA, or gene vaccination.

104. The use of any one of claims 101 and 102, wherein the medicament is a nucleic acid drug.

105. Use of the amino lipid compound of any one of claims 1 to 97, the lipid nanoparticle of any one of claims 98 and 99, or the pharmaceutical composition of claim 100 in the manufacture of a medicament for nucleic acid transfer.

106. The use according to claim 105, wherein the nucleic acid is selected from the group consisting of RNA, antisense oligonucleotide, and DNA;
preferably, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), cas9 mRNA, or a mixture thereof;
preferably, the DNA is a plasmid.
